# EUROPEAN PATENT APPLICATION

(11) **EP 4 707 394 A2**
(43) Date of publication of application: **11.03.2026**
(21) Application number: 26151608.2
(22) Date of filing: 07.02.2020
(51) Int. Cl.: C12N 15/113

(54) **RNAI AGENTS FOR HEPATITIS B VIRUS INFECTION**

(30) Priority: 07.02.2019 US 201962802614 P; 28.05.2019 US 201962853659 P; 07.11.2019 US 201962932315 P
(62) Divisional of application: 20709945.8
(71) Applicant: Arrowhead Pharmaceuticals, Inc., Pasadena, CA 91105 (US); GlaxoSmithKline Intellectual Property (No.3) Limited, Stevenage SG1 2NY (GB)
(72) Inventor: GIVEN, Bruce D., Pasadena, California, 91105 (US); HAMILTON, James C., Pasadena, California, 91105 (US); SCHLUEP, Thomas, Pasadena, California, 91105 (US); BEUMONT-MAUVIEL, Maria, Titusville, New Jersey, 08560 (US); LENZ, Oliver, Titusville, New Jersey, 08560 (US); KALMEIJER, Ronald, Titusville, New Jersey, 08560 (US)
(74) Representative: Bailey, Samuel Sonic

(57) **Abstract**

Described are methods for inhibition of Hepatitis B virus gene expression or treating symptoms and/or diseases associated with Hepatitis B virus infection. Dosing regimens for administering these RNAi agents are also described. RNA interference (RNAi) agents for inhibiting the expression of Hepatitis B virus gene are described. The HBV RNAi agents disclosed herein may be targeted to cells, such as hepatocytes, for example, by using conjugated targeting ligands. Pharmaceutical compositions comprising one or more HBV RNAi agents optionally with one or more additional therapeutics are also described. Delivery of the described HBV RNAi agents to infected liver *in vivo* provides for inhibition of HBV gene expression and treatment of diseases and conditions associated with HBV infection.

## Description

### CROSS-REFERENCE TO RELATED APPLICATIONS

This application claims priority to and the benefit of United States Provisional Patent Application No. 62/802,614, filed February 7, 2019; United States Provisional Patent Application No. 62/853,659, filed May 28, 2019; and United States Provisional Patent Application No. 62/932,315, filed November 7, 2019; the disclosures of each of which are hereby incorporated herein by reference in their entireties.

### SUBMISSION OF SEQUENCE LISTING ON ASCII TEXT FILE

The content of the following submission on ASCII text file is incorporated herein by reference in its entirety: a computer readable form (CRF) of the Sequence Listing (file name: 165002000540SEQLIST.TXT, date recorded: February 5, 2020, size: 78 KB).

### FIELD OF THE INVENTION

Disclosed herein are RNA interference (RNAi) agents for inhibition of Hepatitis B Virus gene expression, compositions that include HBV RNAi agents, and methods of use thereof. Methods of treating and/or preventing symptoms or diseases associated with an infection caused by Hepatitis B Virus in a subject in need thereof are also disclosed.

### BACKGROUND

The Hepatitis B Virus (HBV) is a strict hepatotrophic, double-stranded DNA containing virus. Although DNA is the genetic material, the replication cycle involves a reverse transcription step to copy a pregenomic RNA into DNA. Hepatitis B Virus is classified as one member of the Hepadnaviruses and belongs to the family of Hepadnaviridae. The primary infection of adult humans with Hepatitis B Virus causes an acute hepatitis with symptoms of organ inflammation, fever, jaundice and increased liver transaminases in blood. Those patients that are not able to overcome the virus infection suffer a chronic disease progression over many years with increased risk of developing cirrhotic liver or liver cancer. Perinatal transmission from Hepatitis B Virus-infected mothers to newborns also leads to chronic hepatitis.

Upon uptake by hepatocytes, the nucleocapsid is transferred to the nucleus and DNA is released. There, the DNA strand synthesis is completed and gaps repaired to give the covalently closed circular (ccc) supercoiled DNA of 3.2kb. The cccDNA serves as a template for transcription of five major viral mRNAs, which are 3.5, 3.5, 2.4, 2.1 and 0.7 kb long. All mRNAs are 5'-capped and polyadenylated at the 3'-end. There is sequence overlap at the 3'-end between all five mRNAs.

One 3.5 kb mRNA serves as template for core protein and polymerase production. In addition, the same transcript serves as a pre-genomic replication intermediate and allows the viral polymerase to initiate the reverse transcription into DNA. Core protein is needed for nucleocapsid formation. The other 3.5 kb mRNA encodes pre-core, the secretable e-antigen (HBeAg). In the absence of replication inhibitors, the abundance of e-antigen in blood correlates with Hepatitis B Virus replication in liver and serves as an important diagnostic marker for monitoring the disease progression.

The 2.4 and 2.1 kb mRNAs carry the open reading frames ("ORF") pre-S1, pre-S2 and S for expression of viral large, medium and small surface antigen. The s-antigen is associated with infectious, complete particles. In addition, blood of infected patients also contain non-infectious particles derived from s-antigen alone, free of genomic DNA or polymerase. The function of these particles is not fully understood. The complete and lasting depletion of detectable s-antigen in blood is considered as a reliable indicator for Hepatitis B Virus clearance.

The 0.7 kb mRNA encodes the X protein. This gene product is important for efficient transcription of viral genes and also acts as a transactivator on host gene expression. The latter activity seems to be important for hepatocyte transformation during development of liver cancer.

Patients with detectable s-antigen, e-antigen, and/or viral DNA in the blood for more than 6 months are considered chronically infected. Nucleoside analogs as inhibitors of reverse transcriptase activity are typically the first treatment option for many patients. Administration of lamivudine, tenofovir, and/or entecavir has been shown to suppress Hepatitis B Virus replication, sometimes to undetectable levels, with improvement of liver function and reduction of liver inflammation typically seen as the most important benefits. However, only few patients achieve complete and lasting remission after the end of treatment. Furthermore, the Hepatitis B Virus develops drug resistance with increasing duration of treatment. This is especially difficult for patients co-infected with Hepatitis B and Human Immunodeficiency Virus (HIV). Both viruses are susceptible to nucleoside analogue drugs and may co-develop resistance.

A second treatment option is the administration of interferon-alpha. Here, patients receive high doses of interferon-alpha over a period of 6 months. The Asian genotype B gives very poor response rates. Co-infection with Hepatitis D Virus (HDV) or Human Immunodeficiency Virus has been shown to render interferon-alpha therapy completely ineffective. Patients with strong liver damage and heavy fibrotic conditions are not qualified for interferon-alpha therapy.

Certain Hepatitis B Virus-specific RNA interference (RNAi) agents have been previously shown to inhibit expression of HBV gene expression. For example, U.S. Patent Application Publication No. 2013/0005793, to Chin et al., which is incorporated herein by reference in its entirety, discloses certain double-stranded ribonucleic acid (dsRNA) molecules for inhibiting the expression of Hepatitis B Virus gene.

### SUMMARY

There exists a need for a method of treating and/or preventing symptoms or diseases associated with an infection caused by Hepatitis B Virus (HBV) in a subject in need thereof. Additionally, there exists a need for a method of inhibiting expression of one or more Hepatitis B Virus genes. Particularly, there exists a need for a method of treating or preventing an HBV-associated disease or symptoms in a subject or inhibiting expression of one or more HBV genes using Hepatitis B Virus (HBV)-specific RNA interference (RNAi) agents (also herein termed RNAi agent, RNAi trigger, or trigger) that are able to selectively and efficiently inhibit the expression of a Hepatitis B Virus (HBV) gene. Further, there exists a need for a method of treating or preventing an HBV-associated disease or symptoms in a subject or inhibiting expression of one or more HBV genes using combinations of novel HBV-specific RNAi agents for the treatment of HBV infection and prevention of diseases associated with HBV.

Described herein are methods for treating and/or preventing symptoms or diseases associated with an HBV infection in a subject in need thereof (e.g., a human or animal subject). The method comprises administering one or more HBV gene-specific RNAi agents able to selectively and efficiently decrease expression of an HBV gene. In some embodiments, the symptoms and diseases associated with an HBV infection include but are not limited to chronic liver diseases/disorders, inflammations, fibrotic conditions, proliferative disorders (including cancers, such as hepatocellular carcinoma), Hepatitis D Virus (HDV) infection, and acute HBV infection. In some embodiments, the symptoms and diseases are associated with chronic HBV infection and/or HDV infection.

Additionally, described herein are methods for treating and/or preventing symptoms or diseases associated with an HBV infection in a subject in need thereof comprising administering pharmaceutical compositions comprising one or more of the disclosed HBV RNAi agents that are able to selectively and efficiently decrease expression of an HBV gene.

Also described herein are pharmaceutical compositions for use in treating and/or preventing symptoms or diseases associated with an HBV infection in a human subject, for example by administration of an effective amount of the pharmaceutical composition to the human subject. Further described are uses of the pharmaceutical compositions in the manufacture of a medicament for use in treating and/or preventing symptoms or diseases associated with an HBV infection in a human subject,

In some embodiments, there is a method for treating or preventing a disease associated with an HBV infection in a subject, comprising administering the pharmaceutical composition comprising one or more of the disclosed HBV RNAi agents. In some embodiments, a pharmaceutical composition for use in treating or preventing a disease associated with an HBV infection in a subject comprises one or more of the disclosed HBV RNAi agents. In some embodiments, there is a method for treating a disease associated with an HBV infection in a subject, comprising administering the pharmaceutical composition comprising one or more of the disclosed HBV RNAi agents. In some embodiments, a pharmaceutical composition for use in treating a disease associated with an HBV infection in a subject comprises one or more of the disclosed HBV RNAi agents. In some embodiments, there is a method for preventing a disease associated with an HBV infection in a subject, comprising administering the pharmaceutical composition comprising one or more of the disclosed HBV RNAi agents. In some embodiments, a pharmaceutical composition for use in preventing a disease associated with an HBV infection in a subject comprises one or more of the disclosed HBV RNAi agents.

In some embodiments, the one or more HBV RNAi agents are administered to the subject in an amount of about 25-400 mg per dose administration. In some embodiments, the one or more HBV RNAi agents are administered in a combined amount of about 100 mg per dose administration. In some embodiments, the one or more HBV RNAi agents are administered in a combined amount of about 35 mg, 50 mg, 200 mg, 300 mg or 400 mg per dose administration. In some embodiments, the one or more HBV RNAi agents are administered in a combined amount of about 40 mg per dose administration, about 100 mg per dose administration or about 200 mg per dose administration. In some embodiments, the one or more HBV RNAi agents are administered in a combined amount of about 40 mg per dose administration. In some embodiments, the one or more HBV RNAi agents are administered in a combined amount of about 100 mg per dose administration. In some embodiments, the one or more HBV RNAi agents are administered in a combined amount of about 200 mg per dose administration. In some embodiments, the one or more HBV RNAi agents are administered in a combined amount of about 1-10 mg/kg per dose administration. In some embodiments, the one or more HBV RNAi agents are administered in a combined amount of about 1-5 mg/kg per dose administration. In some embodiments, the one or more HBV RNAi agents are administered in about 1-18 weeks intervals. In some embodiments, the one or more HBV RNAi agents are administered in about 1 month intervals (i.e., monthly dosing). In some embodiments, the one or more HBV RNAi agents are administered in intervals of about once every four weeks. In some embodiments, the one or more HBV RNAi agents are administered in a combined amount of about 40 mg, about 100 mg or about 200 mg per dose administration in intervals of about once every four weeks. In some embodiments, the one or more HBV RNAi agents are administered in about 7-day, 14-day, 21-day or 28-day intervals. In some embodiments, the one or more HBV RNAi agents are administered in 28-day intervals or about 28-day intervals. In some embodiments, the one or more HBV RNAi agents are administered in a combined amount of about 40 mg, about 100 mg or about 200 mg per dose administration in 28-day intervals or about 28-day intervals (i.e., Q4W). In some embodiments, the one or more HBV RNAi agents are administered in a combined amount of about 40 mg per dose administration in 28-day intervals or about 28-day intervals (i.e., Q4W). In some embodiments, the one or more HBV RNAi agents are administered in a combined amount of about 100 mg per dose administration in 28-day intervals or about 28-day intervals (i.e., Q4W). In some embodiments, the one or more HBV RNAi agents are administered in a combined amount of about 200 mg per dose administration in 28-day intervals or about 28-day intervals (i.e., Q4W).

Also described herein are methods for inhibiting expression of one or more Hepatitis B Virus genes. In some embodiments, the methods inhibit expression of one or more HBV transcripts. In some embodiments, the methods inhibit expression of most or all HBV transcripts. In some embodiments, the method comprises administering one or more HBV RNAi agents described herein. In some embodiments, the method comprises administering a pharmaceutical composition comprising one or more of the HBV RNAi agents described herein. In some embodiments, the method comprises administering two HBV RNAi agents described herein. In some embodiments, the method comprises administering a pharmaceutical composition comprising two HBV RNAi agents described herein. In some embodiments, the method comprises contacting a cell infected with HBV with one or more HBV RNAi agents described herein. In some embodiments, the method comprises contacting a cell infected with HBV with two HBV RNAi agents described herein. In some embodiments, the one or more HBV RNAi agents are administered in a combined amount of about 25-400 mg per dose administration. In some embodiments, the one or more HBV RNAi agents are administered in a combined amount of about 100 mg per dose administration. In some embodiments, the one or more HBV RNAi agents are administered in a combined amount of about 35 mg, 50 mg, 200 mg, 300 mg or 400 mg per dose administration. In some embodiments, the one or more HBV RNAi agents are administered in a combined amount of about 40 mg, about 100 mg or about 200 mg per dose administration. In some embodiments, the one or more HBV RNAi agents are administered in a combined amount of about 40 mg per dose administration. In some embodiments, the one or more HBV RNAi agents are administered in a combined amount of about 100 mg per dose administration. In some embodiments, the one or more HBV RNAi agents are administered in a combined amount of about 200 mg per dose administration. In some embodiments, the one or more HBV RNAi agents are administered in a combined amount of about 1-10 mg/kg per dose administration. In some embodiments, the one or more HBV RNAi agents are administered in a combined amount of about 1-5 mg/kg per dose administration. In some embodiments, the one or more HBV RNAi agents are administered in about 1-18 week intervals. In some embodiments, the one or more HBV RNAi agents are administered in about 1 month intervals (i.e., monthly dosing). In some embodiments, the one or more HBV RNAi agents, such as a combined amount of about any one of 40, 100 or 200 mg per dose administration of the one or more HBV RNAi agents, are administered in intervals of about once every four weeks. In some embodiments, the one or more HBV RNAi agents are administered in about 7-day, 14-day, 21-day or 28-day intervals. In some embodiments, the one or more HBV RNAi agents are administered in 28-day intervals or about 28-day intervals. In some embodiments, the one or more HBV RNAi agents are administered in a combined amount of about 40 mg per dose administration, about 100 mg per dose administration or about 200 mg per dose administration in 28-day intervals or about 28-day intervals (i.e., Q4W). In some embodiments, the one or more HBV RNAi agents are administered in a combined amount of about 40 mg per dose administration in 28-day intervals or about 28-day intervals (i.e., Q4W). In some embodiments, the one or more HBV RNAi agents are administered in a combined amount of about 100 mg per dose administration in 28-day intervals or about 28-day intervals (i.e., Q4W). In some embodiments, the one or more HBV RNAi agents are administered in a combined amount of about 200 mg per dose administration in 28-day intervals or about 28-day intervals (i.e., Q4W).

Each HBV RNAi agent disclosed herein includes at least a sense strand and an antisense strand. The sense strand and the antisense strand can be partially, substantially, or fully complementary to each other. The length of the RNAi agent sense and antisense strands described herein each can be 16 to 30 nucleotides in length. In some embodiments, the sense and antisense strands are independently 17 to 26 nucleotides in length. In some embodiments, the sense and antisense strands are independently 19 to 26 nucleotides in length. In some embodiments, the sense and antisense strands are independently 21 to 26 nucleotides in length. In some embodiments, the sense and antisense strands are independently 21 to 24 nucleotides in length. The sense and antisense strands can be either the same length or different lengths. The HBV RNAi agents disclosed herein have been designed to include antisense strand sequences that are at least partially complementary to a sequence in the HBV genome that is conserved across the majority of known serotypes of HBV. The RNAi agents described herein, upon delivery to a cell expressing HBV, inhibit the expression of one or more HBV genes in vivo or in vitro.

An HBV RNAi agent includes a sense strand (also referred to as a passenger strand) that includes a first sequence, and an antisense strand (also referred to as a guide strand) that includes a second sequence. A sense strand of the HBV RNAi agents described herein includes a core stretch having at least about 85% identity to a nucleotide sequence of at least 16 consecutive nucleotides in an HBV mRNA. In some embodiments, the sense strand core nucleotide stretch having at least about 85% identity to a sequence in an HBV mRNA is 16, 17, 18, 19, 20, 21, 22, or 23 nucleotides in length. An antisense strand of an HBV RNAi agent comprises a nucleotide sequence having at least about 85% complementary over a core stretch of at least 16 consecutive nucleotides to a sequence in an HBV mRNA and the corresponding sense strand. In some embodiments, the antisense strand core nucleotide sequence having at least about 85% complementarity to a sequence in an HBV mRNA or the corresponding sense strand is 16, 17, 18, 19, 20, 21, 22, or 23 nucleotides in length.

Examples of HBV RNAi agent sense strands and antisense strands that can be used in HBV RNAi agents are provided in Tables 3 and 4. Examples of HBV RNAi agent duplexes are provided in Table 5. Examples of 19-nucleotide core stretch sequences that consist of or are included in the sense strands and antisense strands of HBV RNAi agents disclosed herein, are provided in Table 2.

In some embodiments, one or more HBV RNAi agents are delivered to target cells or tissues using any oligonucleotide delivery technology known in the art. Nucleic acid delivery methods include, but are not limited to, by encapsulation in liposomes, by iontophoresis, or by incorporation into other vehicles, such as hydrogels, cyclodextrins, biodegradable nanocapsules, and bioadhesive microspheres, proteinaceous vectors or Dynamic Polyconjugates (DPCs) (see, for example WO 2000/053722, WO 2008/0022309, WO 2011/104169, and WO 2012/083185, each of which is incorporated herein by reference). In some embodiments, an HBV RNAi agent is delivered to target cells or tissues by covalently linking the RNAi agent to a targeting group. In some embodiments, the targeting group can include a cell receptor ligand, such as an asialoglycoprotein receptor (ASGPr) ligand. In some embodiments, an ASGPr ligand includes or consists of a galactose derivative cluster. In some embodiments, a galactose derivative cluster includes an N-acetyl-galactosamine trimer or an N-acetyl-galactosamine tetramer. In some embodiments, a galactose derivative cluster is an N-acetyl-galactosamine trimer or an N-acetyl-galactosamine tetramer.

A targeting group can be linked to the 3' or 5' end of a sense strand or an antisense strand of an HBV RNAi agent. In some embodiments, a targeting group is linked to the 3' or 5' end of the sense strand. In some embodiments, a targeting group is linked to the 5' end of the sense strand. In some embodiments, a targeting group is linked to the RNAi agent via a linker.

A targeting group, with or without a linker, can be linked to the 5' or 3' end of any of the sense and/or antisense strands disclosed in Tables 2, 3, and 4. A linker, with or without a targeting group, can be attached to the 5' or 3' end of any of the sense and/or antisense strands disclosed in Tables 2, 3, and 4.

In some embodiments, described herein are methods of treating or preventing an HBV-associated disease or symptoms in a subject or inhibiting expression of one or more HBV genes comprising administering a composition that comprises one or more HBV RNAi agents having the duplex sequences disclosed in Table 5.

In some embodiments, described herein are methods of treating or preventing an HBV-associated disease in a subject comprising administering a composition that comprises one or more HBV RNAi agents having the duplex sequences disclosed in Table 5.

In some embodiments, described herein are methods of treating an HBV-associated disease in a subject comprising administering a composition that comprises one or more HBV RNAi agents having the duplex sequences disclosed in Table 5.

In some embodiments, described herein are methods of preventing an HBV-associated disease in a subject comprising administering a composition that comprises one or more HBV RNAi agents having the duplex sequences disclosed in Table 5.

In some embodiments, the composition comprises a combination or cocktail of at least two HBV RNAi agents having different nucleotide sequences. In some embodiments, the two or more different HBV RNAi agents are each separately and independently linked to targeting groups. In some embodiments, the two or more different HBV RNAi agents are each linked to targeting groups comprised of N-acetyl-galactosamines. In some embodiments, when two or more RNAi agents are included in a composition, each of the RNAi agents is linked to the same targeting group. In some embodiments, when two or more RNAi agents are included in a composition, each of the RNAi agents is linked to different targeting groups, such as targeting groups having different chemical structures.

In some embodiments, targeting groups are linked to the HBV RNAi agents without the use of an additional linker. In some embodiments, the targeting group is designed having a linker readily present to facilitate the linkage to an HBV RNAi agent. In some embodiments, when two or more RNAi agents are included in a composition, the two or more RNAi agents may be linked to the targeting groups using the same linkers. In some embodiments, when two or more RNAi agents are included in a composition, the two or more RNAi agents are linked to the targeting groups using different linkers.

HBV mRNA is known to be polycistronic, resulting in the translation of multiple polypeptides, and separate mRNAs overlap in RNA sequence, therefore a single RNAi agent targeting an HBV gene may result in inhibition of most or all HBV transcripts. However, while not wishing to be bound to any theory, it is hypothesized that a composition that includes two or more HBV RNAi agents targeting different locations or regions of an HBV gene (and, in particular, two or more HBV RNAi agents wherein one HBV RNAi agent targets the S ORF and a second HBV RNAi agent targets the X ORF) may provide for additional advantages over a composition that includes only a single HBV RNAi agent, such as (a) ensuring that all HBV viral transcripts are targeted (i.e., 3.5 kb pre-genomic RNA; 3.5 kb pre-core mRNA; 2.4 kb pre-S1 mRNA; 2.1 kb pre-S2/S mRNA; 0.7 kb X mRNA; as well as any S-antigen expressing mRNAs produced from integrated HBV DNA); (b) serving to expand the genotype coverage to potentially address a larger patient population; and/or (c) potentially decreasing the viral resistance due to mutations in the siRNA binding site.

In some embodiments, described herein are methods of treating or preventing an HBV-associated disease or symptoms in a subject or inhibiting expression of one or more HBV genes comprising administering a pharmaceutical composition that comprises a combination of at least two HBV RNAi agents having different sequences, wherein each HBV RNAi agent targets a different location or different region of an HBV gene. In some embodiments, described herein are methods of treating or preventing an HBV-associated disease or symptoms in a subject or inhibiting expression of one or more HBV genes comprising administering a pharmaceutical composition that comprises a combination of at least two HBV RNAi agents, wherein each HBV RNAi agent is designed to target a different HBV transcript (for example, a composition that includes two HBV RNAi agents, wherein the first HBV RNAi agent includes an antisense strand that is at least partially complementary to a nucleotide sequence located in the S ORF of an HBV gene, while the second HBV RNAi agent includes an antisense strand that is at least partially complementary to a nucleotide sequence located in the X ORF of an HBV gene). As used herein, an RNAi agent that includes an antisense strand at least partially complementary to a nucleotide sequence located in the S ORF targets a portion of the HBV genome of SEQ ID NO:1 between positions 1-1307 and 3185-3221. As used herein, an RNAi agent that includes an antisense strand at least partially complementary to a nucleotide sequence located in the X ORF targets a portion of the HBV genome of SEQ ID NO:1 between positions 1308-1930. In some embodiments, the symptoms and diseases are associated with chronic HBV infection and/or HDV infection. In some embodiments the subject has a diagnosis of HBeAg positive or HBeAg negative chronic HBV infection for at least 6 months.

In some embodiments, described herein are methods of treating or preventing an HBV-associated disease in a subject, comprising administering a pharmaceutical composition that comprises a combination of at least two HBV RNAi agents having different sequences, wherein each HBV RNAi agent targets a different location or different region of an HBV gene. In some embodiments, described herein are methods of treating or preventing an HBV-associated disease in a subject comprising administering a pharmaceutical composition that comprises a combination of at least two HBV RNAi agents, wherein each HBV RNAi agent is designed to target a different HBV transcript (for example, a composition that includes two HBV RNAi agents, wherein the first HBV RNAi agent includes an antisense strand that is at least partially complementary to a nucleotide sequence located in the S ORF of an HBV gene, while the second HBV RNAi agent includes an antisense strand that is at least partially complementary to a nucleotide sequence located in the X ORF of an HBV gene).

In some embodiments, described herein are methods of treating an HBV-associated disease in a subject, comprising administering a pharmaceutical composition that comprises a combination of at least two HBV RNAi agents having different sequences, wherein each HBV RNAi agent targets a different location or different region of an HBV gene. In some embodiments, described herein are methods of treating an HBV-associated disease in a subject comprising administering a pharmaceutical composition that comprises a combination of at least two HBV RNAi agents, wherein each HBV RNAi agent is designed to target a different HBV transcript (for example, a composition that includes two HBV RNAi agents, wherein the first HBV RNAi agent includes an antisense strand that is at least partially complementary to a nucleotide sequence located in the S ORF of an HBV gene, while the second HBV RNAi agent includes an antisense strand that is at least partially complementary to a nucleotide sequence located in the X ORF of an HBV gene).

In some embodiments, the at least two HBV RNAi agents are administered in a combined amount of about 25-400 mg per dose administration. In some embodiments, the at least two HBV RNAi agents are administered in a combined amount of about 25-50 mg, 50-75 mg, 75-100 mg, 100-125 mg, 125-150 mg, 150-175 mg, 175-200 mg, 200-225 mg, 225-250 mg, 250-275 mg, 275-300 mg, 300-325 mg, 325-350 mg, 350-375 mg, 375-400 mg, 25-75 mg, 50-100 mg, 100-150 mg, 150-200 mg, 200-250 mg, 250-300 mg, 300-350 mg, 350-400 mg, 25-100 mg, 50-150 mg, 100-200 mg, 150-250 mg, 200-300 mg, 300-400 mg, 25-200 mg, or 200-400 mg per dose administration. In some embodiments, the at least two HBV RNAi agents are administered in a combined amount of about 30-50 mg per dose administration, about 90-110 mg per dose administration, or about 190-210 mg per dose administration. In some embodiments, the at least two HBV RNAi agents are administered in a combined amount of about 30-50 mg per dose administration. In some embodiments, the at least two HBV RNAi agents are administered in a combined amount of about 90-110 mg per dose administration. In some embodiments, the at least two HBV RNAi agents are administered in a combined amount of about 190-210 mg per dose administration. In some embodiments, the at least two HBV RNAi agents are administered in a combined amount of about 25 mg, about 50 mg, about 100 mg, about 125 mg, about 150 mg, about 175 mg, about 200 mg, about 225 mg, about 250 mg, about 275 mg, about 300 mg, about 325 mg, about 350 mg, about 375 mg, or about 400 mg per dose administration. In some embodiments, the at least two HBV RNAi agents are administered in a combined amount of about 50 mg, about 75 mg, about 100 mg, or about 125 mg per dose administration. In some embodiments, the one or more HBV RNAi agents are administered in a combined amount of about 35 mg, 50 mg, 100 mg, 200 mg, 300 mg or 400 mg per dose administration. In some embodiments, the at least two HBV RNAi agents are administered in a combined amount of about 35 mg, 50 mg, 100 mg, 200 mg, 300 mg or 400 mg per dose administration. In some embodiments, the at least two HBV RNAi agents are administered in a combined amount of about 40 mg, about 100 mg, or about 200 mg per dose administration. In some embodiments, the at least two HBV RNAi agents are administered in a combined amount of about 40 mg per dose administration. In some embodiments, the at least two HBV RNAi agents are administered in a combined amount of about 100 mg per dose administration. In some embodiments, the at least two HBV RNAi agents are administered in a combined amount of about 200 mg per dose administration. In some embodiments, the at least two HBV RNAi agents are administered in about 7-day, about 14-day, about 21-day or about 28-day intervals. In some embodiments, the at least two HBV RNAi agents are administered in 28-day intervals or about 28-day intervals (i.e., Q4W). In some embodiments, the at least two HBV RNAi agents are administered in a combined amount of about 40 mg, about 100 mg or about 200 mg per dose administration in 28-day intervals or about 28-day intervals (i.e., Q4W). In some embodiments, the at least two HBV RNAi agents are administered in a combined amount of about 40 mg per dose administration in 28-day intervals or about 28-day intervals (i.e., Q4W). In some embodiments, the at least two HBV RNAi agents are administered in a combined amount of about 100 mg per dose administration in 28-day intervals or about 28-day intervals (i.e., Q4W). In some embodiments, the at least two HBV RNAi agents are administered in a combined amount of about 200 mg per dose administration in 28-day intervals or about 28-day intervals (i.e., Q4W).

In some embodiments, the at least two HBV RNAi agents are administered in a combined amount of about 1-10 mg/kg per dose administration. In some embodiments, the at least two HBV RNAi agents are administered in a combined amount of about 1-5 mg/kg per dose administration. In some embodiments, the at least two HBV RNAi agents are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, the at least two HBV RNAi agents are administered in about 1-18 week intervals. In some embodiments, the at least two HBV RNAi agents are administered in about 1-week intervals, about 2-week intervals, about 3-week intervals, about 4-week intervals, about 5-week intervals, about 6-week intervals, about 7-week intervals, about 8-week intervals, about 9-week intervals, about 10-week intervals, about 11-week intervals, about 12-week intervals, about 13-week intervals, about 14-week intervals, about 15-week intervals, about 16-week intervals, about 17-week intervals, or about 18-week intervals. In some embodiments, the at least two HBV RNAi agents are administered in about 1-6 month intervals. In some embodiments, the at least two HBV RNAi agents are administered in about 1-month intervals, about 2-month intervals, about 3-month intervals, about 4-month intervals, about 5-month intervals, or about 6-month intervals. In some embodiments, the at least two HBV RNAi agents are administered in about 4-week intervals or 1-month intervals. In some embodiments, the one or more HBV RNAi agents are administered in about 7-day, 14-day, 21-day or 28-day intervals. In some embodiments, the one or more HBV RNAi agents are administered in 28-day intervals or about 28-day intervals.

In some embodiments, the at least two HBV RNAi agents are administered for a duration of about 1-12 months. In some embodiments, the at least two HBV RNAi agents are administered for a duration of at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 12 months. In some embodiments, the at least two HBV RNAi agents are administered for a duration of about 1-18 weeks. In some embodiments, the at least two HBV RNAi agents are administered for a duration of at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about 12 weeks, at least about 13 weeks, at least about 14 weeks, at least about 15 weeks, at least about 16 weeks, at least about 17 weeks, or at least about 18 weeks. In some embodiments, the at least two HBV RNAi agents are administered for a duration of about 12 weeks or 3 months.

In some embodiments, described herein are methods of treating or preventing an HBV-associated disease or symptoms in a subject or inhibiting expression of one or more HBV genes comprising administering a pharmaceutical composition that comprises a combination of one HBV RNAi agent that targets the S ORF of an HBV RNA (i.e., having an antisense strand that targets the S transcripts (S, pre-S1, and pre-S2), the pregenomic RNA (core and polymerase), and the pre-core transcripts (HBeAg) of an HBV genome), and one HBV RNAi agent that targets the X ORF of an HBV RNA (i.e., having an antisense strand that targets the X transcript of an HBV genome, the S transcripts (S, pre-S1, and pre-S2), the pregenomic RNA (core and polymerase), and the pre-core transcripts (HBeAg) of an HBV genome). In some embodiments, the pharmaceutical composition described herein comprises at least one HBV RNAi agent that contains a sequence that targets the S ORF of an HBV gene, and a second HBV RNAi agent that contains a sequence that targets the X ORF of an HBV gene.

In some embodiments, described herein are methods of treating or preventing an HBV-associated disease in a subject comprising administering a pharmaceutical composition that comprises a combination of one HBV RNAi agent that targets the S ORF of an HBV RNA (i.e., having an antisense strand that targets the S transcripts (S, pre-S1, and pre-S2), the pregenomic RNA (core and polymerase), and the pre-core transcripts (HBeAg) of an HBV genome), and one HBV RNAi agent that targets the X ORF of an HBV RNA (i.e., having an antisense strand that targets the X transcript of an HBV genome, the S transcripts (S, pre-S1, and pre-S2), the pregenomic RNA (core and polymerase), and the pre-core transcripts (HBeAg) of an HBV genome). In some embodiments, the pharmaceutical composition described herein comprises at least one HBV RNAi agent that contains a sequence that targets the S ORF of an HBV gene, and a second HBV RNAi agent that contains a sequence that targets the X ORF of an HBV gene.

In some embodiments, the two HBV RNAi agents are administered in a ratio of about 1:1, 2:1, 3:1, 4:1 or 5:1. In some embodiments, the two HBV RNAi agents are administered in a ratio of about 2:1.

In some embodiments, the two HBV RNAi agents are administered in a combined amount of about 25-75 mg per dose administration and in the ratio of about 2:1, about 3:1, about 1:1, about 4:1, about 5:1 or about 1:2. In some embodiments, the two HBV RNAi agents are administered in a combined amount of about 50-125 mg per dose administration and in the ratio of about 2:1, about 3:1, about 1:1, about 4:1, about 5:1 or about 1:2. In some embodiments, the two HBV RNAi agents are administered in a combined amount of about 75-150 mg per dose administration and in the ratio of about 2:1, about 3:1, about 1:1, about 4:1, about 5:1 or about 1:2. In some embodiments, the two HBV RNAi agents are administered in a combined amount of about 100-200 mg per dose administration and in the ratio of about 2:1, about 3:1, about 1:1, about 4:1, about 5:1 or about 1:2. In some embodiments, the two HBV RNAi agents are administered in a combined amount of about 150-250 mg per dose administration and in the ratio of about 2:1, about 3:1, about 1:1, about 4:1, about 5:1 or about 1:2. In some embodiments, the two HBV RNAi agents are administered in a combined amount of about 200-300 mg per dose administration and in the ratio of about 2:1, about 3:1, about 1:1, about 4:1, about 5:1 or about 1:2. In some embodiments, the two HBV RNAi agents are administered in a combined amount of about 300-400 mg per dose administration and in the ratio of about 2:1, about 3:1, about 1:1, about 4:1, about 5:1 or about 1:2. In some embodiments, the two HBV RNAi agents are administered in a combined amount of about 50-100 mg per dose administration and in the ratio of about 2:1, about 3:1, about 1:1, about 4:1, about 5:1 or about 1:2. In some embodiments, the two HBV RNAi agents are administered in a combined amount of about 25-400 mg per dose administration and in the ratio of about 2:1. In some embodiments, the two HBV RNAi agents are administered in a combined amount of about 25-75 mg per dose administration and in the ratio of about 2:1. In some embodiments, the two HBV RNAi agents are administered in a combined amount of about 50-125 mg per dose administration and in the ratio of about 2:1. In some embodiments, the two HBV RNAi agents are administered in a combined amount of about 75-150 mg per dose administration and in the ratio of about 2:1. In some embodiments, the two HBV RNAi agents are administered in a combined amount of about 100-200 mg per dose administration and in the ratio of about 2:1. In some embodiments, the two HBV RNAi agents are administered in a combined amount of about 125-225 mg per dose administration and in the ratio of about 2:1. In some embodiments, the two HBV RNAi agents are administered in a combined amount of about 150-250 mg per dose administration and in the ratio of about 2:1. In some embodiments, the two HBV RNAi agents are administered in a combined amount of about 200-300 mg per dose administration and in the ratio of about 2: 1. In some embodiments, the two HBV RNAi agents are administered in a combined amount of about 300-400 mg per dose administration and in the ratio of about 2:1. In some embodiments, the two HBV RNAi agents are administered in a combined amount of about 35 mg per dose administration and in the ratio of about 2:1. In some embodiments, the two HBV RNAi agents are administered in a combined amount of about 40 mg per dose administration and in the ratio of about 2:1. In some embodiments, the two HBV RNAi agents are administered in a combined amount of about 100 mg per dose administration and in the ratio of about 2:1. In some embodiments, the two HBV RNAi agents are administered in a combined amount of about 200 mg per dose administration and in the ratio of about 2:1. In some embodiments, the two HBV RNAi agents are administered in a combined amount of about 300 mg per dose administration and in the ratio of about 2:1. In some embodiments, the two HBV RNAi agents are administered in a combined amount of about 400 mg per dose administration and in the ratio of about 2: 1. In some embodiments, the two HBV RNAi agents are administered in about 7-day, about 14-day, about 21-day or about 28-day intervals. In some embodiments, the two HBV RNAi agents are administered in 28-day intervals or about 28-day intervals (i.e., Q4W). In some embodiments, the two HBV RNAi agents are administered in a combined amount of any one of about 40 mg, about 100 mg or about 200 mg per dose administration, in the ratio of about 2:1, and in 28-day intervals or about 28-day intervals (i.e., Q4W). In some embodiments, the two HBV RNAi agents are administered in a combined amount of about 40 mg per dose administration, in the ratio of about 2:1, and in 28-day intervals or about 28-day intervals (i.e., Q4W). In some embodiments, the two HBV RNAi agents are administered in a combined amount of about 100 mg per dose administration, in the ratio of about 2:1, and in 28-day intervals or about 28-day intervals (i.e., Q4W). In some embodiments, the two HBV RNAi agents are administered in a combined amount of about 200 mg per dose administration, in the ratio of about 2:1, and in 28-day intervals or about 28-day intervals (i.e., Q4W).

In some embodiments, the first RNAi agent is administered in an amount of about 3-650 mg per dose administration, and the second RNAi agent is administered in an amount of about 2-325 mg per dose administration. In some embodiments, the first RNAi agent is administered in an amount of about 35-265 mg per dose administration. In some embodiments, the first RNAi agent is administered in an amount of about 50-75 mg per dose administration. In some embodiments, the second RNAi agent is administered in an amount of about 20-125 mg per dose administration. In some embodiments, the second RNAi agent is administered in an amount of about 25-50 mg per dose administration.

In some embodiments, two RNAi agents are administered at a combined dose of 25-400 mg per dose administration. In an embodiment, two RNAi agents are administered at a combined dose of 25-400 mg, and the first RNAi agent is administered with the second RNAi agent at a ratio of 1:1. In an embodiment, the dose of each of the first and second RNAi agents is in an amount of about 12 mg for a combined dose of about 25 mg. In an embodiment, the dose of each of the first and second RNAi agents is in an amount of about 17 mg for a combined dose of about 35 mg. In an embodiment, the dose of each of the first and second RNAi agents is in an amount of about 20 mg for a combined dose of about 40 mg. In an embodiment, the dose of each of the first and second RNAi agents is in an amount of about 25 mg for a combined dose of about 50 mg. In an embodiment, the dose of each of the first and second RNAi agents is in an amount of about 50 mg for a combined dose of about 100 mg. In an embodiment, the dose of each of the first and second RNAi agents is in an amount of about 100 mg for a combined dose of about 200 mg. In an embodiment, the dose of each of the first and second RNAi agents is in an amount of about 150 mg for a combined dose of about 300 mg. In an embodiment, the dose of each of the first and second RNAi agents is in an amount of about 200 mg for a combined dose of about 400 mg. In some embodiments, two RNAi agents are administered in about 7-day, about 14-day, about 21-day or about 28-day intervals. In some embodiments, two RNAi agents are administered in 28-day intervals or about 28-day intervals (i.e., Q4W). In an embodiment, the dose of each of the first and second RNAi agents is in an amount of about 20 mg for a combined dose of about 40 mg, or in an amount of about 50 mg for a combined dose of about 100 mg, or in an amount of about 100 mg for a combined dose of about 200 mg, and the two RNAi agents are administered in 28-day intervals or about 28-day intervals (i.e., Q4W). In an embodiment, the dose of each of the first and second RNAi agents is in an amount of about 20 mg for a combined dose of about 40 mg and the two RNAi agents are administered in 28-day intervals or about 28-day intervals (i.e., Q4W). In an embodiment, the dose of each of the first and second RNAi agents is in an amount of about 50 mg for a combined dose of about 100 mg and the two RNAi agents are administered in 28-day intervals or about 28-day intervals (i.e., Q4W). In an embodiment, the dose of each of the first and second RNAi agents is in an amount of about 100 mg for a combined dose of about 200 mg and the two RNAi agents are administered in 28-day intervals or about 28-day intervals (i.e., Q4W).

In an embodiment, two RNAi agents are administered at a combined dose of 25-400 mg per dose, and the first RNAi agent is administered with the second RNAi agent at a ratio of 2:1. In an embodiment, the dose of the first RNAi agent is in an amount of about 16 mg, and the dose of the second RNAi agent is in an amount of about 8 mg for a combined dose of about 25 mg. In an embodiment, the dose of the first RNAi agent is in an amount of about 24 mg, and the dose of the second RNAi agent is in an amount of about 12 mg for a combined dose of about 35 mg. In an embodiment, the dose of the first RNAi agent is in an amount of about 27 mg, and the dose of the second RNAi agent is in an amount of about 13 mg for a combined dose of about 40 mg. In an embodiment, the dose of the first RNAi agent is in an amount of about 33 mg, and the dose of the second RNAi agent is in an amount of about 17 mg for a combined dose of about 50 mg. In an embodiment, the dose of the first RNAi agent is in an amount of about 65 mg, and the dose of the second RNAi agent is in an amount of about 35 mg for a combined dose of about 100 mg. In an embodiment, the dose of the first RNAi agent is in an amount of about 133 mg, and the dose of the second RNAi agent is in an amount of about 67 mg for a combined dose of about 200 mg. In an embodiment, the dose of the first RNAi agent is in an amount of about 200 mg, and the dose of the second RNAi agent is in an amount of about 100 mg for a combined dose of about 300 mg. In an embodiment, the dose of the first RNAi agent is in an amount of about 270 mg, and the dose of the second RNAi agent is in an amount of about 135 mg for a combined dose of about 400 mg. In some embodiments, two RNAi agents are administered in about 7-day, about 14-day, about 21-day or about 28-day intervals. In some embodiments, two RNAi agents are administered in 28-day intervals or about 28-day intervals (i.e., Q4W). In an embodiment, the dose of the first RNAi agent is in an amount of about 27 mg, and the dose of the second RNAi agent is in an amount of about 13 mg for a combined dose of about 40 mg and the two RNAi agents are administered in 28-day intervals or about 28-day intervals (i.e., Q4W). In an embodiment, the dose of the first RNAi agent is in an amount of about 65 mg, and the dose of the second RNAi agent is in an amount of about 35 mg for a combined dose of about 100 mg and the two RNAi agents are administered in 28-day intervals or about 28-day intervals (i.e., Q4W). In an embodiment, the dose of the first RNAi agent is in an amount of about 133 mg, and the dose of the second RNAi agent is in an amount of about 67 mg for a combined dose of about 200 mg and the two RNAi agents are administered in 28-day intervals or about 28-day intervals (i.e., Q4W).

In an embodiment, two RNAi agents are administered at a combined dose of 25-400 mg per dose, the first RNAi agent is administered with the second RNAi agent at a ratio of 3:1. In an embodiment, the dose of the first RNAi agent is in an amount of about 18 mg, and the dose of the second RNAi agent is in an amount of about 6 mg for a combined dose of about 25 mg. In an embodiment, the dose of the first RNAi agent is in an amount of about 27 mg, and the dose of the second RNAi agent is in an amount of about 9 mg for a combined dose of about 35 mg. In an embodiment, the dose of the first RNAi agent is in an amount of about 30 mg, and the dose of the second RNAi agent is in an amount of about 10 mg for a combined dose of about 40 mg. In an embodiment, the dose of the first RNAi agent is in an amount of about 36 mg, and the dose of the second RNAi agent is in an amount of about 12 mg for a combined dose of about 50 mg. In an embodiment, the dose of the first RNAi agent is in an amount of about 75 mg, and the dose of the second RNAi agent is in an amount of about 25 mg for a combined dose of about 100 mg. In an embodiment, the dose of the first RNAi agent is in an amount of about 150 mg, and the dose of the second RNAi agent is in an amount of about 50 mg for a combined dose of about 200 mg. In an embodiment, the dose of the first RNAi agent is in an amount of about 225 mg, and the dose of the second RNAi agent is in an amount of about 75 mg for a combined dose of about 300 mg. In an embodiment, the dose of the first RNAi agent is in an amount of about 300 mg, and the dose of the second RNAi agent is in an amount of about 100 mg for a combined dose of about 400 mg. In some embodiments, two RNAi agents are administered in about 7-day, about 14-day, about 21-day or about 28-day intervals. In some embodiments, two RNAi agents are administered in 28-day intervals or about 28-day intervals (i.e., Q4W). In an embodiment, the dose of the first RNAi agent is in an amount of about 30 mg, and the dose of the second RNAi agent is in an amount of about 10 mg for a combined dose of about 40 mg and the two RNAi agents are administered in 28-day intervals or about 28-day intervals (i.e., Q4W). In an embodiment, the dose of the first RNAi agent is in an amount of about 75 mg, and the dose of the second RNAi agent is in an amount of about 25 mg for a combined dose of about 100 mg and the two RNAi agents are administered in 28-day intervals or about 28-day intervals (i.e., Q4W). In an embodiment, the dose of the first RNAi agent is in an amount of about 150 mg, and the dose of the second RNAi agent is in an amount of about 50 mg for a combined dose of about 200 mg and the two RNAi agents are administered in 28-day intervals or about 28-day intervals (i.e., Q4W).

In some embodiments, the two HBV RNAi agents are administered in a combined amount of about 1-10 mg/kg per dose administration. In some embodiments, the two HBV RNAi agents are administered in a combined amount of about 1-5 mg/kg per dose administration. In some embodiments, the two HBV RNAi agents are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration. In some embodiments, the two HBV RNAi agents are administered in about 7-day, about 14-day, about 21-day or about 28-day intervals. In some embodiments, the two HBV RNAi agents are administered in 28-day intervals or about 28-day intervals (i.e., Q4W).

In some embodiments, the first RNAi agent is administered in an amount of about 0.6-7 mg/kg per dose administration, and the second RNAi agent is administered in an amount of about 0.3-5 mg/kg per dose administration. In some embodiments, the second RNAi agent is administered in an amount of about 0.5-2.5 mg/kg per dose administration. In some embodiments, the second RNAi agent is administered in an amount of about 0.3-1.5 mg/kg per dose administration. In some embodiments, the first RNAi agent is administered in an amount of about 0.6-5 mg/kg per dose administration. In some embodiments, the first RNAi agent is administered in an amount of about 1-2.5 mg/kg per dose administration. In some embodiments, the first RNAi agent and the second RNAi agent are administered in about 7-day, about 14-day, about 21-day or about 28-day intervals. In some embodiments, the first RNAi agent and the second RNAi agent are administered in 28-day intervals or about 28-day intervals (i.e., Q4W).

Disclosed herein are methods for inhibiting expression of an HBV gene, the method comprising administering one or more HBV RNAi agents having an antisense strand comprising the sequence of any of the sequences in Table 3.

Disclosed herein are methods for inhibiting expression of an HBV gene, the method comprising administering one or more HBV RNAi agents having a sense strand comprising the sequence of any of the sequences in Table 4.

Disclosed herein are methods for inhibiting expression of an HBV gene, the method comprising administering one or more HBV RNAi agents having an antisense strand comprising the sequence of any of the sequences in Table 3, and a sense strand comprising the sequence of any of the sequences in Table 4 that is at least partially complementary to the antisense strand.

Disclosed herein are methods for inhibiting expression of an HBV gene, the method comprising administering one or more HBV RNAi agents having an antisense strand that consists of the sequence of any of the sequences in Table 3, and a sense strand that consists of the sequence of any of the sequences in Table 4 that is at least partially complementary to the antisense strand.

Disclosed herein are methods for inhibiting expression of an HBV gene in a cell, the method comprising administering one or more HBV RNAi agents having the duplex structure of Table 5.

Disclosed herein are methods of treatment of an HBV infection or prevention of disease or symptoms caused by an HBV infection, the method comprising administering one or more HBV RNAi agents having an antisense strand comprising the sequence of any of the sequences in Table 3.

Disclosed herein are methods of treating or preventing a disease associated with an infection caused by HBV, comprising administering one or more HBV RNAi agents having an antisense strand comprising the sequence of any of the sequences in Table 3.

Disclosed herein are methods of treating a disease associated with an infection caused by HBV, comprising administering one or more HBV RNAi agents having an antisense strand comprising the sequence of any of the sequences in Table 3.

Disclosed herein are methods of treatment of an HBV infection or prevention of disease or symptoms caused by an HBV infection, the method comprising administering one or more HBV RNAi agents having a sense strand comprising the sequence of any of the sequences in Table 4.

Disclosed herein are methods of treating or preventing a disease associated with an infection caused by HBV, comprising administering one or more HBV RNAi agents having a sense strand comprising the sequence of any of the sequences in Table 4.

Disclosed herein are methods of treating a disease associated with an infection caused by HBV, comprising administering one or more HBV RNAi agents having a sense strand comprising the sequence of any of the sequences in Table 4.

Disclosed herein are methods of treatment of an HBV infection or prevention of disease or symptoms caused by an HBV infection, the method comprising administering one or more HBV RNAi agents having an antisense strand comprising the sequence of any of the sequences in Table 3, and a sense strand comprising the sequence of any of the sequences in Table 4 that is at least partially complementary to the antisense strand.

Disclosed herein are methods of treating or preventing a disease associated with an infection caused by HBV, comprising administering one or more HBV RNAi agents having an antisense strand comprising the sequence of any of the sequences in Table 3, and a sense strand comprising the sequence of any of the sequences in Table 4 that is at least partially complementary to the antisense strand.

Disclosed herein are methods of treating a disease associated with an infection caused by HBV, comprising administering one or more HBV RNAi agents having an antisense strand comprising the sequence of any of the sequences in Table 3, and a sense strand comprising the sequence of any of the sequences in Table 4 that is at least partially complementary to the antisense strand.

Disclosed herein are methods of treatment of an HBV infection or prevention of disease or symptoms caused by an HBV infection, the method comprising administering one or more HBV RNAi agents having an antisense strand that consists of the sequence of any of the sequences in Table 3, and a sense strand that consists of the sequence of any of the sequences in Table 4 that is at least partially complementary to the antisense strand.

Disclosed herein are methods of treating or preventing a disease associated with an infection caused by HBV, comprising administering one or more HBV RNAi agents having an antisense strand that consists of the sequence of any of the sequences in Table 3, and a sense strand that consists of the sequence of any of the sequences in Table 4 that is at least partially complementary to the antisense strand.

Disclosed herein are methods of treating a disease associated with an infection caused by HBV, comprising administering one or more HBV RNAi agents having an antisense strand that consists of the sequence of any of the sequences in Table 3, and a sense strand that consists of the sequence of any of the sequences in Table 4 that is at least partially complementary to the antisense strand.

Disclosed herein are methods of treatment of an HBV infection or prevention of disease or symptoms caused by an HBV infection, the method comprising administering one or more HBV RNAi agents having the duplex structure of Table 5.

Disclosed herein are methods of treating or preventing a disease associated with an infection caused by HBV, comprising administering one or more HBV RNAi agents having the duplex structure of Table 5.

Disclosed herein are methods of treating a disease associated with an infection caused by HBV, comprising administering one or more HBV RNAi agents having the duplex structure of Table 5.

Disclosed herein are methods for inhibiting expression of an HBV gene, the method comprising administering (i) an HBV RNAi agent having an antisense strand comprising or consisting of the sequence of any of the sequences in Table 2 or Table 3, and (ii) a second HBV RNAi agent having an antisense strand comprising or consisting of the sequence of any of the sequences in Table 2 or Table 3.

Disclosed herein are methods of treatment of an HBV infection or prevention of disease or symptoms caused by an HBV infection, the method comprising administering (i) an HBV RNAi agent having an antisense strand comprising or consisting of the sequence of any of the sequences in Table 2 or Table 3, and (ii) a second HBV RNAi agent having an antisense strand comprising or consisting of the sequence of any of the sequences in Table 2 or Table 3.

Disclosed herein are methods of treating or preventing a disease associated with an infection caused by HBV, comprising administering (i) an HBV RNAi agent having an antisense strand comprising or consisting of the sequence of any of the sequences in Table 2 or Table 3, and (ii) a second HBV RNAi agent having an antisense strand comprising or consisting of the sequence of any of the sequences in Table 2 or Table 3.

Disclosed herein are methods of treating a disease associated with an infection caused by HBV, comprising administering (i) an HBV RNAi agent having an antisense strand comprising or consisting of the sequence of any of the sequences in Table 2 or Table 3, and (ii) a second HBV RNAi agent having an antisense strand comprising or consisting of the sequence of any of the sequences in Table 2 or Table 3.

Disclosed herein are methods for inhibiting expression of an HBV gene, the method comprising administering (i) a first HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the first HBV RNAi agent, and (ii) a second HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the second HBV RNAi agent.

Disclosed herein are methods of treatment of an HBV infection or prevention of disease or symptoms caused by an HBV infection, the method comprising administering (i) a first HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the first HBV RNAi agent, and (ii) a second HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the second HBV RNAi agent.

Disclosed herein are methods of treating or preventing a disease associated with an infection caused by HBV, comprising administering (i) a first HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the first HBV RNAi agent, and (ii) a second HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the second HBV RNAi agent.

Disclosed herein are methods of treating a disease associated with an infection caused by HBV, comprising administering (i) a first HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the first HBV RNAi agent, and (ii) a second HBV RNAi agent having an antisense strand comprising or consisting of the sequence of any of the sequences in Table 2 or Table 3 and a sense strand comprising or consisting of the sequence of any of the sequences in Table 2 or Table 4 that is at least partially complementary to the antisense strand of the second HBV RNAi agent.

In some embodiments, an HBV RNAi agent disclosed herein comprises:
a. an antisense strand that comprises the nucleobase sequence differing by 0, 1, 2 or 3 nucleobases from the sequence (5'→3') AUUGAGAGAAGUCCACCAC (SEQ ID NO: 7), and a sense strand that comprises the nucleobase sequence differing by 0, 1, 2 or 3 nucleobases from the sequence (5'→3') GUGGUGGACUUCUCUCAAU (SEQ ID NO: 34); or
b. an antisense strand that comprises the nucleobase sequence differing by 0, 1, 2 or 3 nucleobases from the sequence (5'→3') UUUGAGAGAAGUCCACCAC (SEQ ID NO: 8), and a sense strand that comprises the nucleobase sequence differing by 0, 1, 2 or 3 nucleobases from the sequence (5'→3') GUGGUGGACUUCUCUCAAA (SEQ ID NO: 35); or
c. an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
d. an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40); or
e. an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
f. an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45); or
g. an antisense strand that comprises the nucleobase sequence differing by 0, 1, 2 or 3 nucleobases from the sequence (5'→3') ACCAAUUUAUGCCUACAGC (SEQ ID NO: 22), and a sense strand that comprises the nucleobase sequence differing by 0, 1, 2 or 3 nucleobases from the sequence (5'→3') GCUGUAGGCAUAAAUUGGU (SEQ ID NO: 49); or
h. an antisense strand that comprises the nucleobase sequence differing by 0, 1, 2 or 3 nucleobases from the sequence (5'→3') UCCAAUUUAUGCCUACAGC (SEQ ID NO: 23), and a sense strand that comprises the nucleobase sequence differing by 0, 1, 2 or 3 nucleobases from the sequence (5'→3') GCUGUAGGCAUAAAUUGGA (SEQ ID NO: 50); or
i. an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
j. an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
k. an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises an HBV RNAi agent.

In some embodiments, disclosed herein are methods of treating or preventing a disease associated with an infection caused by HBV, comprising administering a composition that inhibits expression of an HBV gene in a cell, wherein the composition comprises an HBV RNAi agent.

In some embodiments, disclosed herein are methods of treating a disease associated with an infection caused by HBV, comprising administering a composition that inhibits expression of an HBV gene in a cell, wherein the composition comprises an HBV RNAi agent.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two or more HBV RNAi agents, wherein a first HBV RNAi agent comprises:
i) an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
ii) an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40);
and wherein a second HBV RNAi agent comprises:
i) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
ii) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
iii) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating or preventing a disease associated with an infection caused by HBV, comprising administering a composition that inhibits expression of an HBV gene in a cell, wherein the composition comprises two or more HBV RNAi agents, wherein a first HBV RNAi agent comprises:
iii) an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
iv) an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40);
and wherein a second HBV RNAi agent comprises:
iv) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
v) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
vi) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating a disease associated with an infection caused by HBV, comprising administering a composition that inhibits expression of an HBV gene in a cell, wherein the composition comprises two or more HBV RNAi agents, wherein a first HBV RNAi agent comprises:
v) an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
vi) an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40);
and wherein a second HBV RNAi agent comprises:
vii) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
viii) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
ix) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two or more HBV RNAi agents, wherein a first HBV RNAi agent comprises:
i) an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
ii) an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45);
and wherein a second HBV RNAi agent comprises:
i) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
ii) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
iii) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating or preventing a disease associated with an infection caused by HBV, comprising administering a composition that inhibits expression of an HBV gene in a cell, wherein the composition comprises two or more HBV RNAi agents, wherein a first HBV RNAi agent comprises:
iii) an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
iv) an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45);
and wherein a second HBV RNAi agent comprises:
iv) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
v) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
vi) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating a disease associated with an infection caused by HBV, comprising administering a composition that inhibits expression of an HBV gene in a cell, wherein the composition comprises two or more HBV RNAi agents, wherein a first HBV RNAi agent comprises:
v) an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
vi) an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45);
and wherein a second HBV RNAi agent comprises:
vii) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
viii) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
ix) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are a method of treating or preventing an HBV-associated disease or symptoms in a subject or inhibiting expression of one or more HBV genes comprising administering a composition for inhibiting expression of an HBV gene in a cell, wherein the composition comprises two or more HBV RNAi agents, wherein a first HBV RNAi agent comprises:
i) an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
ii) an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40);
and wherein a second HBV RNAi agent comprises an antisense strand having a sequence that is at least partially complementary to a portion of the X ORF of an HBV mRNA.

In some embodiments, disclosed herein are methods of treating or preventing a disease associated with an infection caused by HBV, comprising administering a composition for inhibiting expression of an HBV gene in a cell, wherein the composition comprises two or more HBV RNAi agents, wherein a first HBV RNAi agent comprises:
iii) an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
iv) an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40);
and wherein a second HBV RNAi agent comprises an antisense strand having a sequence that is at least partially complementary to a portion of the X ORF of an HBV mRNA.

In some embodiments, disclosed herein are methods of treating a disease associated with an infection caused by HBV, comprising administering a composition for inhibiting expression of an HBV gene in a cell, wherein the composition comprises two or more HBV RNAi agents, wherein a first HBV RNAi agent comprises:
v) an antisense strand that comprises the nucleobase sequence differing by 0, 1, 2 or 3 nucleobases from the sequence (5'→3') AAUUGAGAGAAGUCCACCA (SEQ ID NO: 12), and a sense strand that comprises the nucleobase sequence differing by 0, 1, 2 or 3 nucleobases from the sequence (5'→3') UGGUGGACUUCUCUCAAUU (SEQ ID NO: 39); or
vi) an antisense strand that comprises the nucleobase sequence differing by 0, 1, 2 or 3 nucleobases from the sequence (5'→3') UAUUGAGAGAAGUCCACCA (SEQ ID NO: 13), and a sense strand that comprises the nucleobase sequence differing by 0, 1, 2 or 3 nucleobases from the sequence (5'→3') UGGUGGACUUCUCUCAAUA (SEQ ID NO: 40);
and wherein a second HBV RNAi agent comprises an antisense strand having a sequence that is at least partially complementary to a portion of the X ORF of an HBV mRNA.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two or more HBV RNAi agents, wherein a first HBV RNAi agent comprises:
i) an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
ii) an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45);
and wherein a second HBV RNAi agent comprises an antisense strand having a sequence that is at least partially complementary to a portion of the X ORF of an HBV mRNA.

In some embodiments, disclosed herein are methods of treating or preventing a disease associated with an infection caused by HBV, comprising administering a composition that inhibits expression of an HBV gene in a cell, wherein the composition comprises two or more HBV RNAi agents, wherein a first HBV RNAi agent comprises:
iii) an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
iv) an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45);
and wherein a second HBV RNAi agent comprises an antisense strand having a sequence that is at least partially complementary to a portion of the X ORF of an HBV mRNA.

In some embodiments, disclosed herein are methods of treating a disease associated with an infection caused by HBV, comprising administering a composition that inhibits expression of an HBV gene in a cell, wherein the composition comprises two or more HBV RNAi agents, wherein a first HBV RNAi agent comprises:
v) an antisense strand that comprises the nucleobase sequence differing by 0, 1, 2 or 3 nucleobases from the sequence (5'→3') AGAAAAUUGAGAGAAGUCC (SEQ ID NO: 17), and a sense strand that comprises the nucleobase sequence differing by 0, 1, 2 or 3 nucleobases from the sequence (5'→3') GGACUUCUCUCAAUUUUCU (SEQ ID NO: 44); or
vi) an antisense strand that comprises the nucleobase sequence differing by 0, 1, 2 or 3 nucleobases from the sequence (5'→3') UGAAAAUUGAGAGAAGUCC (SEQ ID NO: 18), and a sense strand that comprises the nucleobase sequence differing by 0, 1, 2 or 3 nucleobases from the sequence (5'→3') GGACUUCUCUCAAUUUUCA (SEQ ID NO: 45);
and wherein a second HBV RNAi agent comprises an antisense strand having a sequence that is at least partially complementary to a portion of the X ORF of an HBV mRNA.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two or more HBV RNAi agents, wherein a first HBV RNAi agent comprises an antisense strand having a sequence that is at least partially complementary to a portion of the S ORF of an HBV mRNA, and wherein a second HBV RNAi agent comprises:
i) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
ii) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
iii) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating or preventing a disease associated with an infection caused by HBV, comprising administering a composition that inhibits expression of an HBV gene in a cell, wherein the composition comprises two or more HBV RNAi agents, wherein a first HBV RNAi agent comprises an antisense strand having a sequence that is at least partially complementary to a portion of the S ORF of an HBV mRNA, and wherein a second HBV RNAi agent comprises:
iv) an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
v) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
vi) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, disclosed herein are methods of treating a disease associated with an infection caused by HBV, comprising administering a composition that inhibits expression of an HBV gene in a cell, wherein the composition comprises two or more HBV RNAi agents, wherein a first HBV RNAi agent comprises an antisense strand having a sequence that is at least partially complementary to a portion of the S ORF of an HBV mRNA, and wherein a second HBV RNAi agent comprises:
vii)an antisense strand that comprises the nucleobase sequence differing by 0, 1, 2 or 3 nucleobases from the sequence (5'→3') GACCAAUUUAUGCCUACAG (SEQ ID NO: 27), and a sense strand that comprises the nucleobase sequence differing by 0, 1, 2 or 3 nucleobases from the sequence (5'→3') CUGUAGGCAUAAAUUGGUC (SEQ ID NO: 54); or
viii) an antisense strand that comprises the nucleobase sequence differing by 0, 1, 2 or 3 nucleobases from the sequence (5'→3') AACCAAUUUAUGCCUACAG (SEQ ID NO: 28), and a sense strand that comprises the nucleobase sequence differing by 0, 1, 2 or 3 nucleobases from the sequence (5'→3') CUGUAGGCAUAAAUUGGUU (SEQ ID NO: 55); or
ix) an antisense strand that comprises the nucleobase sequence differing by 0, 1, 2 or 3 nucleobases from the sequence (5'→3') UACCAAUUUAUGCCUACAG (SEQ ID NO: 29), and a sense strand that comprises the nucleobase sequence differing by 0, 1, 2 or 3 nucleobases from the sequence (5'→3') CUGUAGGCAUAAAUUGGUA (SEQ ID NO: 56).

In some embodiments, an HBV RNAi agent disclosed herein comprises:
a. an antisense strand that comprises the nucleobase sequence differing by 0, 1, 2 or 3 nucleobases from the sequence (5'→3') UACCAAUUUAUGCCUACAGGCCUUAU (SEQ ID NO: 149); or
b. an antisense strand that comprises the nucleobase sequence differing by 0, 1, 2 or 3 nucleobases from the sequence (5'→3') UACCAAUUUAUGCCUACAGGCCU (SEQ ID NO: 150); or
c. an antisense strand that comprises the nucleobase sequence differing by 0, 1, 2 or 3 nucleobases from the sequence (5'→3') UACCAAUUUAUGCCUACAGGC (SEQ ID NO: 151); or
d. an antisense strand that comprises the nucleobase sequence differing by 0, 1, 2 or 3 nucleobases from the sequence (5'→3') UGAAAAUUGAGAGAAGUCCUU (SEQ ID NO: 152); or
e. an antisense strand that comprises the nucleobase sequence differing by 0, 1, 2 or 3 nucleobases from the sequence (5'→3') UACCAAUUUAUGCCUACAGUU (SEQ ID NO: 154); or
f. an antisense strand that comprises the nucleobase sequence differing by 0, 1, 2 or 3 nucleobases from the sequence (5'→3') UAUUGAGAGAAGUCCACCACG (SEQ ID NO: 160); or
g. an antisense strand that comprises the nucleobase sequence differing by 0, 1, 2 or 3 nucleobases from the sequence (5'→3') UACCAAUUUAUGCCUACAGCC (SEQ ID NO: 162); or
h. an antisense strand that comprises the nucleobase sequence differing by 0, 1, 2 or 3 nucleobases from the sequence (5'→3') UACCAAUUUAUGCCUACAGCCUU (SEQ ID NO: 163); or
i. an antisense strand that comprises the nucleobase sequence differing by 0, 1, 2 or 3 nucleobases from the sequence (5'→3') UAUUGAGAGAAGUCCACCACGA (SEQ ID NO: 170); or
j. an antisense strand that comprises the nucleobase sequence differing by 0, 1, 2 or 3 nucleobases from the sequence (5'→3') AGAAAAUUGAGAGAAGUCCAC (SEQ ID NO: 171); or
k. an antisense strand that comprises the nucleobase sequence differing by 0, 1, 2 or 3 nucleobases from the sequence (5'→3') UACCAAUUUAUGCCUACAGCUU (SEQ ID NO: 172); or
l. an antisense strand that comprises the nucleobase sequence differing by 0, 1, 2 or 3 nucleobases from the sequence (5'→3') UACCAAUUUAUGCCUACAGCCU (SEQ ID NO: 173); or
m. an antisense strand that comprises the nucleobase sequence differing by 0, 1, 2 or 3 nucleobases from the sequence (5'→3') UAUUGAGAGAAGUCCACCAUU (SEQ ID NO: 174); or
n. an antisense strand that comprises the nucleobase sequence differing by 0, 1, 2 or 3 nucleobases from the sequence (5'→3') UAUUGAGAGAAGUCCACCACUU (SEQ ID NO: 175); or
o. an antisense strand that comprises the nucleobase sequence differing by 0, 1, 2 or 3 nucleobases from the sequence (5'→3') AGAAAAUUGAGAGAAGUCCUU (SEQ ID NO: 178); or
p. an antisense strand that comprises the nucleobase sequence differing by 0, 1, 2 or 3 nucleobases from the sequence (5'→3') AGAAAAUUGAGAGAAGUCCACUU (SEQ ID NO: 179); or
q. an antisense strand that comprises the nucleobase sequence differing by 0, 1, 2 or 3 nucleobases from the sequence (5'→3') AGAAAAUUGAGAGAAGUCCACC (SEQ ID NO: 180); or
r. an antisense strand that comprises the nucleobase sequence differing by 0, 1, 2 or 3 nucleobases from the sequence (5'→3') UGAAAAUUGAGAGAAGUCCAC (SEQ ID NO: 181); or
s. an antisense strand that comprises the nucleobase sequence differing by 0, 1, 2 or 3 nucleobases from the sequence (5'→3') ACCAAUUUAUGCCUACAGCUU (SEQ ID NO: 182); or
t. an antisense strand that comprises the nucleobase sequence differing by 0, 1, 2 or 3 nucleobases from the sequence (5'→3') ACCAAUUUAUGCCUACAGCCUU (SEQ ID NO: 183); or
u. an antisense strand that comprises the nucleobase sequence differing by 0, 1, 2 or 3 nucleobases from the sequence (5'→3') ACCAAUUUAUGCCUACAGCCUC (SEQ ID NO: 184); or
v. an antisense strand that comprises the nucleobase sequence differing by 0, 1, 2 or 3 nucleobases from the sequence (5'→3') UCCAAUUUAUGCCUACAGCUU (SEQ ID NO: 185); or
w. an antisense strand that comprises the nucleobase sequence differing by 0, 1, 2 or 3 nucleobases from the sequence (5'→3') UCCAAUUUAUGCCUACAGCCUU (SEQ ID NO: 186); or
x. an antisense strand that comprises the nucleobase sequence differing by 0, 1, 2 or 3 nucleobases from the sequence (5'→3') UACCAAUUUAUGCCUACAGCU (SEQ ID NO: 187); or
y. an antisense strand that comprises the nucleobase sequence differing by 0, 1, 2 or 3 nucleobases from the sequence (5'→3') UACCAAUUUAUGCCUACAGCG (SEQ ID NO: 188); or
z. an antisense strand that comprises the nucleobase sequence differing by 0, 1, 2 or 3 nucleobases from the sequence (5'→3') AACCAAUUUAUGCCUACAGCC (SEQ ID NO: 189); or
aa. an antisense strand that comprises the nucleobase sequence differing by 0, 1, 2 or 3 nucleobases from the sequence (5'→3') ACCAAUUUAUGCCUACAGCCU (SEQ ID NO: 190); or
bb. an antisense strand that comprises the nucleobase sequence differing by 0, 1, 2 or 3 nucleobases from the sequence (5'→3') UCCAAUUUAUGCCUACAGCCU (SEQ ID NO: 191); or
cc. an antisense strand that comprises the nucleobase sequence differing by 0, 1, 2 or 3 nucleobases from the sequence (5'→3') ACCAAUUUAUGCCUACAGCCG (SEQ ID NO: 192); or
dd. an antisense strand that comprises the nucleobase sequence differing by 0, 1, 2 or 3 nucleobases from the sequence (5'→3') UCCAAUUUAUGCCUACAGCCG (SEQ ID NO: 193); or
ee. an antisense strand that comprises the nucleobase sequence differing by 0, 1, 2 or 3 nucleobases from the sequence (5'→3') UACCAAUUUAUGCCUACAGGG (SEQ ID NO: 194);
and wherein the HBV RNAi agent further comprises a sense strand at least partially complementary to the respective antisense strand.

In some embodiments, an HBV RNAi agent disclosed herein comprises:
a. an antisense strand that consists of the nucleobase sequence differing by 0, 1, 2 or 3 nucleobases from the sequence (5'→3') UACCAAUUUAUGCCUACAGGCCUUAU (SEQ ID NO: 149); or
b. an antisense strand that consists of the nucleobase sequence differing by 0, 1, 2 or 3 nucleobases from the sequence (5'→3') UACCAAUUUAUGCCUACAGGCCU (SEQ ID NO: 150); or
c. an antisense strand that consists of the nucleobase sequence differing by 0, 1, 2 or 3 nucleobases from the sequence (5'→3') UACCAAUUUAUGCCUACAGGC (SEQ ID NO: 151); or
d. an antisense strand that consists of the nucleobase sequence differing by 0, 1, 2 or 3 nucleobases from the sequence (5'→3') UGAAAAUUGAGAGAAGUCCUU (SEQ ID NO: 152); or
e. an antisense strand that consists of the nucleobase sequence differing by 0, 1, 2 or 3 nucleobases from the sequence (5'→3') UACCAAUUUAUGCCUACAGUU (SEQ ID NO: 154); or
f. an antisense strand that consists of the nucleobase sequence differing by 0, 1, 2 or 3 nucleobases from the sequence (5'→3') UAUUGAGAGAAGUCCACCACG (SEQ ID NO: 160); or
g. an antisense strand that consists of the nucleobase sequence differing by 0, 1, 2 or 3 nucleobases from the sequence (5'→3') UACCAAUUUAUGCCUACAGCC (SEQ ID NO: 162); or
h. an antisense strand that consists of the nucleobase sequence differing by 0, 1, 2 or 3 nucleobases from the sequence (5'→3') UACCAAUUUAUGCCUACAGCCUU (SEQ ID NO: 163); or
i. an antisense strand that consists of the nucleobase sequence differing by 0, 1, 2 or 3 nucleobases from the sequence (5'→3') UAUUGAGAGAAGUCCACCACGA (SEQ ID NO: 170); or
j. an antisense strand that consists of the nucleobase sequence differing by 0, 1, 2 or 3 nucleobases from the sequence (5'→3') AGAAAAUUGAGAGAAGUCCAC (SEQ ID NO: 171); or
k. an antisense strand that consists of the nucleobase sequence differing by 0, 1, 2 or 3 nucleobases from the sequence (5'→3') UACCAAUUUAUGCCUACAGCUU (SEQ ID NO: 172); or
l. an antisense strand that consists of the nucleobase sequence differing by 0, 1, 2 or 3 nucleobases from the sequence (5'→3') UACCAAUUUAUGCCUACAGCCU (SEQ ID NO: 173); or
m. an antisense strand that consists of the nucleobase sequence differing by 0, 1, 2 or 3 nucleobases from the sequence (5'→3') UAUUGAGAGAAGUCCACCAUU (SEQ ID NO: 174); or
n. an antisense strand that consists of the nucleobase sequence differing by 0, 1, 2 or 3 nucleobases from the sequence (5'→3') UAUUGAGAGAAGUCCACCACUU (SEQ ID NO: 175); or
o. an antisense strand that consists of the nucleobase sequence differing by 0, 1, 2 or 3 nucleobases from the sequence (5'→3') AGAAAAUUGAGAGAAGUCCUU (SEQ ID NO: 178); or
p. an antisense strand that consists of the nucleobase sequence differing by 0, 1, 2 or 3 nucleobases from the sequence (5'→3') AGAAAAUUGAGAGAAGUCCACUU (SEQ ID NO: 179); or
q. an antisense strand that consists of the nucleobase sequence differing by 0, 1, 2 or 3 nucleobases from the sequence (5'→3') AGAAAAUUGAGAGAAGUCCACC (SEQ ID NO: 180); or
r. an antisense strand that consists of the nucleobase sequence differing by 0, 1, 2 or 3 nucleobases from the sequence (5'→3') UGAAAAUUGAGAGAAGUCCAC (SEQ ID NO: 181); or
s. an antisense strand that consists of the nucleobase sequence differing by 0, 1, 2 or 3 nucleobases from the sequence (5'→3') ACCAAUUUAUGCCUACAGCUU (SEQ ID NO: 182); or
t. an antisense strand that consists of the nucleobase sequence differing by 0, 1, 2 or 3 nucleobases from the sequence (5'→3') ACCAAUUUAUGCCUACAGCCUU (SEQ ID NO: 183); or
u. an antisense strand that comprises the nucleobase sequence differing by 0, 1, 2 or 3 nucleobases from the sequence (5'→3') ACCAAUUUAUGCCUACAGCCUC (SEQ ID NO: 184); or
v. an antisense strand that consists of the nucleobase sequence differing by 0, 1, 2 or 3 nucleobases from the sequence (5'→3') UCCAAUUUAUGCCUACAGCUU (SEQ ID NO: 185); or
w. an antisense strand that consists of the nucleobase sequence differing by 0, 1, 2 or 3 nucleobases from the sequence (5'→3') UCCAAUUUAUGCCUACAGCCUU (SEQ ID NO: 186); or
x. an antisense strand that consists of the nucleobase sequence differing by 0, 1, 2 or 3 nucleobases from the sequence (5'→3') UACCAAUUUAUGCCUACAGCU (SEQ ID NO: 187); or
y. an antisense strand that comprises the nucleobase sequence differing by 0, 1, 2 or 3 nucleobases from the sequence (5'→3') UACCAAUUUAUGCCUACAGCG (SEQ ID NO: 188); or
z. an antisense strand that consists of the nucleobase sequence differing by 0, 1, 2 or 3 nucleobases from the sequence (5'→3') AACCAAUUUAUGCCUACAGCC (SEQ ID NO: 189); or
aa. an antisense strand that consists of the nucleobase sequence differing by 0, 1, 2 or 3 nucleobases from the sequence (5'→3') ACCAAUUUAUGCCUACAGCCU (SEQ ID NO: 190); or
bb. an antisense strand that consists of the nucleobase sequence differing by 0, 1, 2 or 3 nucleobases from the sequence (5'→3') UCCAAUUUAUGCCUACAGCCU (SEQ ID NO: 191); or
cc. an antisense strand that consists of the nucleobase sequence differing by 0, 1, 2 or 3 nucleobases from the sequence (5'→3') ACCAAUUUAUGCCUACAGCCG (SEQ ID NO: 192); or
dd. an antisense strand that comprises the nucleobase sequence differing by 0, 1, 2 or 3 nucleobases from the sequence (5'→3') UCCAAUUUAUGCCUACAGCCG (SEQ ID NO: 193); or.
ee. an antisense strand that consists of the nucleobase sequence differing by 0, 1, 2 or 3 nucleobases from the sequence (5'→3') UACCAAUUUAUGCCUACAGGG (SEQ ID NO: 194);
and wherein the HBV RNAi agent further comprises a sense strand at least partially complementary to the respective antisense strand.

In some embodiments, an HBV RNAi agent disclosed herein comprises:
i. an antisense strand that comprises the sequence differing by 0, 1, 2 or 3 nucleotides from the sequence (5'→3') usAfscCfaAfuUfuAfuGfcCfuAfcAfgGfccsusuAu (SEQ ID NO: 61); or
ii. an antisense strand that comprises the sequence differing by 0, 1, 2 or 3 nucleotides from the sequence (5'→3') usAfscCfaAfuUfuAfuGfcCfuAfcAfgGfcscsu (SEQ ID NO: 62); or
iii. an antisense strand that comprises the sequence differing by 0, 1, 2 or 3 nucleotides from the sequence (5'→3') usAfscsCfaAfuUfuAfuGfcCfuAfcAfgGfccsu (SEQ ID NO: 63); or
iv. an antisense strand that comprises the sequence differing by 0, 1, 2 or 3 nucleotides from the sequence (5'→3') usAfscsCfaAfuUfuAfuGfcCfuAfcAfgGfsc (SEQ ID NO: 64); or
v. an antisense strand that comprises the sequence differing by 0, 1, 2 or 3 nucleotides from the sequence (5'→3') usAfscsCfaAfuUfuAfuGfcCfuAfcAfgusu (SEQ ID NO: 68); or
vi. an antisense strand that comprises the sequence differing by 0, 1, 2 or 3 nucleotides from the sequence (5'→3') usAfscscaauUfuAfuGfcCfuacagcsc (SEQ ID NO: 85); or
vii. an antisense strand that comprises the sequence differing by 0, 1, 2 or 3 nucleotides from the sequence (5'→3') usAfsusugagAfgAfaGfuCfcaccacsg (SEQ ID NO: 94); or
viii. an antisense strand that comprises the sequence differing by 0, 1, 2 or 3 nucleotides from the sequence (5'→3') usAfsusUfgAfgAfgAfaGfuCfcAfcCfaCfgsa (SEQ ID NO: 98); or
ix. an antisense strand that comprises the sequence differing by 0, 1, 2 or 3 nucleotides from the sequence (5'→3') usAfscsCfaAfuuuauGfcCfuAfcAfgcsc (SEQ ID NO: 102); or
x. an antisense strand that comprises the sequence differing by 0, 1, 2 or 3 nucleotides from the sequence (5'→3') usAfscsCfaAfuuuauGfcCfuAfcAfgcusu (SEQ ID NO: 103); or
xi. an antisense strand that comprises the sequence differing by 0, 1, 2 or 3 nucleotides from the sequence (5'→3') usAfscsCfaAfuuuauGfcCfuAfcAfgccsu (SEQ ID NO: 104); or
xii. an antisense strand that comprises the sequence differing by 0, 1, 2 or 3 nucleotides from the sequence (5'→3') usAfscsCfaAfuuuauGfcCfuAfcAfgccusu (SEQ ID NO: 105); or
xiii. an antisense strand that comprises the sequence differing by 0, 1, 2 or 3 nucleotides from the sequence (5'→3') cPrpusAfscsCfaAfuUfuAfuGfcCfuAfcAfgusu (SEQ ID NO: 107); or
xiv. an antisense strand that comprises the sequence differing by 0, 1, 2 or 3 nucleotides from the sequence (5'→3') cPrpusAfsusUfgAfgAfgAfaGfuCfcAfcCfaCfsg (SEQ ID NO: 108); or
xv. an antisense strand that comprises the sequence differing by 0, 1, 2 or 3 nucleotides from the sequence (5'→3') usAfsusUfgAfgagaaGfuCfcAfcCfausu (SEQ ID NO: 109); or
xvi. an antisense strand that comprises the sequence differing by 0, 1, 2 or 3 nucleotides from the sequence (5'→3') usAfsusUfgAfgagaaGfuCfcAfcCfacsg (SEQ ID NO: 110); or
xvii. an antisense strand that comprises the sequence differing by 0, 1, 2 or 3 nucleotides from the sequence (5'→3') usAfsusUfgAfgagaaGfuCfcAfcCfacsusu (SEQ ID NO: 111); or
xviii. an antisense strand that comprises the sequence differing by 0, 1, 2 or 3 nucleotides from the sequence (5'→3') usAfsusUfgAfgagaaGfuCfcAfcCfacsgsa (SEQ ID NO: 112); or
xix. an antisense strand that comprises the sequence differing by 0, 1, 2 or 3 nucleotides from the sequence (5'→3') usAfsusUfgAfgagaaGfuCfcAfcCfacusu (SEQ ID NO: 120); or
xx. an antisense strand that comprises the sequence differing by 0, 1, 2 or 3 nucleotides from the sequence (5'→3') asGfsasAfaAfuUfgAfgAfgAfaGfuCfcusu (SEQ ID NO: 125);
xxi. an antisense strand that comprises the sequence differing by 0, 1, 2 or 3 nucleotides from the sequence (5'→3') asGfsasAfaAfuUfgAfgAfgAfaGfuCfcasc (SEQ ID NO: 126); or
xxii. an antisense strand that comprises the sequence differing by 0, 1, 2 or 3 nucleotides from the sequence (5'→3') asGfsasAfaAfuUfgAfgAfgAfaGfuCfcacusu (SEQ ID NO: 127); or
xxiii. an antisense strand that comprises the sequence differing by 0, 1, 2 or 3 nucleotides from the sequence (5'→3') asGfsasAfaAfuUfgAfgAfgAfaGfuCfcacsc (SEQ ID NO: 128); or
xxiv. an antisense strand that comprises the sequence differing by 0, 1, 2 or 3 nucleotides from the sequence (5'→3') usGfsasAfaAfuUfgAfgAfgAfaGfuCfcusu (SEQ ID NO: 129); or
xxv. an antisense strand that comprises the sequence differing by 0, 1, 2 or 3 nucleotides from the sequence (5'→3') usGfsasAfaAfuUfgAfgAfgAfaGfuCfcasc (SEQ ID NO: 130); or
xxvi. an antisense strand that comprises the sequence differing by 0, 1, 2 or 3 nucleotides from the sequence (5'→3') asCfscsAfaUfuUfaUfgCfcUfaCfaGfcusu (SEQ ID NO: 131); or
xxvii. an antisense strand that comprises the sequence differing by 0, 1, 2 or 3 nucleotides from the sequence (5'→3') asCfscsAfaUfuUfaUfgCfcUfaCfaGfccusu (SEQ ID NO: 132); or
xxviii. an antisense strand that comprises the sequence differing by 0, 1, 2 or 3 nucleotides from the sequence (5'→3') asCfscsAfaUfuUfaUfgCfcUfaCfaGfccusc (SEQ ID NO: 133); or
xxix. an antisense strand that comprises the sequence differing by 0, 1, 2 or 3 nucleotides from the sequence (5'→3') usCfscsAfaUfuUfaUfgCfcUfaCfaGfcusu (SEQ ID NO: 134); or
xxx. an antisense strand that comprises the sequence differing by 0, 1, 2 or 3 nucleotides from the sequence (5'→3') usCfscsAfaUfuUfaUfgCfcUfaCfaGfccusu (SEQ ID NO: 135); or
xxxi. an antisense strand that comprises the sequence differing by 0, 1, 2 or 3 nucleotides from the sequence (5'→3') cPrpusAfscsCfaAfuUfuAfuGfcCfuAfcAfgcsc (SEQ ID NO: 136); or
xxxii. an antisense strand that comprises the sequence differing by 0, 1, 2 or 3 nucleotides from the sequence (5'→3') usAfscsCfaAfuUfuAfuGfcCfuAfcAfgscsc (SEQ ID NO: 137); or
xxxiii. an antisense strand that comprises the sequence differing by 0, 1, 2 or 3 nucleotides from the sequence (5'→3') cPrpusAfscsCfaAfuUfuAfuGfcCfuAfcAfgscsc (SEQ ID NO: 138); or
xxxiv. an antisense strand that comprises the sequence differing by 0, 1, 2 or 3 nucleotides from the sequence (5'→3') usAfscsCfaAfuUfuAfuGfcCfuAfcAfgcsu (SEQ ID NO: 139); or
xxxv. an antisense strand that comprises the sequence differing by 0, 1, 2 or 3 nucleotides from the sequence (5'→3') usAfscsCfaAfuUfuAfuGfcCfuAfcAfgcsg (SEQ ID NO: 140); or
xxxvi. an antisense strand that comprises the sequence differing by 0, 1, 2 or 3 nucleotides from the sequence (5'→3') asAfscsCfaAfuUfuAfuGfcCfuAfcAfgcsc (SEQ ID NO: 141); or
xxxvii. an antisense strand that comprises the sequence differing by 0, 1, 2 or 3 nucleotides from the sequence (5'→3') usAfscsCfaAfuUfUfAfuGfcCfuAfcAfgusu (SEQ ID NO: 142); or
xxxviii. an antisense strand that comprises the sequence differing by 0, 1, 2 or 3 nucleotides from the sequence (5'→3') usAfscsCfaAfuUfuAfuGfcCfuAfcAfgCfsc (SEQ ID NO: 143); or
xxxix. an antisense strand that comprises the sequence differing by 0, 1, 2 or 3 nucleotides from the sequence (5'→3') asCfscAfaUfuUfaUfgCfcUfaCfaGfcCfsu (SEQ ID NO: 144); or
xl. an antisense strand that comprises the sequence differing by 0, 1, 2 or 3 nucleotides from the sequence (5'→3') usCfscAfaUfuUfaUfgCfcUfaCfaGfcCfsu (SEQ ID NO: 145); or
xli. an antisense strand that comprises the sequence differing by 0, 1, 2 or 3 nucleotides from the sequence (5'→3') asCfscAfaUfuUfaUfgCfcUfaCfaGfccsg (SEQ ID NO: 146); or
xlii. an antisense strand that comprises the sequence differing by 0, 1, 2 or 3 nucleotides from the sequence (5'→3') usCfscAfaUfuUfaUfgCfcUfaCfaGfccsg (SEQ ID NO: 147); or
xliii. an antisense strand that comprises the sequence differing by 0, 1, 2 or 3 nucleotides from the sequence (5'→3') usAfscsCfaAfuUfuAfuGfcCfuAfcAfggsg (SEQ ID NO: 148);
wherein a, g, c and u are 2'-O-methyl (2'-OMe) modified nucleotides; Af, Cf, Gf, and Uf are 2'-fluoro modified nucleotides; s is a phosphorothioate internucleoside linkage and the remaining nucleotide monomers are linked by phosphodiester bonds; and cPrpu is 5'-cyclopropyl phosphonate-2'-O-methyl modified nucleotide; and wherein the HBV RNAi agent further comprises a sense strand at least partially complementary to the respective antisense strand.

In some embodiments, an HBV RNAi agent disclosed herein comprises:
i. an antisense strand that consists of the sequence (5'→3') usAfscCfaAfuUfuAfuGfcCfuAfcAfgGfccsusuAu (SEQ ID NO: 61); or
ii. an antisense strand that consists of the sequence (5'→3') usAfscCfaAfuUfuAfuGfcCfuAfcAfgGfcscsu (SEQ ID NO: 62); or
iii. an antisense strand that consists of the sequence (5'→3') usAfscsCfaAfuUfuAfuGfcCfuAfcAfgGfccsu (SEQ ID NO: 63); or
iv. an antisense strand that consists of the sequence (5'→3') usAfscsCfaAfuUfuAfuGfcCfuAfcAfgGfsc (SEQ ID NO: 64); or
v. an antisense strand that consists of the sequence (5'→3') usAfscsCfaAfuUfuAfuGfcCfuAfcAfgusu (SEQ ID NO: 68); or
vi. an antisense strand that consists of the sequence (5'→3') usAfscscaauUfuAfuGfcCfuacagcsc (SEQ ID NO: 85); or
vii. an antisense strand that consists of the sequence (5'→3') usAfsusugagAfgAfaGfuCfcaccacsg (SEQ ID NO: 94); or
viii. an antisense strand that consists of the sequence (5'→3') usAfsusUfgAfgAfgAfaGfuCfcAfcCfaCfgsa (SEQ ID NO: 98); or
ix. an antisense strand that consists of the sequence (5'→3') usAfscsCfaAfuuuauGfcCfuAfcAfgcsc (SEQ ID NO: 102); or
x. an antisense strand that consists of the sequence (5'→3') usAfscsCfaAfuuuauGfcCfuAfcAfgcusu (SEQ ID NO: 103); or
xi. an antisense strand that consists of the sequence (5'→3') usAfscsCfaAfuuuauGfcCfuAfcAfgccsu (SEQ ID NO: 104); or
xii. an antisense strand that consists of the sequence (5'→3') usAfscsCfaAfuuuauGfcCfuAfcAfgccusu (SEQ ID NO: 105); or
xiii. an antisense strand that consists of the sequence (5'→3') cPrpusAfscsCfaAfuUfuAfuGfcCfuAfcAfgusu (SEQ ID NO: 107); or
xiv. an antisense strand that consists of the sequence (5'→3') cPrpusAfsusUfgAfgAfgAfaGfuCfcAfcCfaCfsg (SEQ ID NO: 108); or
xv. an antisense strand that consists of the sequence (5'→3') usAfsusUfgAfgagaaGfuCfcAfcCfausu (SEQ ID NO: 109); or
xvi. an antisense strand that consists of the sequence (5'→3') usAfsusUfgAfgagaaGfuCfcAfcCfacsg (SEQ ID NO: 110); or
xvii. an antisense strand that consists of the sequence (5'→3') usAfsusUfgAfgagaaGfuCfcAfcCfacsusu (SEQ ID NO: 111); or
xviii. an antisense strand that consists of the sequence (5'→3') usAfsusUfgAfgagaaGfuCfcAfcCfacsgsa (SEQ ID NO: 112); or
xix. an antisense strand that consists of the sequence (5'→3') usAfsusUfgAfgagaaGfuCfcAfcCfacusu (SEQ ID NO: 120); or
xx. an antisense strand that consists of the sequence (5'→3') asGfsasAfaAfuUfgAfgAfgAfaGfuCfcusu (SEQ ID NO: 125);
xxi. an antisense strand that consists of the sequence (5'→3') asGfsasAfaAfuUfgAfgAfgAfaGfuCfcasc (SEQ ID NO: 126); or
xxii. an antisense strand that consists of the sequence (5'→3') asGfsasAfaAfuUfgAfgAfgAfaGfuCfcacusu (SEQ ID NO: 127); or
xxiii. an antisense strand that consists of the sequence (5'→3') asGfsasAfaAfuUfgAfgAfgAfaGfuCfcacsc (SEQ ID NO: 128); or
xxiv. an antisense strand that consists of the sequence (5'→3') usGfsasAfaAfuUfgAfgAfgAfaGfuCfcusu (SEQ ID NO: 129); or
xxv. an antisense strand that consists of the sequence (5'→3') usGfsasAfaAfuUfgAfgAfgAfaGfuCfcasc (SEQ ID NO: 130); or
xxvi. an antisense strand that consists of the sequence (5'→3') asCfscsAfaUfuUfaUfgCfcUfaCfaGfcusu (SEQ ID NO: 131); or
xxvii. an antisense strand that consists of the sequence (5'→3') asCfscsAfaUfuUfaUfgCfcUfaCfaGfccusu (SEQ ID NO: 132); or
xxviii. an antisense strand that consists of the sequence (5'→3') asCfscsAfaUfuUfaUfgCfcUfaCfaGfccusc (SEQ ID NO: 133); or
xxix. an antisense strand that consists of the sequence (5'→3') usCfscsAfaUfuUfaUfgCfcUfaCfaGfcusu (SEQ ID NO: 134); or
xxx. an antisense strand that consists of the sequence (5'→3') usCfscsAfaUfuUfaUfgCfcUfaCfaGfccusu (SEQ ID NO: 135); or
xxxi. an antisense strand that consists of the sequence (5'→3') cPrpusAfscsCfaAfuUfuAfuGfcCfuAfcAfgcsc (SEQ ID NO: 136); or
xxxii. an antisense strand that consists of the sequence (5'→3') usAfscsCfaAfuUfuAfuGfcCfuAfcAfgscsc (SEQ ID NO: 137); or
xxxiii. an antisense strand that consists of the sequence (5'→3') cPrpusAfscsCfaAfuUfuAfuGfcCfuAfcAfgscsc (SEQ ID NO: 138); or
xxxiv. an antisense strand that consists of the sequence (5'→3') usAfscsCfaAfuUfuAfuGfcCfuAfcAfgcsu (SEQ ID NO: 139); or
xxxv. an antisense strand that consists of the sequence (5'→3') usAfscsCfaAfuUfuAfuGfcCfuAfcAfgcsg (SEQ ID NO: 140); or
xxxvi. an antisense strand that consists of the sequence (5'→3') asAfscsCfaAfuUfuAfuGfcCfuAfcAfgcsc (SEQ ID NO: 141); or
xxxvii. an antisense strand that consists of the sequence (5'→3') usAfscsCfaAfuUfUfAfuGfcCfuAfcAfgusu (SEQ ID NO: 142); or
xxxviii. an antisense strand that consists of the sequence (5'→3') usAfscsCfaAfuUfuAfuGfcCfuAfcAfgCfsc (SEQ ID NO: 143); or
xxxix. an antisense strand that consists of the sequence (5'→3') asCfscAfaUfuUfaUfgCfcUfaCfaGfcCfsu (SEQ ID NO: 144); or
xl. an antisense strand that consists of the sequence (5'→3') usCfscAfaUfuUfaUfgCfcUfaCfaGfcCfsu (SEQ ID NO: 145); or
xli. an antisense strand that consists of the sequence (5'→3') asCfscAfaUfuUfaUfgCfcUfaCfaGfccsg (SEQ ID NO: 146); or
xlii. an antisense strand that consists of the sequence (5'→3') usCfscAfaUfuUfaUfgCfcUfaCfaGfccsg (SEQ ID NO: 147); or
xliii. an antisense strand that consists of the sequence (5'→3') usAfscsCfaAfuUfuAfuGfcCfuAfcAfggsg (SEQ ID NO: 148);
wherein a, g, c and u are 2'-O-methyl (2'-OMe) modified nucleotides; Af, Cf, Gf, and Uf are 2'-fluoro modified nucleotides; s is a phosphorothioate internucleoside linkage and the remaining nucleotide monomers are linked by phosphodiester bonds; and cPrpu is 5'-cyclopropyl phosphonate-2'-O-methyl modified nucleotide; and wherein the HBV RNAi agent further comprises a sense strand at least partially complementary to the respective antisense strand.

In some embodiments, an HBV RNAi agent disclosed herein comprises:
a. a sense strand that comprises the nucleobase sequence differing by 0, 1, 2 or 3 nucleobases from the sequence (5'→3') UUGCCUGUAGGCAUAAAUUGGUAUT (SEQ ID NO: 275); or
b. a sense strand that comprises the nucleobase sequence differing by 0, 1, 2 or 3 nucleobases from the sequence (5'→3') UAUAUGCCUGUAGGCAUAAAUUGGUA (SEQ ID NO: 276); or
c. a sense strand that comprises the nucleobase sequence differing by 0, 1, 2 or 3 nucleobases from the sequence (5'→3') CUGUAGGCAUAAAUUGGUAUU (SEQ ID NO: 278); or
d. a sense strand that comprises the nucleobase sequence differing by 0, 1, 2 or 3 nucleobases from the sequence (5'→3') CGUGGUGGACUUCUCUCAAUU (SEQ ID NO: 285); or
e. a sense strand that comprises the nucleobase sequence differing by 0, 1, 2 or 3 nucleobases from the sequence (5'→3') CGUGGUGGACUUCUCUCAAUA (SEQ ID NO: 289); or
f. a sense strand that comprises the nucleobase sequence differing by 0, 1, 2 or 3 nucleobases from the sequence (5'→3') CUGUAGGCAUAAAUUGGUA (SEQ ID NO: 292); or
g. a sense strand that comprises the nucleobase sequence differing by 0, 1, 2 or 3 nucleobases from the sequence (5'→3') GGCUGUAGGCAUAAAUUGGUA (SEQ ID NO: 294); or
h. a sense strand that comprises the nucleobase sequence differing by 0, 1, 2 or 3 nucleobases from the sequence (5'→3') UCGUGGUGGACUUCUCUCAAUU (SEQ ID NO: 300); or
i. a sense strand that comprises the nucleobase sequence differing by 0, 1, 2 or 3 nucleobases from the sequence (5'→3') GUGGACUUCUCUCAAUUUUCU (SEQ ID NO: 302); or
j. a sense strand that comprises the nucleobase sequence differing by 0, 1, 2 or 3 nucleobases from the sequence (5'→3') GCUGUAGGCAUAAAUUGGUAUU (SEQ ID NO: 303); or
k. a sense strand that comprises the nucleobase sequence differing by 0, 1, 2 or 3 nucleobases from the sequence (5'→3') GGCUGUAGGCAUAAAUUGGUAUU (SEQ ID NO: 304); or
l. a sense strand that comprises the nucleobase sequence differing by 0, 1, 2 or 3 nucleobases from the sequence (5'→3') UGGUGGACUUCUCUCAAUAUU (SEQ ID NO: 306); or
m. a sense strand that comprises the nucleobase sequence differing by 0, 1, 2 or 3 nucleobases from the sequence (5'→3') GUGGUGGACUUCUCUCAAUAUU (SEQ ID NO: 307); or
n. a sense strand that comprises the nucleobase sequence differing by 0, 1, 2 or 3 nucleobases from the sequence (5'→3') AAUGGUGGACUUCUCUCAAUAUU (SEQ ID NO: 308); or
o. a sense strand that comprises the nucleobase sequence differing by 0, 1, 2 or 3 nucleobases from the sequence (5'→3') GGACUUCUCUCAAUUUUCU (SEQ ID NO: 318); or
p. a sense strand that comprises the nucleobase sequence differing by 0, 1, 2 or 3 nucleobases from the sequence (5'→3') GGUGGACUUCUCUCAAUUUUCU (SEQ ID NO: 319); or
q. a sense strand that comprises the nucleobase sequence differing by 0, 1, 2 or 3 nucleobases from the sequence (5'→3') GGACUUCUCUCAAUUUUCA (SEQ ID NO: 320); or
r. a sense strand that comprises the nucleobase sequence differing by 0, 1, 2 or 3 nucleobases from the sequence (5'→3') GUGGACUUCUCUCAAUUUUCA (SEQ ID NO: 321); or
s. a sense strand that comprises the nucleobase sequence differing by 0, 1, 2 or 3 nucleobases from the sequence (5'→3') GCUGUAGGCAUAAAUUGGU (SEQ ID NO: 322); or
t. a sense strand that comprises the nucleobase sequence differing by 0, 1, 2 or 3 nucleobases from the sequence (5'→3') GGCUGUAGGCAUAAAUUGGU (SEQ ID NO: 323); or
u. a sense strand that comprises the nucleobase sequence differing by 0, 1, 2 or 3 nucleobases from the sequence (5'→3') GAGGCUGUAGGCAUAAAUUGGU (SEQ ID NO: 324); or
v. a sense strand that comprises the nucleobase sequence differing by 0, 1, 2 or 3 nucleobases from the sequence (5'→3') GCUGUAGGCAUAAAUUGGA (SEQ ID NO: 325); or
w. a sense strand that comprises the nucleobase sequence differing by 0, 1, 2 or 3 nucleobases from the sequence (5'→3') GGCUGUAGGCAUAAAUUGGA (SEQ ID NO: 326); or
x. a sense strand that comprises the nucleobase sequence differing by 0, 1, 2 or 3 nucleobases from the sequence (5'→3') AGCUGUAGGCAUAAAUUGGUA (SEQ ID NO: 327); or
y. a sense strand that comprises the nucleobase sequence differing by 0, 1, 2 or 3 nucleobases from the sequence (5'→3') CGCUGUAGGCAUAAAUUGGUA (SEQ ID NO: 328); or
z. a sense strand that comprises the nucleobase sequence differing by 0, 1, 2 or 3 nucleobases from the sequence (5'→3') GGCUGUAGGCAUAAAUUGGUU (SEQ ID NO: 329); or
aa. an antisense strand that comprises the nucleobase sequence differing by 0, 1, 2 or 3 nucleobases from the sequence (5'→3') AGGCUGUAGGCAUAAAUUGGU (SEQ ID NO: 330); or
bb. a sense strand that comprises the nucleobase sequence differing by 0, 1, 2 or 3 nucleobases from the sequence (5'→3') AGGCUGUAGGCAUAAAUUGGA (SEQ ID NO: 331); or
cc. a sense strand that comprises the nucleobase sequence differing by 0, 1, 2 or 3 nucleobases from the sequence (5'→3') CGGCUGUAGGCAUAAAUUGGU (SEQ ID NO: 332); or
dd. a sense strand that comprises the nucleobase sequence differing by 0, 1, 2 or 3 nucleobases from the sequence (5'→3') CGGCUGUAGGCAUAAAUUGGA (SEQ ID NO: 333); or
ee. a sense strand that comprises the nucleobase sequence differing by 0, 1, 2 or 3 nucleobases from the sequence (5'→3') CCCUGUAGGCAUAAAUUGGUA (SEQ ID NO: 334);
and wherein the HBV RNAi agent further comprises an antisense strand at least partially complementary to the respective antisense strand.

In some embodiments, an HBV RNAi agent disclosed herein comprises:
a. a sense strand that consists of the nucleobase sequence (5'→3') UUGCCUGUAGGCAUAAAUUGGUAUT (SEQ ID NO: 275); or
b. a sense strand that consists of the nucleobase sequence (5'→3') UAUAUGCCUGUAGGCAUAAAUUGGUA (SEQ ID NO: 276); or
c. a sense strand that consists of the nucleobase sequence (5'→3') CUGUAGGCAUAAAUUGGUAUU (SEQ ID NO: 278); or
d. a sense strand that consists of the nucleobase sequence (5'→3') CGUGGUGGACUUCUCUCAAUU (SEQ ID NO: 285); or
e. a sense strand that consists of the nucleobase sequence (5'→3') CGUGGUGGACUUCUCUCAAUA (SEQ ID NO: 289); or
f. a sense strand that consists of the nucleobase sequence (5'→3') CUGUAGGCAUAAAUUGGUA (SEQ ID NO: 292); or
g. a sense strand that consists of the nucleobase sequence (5'→3') GGCUGUAGGCAUAAAUUGGUA (SEQ ID NO: 294); or
h. a sense strand that consists of the nucleobase sequence (5'→3') UCGUGGUGGACUUCUCUCAAUU (SEQ ID NO: 300); or
i. a sense strand that consists of the nucleobase sequence (5'→3') GUGGACUUCUCUCAAUUUUCU (SEQ ID NO: 302); or
j. a sense strand that consists of the nucleobase sequence (5'→3') GCUGUAGGCAUAAAUUGGUAUU (SEQ ID NO: 303); or
k. a sense strand that consists of the nucleobase sequence (5'→3') GGCUGUAGGCAUAAAUUGGUAUU (SEQ ID NO: 304); or
1. a sense strand that consists of the nucleobase sequence (5'→3') UGGUGGACUUCUCUCAAUAUU (SEQ ID NO: 306); or
m. a sense strand that consists of the nucleobase sequence (5'→3') GUGGUGGACUUCUCUCAAUAUU (SEQ ID NO: 307); or
n. a sense strand that consists of the nucleobase sequence (5'→3') AAUGGUGGACUUCUCUCAAUAUU (SEQ ID NO: 308); or
o. a sense strand that comprises the nucleobase sequence (5'→3') GGACUUCUCUCAAUUUUCU (SEQ ID NO: 318); or
p. a sense strand that consists of the nucleobase sequence (5'→3') GGUGGACUUCUCUCAAUUUUCU (SEQ ID NO: 319); or
q. a sense strand that consists of the nucleobase sequence (5'→3') GGACUUCUCUCAAUUUUCA (SEQ ID NO: 320); or
r. a sense strand that consists of the nucleobase sequence (5'→3') GUGGACUUCUCUCAAUUUUCA (SEQ ID NO: 321); or
s. a sense strand that consists of the nucleobase sequence (5'→3') GCUGUAGGCAUAAAUUGGU (SEQ ID NO: 322); or
t. a sense strand that consists of the nucleobase sequence (5'→3') GGCUGUAGGCAUAAAUUGGU (SEQ ID NO: 323); or
u. a sense strand that consists of the nucleobase sequence (5'→3') GAGGCUGUAGGCAUAAAUUGGU (SEQ ID NO: 324); or
v. a sense strand that consists of the nucleobase sequence (5'→3') GCUGUAGGCAUAAAUUGGA (SEQ ID NO: 325); or
w. a sense strand that consists of the nucleobase sequence (5'→3') GGCUGUAGGCAUAAAUUGGA (SEQ ID NO: 326); or
x. a sense strand that consists of the nucleobase sequence (5'→3') AGCUGUAGGCAUAAAUUGGUA (SEQ ID NO: 327); or
y. a sense strand that consists of the nucleobase sequence (5'→3') CGCUGUAGGCAUAAAUUGGUA (SEQ ID NO: 328); or
z. a sense strand that consists of the nucleobase sequence (5'→3') GGCUGUAGGCAUAAAUUGGUU (SEQ ID NO: 329); or
aa. an antisense strand that comprises the nucleobase sequence (5'→3') AGGCUGUAGGCAUAAAUUGGU (SEQ ID NO: 330); or
bb. a sense strand that consists of the nucleobase sequence (5'→3') AGGCUGUAGGCAUAAAUUGGA (SEQ ID NO: 331); or
cc. a sense strand that consists of the nucleobase sequence (5'→3') CGGCUGUAGGCAUAAAUUGGU (SEQ ID NO: 332); or
dd. a sense strand that consists of the nucleobase sequence (5'→3') CGGCUGUAGGCAUAAAUUGGA (SEQ ID NO: 333); or
ee. a sense strand that consists of the nucleobase sequence (5'→3') CCCUGUAGGCAUAAAUUGGUA (SEQ ID NO: 334);
and wherein the HBV RNAi agent further comprises an antisense strand at least partially complementary to the respective antisense strand.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein a second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein a second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292).

In some embodiments, disclosed herein are methods of treating a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein a second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein a first HBV RNAi agent comprises an antisense strand that consists of the nucleobase sequence (5'→3') UAUUGAGAGAAGUCCACCACUU (SEQ ID NO: 175), and a sense strand that consists of the nucleobase sequence (5'→3') GUGGUGGACUUCUCUCAAUAUU (SEQ ID NO: 307); and wherein a second HBV RNAi agent comprises an antisense strand that consists of the nucleobase sequence (5'→3') UACCAAUUUAUGCCUACAGUU (SEQ ID NO: 154), and a sense strand that consists of the nucleobase sequence (5'→3') CUGUAGGCAUAAAUUGGUA (SEQ ID NO: 292).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein a first HBV RNAi agent comprises an antisense strand that consists of the nucleobase sequence (5'→3') UAUUGAGAGAAGUCCACCACUU (SEQ ID NO: 175), and a sense strand that consists of the nucleobase sequence (5'→3') GUGGUGGACUUCUCUCAAUAUU (SEQ ID NO: 307); and wherein a second HBV RNAi agent comprises an antisense strand that consists of the nucleobase sequence (5'→3') UACCAAUUUAUGCCUACAGUU (SEQ ID NO: 154), and a sense strand that consists of the nucleobase sequence (5'→3') CUGUAGGCAUAAAUUGGUA (SEQ ID NO: 292).

In some embodiments, disclosed herein are methods of treating a disease associated with an infection caused by HBV, comprising administering a composition that inhibits expression of an HBV gene in a cell, wherein the composition comprises two HBV RNAi agents, wherein a first HBV RNAi agent comprises an antisense strand that consists of the nucleobase sequence (5'→3') UAUUGAGAGAAGUCCACCACUU (SEQ ID NO: 175), and a sense strand that consists of the nucleobase sequence (5'→3') GUGGUGGACUUCUCUCAAUAUU (SEQ ID NO: 307); and wherein a second HBV RNAi agent comprises an antisense strand that consists of the nucleobase sequence (5'→3') UACCAAUUUAUGCCUACAGUU (SEQ ID NO: 154), and a sense strand that consists of the nucleobase sequence (5'→3') CUGUAGGCAUAAAUUGGUA (SEQ ID NO: 292).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328).

In some embodiments, disclosed herein are methods of treating a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328).

In some embodiments, disclosed herein are methods of treating or preventing an HBV-associated disease or symptoms in a subject or inhibiting expression of one or more HBV genes comprising administering a compositions for inhibiting expression of an HBV gene in a cell, wherein the composition comprises two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCG (SEQ ID NO: 188), and a sense strand that consists of the nucleobase sequence (5'→3') CGCUGUAGGCAUAAAUUGGUA (SEQ ID NO: 328).

In some embodiments, disclosed herein are methods of treating or preventing a disease associated with an infection caused by HBV, comprising administering a compositions for inhibiting expression of an HBV gene in a cell, wherein the composition comprises two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCG (SEQ ID NO: 188), and a sense strand that consists of the nucleobase sequence (5'→3') CGCUGUAGGCAUAAAUUGGUA (SEQ ID NO: 328).

In some embodiments, disclosed herein are methods of treating a disease associated with an infection caused by HBV, comprising administering a compositions for inhibiting expression of an HBV gene in a cell, wherein the composition comprises two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCG (SEQ ID NO: 188), and a sense strand that consists of the nucleobase sequence (5'→3') CGCUGUAGGCAUAAAUUGGUA (SEQ ID NO: 328).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294).

In some embodiments, disclosed herein are methods of treating a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294).

In some embodiments, disclosed herein are methods of treating or preventing an HBV-associated disease or symptoms in a subject or inhibiting expression of one or more HBV genes comprising administering a composition that inhibits expression of an HBV gene in a cell, the composition comprising two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCC (SEQ ID NO: 162), and a sense strand that consists of the nucleobase sequence (5'→3') GGCUGUAGGCAUAAAUUGGUA (SEQ ID NO: 294).

In some embodiments, disclosed herein are methods of treating or preventing a disease associated with an infection caused by HBV, comprising administering a composition that inhibits expression of an HBV gene in a cell, the composition comprising two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCC (SEQ ID NO: 162), and a sense strand that consists of the nucleobase sequence (5'→3') GGCUGUAGGCAUAAAUUGGUA (SEQ ID NO: 294).

In some embodiments, disclosed herein are methods of treating a disease associated with an infection caused by HBV, comprising administering a composition that inhibits expression of an HBV gene in a cell, the composition comprising two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCC (SEQ ID NO: 162), and a sense strand that consists of the nucleobase sequence (5'→3') GGCUGUAGGCAUAAAUUGGUA (SEQ ID NO: 294).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307).

In some embodiments, disclosed herein are methods of treating a disease associated with an infection caused by HBV, comprising administering a composition that inhibits expression of an HBV gene in a cell, wherein the composition comprises two HBV RNAi agents, wherein a first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein a second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCC (SEQ ID NO: 162), and a sense strand that consists of the nucleobase sequence (5'→3') GUGGUGGACUUCUCUCAAUAUU (SEQ ID NO: 307).In some embodiments, disclosed herein are methods of treating or preventing a disease associated with an infection caused by HBV, comprising administering a composition that inhibits expression of an HBV gene in a cell, wherein the composition comprises two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCC (SEQ ID NO: 162), and a sense strand that consists of the nucleobase sequence (5'→3') GUGGUGGACUUCUCUCAAUAUU (SEQ ID NO: 307).

In some embodiments, disclosed herein are methods of treating a disease associated with an infection caused by HBV, comprising administering a composition that inhibits expression of an HBV gene in a cell, wherein the composition comprises two HBV RNAi agents, wherein a first HBV RNAi agent comprises an antisense strand that consists of the nucleobase sequence (5'→3') AGAAAAUUGAGAGAAGUCCAC (SEQ ID NO: 171), and a sense strand that consists of the nucleobase sequence (5'→3') GUGGACUUCUCUCAAUUUUCU (SEQ ID NO: 302); and wherein a second HBV RNAi agent comprises an antisense strand that consists of the nucleobase sequence (5'→3') UACCAAUUUAUGCCUACAGCC (SEQ ID NO: 162), and a sense strand that consists of the nucleobase sequence (5'→3') GUGGUGGACUUCUCUCAAUAUU (SEQ ID NO: 307).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein the second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein the second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292).

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein the second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328).

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294).

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307).

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307).

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307).

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein the second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292), and wherein the sense strand of the first HBV RNAi agent and the second HBV RNAi agent are conjugated to a targeting ligand comprising N-acetyl-galactosamine.

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein the second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292), and wherein the sense strand of the first HBV RNAi agent and the second HBV RNAi agent are conjugated to a targeting ligand comprising N-acetyl-galactosamine.

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') UAUUGAGAGAAGUCCACCACUU (SEQ ID NO: 175), and a sense strand that comprises the nucleobase sequence differing by 0, 1, 2 or 3 nucleobases from the sequence (5'→3') GUGGUGGACUUCUCUCAAUAUU (SEQ ID NO: 307); and wherein the second HBV RNAi agent comprises an antisense strand that comprises the nucleobase sequence differing by 0, 1, 2 or 3 nucleobases from the sequence (5'→3') UACCAAUUUAUGCCUACAGUU (SEQ ID NO: 154), and a sense strand that comprises the nucleobase sequence differing by 0, 1, 2 or 3 nucleobases from the sequence (5'→3') CUGUAGGCAUAAAUUGGUA (SEQ ID NO: 292), and wherein the sense strand of the first HBV RNAi agent and the second HBV RNAi agent are conjugated to a targeting ligand comprising N-acetyl-galactosamine.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328), and wherein the sense strand of the first HBV RNAi agent and the second HBV RNAi agent are conjugated to a targeting ligand comprising N-acetyl-galactosamine.

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328), and wherein the sense strand of the first HBV RNAi agent and the second HBV RNAi agent are conjugated to a targeting ligand comprising N-acetyl-galactosamine.

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCG (SEQ ID NO: 188), and a sense strand that comprises the nucleobase sequence differing by 0, 1, 2 or 3 nucleobases from the sequence (5'→3') CGCUGUAGGCAUAAAUUGGUA (SEQ ID NO: 328), and wherein the sense strand of the first HBV RNAi agent and the second HBV RNAi agent are conjugated to a targeting ligand comprising N-acetyl-galactosamine.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294), and wherein the sense strand of the first HBV RNAi agent and the second HBV RNAi agent are conjugated to a targeting ligand comprising N-acetyl-galactosamine.

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294), and wherein the sense strand of the first HBV RNAi agent and the second HBV RNAi agent are conjugated to a targeting ligand comprising N-acetyl-galactosamine.

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GGCUGUAGGCAUAAAUUGGUA (SEQ ID NO: 294), and wherein the sense strand of the first HBV RNAi agent and the second HBV RNAi agent are conjugated to a targeting ligand comprising N-acetyl-galactosamine.

In some embodiments, disclosed herein are methods of treating or preventing an HBV-associated disease or symptoms in a subject or inhibiting expression of one or more HBV genes comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307), and wherein the sense strand of the first HBV RNAi agent and the second HBV RNAi agent are conjugated to a targeting ligand comprising N-acetyl-galactosamine.

In some embodiments, disclosed herein are methods of treating or preventing a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307), and wherein the sense strand of the first HBV RNAi agent and the second HBV RNAi agent are conjugated to a targeting ligand comprising N-acetyl-galactosamine.

In some embodiments, disclosed herein are methods of treating a disease associated with an infection caused by HBV, comprising administering a composition that inhibits expression of an HBV gene in a cell, wherein the composition comprises two HBV RNAi agents, wherein all or substantially all of the nucleotides in the sense strand are modified and/or all or substantially all of the nucleotides in the antisense strand in the first and/or second HBV RNAi agent are modified nucleotides, and wherein the first HBV RNAi agent comprises an antisense strand that comprises the nucleobase sequence differing by 0, 1, 2 or 3 nucleobases from the sequence (5'→3') AGAAAAUUGAGAGAAGUCCAC (SEQ ID NO: 171), and a sense strand that comprises the nucleobase sequence differing by 0, 1, 2 or 3 nucleobases from the sequence (5'→3') GUGGACUUCUCUCAAUUUUCU (SEQ ID NO: 302); and wherein the second HBV RNAi agent comprises an antisense strand that comprises the nucleobase sequence differing by 0, 1, 2 or 3 nucleobases from the sequence (5'→3') UACCAAUUUAUGCCUACAGCC (SEQ ID NO: 162), and a sense strand that comprises the nucleobase sequence differing by 0, 1, 2 or 3 nucleobases from the sequence (5'→3') GUGGUGGACUUCUCUCAAUAUU (SEQ ID NO: 307), and wherein the sense strand of the first HBV RNAi agent and the second HBV RNAi agent are conjugated to a targeting ligand comprising N-acetyl-galactosamine.

In some embodiments, disclosed herein are methods of treatment of an HBV infection or prevention of disease or symptoms caused by an HBV infection comprising administering to a subject in need thereof an effective amount of AD04872 and an effective amount of AD05070. In some embodiments, the ratio of AD04872 to AD05070 administered to a subject in need thereof is about 2:1. In some embodiments, the ratio of AD04872 to AD05070 administered to a subject in need thereof is about 3:1. In some embodiments, the ratio of AD04872 to AD05070 administered to a subject in need thereof is about 1:1. In some embodiments, the ratio of AD04872 to AD05070 administered to a subject in need thereof is about 4:1. In some embodiments, the ratio of AD04872 to AD05070 administered to a subject in need thereof is about 5:1. In some embodiments, the ratio of AD04872 to AD05070 administered to a subject in need thereof is about 1:2.

In some embodiments, disclosed herein are methods of treating or preventing a disease associated with an infection caused by HBV, comprising administering to a subject in need thereof an effective amount of AD04872 and an effective amount of AD05070. In some embodiments, the ratio of AD04872 to AD05070 administered to a subject in need thereof is about 2:1. In some embodiments, the ratio of AD04872 to AD05070 administered to a subject in need thereof is about 3:1. In some embodiments, the ratio of AD04872 to AD05070 administered to a subject in need thereof is about 1:1. In some embodiments, the ratio of AD04872 to AD05070 administered to a subject in need thereof is about 4:1. In some embodiments, the ratio of AD04872 to AD05070 administered to a subject in need thereof is about 5:1. In some embodiments, the ratio of AD04872 to AD05070 administered to a subject in need thereof is about 1:2.

In some embodiments, disclosed herein are methods of treating a disease associated with an infection caused by HBV, comprising administering to a subject in need thereof an effective amount of AD04872 and an effective amount of AD05070. In some embodiments, the ratio of AD04872 to AD05070 administered to a subject in need thereof is about 2:1. In some embodiments, the ratio of AD04872 to AD05070 administered to a subject in need thereof is about 3:1. In some embodiments, the ratio of AD04872 to AD05070 administered to a subject in need thereof is about 1:1. In some embodiments, the ratio of AD04872 to AD05070 administered to a subject in need thereof is about 4:1. In some embodiments, the ratio of AD04872 to AD05070 administered to a subject in need thereof is about 5:1. In some embodiments, the ratio of AD04872 to AD05070 administered to a subject in need thereof is about 1:2.

In some embodiments, AD04872 and AD05070 are administered in a combined amount of about 25-400 mg per dose administration. In some embodiments, AD04872 and AD05070 are administered in a combined amount of about 25-50 mg, 50-75 mg, 75-100 mg, 100-125 mg, 125-150 mg, 150-175 mg, 175-200 mg, 200-225 mg, 225-250 mg, 250-275 mg, 275-300 mg, 300-325 mg, 325-350 mg, 350-375 mg, 375-400 mg, 25-75 mg, 50-100 mg, 100-150 mg, 150-200 mg, 200-250 mg, 250-300 mg, 300-350 mg, 350-400 mg, 25-100 mg, 50-150 mg, 100-200 mg, 150-250 mg, 200-300 mg, 300-400 mg, 25-200 mg, or 200-400 mg per dose administration. In some embodiments, AD04872 and AD05070 are administered in a combined amount of about 30-50 mg, about 90-110 mg, or about 190-210 mg per dose administration. In some embodiments, AD04872 and AD05070 are administered in a combined amount of about 30-50 mg per dose administration. In some embodiments, AD04872 and AD05070 are administered in a combined amount of about 90-110 mg per dose administration. In some embodiments, AD04872 and AD05070 are administered in a combined amount of about 190-210 mg per dose administration. In some embodiments, AD04872 to AD05070 are administered in a combined amount of about 25 mg, about 50 mg, about 100 mg, about 125 mg, about 150 mg, about 175 mg, about 200 mg, about 225 mg, about 250 mg, about 275 mg, about 300 mg, about 325 mg, about 350 mg, about 375 mg, or about 400 mg per dose administration. In some embodiments, AD04872 and AD05070 are administered in a combined amount of about 50 mg, about 75 mg, about 100 mg, or about 125 mg per dose administration. In some embodiments, AD04872 and AD05070 are administered in a combined amount of about 35 mg, about 50 mg, about 100 mg, or about 125 mg per dose administration. In some embodiments, AD04872 and AD05070 are administered in a combined amount of about 40 mg, about 100 mg, or about 200 mg per dose administration. In some embodiments, AD04872 and AD05070 are administered in a combined amount of about 40 mg per dose administration. In some embodiments, AD04872 and AD05070 are administered in a combined amount of about 100 mg per dose administration. In some embodiments, AD04872 and AD05070 are administered in a combined amount of about 200 mg per dose administration. In some embodiments, AD04872 and AD05070 are administered in about 7-day, about 14-day, about 21-day or about 28-day intervals. In some embodiments, AD04872 and AD05070 are administered in 28-day intervals or about 28-day intervals (i.e., Q4W). In some embodiments, AD04872 and AD05070 are administered in a combined amount of about 40 mg, about 100 mg or about 200 mg per dose administration in 28-day intervals or about 28-day intervals (i.e., Q4W). In some embodiments, AD04872 and AD05070 are administered in a combined amount of about 40 mg per dose administration in 28-day intervals or about 28-day intervals (i.e., Q4W). In some embodiments, AD04872 and AD05070 are administered in a combined amount of about 100 mg per dose administration in 28-day intervals or about 28-day intervals (i.e., Q4W). In some embodiments, AD04872 and AD05070 are administered in a combined amount of about 200 mg per dose administration in 28-day intervals or about 28-day intervals (i.e., Q4W).

In some embodiments, AD04872 and AD05070 are administered in about 1-18 week intervals. In some embodiments, AD04872 and AD05070 are administered in about 1-week intervals, about 2-week intervals, about 3-week intervals, about 4-week intervals, about 5-week intervals, about 6-week intervals, about 7-week intervals, about 8-week intervals, about 9-week intervals, about 10-week intervals, about 11-week intervals, about 12-week intervals, about 13-week intervals, about 14-week intervals, about 15-week intervals, about 16-week intervals, about 17-week intervals, or about 18-week intervals. In some embodiments, AD04872 and AD05070 are administered in about 1-6 month intervals. In some embodiments, AD04872 and AD05070 are administered in about 1- month intervals, about 2-month intervals, about 3-month intervals, about 4-month intervals, about 5-month intervals, or about 6-month intervals. In some embodiments, AD04872 and AD05070 are administered in about 4-week intervals or 1-month intervals. In some embodiments, AD04872 and AD05070 are administered in about 7-day, 14-day, 21-day or 28-day intervals. In some embodiments, AD04872 and AD05070 are administered in about 28-day intervals.

In some embodiments, AD04872 and AD05070 are administered for a duration of about 1-12 months. In some embodiments, AD04872 and AD05070 are administered for a duration of at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 12 months. In some embodiments, AD04872 and AD05070 are administered for a duration of about 1-18 weeks. In some embodiments, AD04872 and AD05070 are administered for a duration of at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about 12 weeks, at least about 13 weeks, at least about 14 weeks, at least about 15 weeks, at least about 16 weeks, at least about 17 weeks, or at least about 18 weeks. In some embodiments, AD04872 and AD05070 are administered for a duration of about 12 weeks or 3 months.

In some embodiments, AD04872 and AD05070 are administered in a combined amount of about 25-75 mg per dose administration and in the ratio of about 2:1, about 3:1, about 1:1, about 4:1, about 5:1 or about 1:2. In some embodiments, AD04872 and AD05070 are administered in a combined amount of about 50-125 mg per dose administration and in the ratio of about 2:1, about 3:1, about 1:1, about 4:1, about 5:1 or about 1:2. In some embodiments, AD04872 and AD05070 are administered in a combined amount of about 75-150 mg per dose administration and in the ratio of about 2:1, about 3:1,about 1:1, about 4:1, about 5:1 or about 1:2. In some embodiments, AD04872 and AD05070 are administered in a combined amount of about 100-200 mg per dose administration and in the ratio of about 2:1, about 3:1, about 1:1, about 4:1, about 5:1 or about 1:2. In some embodiments, AD04872 and AD05070 are administered in a combined amount of about 150-250 mg per dose administration and in the ratio of about 2:1, about 3:1, about 1:1, about 4:1, about 5:1 or about 1:2. In some embodiments, AD04872 and AD05070 are administered in a combined amount of about 200-300 mg per dose administration and in the ratio of about 2:1, about 3:1, about 1: 1, about 4:1, about 5:1 or about 1:2. In some embodiments, AD04872 and AD05070 are administered in a combined amount of about 300-400 mg per dose administration and in the ratio of about 2:1, about 3:1, about 1:1, about 4:1, about 5:1 or about 1:2. In some embodiments, AD04872 and AD05070 are administered in a combined amount of about 50-100 mg per dose administration and in the ratio of about 2:1, about 3:1, about 1:1, about 4:1, about 5:1 or about 1:2. In some embodiments, AD04872 and AD05070 are administered in a combined amount of about 25-400 mg per dose administration and in the ratio of about 2:1. In some embodiments, AD04872 and AD05070 are administered in a combined amount of about 25-75 mg per dose administration and in the ratio of about 2:1. In some embodiments, AD04872 and AD05070 are administered in a combined amount of about 50-125 mg per dose administration and in the ratio of about 2:1. In some embodiments, AD04872 and AD05070 are administered in a combined amount of about 75-150 mg per dose administration and in the ratio of about 2:1. In some embodiments, AD04872 and AD05070 are administered in a combined amount of about 100-200 mg per dose administration and in the ratio of about 2:1. In some embodiments, AD04872 and AD05070 are administered in a combined amount of about 125-225 mg per dose administration and in the ratio of about 2:1. In some embodiments, AD04872 and AD05070 are administered in a combined amount of about 150-250 mg per dose administration and in the ratio of about 2:1. In some embodiments, AD04872 and AD05070 are administered in a combined amount of about 200-300 mg per dose administration and in the ratio of about 2:1. In some embodiments, AD04872 and AD05070 are administered in a combined amount of about 300-400 mg per dose administration and in the ratio of about 2:1. In some embodiments, AD04872 and AD05070 are administered in a combined amount of about 35 mg per dose administration and in the ratio of about 2:1. In some embodiments, AD04872 and AD05070 are administered in a combined amount of about 40 mg per dose administration and in the ratio of about 2:1. In some embodiments, AD04872 and AD05070 are administered in a combined amount of about 100 mg per dose administration and in the ratio of about 2:1. In some embodiments, AD04872 and AD05070 are administered in a combined amount of about 200 mg per dose administration and in the ratio of about 2:1. In some embodiments, AD04872 and AD05070 are administered in a combined amount of about 300 mg per dose administration and in the ratio of about 2:1. In some embodiments, AD04872 and AD05070 are administered in a combined amount of about 400 mg per dose administration and in the ratio of about 2:1. In some embodiments, AD04872 and AD05070 are administered in about 7-day, about 14-day, about 21-day or about 28-day intervals. In some embodiments, AD04872 and AD05070 are administered in 28-day intervals or about 28-day intervals (i.e., Q4W). In some embodiments, AD04872 and AD05070 are administered in a combined amount of about 40 mg, about 100 mg or about 200 mg per dose administration, in the ratio of about 2:1, and in 28-day intervals or about 28-day intervals. In some embodiments, AD04872 and AD05070 are administered in a combined amount of about 40 mg per dose administration, in the ratio of about 2:1, and in 28-day intervals or about 28-day intervals (i.e., Q4W). In some embodiments, AD04872 and AD05070 are administered in a combined amount of about 100 mg per dose administration, in the ratio of about 2:1, and in 28-day intervals or about 28-day intervals (i.e., Q4W). In some embodiments, AD04872 and AD05070 are administered in a combined amount of about 200 mg per dose administration, in the ratio of about 2:1, and in 28-day intervals or about 28-day intervals (i.e., Q4W).

In some embodiments, AD04872 is administered in an amount of about 3-650 mg, and AD05070 is administered in an amount of about 2-325 mg per dose administration. In some embodiments, AD04872 is administered in an amount of about 35-265 mg per dose administration. In some embodiments, AD04872 is administered in an amount of about 50-75 mg per dose administration. In some embodiments, AD05070 is administered in an amount of about 20-125 mg per dose administration. In some embodiments, AD05070 is administered in an amount of about 25-50 mg per dose administration.

In some embodiments, AD04872 and AD05070 are administered in a combined amount of about 1-10 mg/kg per dose administration. In some embodiments, AD04872 and AD05070 are administered in a combined amount of about 1-5 mg/kg per dose administration. In some embodiments, AD04872 and AD05070 are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, AD05070 is administered in an amount of about 0.3-5 mg/kg per dose administration, and AD04872 is administered in an amount of about 0.6-7 mg/kg per dose administration. In some embodiments, AD05070 is administered in an amount of about 0.5-2.5 mg/kg per dose administration. In some embodiments, AD05070 is administered in an amount of about 0.3-1.5 mg/kg per dose administration. In some embodiments, AD04872 is administered in an amount of about 0.6-5 mg/kg per dose administration. In some embodiments, AD04872 is administered in an amount of about 1-2.5 mg/kg per dose administration.

In some embodiments, AD04872 and AD05070 are administered at a combined dose of 25-400 mg per dose administration. In an embodiment, AD04872 and AD05070 are administered at a combined dose of 25-400 mg, and AD04872 is administered with AD05070 at a ratio of 1:1. In an embodiment, the dose of AD04872 is administered with AD05070 is in an amount of about 12 mg for a combined dose of about 25 mg. In an embodiment, the dose of each of AD04872 and AD05070 is in an amount of about 17 mg for a combined dose of about 35 mg. In an embodiment, the dose of each of AD04872 and AD05070 is in an amount of about 20 mg for a combined dose of about 40 mg. In an embodiment, the dose of each of AD04872 and AD05070 is in an amount of about 25 mg for a combined dose of about 50 mg. In an embodiment, the dose of each of AD04872 and AD05070 is in an amount of about 50 mg for a combined dose of about 100 mg. In an embodiment, the dose of each of AD04872 and AD05070 is in an amount of about 100 mg for a combined dose of about 200 mg. In an embodiment, the dose of each of AD04872 and AD05070 is in an amount of about 150 mg for a combined dose of about 300 mg. In an embodiment, the dose of each of AD04872 and AD05070 is in an amount of about 200 mg for a combined dose of about 400 mg. In some embodiments, AD04872 and AD05070 are administered in about 7-day, about 14-day, about 21-day or about 28-day intervals. In some embodiments, AD04872 and AD05070 are administered in 28-day intervals or about 28-day intervals (i.e., Q4W). In an embodiment, the dose of each of AD04872 and AD05070 is in an amount of about 20 mg for a combined dose of about 40 mg, or in an amount of about 50 mg for a combined dose of about 100 mg, or in an amount of about 100 mg for a combined dose of about 200 mg, and AD04872 and AD05070 are administered in 28-day intervals or about 28-day intervals (i.e., Q4W). In an embodiment, the dose of each of AD04872 and AD05070 is in an amount of about 20 mg for a combined dose of about 40 mg and AD04872 and AD05070 are administered in 28-day intervals or about 28-day intervals (i.e., Q4W). In an embodiment, the dose of each of AD04872 and AD05070 is in an amount of about 50 mg for a combined dose of about 100 mg and AD04872 and AD05070 are administered in 28-day intervals or about 28-day intervals (i.e., Q4W). In an embodiment, the dose of each of AD04872 and AD05070 is in an amount of about 100 mg for a combined dose of about 200 mg and AD04872 and AD05070 are administered in 28-day intervals or about 28-day intervals (i.e., Q4W).

In an embodiment, AD04872 and AD05070 are administered at a combined dose of 25-400 mg per dose, and AD05070 is administered with AD04872 at a ratio of 1:2. In an embodiment, the dose of AD04872 is in an amount of about 16 mg, and the dose of AD05070 is in an amount of about 8 mg for a combined dose of about 25 mg. In an embodiment, the dose of AD05070 is in an amount of about 12 mg, and the dose of the AD04872 is in an amount of about 24 mg for a combined dose of about 35 mg. In an embodiment, the dose of AD05070 is in an amount of about 13 mg, and the dose of the AD04872 is in an amount of about 27 mg for a combined dose of about 40 mg. In an embodiment, the dose of AD04872 is in an amount of about 33 mg, and the dose of AD05070 is in an amount of about 17 mg for a combined dose of about 50 mg. In an embodiment, the dose of AD05070 is in an amount of about 35 mg, and the dose of AD04872 is in an amount of about 65 mg for a combined dose of about 100 mg. In an embodiment, the dose of AD05070 is in an amount of about 67 mg, and the dose of AD04872 is in an amount of about 133 mg for a combined dose of about 200 mg. In an embodiment, the dose of the AD05070 is in an amount of about 100 mg, and the dose of AD04872 is in an amount of about 200 mg for a combined dose of about 300 mg. In an embodiment, the dose of AD05070 is in an amount of about 135 mg, and the dose of AD04872 is in an amount of about 270 mg for a combined dose of about 400 mg. In some embodiments, AD04872 and AD05070 are administered in about 7-day, about 14-day, about 21-day or about 28-day intervals. In some embodiments, AD04872 and AD05070 are administered in 28-day intervals or about 28-day intervals (i.e., Q4W). In an embodiment, the dose of AD05070 is in an amount of about 13 mg, and the dose of the AD04872 is in an amount of about 27 mg for a combined dose of about 40 mg, and AD04872 and AD05070 are administered in 28-day intervals or about 28-day intervals (i.e., Q4W). In an embodiment, the dose of AD05070 is in an amount of about 35 mg, and the dose of AD04872 is in an amount of about 65 mg for a combined dose of about 100 mg, and AD04872 and AD05070 are administered in 28-day intervals or about 28-day intervals (i.e., Q4W). In an embodiment, the dose of AD05070 is in an amount of about 67 mg, and the dose of AD04872 is in an amount of about 133 mg for a combined dose of about 200 mg, and AD04872 and AD05070 are administered in 28-day intervals or about 28-day intervals (i.e., Q4W).

In an embodiment, AD04872 and AD05070 are administered at a combined dose of 25-400 mg per dose, AD05070 is administered with AD04872 at a ratio of 1:3. In an embodiment, the dose of AD04872 is in an amount of about 18 mg, and the dose of AD05070 is in an amount of about 6 mg for a combined dose of about 25 mg. In an embodiment, the dose of AD05070 is in an amount of about 9 mg, and the dose of AD04872 is in an amount of about 27 mg for a combined dose of about 35 mg. In an embodiment, the dose of AD05070 is in an amount of about 10 mg, and the dose of AD04872 is in an amount of about 30 mg for a combined dose of about 40 mg. In an embodiment, the dose of AD04872 is in an amount of about 36 mg, and the dose of AD05070 is in an amount of about 12 mg for a combined dose of about 50 mg. In an embodiment, the dose of AD05070 is in an amount of about 25 mg, and the dose of AD04872 is in an amount of about 75 mg for a combined dose of about 100 mg. In an embodiment, the dose of AD05070 is in an amount of about 50 mg, and the dose of AD04872 is in an amount of about 150 mg for a combined dose of about 200 mg. In an embodiment, the dose of AD05070 is in an amount of about 75 mg, and the dose of AD04872 is in an amount of about 225 mg for a combined dose of about 300 mg. In an embodiment, the dose of AD05070 is in an amount of about 100 mg, and the dose of AD04872 is in an amount of about 300 mg for a combined dose of about 400 mg. In some embodiments, AD04872 and AD05070 are administered in about 7-day, about 14-day, about 21-day or about 28-day intervals. In some embodiments, AD04872 and AD05070 are administered in 28-day intervals or about 28-day intervals (i.e., Q4W). In an embodiment, the dose of AD05070 is in an amount of about 10 mg, and the dose of AD04872 is in an amount of about 30 mg for a combined dose of about 40 mg, and AD04872 and AD05070 are administered in 28-day intervals or about 28-day intervals (i.e., Q4W). In an embodiment, the dose of AD05070 is in an amount of about 25 mg, and the dose of AD04872 is in an amount of about 75 mg for a combined dose of about 100 mg, and AD04872 and AD05070 are administered in 28-day intervals or about 28-day intervals (i.e., Q4W). In an embodiment, the dose of AD05070 is in an amount of about 50 mg, and the dose of AD04872 is in an amount of about 150 mg for a combined dose of about 200 mg, and AD04872 and AD05070 are administered in 28-day intervals or about 28-day intervals (i.e., Q4W).

In some embodiments, about 1 mg/kg (mpk) of AD04872 and about 1 mg/kg of AD05070 are administered to a subject in need thereof. In some embodiments, about 1.5 mg/kg of AD04872 and about 1.5 mg/kg of AD05070 are administered to a subject in need thereof. In some embodiments, about 2.0 mg/kg of AD04872 and about 1.0 mg/kg of AD05070 are administered to a subject in need thereof. In some embodiments, about 3.0 mg/kg of AD04872 and about 1.0 mg/kg of AD05070 are administered to a subject in need thereof. In some embodiments, about 3.2 mg/kg of AD04872 and about 0.8 mg/kg of AD05070 are administered to a subject in need thereof. In some embodiments, about 2.7 mg/kg of AD04872 and about 1.3 mg/kg of AD05070 are administered to a subject in need thereof. In some embodiments, about 4.0 mg/kg of AD04872 and about 1.0 mg/kg of AD05070 are administered to a subject in need thereof. In some embodiments, about 3.3 mg/kg of AD04872 and about 1.7 mg/kg of AD05070 are administered to a subject in need thereof. In some embodiments, between about 0.05 and about 5 mg/kg of AD04872 and between about 0.05 and about 5 mg/kg of AD05070 are administered to a subject in need thereof. In some embodiments, about AD04872 and about AD05070 are administered separately (e.g., in separate injections). In some embodiments, the respective dose of AD04872 and the respective dose of AD05070 are administered together (e.g., in the same injection). In some embodiments, the respective dose of AD04872 and the respective dose of AD05070 are prepared in a single pharmaceutical composition.

In some embodiments, disclosed herein are methods of treatment of an HBV infection or prevention of diseases or symptoms caused by an HBV infection comprising administering to a subject in need thereof an effective amount of AD04872 and an effective amount of AD04776. In some embodiments, the ratio of AD04872 to AD04776 administered to a subject in need thereof is about 2:1. In some embodiments, the ratio of AD04872 to AD04776 administered to a subject in need thereof is about 3:1. In some embodiments, the ratio of AD04872 to AD04776 administered to a subject in need thereof is about 4:1. In some embodiments, the ratio of AD04872 to AD04776 administered to a subject in need thereof is about 1:1. In some embodiments, the ratio of AD04872 to AD04776 administered to a subject in need thereof is 5:1. In some embodiments, the ratio of AD04872 to AD04776 administered to a subject in need thereof is 1:2.

In some embodiments, disclosed herein are methods of treating or preventing a disease associated with an infection caused by HBV, comprising administering to a subject in need thereof an effective amount of AD04872 and an effective amount of AD04776. In some embodiments, the ratio of AD04872 to AD04776 administered to a subject in need thereof is about 2:1. In some embodiments, the ratio of AD04872 to AD04776 administered to a subject in need thereof is about 3:1. In some embodiments, the ratio of AD04872 to AD04776 administered to a subject in need thereof is about 4:1. In some embodiments, the ratio of AD04872 to AD04776 administered to a subject in need thereof is about 1:1. In some embodiments, the ratio of AD04872 to AD04776 administered to a subject in need thereof is 5:1. In some embodiments, the ratio of AD04872 to AD04776 administered to a subject in need thereof is 1:2.

In some embodiments, disclosed herein are methods of treating a disease associated with an infection caused by HBV, comprising administering to a subject in need thereof an effective amount of AD04872 and an effective amount of AD04776. In some embodiments, the ratio of AD04872 to AD04776 administered to a subject in need thereof is about 2:1. In some embodiments, the ratio of AD04872 to AD04776 administered to a subject in need thereof is about 3:1. In some embodiments, the ratio of AD04872 to AD04776 administered to a subject in need thereof is about 4:1. In some embodiments, the ratio of AD04872 to AD04776 administered to a subject in need thereof is about 1:1. In some embodiments, the ratio of AD04872 to AD04776 administered to a subject in need thereof is 5:1. In some embodiments, the ratio of AD04872 to AD04776 administered to a subject in need thereof is 1:2.

In some embodiments, AD04872 and AD04776 are administered in a combined amount of about 25-400 mg per dose administration. In some embodiments, AD04872 and AD04776 are administered in a combined amount of about 25-50 mg, 50-75 mg, 75-100 mg, 100-125 mg, 125-150 mg, 150-175 mg, 175-200 mg, 200-225 mg, 225-250 mg, 250-275 mg, 275-300 mg, 300-325 mg, 325-350 mg, 350-375 mg, 375-400 mg, 25-75 mg, 50-100 mg, 100-150 mg, 150-200 mg, 200-250 mg, 250-300 mg, 300-350 mg, 350-400 mg, 25-100 mg, 50-150 mg, 100-200 mg, 150-250 mg, 200-300 mg, 300-400 mg, 25-200 mg, or 200-400 mg per dose administration. In some embodiments, AD04872 and AD04776 are administered in a combined amount of about 30-50 mg, about 90-110 mg, or about 190-210 mg per dose administration. In some embodiments, AD04872 and AD04776 are administered in a combined amount of about 30-50 mg per dose administration. In some embodiments, AD04872 and AD04776 are administered in a combined amount of about 90-110 mg per dose administration. In some embodiments, AD04872 and AD04776 are administered in a combined amount of about 190-210 mg per dose administration. In some embodiments, AD04872 and AD04776 are administered in a combined amount of about 25 mg, about 50 mg, about 100 mg, about 125 mg, about 150 mg, about 175 mg, about 200 mg, about 225 mg, about 250 mg, about 275 mg, about 300 mg, about 325 mg, about 350 mg, about 375 mg, or about 400 mg per dose administration. In some embodiments, AD04872 and AD04776 are administered in a combined amount of about 50 mg, about 75 mg, about 100 mg, or about 125 mg per dose administration. In some embodiments, AD04872 and AD04776 are administered in a combined amount of about 35 mg, about 50 mg, about 100 mg, about 200 mg, about 300 mg or about 400 mg per dose administration. In some embodiments, AD04872 and AD04776 are administered in a combined amount of about 40 mg, about 100 mg, or about 200 mg per dose administration. In some embodiments, AD04872 and AD04776 are administered in a combined amount of about 40 mg per dose administration. In some embodiments, AD04872 and AD04776 are administered in a combined amount of about 100 mg per dose administration. In some embodiments, AD04872 and AD04776 are administered in a combined amount of about 200 mg per dose administration. In some embodiments, AD04872 and AD04776 are administered in about 7-day, about 14-day, about 21-day or about 28-day intervals. In some embodiments, AD04872 and AD04776 are administered in 28-day intervals or about 28-day intervals (i.e., Q4W). In some embodiments, AD04872 and AD04776 are administered in a combined amount of about 40 mg, about 100 mg or about 200 mg per dose administration in 28-day intervals or about 28-day intervals (i.e., Q4W). In some embodiments, AD04872 and AD04776 are administered in a combined amount of about 40 mg per dose administration in 28-day intervals or about 28-day intervals (i.e., Q4W). In some embodiments, AD04872 and AD04776 are administered in a combined amount of about 100 mg per dose administration in 28-day intervals or about 28-day intervals (i.e., Q4W). In some embodiments, AD04872 and AD04776 are administered in a combined amount of about 200 mg per dose administration in 28-day intervals or about 28-day intervals (i.e., Q4W).

In some embodiments, AD04872 and AD04776 are administered in about 1-18 week intervals. In some embodiments, AD04872 and AD04776 are administered in about 1-week intervals, about 2-week intervals, about 3-week intervals, about 4-week intervals, about 5-week intervals, about 6-week intervals, about 7-week intervals, about 8-week intervals, about 9-week intervals, about 10-week intervals, about 11-week intervals, about 12-week intervals, about 13-week intervals, about 14-week intervals, about 15-week intervals, about 16-week intervals, about 17-week intervals, or about 18-week intervals. In some embodiments, AD04872 and AD04776 are administered in about 1-6 month intervals. In some embodiments, AD04872 and AD04776 are administered in about 1- month intervals, about 2-month intervals, about 3- month intervals, about 4- month intervals, about 5- month intervals, or about 6- month intervals. In some embodiments, AD04872 and AD04776 are administered in about 4-week intervals or 1-month intervals. In some embodiments, AD04872 and AD04776 are administered in about 7-day, 14-day, 21-day or 28-day intervals. In some embodiments, AD04872 and AD04776 are administered in about 28-day intervals (i.e., Q4W).

In some embodiments, AD04872 and AD04776 are administered for a duration of about 1-12 months. In some embodiments, AD04872 and AD04776 are administered for a duration of at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 12 months. In some embodiments, AD04872 and AD04776 are administered for a duration of about 1-18 weeks. In some embodiments, AD04872 and AD04776 are administered for a duration of at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about 12 weeks, at least about 13 weeks, at least about 14 weeks, at least about 15 weeks, at least about 16 weeks, at least about 17 weeks, or at least about 18 weeks. In some embodiments, AD04872 and AD04776 are administered for a duration of about 12 weeks or 3 months.

In some embodiments, AD04872 and AD04776 are administered in a combined amount of about 25-75 mg per dose administration and in the ratio of about 2:1, about 3:1, about 1:1, about 4:1, about 5:1 or about 1:2. In some embodiments, AD04872 and AD04776 are administered in a combined amount of about 50-125 mg per dose administration and in the ratio of about 2:1, about 3:1, about 1:1, about 4:1, about 5:1 or about 1:2. In some embodiments, AD04872 and AD04776 are administered in a combined amount of about 75-150 mg per dose administration and in the ratio of about 2:1, about 3:1, about 1:1, about 4:1, about 5:1 or about 1:2. In some embodiments, AD04872 and AD04776 are administered in a combined amount of about 100-200 mg per dose administration and in the ratio of about 2:1, about 3:1, about 1:1, about 4:1, about 5:1 or about 1:2. In some embodiments, AD04872 and AD04776 are administered in a combined amount of about 150-250 mg per dose administration and in the ratio of about 2:1, about 3:1, about 1:1, about 4:1, about 5:1 or about 1:2. In some embodiments, AD04872 and AD04776 are administered in a combined amount of about 200-300 mg per dose administration and in the ratio of about 2:1, about 3:1, about 1:1, about 4:1, about 5:1 or about 1:2. In some embodiments, AD04872 and AD04776 are administered in a combined amount of about 300-400 mg per dose administration and in the ratio of about 2:1, about 3:1, about 1:1, about 4:1, about 5:1 or about 1:2. In some embodiments, AD04872 and AD04776 are administered in a combined amount of about 50-100 mg per dose administration and in the ratio of about 2:1, about 3:1, about 1:1, about 4:1, about 5:1 or about 1:2. In some embodiments, AD04872 and AD04776 are administered in a combined amount of about 25-400 mg per dose administration and in the ratio of about 2:1. In some embodiments, AD04872 and AD04776 are administered in a combined amount of about 25-75 mg per dose administration and in the ratio of about 2:1. In some embodiments, AD04872 and AD04776 are administered in a combined amount of about 50-125 mg per dose administration and in the ratio of about 2:1. In some embodiments, AD04872 and AD04776 are administered in a combined amount of about 75-150 mg per dose administration and in the ratio of about 2:1. In some embodiments, AD04872 and AD04776 are administered in a combined amount of about 100-200 mg per dose administration and in the ratio of about 2:1. In some embodiments, AD04872 and AD04776 are administered in a combined amount of about 125-225 mg per dose administration and in the ratio of about 2:1. In some embodiments, AD04872 and AD04776 are administered in a combined amount of about 150-250 mg per dose administration and in the ratio of about 2:1. In some embodiments, AD04872 and AD04776 are administered in a combined amount of about 200-300 mg per dose administration and in the ratio of about 2:1. In some embodiments, AD04872 and AD04776 are administered in a combined amount of about 300-400 mg per dose administration and in the ratio of about 2:1. In some embodiments, AD04872 and AD04776 are administered in a combined amount of about 35 mg per dose administration and in the ratio of about 2:1. In some embodiments, AD04872 and AD04776 are administered in a combined amount of about 40 mg per dose administration and in the ratio of about 2:1. In some embodiments, AD04872 and AD04776 are administered in a combined amount of about 100 mg per dose administration and in the ratio of about 2:1. In some embodiments, AD04872 and AD04776 are administered in a combined amount of about 200 mg per dose administration and in the ratio of about 2:1. In some embodiments, AD04872 and AD04776 are administered in a combined amount of about 300 mg per dose administration and in the ratio of about 2:1. In some embodiments, AD04872 and AD04776 are administered in a combined amount of about 400 mg per dose administration and in the ratio of about 2:1. In some embodiments, AD04872 and AD04776 are administered in about 7-day, about 14-day, about 21-day or about 28-day intervals. In some embodiments, AD04872 and AD04776 are administered in 28-day intervals or about 28-day intervals (i.e., Q4W). In some embodiments, AD04872 and AD04776 are administered in a combined amount of about 40 mg, about 100 mg or about 200 mg per dose administration, in the ratio of about 2:1, and in 28-day intervals or about 28-day intervals (i.e., Q4W). In some embodiments, AD04872 and AD04776 are administered in a combined amount of about 40 mg per dose administration, in the ratio of about 2:1, and in 28-day intervals or about 28-day intervals (i.e., Q4W). In some embodiments, AD04872 and AD04776 are administered in a combined amount of about 100 mg per dose administration, in the ratio of about 2:1, and in 28-day intervals or about 28-day intervals (i.e., Q4W). In some embodiments, AD04872 and AD04776 are administered in a combined amount of about 200 mg per dose administration, in the ratio of about 2:1, and in 28-day intervals or about 28-day intervals (i.e., Q4W).

In some embodiments, AD04872 is administered in an amount of about 3-650 mg, and AD04776 is administered in an amount of about 2-325 mg per dose administration. In some embodiments, AD04872 is administered in an amount of about 35-265 mg per dose administration. In some embodiments, AD04872 is administered in an amount of about 50-75 mg per dose administration. In some embodiments, AD04776 is administered in an amount of about 20-125 mg per dose administration. In some embodiments, AD04776 is administered in an amount of about 25-50 mg per dose administration.

In some embodiments, AD04872 and AD04776 are administered in a combined amount of about 1-10 mg/kg per dose administration. In some embodiments, AD04872 and AD04776 are administered in a combined amount of about 1-5 mg/kg per dose administration. In some embodiments, AD04872 and AD04776 are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, AD04776 is administered in an amount of about 0.3-5 mg/kg per dose administration, and AD04872 is administered in an amount of about 0.6-7 mg/kg per dose administration. In some embodiments, AD04776 is administered in an amount of about 0.5-2.5 mg per dose administration. In some embodiments, AD04776 is administered in an amount of about 0.3-1.5 mg per dose administration. In some embodiments, AD04872 is administered in an amount of about 0.6-5 mg per dose administration. In some embodiments, AD04872 is administered in an amount of about 1-2.5 mg per dose administration.

In some embodiments, AD04872 and AD04776 are administered at a combined dose of 25-400 mg per dose administration. In an embodiment, AD04872 and AD04776 are administered at a combined dose of 25-400 mg, and AD04872 is administered with AD04776 at a ratio of 1:1. In an embodiment, the dose of each of AD04872 and AD04776 is in an amount of about 12 mg for a combined dose of about 25 mg. In an embodiment, the dose of each of AD04872 and AD04776 is in an amount of about 17 mg for a combined dose of about 35 mg. In an embodiment, the dose of each of AD04872 and AD04776 is in an amount of about 20 mg for a combined dose of about 40 mg. In an embodiment, the dose of each of AD04872 and AD04776 is in an amount of about 25 mg for a combined dose of about 50 mg. In an embodiment, the dose of each of AD04872 and AD04776 is in an amount of about 50 mg for a combined dose of about 100 mg. In an embodiment, the dose of each of AD04872 and AD04776 is in an amount of about 100 mg for a combined dose of about 200 mg. In an embodiment, the dose of each of AD04872 and AD04776 is in an amount of about 150 mg for a combined dose of about 300 mg. In an embodiment, the dose of each of AD04872 and AD04776 is in an amount of about 200 mg for a combined dose of about 400 mg. In some embodiments, AD04872 and AD04776 are administered in about 7-day, about 14-day, about 21-day or about 28-day intervals. In some embodiments, AD04872 and AD04776 are administered in 28-day intervals or about 28-day intervals (i.e., Q4W). In an embodiment, the dose of each of AD04872 and AD04776 is in an amount of about 20 mg for a combined dose of about 40 mg, or in an amount of about 50 mg for a combined dose of about 100 mg, or in an amount of about 100 mg for a combined dose of about 200 mg, and AD04872 and AD04776 are administered in 28-day intervals or about 28-day intervals (i.e., Q4W). In an embodiment, the dose of each of AD04872 and AD04776 is in an amount of about 20 mg for a combined dose of about 40 mg and AD04872 and AD04776 are administered in 28-day intervals or about 28-day intervals (i.e., Q4W). In an embodiment, the dose of each of AD04872 and AD04776 is in an amount of about 50 mg for a combined dose of about 100 mg and AD04872 and AD04776 are administered in 28-day intervals or about 28-day intervals (i.e., Q4W). In an embodiment, the dose of each of AD04872 and AD04776 is in an amount of about 100 mg for a combined dose of about 200 mg and AD04872 and AD04776 are administered in 28-day intervals or about 28-day intervals (i.e., Q4W).

In an embodiment, AD04872 and AD04776 are administered at a combined dose of 25-400 mg per dose, and AD04776 is administered with AD04872 at a ratio of 1:2. In an embodiment, the dose of AD04872 is in an amount of about 16 mg, and the dose of AD04776 is in an amount of about 8 mg for a combined dose of about 25 mg. In an embodiment, the dose of AD04776 is in an amount of about 12 mg, and the dose of the AD04872 is in an amount of about 24 mg for a combined dose of about 35 mg. In an embodiment, the dose of AD04776 is in an amount of about 13 mg, and the dose of the AD04872 is in an amount of about 27 mg for a combined dose of about 40 mg. In an embodiment, the dose of AD04872 is in an amount of about 33 mg, and the dose of AD04776 is in an amount of about 17 mg for a combined dose of about 50 mg. In an embodiment, the dose of AD04776 is in an amount of about 35 mg, and the dose of AD04872 is in an amount of about 65 mg for a combined dose of about 100 mg. In an embodiment, the dose of AD04776 is in an amount of about 67 mg, and the dose of AD04872 is in an amount of about 133 mg for a combined dose of about 200 mg. In an embodiment, the dose of the AD04776 is in an amount of about 100 mg, and the dose of AD04872 is in an amount of about 200 mg for a combined dose of about 300 mg. In an embodiment, the dose of AD04776 is in an amount of about 135 mg, and the dose of AD04872 is in an amount of about 270 mg for a combined dose of about 400 mg. In some embodiments, AD04872 and AD04776 are administered in about 7-day, about 14-day, about 21-day or about 28-day intervals. In some embodiments, AD04872 and AD04776 are administered in 28-day intervals or about 28-day intervals (i.e., Q4W). In an embodiment, the dose of AD04776 is in an amount of about 13 mg, and the dose of the AD04872 is in an amount of about 27 mg for a combined dose of about 40 mg, and AD04872 and AD04776 are administered in 28-day intervals or about 28-day intervals (i.e., Q4W). In an embodiment, the dose of AD04776 is in an amount of about 35 mg, and the dose of AD04872 is in an amount of about 65 mg for a combined dose of about 100 mg, and AD04872 and AD04776 are administered in 28-day intervals or about 28-day intervals (i.e., Q4W). In an embodiment, the dose of AD04776 is in an amount of about 67 mg, and the dose of AD04872 is in an amount of about 133 mg for a combined dose of about 200 mg, and AD04872 and AD04776 are administered in 28-day intervals or about 28-day intervals (i.e., Q4W).

In an embodiment, AD04872 and AD04776 are administered at a combined dose of 25-400 mg per dose, AD04776 is administered with AD04872 at a ratio of 1:3. In an embodiment, the dose of AD04872 is in an amount of about 18 mg, and the dose of AD04776 is in an amount of about 6 mg for a combined dose of about 25 mg. In an embodiment, the dose of AD04776 is in an amount of about 9 mg, and the dose of AD04872 is in an amount of about 27 mg for a combined dose of about 35 mg. In an embodiment, the dose of AD04776 is in an amount of about 10 mg, and the dose of AD04872 is in an amount of about 30 mg for a combined dose of about 40 mg. In an embodiment, the dose of AD04872 is in an amount of about 36 mg, and the dose of AD04776 is in an amount of about 12 mg for a combined dose of about 50 mg. In an embodiment, the dose of AD04776 is in an amount of about 25 mg, and the dose of AD04872 is in an amount of about 75 mg for a combined dose of about 100 mg. In an embodiment, the dose of AD04776 is in an amount of about 50 mg, and the dose of AD04872 is in an amount of about 150 mg for a combined dose of about 200 mg. In an embodiment, the dose of AD04776 is in an amount of about 75 mg, and the dose of AD04872 is in an amount of about 225 mg for a combined dose of about 300 mg. In an embodiment, the dose of AD04776 is in an amount of about 100 mg, and the dose of AD04872 is in an amount of about 300 mg for a combined dose of about 400 mg. In some embodiments, AD04872 and AD04776 are administered in about 7-day, about 14-day, about 21-day or about 28-day intervals. In some embodiments, AD04872 and AD04776 are administered in 28-day intervals or about 28-day intervals (i.e., Q4W). In an embodiment, the dose of AD04776 is in an amount of about 10 mg, and the dose of AD04872 is in an amount of about 30 mg for a combined dose of about 40 mg, and AD04872 and AD04776 are administered in 28-day intervals or about 28-day intervals (i.e., Q4W). In an embodiment, the dose of AD04776 is in an amount of about 25 mg, and the dose of AD04872 is in an amount of about 75 mg for a combined dose of about 100 mg, and AD04872 and AD04776 are administered in 28-day intervals or about 28-day intervals (i.e., Q4W). In an embodiment, the dose of AD04776 is in an amount of about 50 mg, and the dose of AD04872 is in an amount of about 150 mg for a combined dose of about 200 mg, and AD04872 and AD04776 are administered in 28-day intervals or about 28-day intervals (i.e., Q4W).

In some embodiments, about 1 mg/kg (mpk) of AD04872 and about 1 mg/kg of AD04776 are administered to a subject in need thereof. In some embodiments, about 1.5 mg/kg of AD04872 and about 1.5 mg/kg of AD04776 are administered to a subject in need thereof. In some embodiments, about 2.0 mg/kg of AD04872 and about 1.0 mg/kg of AD04776 are administered to a subject in need thereof. In some embodiments, about 3.0 mg/kg of AD04872 and about 1.0 mg/kg of AD04776 are administered to a subject in need thereof. In some embodiments, about 3.2 mg/kg of AD04872 and about 0.8 mg/kg of AD04776 are administered to a subject in need thereof. In some embodiments, about 2.7 mg/kg of AD04872 and about 1.3 mg/kg of AD04776 are administered to a subject in need thereof. In some embodiments, about 4.0 mg/kg of AD04872 and about 1.0 mg/kg of AD04776 are administered to a subject in need thereof. In some embodiments, about 3.3 mg/kg of AD04872 and about 1.7 mg/kg of AD04776 are administered to a subject in need thereof. In some embodiments, between about 0.05 and about 5 mg/kg of AD04872 and between about 0.05 and about 5 mg/kg of AD04776 are administered to a subject in need thereof. In some embodiments, the respective doses of AD04872 and AD04776 are administered separately (e.g., in separate injections). In some embodiments, the respective doses of AD04872 and AD04776 are administered together (e.g., in the same injection). In some embodiments, the respective doses of AD04872 and AD04776 are prepared in a single pharmaceutical composition.

In some embodiments, disclosed herein are methods of treatment of an HBV infection or prevention of disease or symptoms caused by an HBV infection comprising administering to a subject in need thereof an effective amount of AD04872 and an effective amount of AD04982. In some embodiments, the ratio of AD04872 to AD04982 administered to a subject in need thereof is about 2:1. In some embodiments, the ratio of AD04872 to AD04982 administered to a subject in need thereof is about 3:1. In some embodiments, the ratio of AD04872 to AD04982 administered to a subject in need thereof is about 4:1. In some embodiments, the ratio of AD04872 to AD04982 administered to a subject in need thereof is about 1:1. In some embodiments, the ratio of AD04872 to AD04982 administered to a subject in need thereof is about 5:1. In some embodiments, the ratio of AD04872 to AD04982 administered to a subject in need thereof is 1:2.

In some embodiments, disclosed herein are methods of treating or preventing a disease associated with an infection caused by HBV, comprising administering to a subject in need thereof an effective amount of AD04872 and an effective amount of AD04982. In some embodiments, the ratio of AD04872 to AD04982 administered to a subject in need thereof is about 2:1. In some embodiments, the ratio of AD04872 to AD04982 administered to a subject in need thereof is about 3:1. In some embodiments, the ratio of AD04872 to AD04982 administered to a subject in need thereof is about 4:1. In some embodiments, the ratio of AD04872 to AD04982 administered to a subject in need thereof is about 1:1. In some embodiments, the ratio of AD04872 to AD04982 administered to a subject in need thereof is about 5:1. In some embodiments, the ratio of AD04872 to AD04982 administered to a subject in need thereof is 1:2.

In some embodiments, disclosed herein are methods of treating a disease associated with an infection caused by HBV, comprising administering to a subject in need thereof an effective amount of AD04872 and an effective amount of AD04982. In some embodiments, the ratio of AD04872 to AD04982 administered to a subject in need thereof is about 2:1. In some embodiments, the ratio of AD04872 to AD04982 administered to a subject in need thereof is about 3:1. In some embodiments, the ratio of AD04872 to AD04982 administered to a subject in need thereof is about 4:1. In some embodiments, the ratio of AD04872 to AD04982 administered to a subject in need thereof is about 1:1. In some embodiments, the ratio of AD04872 to AD04982 administered to a subject in need thereof is about 5:1. In some embodiments, the ratio of AD04872 to AD04982 administered to a subject in need thereof is 1:2.

In some embodiments, AD04872 and AD04982 are administered in a combined amount of about 25-400 mg per dose administration. In some embodiments, AD04872 and AD04982 are administered in a combined amount of about 25-50 mg, 50-75 mg, 75-100 mg, 100-125 mg, 125-150 mg, 150-175 mg, 175-200 mg, 200-225 mg, 225-250 mg, 250-275 mg, 275-300 mg, 300-325 mg, 325-350 mg, 350-375 mg, 375-400 mg, 25-75 mg, 50-100 mg, 100-150 mg, 150-200 mg, 200-250 mg, 250-300 mg, 300-350 mg, 350-400 mg, 25-100 mg, 50-150 mg, 100-200 mg, 150-250 mg, 200-300 mg, 300-400 mg, 25-200 mg, or 200-400 mg. In some embodiments, AD04872 and AD04982 are administered in a combined amount of about 30-50 mg, about 90-110 mg, or about 190-210 mg per dose administration. In some embodiments, AD04872 and AD04982 are administered in a combined amount of about 30-50 mg per dose administration. In some embodiments, AD04872 and AD04982 are administered in a combined amount of about 90-110 mg per dose administration. In some embodiments, AD04872 and AD04982 are administered in a combined amount of about 190-210 mg per dose administration. In some embodiments, AD04872 and AD04982 are administered in a combined amount of about 25 mg, about 50 mg, about 100 mg, about 125 mg, about 150 mg, about 175 mg, about 200 mg, about 225 mg, about 250 mg, about 275 mg, about 300 mg, about 325 mg, about 350 mg, about 375 mg, or about 400 mg per dose administration. In some embodiments, AD04872 and AD04982 are administered in a combined amount of about 50 mg, about 75 mg, about 100 mg, or about 125 mg per dose administration. In some embodiments, AD04872 and AD04982 are administered in a combined amount of about 35 mg, about 50 mg, about 100 mg, about 200 mg, about 300 mg or about 400 mg per dose administration. In some embodiments, AD04872 and AD04982 are administered in a combined amount of about 40 mg, about 100 mg, or about 200 mg per dose administration. In some embodiments, AD04872 and AD04982 are administered in a combined amount of about 40 mg per dose administration. In some embodiments, AD04872 and AD04982 are administered in a combined amount of about 100 mg per dose administration. In some embodiments, AD04872 and AD04982 are administered in a combined amount of about 200 mg per dose administration. In some embodiments, AD04872 and AD04982 are administered in about 7-day, about 14-day, about 21-day or about 28-day intervals. In some embodiments, AD04872 and AD04982 are administered in 28-day intervals or about 28-day intervals (i.e., Q4W). In some embodiments, AD04872 and AD04982 are administered in a combined amount of about 40 mg, about 100 mg or about 200 mg per dose administration in 28-day intervals or about 28-day intervals (i.e., Q4W). In some embodiments, AD04872 and AD04982 are administered in a combined amount of about 40 mg per dose administration in 28-day intervals or about 28-day intervals (i.e., Q4W). In some embodiments, AD04872 and AD04982 are administered in a combined amount of about 100 mg per dose administration in 28-day intervals or about 28-day intervals (i.e., Q4W). In some embodiments, AD04872 and AD04982 are administered in a combined amount of about 200 mg per dose administration in 28-day intervals or about 28-day intervals (i.e., Q4W).

In some embodiments, AD04872 and AD04982 are administered in about 1-18 week intervals. In some embodiments, AD04872 and AD04982 are administered in about 1-week intervals, about 2-week intervals, about 3-week intervals, about 4-week intervals, about 5-week intervals, about 6-week intervals, about 7-week intervals, about 8-week intervals, about 9-week intervals, about 10-week intervals, about 11-week intervals, about 12-week intervals, about 13-week intervals, about 14-week intervals, about 15-week intervals, about 16-week intervals, about 17-week intervals, or about 18-week intervals. In some embodiments, AD04872 and AD04982 are administered in about 1-6 month intervals. In some embodiments, AD04872 and AD04982 are administered in about 1- month intervals, about 2-month intervals, about 3- month intervals, about 4- month intervals, about 5- month intervals, or about 6- month intervals. In some embodiments, AD04872 and AD04982 are administered in about 4-week intervals or 1-month intervals. In some embodiments, AD04872 and AD04982 are administered in about 7-day, 14-day, 21-day or 28-day intervals. In some embodiments, AD04872 and AD04982 are administered in about 28-day intervals (i.e., Q4W).

In some embodiments, AD04872 and AD04982 are administered for a duration of about 1-12 months. In some embodiments, AD04872 and AD04982 are administered for a duration of at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 12 months. In some embodiments, AD04872 and AD04982 are administered for a duration of about 1-18 weeks. In some embodiments, AD04872 and AD04982 are administered for a duration of at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about 12 weeks, at least about 13 weeks, at least about 14 weeks, at least about 15 weeks, at least about 16 weeks, at least about 17 weeks, or at least about 18 weeks. In some embodiments, AD04872 and AD04982 are administered for a duration of about 12 weeks or 3 months.

In some embodiments, AD04872 and AD04982 are administered in a combined amount of about 25-75 mg per dose administration and in the ratio of about 2:1, about 3:1, about 1:1, about 4:1, about 5:1 or about 1:2. In some embodiments, AD04872 and AD04982 are administered in a combined amount of about 50-125 mg per dose administration and in the ratio of about 2:1, about 3:1, about 1:1, about 4:1, about 5:1 or about 1:2. In some embodiments, AD04872 and AD04982 are administered in a combined amount of about 75-150 mg per dose administration and in the ratio of about 2:1, about 3:1, about 1:1, about 4:1, about 5:1 or about 1:2. In some embodiments, AD04872 and AD04982 are administered in a combined amount of about 100-200 mg per dose administration and in the ratio of about 2:1, about 3:1,about 1:1, about 4:1, about 5:1 or about 1:2. In some embodiments, AD04872 and AD04982 are administered in a combined amount of about 150-250 mg per dose administration and in the ratio of about 2:1, about 3:1, about 1:1, about 4:1, about 5:1 or about 1:2. In some embodiments, AD04872 and AD04982 are administered in a combined amount of about 200-300 mg per dose administration and in the ratio of about 2:1, about 3:1, about 1:1, about 4:1, about 5:1 or about 1:2. In some embodiments, AD04872 and AD04982 are administered in a combined amount of about 300-400 mg per dose administration and in the ratio of about 2:1, about 3:1, about 1:1, about 4:1, about 5:1 or about 1:2. In some embodiments, AD04872 and AD04982 are administered in a combined amount of about 50-100 mg per dose administration and in the ratio of about 2:1, about 3:1, about 1:1, about 4:1, about 5:1 or about 1:2. In some embodiments, AD04872 and AD04982 are administered in a combined amount of about 25-400 mg per dose administration and in the ratio of about 2:1. In some embodiments, AD04872 and AD04982 are administered in a combined amount of about 25-75 mg per dose administration and in the ratio of about 2:1. In some embodiments, AD04872 and AD04982 are administered in a combined amount of about 50-125 mg per dose administration and in the ratio of about 2:1. In some embodiments, AD04872 and AD04982 are administered in a combined amount of about 75-150 mg per dose administration and in the ratio of about 2:1. In some embodiments, AD04872 and AD04982 are administered in a combined amount of about 100-200 mg per dose administration and in the ratio of about 2:1. In some embodiments, AD04872 and AD04982 are administered in a combined amount of about 125-225 mg per dose administration and in the ratio of about 2:1. In some embodiments, AD04872 and AD04982 are administered in a combined amount of about 150-250 mg per dose administration and in the ratio of about 2:1. In some embodiments, AD04872 and AD04982 are administered in a combined amount of about 200-300 mg per dose administration and in the ratio of about 2:1. In some embodiments, AD04872 and AD04982 are administered in a combined amount of about 300-400 mg per dose administration and in the ratio of about 2:1. In some embodiments, AD04872 and AD04982 are administered in a combined amount of about 35 mg per dose administration and in the ratio of about 2:1. In some embodiments, AD04872 and AD04982 are administered in a combined amount of about 40 mg per dose administration and in the ratio of about 2:1. In some embodiments, AD04872 and AD04982 are administered in a combined amount of about 100 mg per dose administration and in the ratio of about 2:1. In some embodiments, AD04872 and AD04982 are administered in a combined amount of about 200 mg per dose administration and in the ratio of about 2:1. In some embodiments, AD04872 and AD04982 are administered in a combined amount of about 300 mg per dose administration and in the ratio of about 2:1. In some embodiments, AD04872 and AD04982 are administered in a combined amount of about 400 mg per dose administration and in the ratio of about 2:1. In some embodiments, AD04872 and AD04982 are administered in about 7-day, about 14-day, about 21-day or about 28-day intervals. In some embodiments, AD04872 and AD04982 are administered in 28-day intervals or about 28-day intervals (i.e., Q4W). In some embodiments, AD04872 and AD04982 are administered in a combined amount of about 40 mg, about 100 mg or about 200 mg per dose administration, in the ratio of about 2:1, and in 28-day intervals or about 28-day intervals (i.e., Q4W). In some embodiments, AD04872 and AD04982 are administered in a combined amount of about 40 mg per dose administration, in the ratio of about 2:1, and in 28-day intervals or about 28-day intervals (i.e., Q4W). In some embodiments, AD04872 and AD04982 are administered in a combined amount of about 100 mg per dose administration, in the ratio of about 2:1, and in 28-day intervals or about 28-day intervals (i.e., Q4W). In some embodiments, AD04872 and AD04982 are administered in a combined amount of about 200 mg per dose administration, in the ratio of about 2:1, and in 28-day intervals or about 28-day intervals (i.e., Q4W).

In some embodiments, AD04872 is administered in an amount of about 3-650 mg, and AD04982 is administered in an amount of about 2-325 mg per dose administration. In some embodiments, AD04872 is administered in an amount of about 35-265 mg per dose administration. In some embodiments, AD04872 is administered in an amount of about 50-75 mg per dose administration. In some embodiments, AD04982 is administered in an amount of about 20-125 mg per dose administration. In some embodiments, AD04982 is administered in an amount of about 25-50 mg per dose administration.

In some embodiments, AD04872 and AD04982 are administered in a combined amount of about 1-10 mg/kg per dose administration. In some embodiments, AD04872 and AD04982 are administered in a combined amount of about 1-5 mg/kg per dose administration. In some embodiments, AD04872 and AD04982 are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, AD04982 is administered in an amount of about 0.3-5 mg/kg per dose administration, and AD04872 is administered in an amount of about 0.6-7 mg/kg per dose administration. In some embodiments, AD04982 is administered in an amount of about 0.5-2.5 mg/kg per dose administration. In some embodiments, AD04982 is administered in an amount of about 0.3-1.5 mg/kg per dose administration. In some embodiments, AD04872 is administered in an amount of about 0.6-5 mg/kg per dose administration. In some embodiments, AD04872 is administered in an amount of about 1-2.5 mg/kg per dose administration.

In some embodiments, AD04872 and AD04982 are administered at a combined dose of 25-400 mg per dose administration. In an embodiment, AD04872 and AD04982 are administered at a combined dose of 25-400 mg, and AD04872 is administered with AD04982 at a ratio of 1:1. In an embodiment, the dose of each of AD04872 and AD04982 is in an amount of about 12 mg for a combined dose of about 25 mg. In an embodiment, the dose of each of AD04872 and AD04982 is in an amount of about 17 mg for a combined dose of about 35 mg. In an embodiment, the dose of each of AD04872 and AD04982 is in an amount of about 20 mg for a combined dose of about 40 mg. In an embodiment, the dose of each of AD04872 and AD04982 is in an amount of about 25 mg for a combined dose of about 50 mg. In an embodiment, the dose of each of AD04872 and AD04982 is in an amount of about 50 mg for a combined dose of about 100 mg. In an embodiment, the dose of each of AD04872 and AD04982 is in an amount of about 100 mg for a combined dose of about 200 mg. In an embodiment, the dose of each of AD04872 and AD04982 is in an amount of about 150 mg for a combined dose of about 300 mg. In an embodiment, the dose of each of AD04872 and AD04982 is in an amount of about 200 mg for a combined dose of about 400 mg. In some embodiments, AD04872 and AD04982 are administered in about 7-day, about 14-day, about 21-day or about 28-day intervals. In some embodiments, AD04872 and AD04982 are administered in 28-day intervals or about 28-day intervals (i.e., Q4W). In an embodiment, the dose of each of AD04872 and AD04982 is in an amount of about 20 mg for a combined dose of about 40 mg, or in an amount of about 50 mg for a combined dose of about 100 mg, or in an amount of about 100 mg for a combined dose of about 200 mg, and AD04872 and AD04982 are administered in 28-day intervals or about 28-day intervals (i.e., Q4W). In an embodiment, the dose of each of AD04872 and AD04982 is in an amount of about 20 mg for a combined dose of about 40 mg and AD04872 and AD04982 are administered in 28-day intervals or about 28-day intervals (i.e., Q4W). In an embodiment, the dose of each of AD04872 and AD04982 is in an amount of about 50 mg for a combined dose of about 100 mg and AD04872 and AD04982 are administered in 28-day intervals or about 28-day intervals (i.e., Q4W). In an embodiment, the dose of each of AD04872 and AD04982 is in an amount of about 100 mg for a combined dose of about 200 mg and AD04872 and AD04982 are administered in 28-day intervals or about 28-day intervals (i.e., Q4W).

In an embodiment, AD04872 and AD04982 are administered at a combined dose of 25-400 mg per dose, and AD04982 is administered with AD04872 at a ratio of 1:2. In an embodiment, the dose of AD04872 is in an amount of about 16 mg, and the dose of AD04982 is in an amount of about 8 mg for a combined dose of about 25 mg. In an embodiment, the dose of AD04982 is in an amount of about 12 mg, and the dose of the AD04872 is in an amount of about 24 mg for a combined dose of about 35 mg. In an embodiment, the dose of AD04982 is in an amount of about 13 mg, and the dose of the AD04872 is in an amount of about 27 mg for a combined dose of about 40 mg. In an embodiment, the dose of AD04872 is in an amount of about 33 mg, and the dose of AD04982 is in an amount of about 17 mg for a combined dose of about 50 mg. In an embodiment, the dose of AD04982 is in an amount of about 35 mg, and the dose of AD04872 is in an amount of about 65 mg for a combined dose of about 100 mg. In an embodiment, the dose of AD04982 is in an amount of about 67 mg, and the dose of AD04872 is in an amount of about 133 mg for a combined dose of about 200 mg. In an embodiment, the dose of the AD04982 is in an amount of about 100 mg, and the dose of AD04872 is in an amount of about 200 mg for a combined dose of about 300 mg. In an embodiment, the dose of AD04982 is in an amount of about 135 mg, and the dose of AD04872 is in an amount of about 270 mg for a combined dose of about 400 mg. In some embodiments, AD04872 and AD04982 are administered in about 7-day, about 14-day, about 21-day or about 28-day intervals. In some embodiments, AD04872 and AD04982 are administered in 28-day intervals or about 28-day intervals (i.e., Q4W). In an embodiment, the dose of AD04982 is in an amount of about 13 mg, and the dose of the AD04872 is in an amount of about 27 mg for a combined dose of about 40 mg, and AD04872 and AD04982 are administered in 28-day intervals or about 28-day intervals (i.e., Q4W). In an embodiment, the dose of AD04982 is in an amount of about 35 mg, and the dose of AD04872 is in an amount of about 65 mg for a combined dose of about 100 mg, and AD04872 and AD04982 are administered in 28-day intervals or about 28-day intervals (i.e., Q4W). In an embodiment, the dose of AD04982 is in an amount of about 67 mg, and the dose of AD04872 is in an amount of about 133 mg for a combined dose of about 200 mg, and AD04872 and AD04982 are administered in 28-day intervals or about 28-day intervals (i.e., Q4W).

In an embodiment, AD04872 and AD04982 are administered at a combined dose of 25-400 mg per dose, AD04982 is administered with AD04872 at a ratio of 1:3. In an embodiment, the dose of AD04872 is in an amount of about 18 mg, and the dose of AD04982 is in an amount of about 6 mg for a combined dose of about 25 mg. In an embodiment, the dose of AD04982 is in an amount of about 9 mg, and the dose of AD04872 is in an amount of about 27 mg for a combined dose of about 35 mg. In an embodiment, the dose of AD04982 is in an amount of about 10 mg, and the dose of AD04872 is in an amount of about 30 mg for a combined dose of about 40 mg. In an embodiment, the dose of AD04872 is in an amount of about 36 mg, and the dose of AD04982 is in an amount of about 12 mg for a combined dose of about 50 mg. In an embodiment, the dose of AD04982 is in an amount of about 25 mg, and the dose of AD04872 is in an amount of about 75 mg for a combined dose of about 100 mg. In an embodiment, the dose of AD04982 is in an amount of about 50 mg, and the dose of AD04872 is in an amount of about 150 mg for a combined dose of about 200 mg. In an embodiment, the dose of AD04982 is in an amount of about 75 mg, and the dose of AD04872 is in an amount of about 225 mg for a combined dose of about 300 mg. In an embodiment, the dose of AD04982 is in an amount of about 100 mg, and the dose of AD04872 is in an amount of about 300 mg for a combined dose of about 400 mg. In some embodiments, AD04872 and AD04982 are administered in about 7-day, about 14-day, about 21-day or about 28-day intervals. In some embodiments, AD04872 and AD04982 are administered in 28-day intervals or about 28-day intervals (i.e., Q4W). In an embodiment, the dose of AD04982 is in an amount of about 10 mg, and the dose of AD04872 is in an amount of about 30 mg for a combined dose of about 40 mg, and AD04872 and AD04982 are administered in 28-day intervals or about 28-day intervals (i.e., Q4W). In an embodiment, the dose of AD04982 is in an amount of about 25 mg, and the dose of AD04872 is in an amount of about 75 mg for a combined dose of about 100 mg, and AD04872 and AD04982 are administered in 28-day intervals or about 28-day intervals (i.e., Q4W). In an embodiment, the dose of AD04982 is in an amount of about 50 mg, and the dose of AD04872 is in an amount of about 150 mg for a combined dose of about 200 mg, and AD04872 and AD04982 are administered in 28-day intervals or about 28-day intervals (i.e., Q4W).

In some embodiments, about 1 mg/kg (mpk) of AD04872 and about 1 mg/kg of AD04982 are administered to a subject in need thereof. In some embodiments, about 1.5 mg/kg of AD04872 and about 1.5 mg/kg of AD04982 are administered to a subject in need thereof. In some embodiments, about 2.0 mg/kg of AD04872 and about 1.0 mg/kg of AD04982 are administered to a subject in need thereof. In some embodiments, about 3.0 mg/kg of AD04872 and about 1.0 mg/kg of AD04982 are administered to a subject in need thereof. In some embodiments, about 3.2 mg/kg of AD04872 and about 0.8 mg/kg of AD04982 are administered to a subject in need thereof. In some embodiments, about 2.7 mg/kg of AD04872 and about 1.3 mg/kg of AD04982 are administered to a subject in need thereof. In some embodiments, about 4.0 mg/kg of AD04872 and about 1.0 mg/kg of AD04982 are administered to a subject in need thereof. In some embodiments, about 3.3 mg/kg of AD04872 and about 1.7 mg/kg of AD04982 are administered to a subject in need thereof. In some embodiments, between about 0.05 and about 5 mg/kg of AD04872 and between about 0.05 and about 5 mg/kg of AD04982 are administered to a subject in need thereof. In some embodiments, the respective doses of AD04872 and AD04982 are administered separately (e.g., in separate injections). In some embodiments, the respective doses of AD04872 and AD04982 are administered together (e.g., in the same injection). In some embodiments, the respective doses of AD04872 and AD04982 are prepared in a single pharmaceutical composition.

In some embodiments, disclosed herein are methods of treatment of an HBV infection or prevention of disease or symptoms caused by an HBV infection comprising administering to a subject in need thereof an effective amount of AD04580 and an effective amount of AD04585. In some embodiments, the ratio of AD04580 to AD04585 administered to a subject in need thereof is about 2:1. In some embodiments, the ratio of AD04580 to AD04585 administered to a subject in need thereof is about 3:1. In some embodiments, the ratio of AD04580 to AD04585 administered to a subject in need thereof is about 4:1. In some embodiments, the ratio of AD04580 to AD04585 administered to a subject in need thereof is about 5:1. In some embodiments, the ratio of AD04580 to AD04585 administered to a subject in need thereof is about 1:1. In some embodiments, the ratio of AD04580 to AD04585 administered to a subject in need thereof is about 1:2. In some embodiments, about 1 mg/kg (mpk) of AD04580 and about 1 mg/kg of AD04585 are administered to a subject in need thereof. In some embodiments, about 1.5 mg/kg of AD04580 and about 1.5 mg/kg of AD04585 are administered to a subject in need thereof. In some embodiments, between about 0.05 and about 5 mg/kg of AD04580 and between about 0.05 and about 5 mg/kg of AD04585 are administered to a subject in need thereof.

In some embodiments, disclosed herein are methods of treating or preventing a disease associated with an infection caused by HBV, comprising administering to a subject in need thereof an effective amount of AD04580 and an effective amount of AD04585. In some embodiments, the ratio of AD04580 to AD04585 administered to a subject in need thereof is about 2:1. In some embodiments, the ratio of AD04580 to AD04585 administered to a subject in need thereof is about 3:1. In some embodiments, the ratio of AD04580 to AD04585 administered to a subject in need thereof is about 4:1. In some embodiments, the ratio of AD04580 to AD04585 administered to a subject in need thereof is about 5:1. In some embodiments, the ratio of AD04580 to AD04585 administered to a subject in need thereof is about 1:1. In some embodiments, the ratio of AD04580 to AD04585 administered to a subject in need thereof is about 1:2. In some embodiments, about 1 mg/kg (mpk) of AD04580 and about 1 mg/kg of AD04585 are administered to a subject in need thereof. In some embodiments, about 1.5 mg/kg of AD04580 and about 1.5 mg/kg of AD04585 are administered to a subject in need thereof. In some embodiments, between about 0.05 and about 5 mg/kg of AD04580 and between about 0.05 and about 5 mg/kg of AD04585 are administered to a subject in need thereof.

In some embodiments, disclosed herein are methods of treating a disease associated with an infection caused by HBV, comprising administering to a subject in need thereof an effective amount of AD04580 and an effective amount of AD04585. In some embodiments, the ratio of AD04580 to AD04585 administered to a subject in need thereof is about 2:1. In some embodiments, the ratio of AD04580 to AD04585 administered to a subject in need thereof is about 3: 1. In some embodiments, the ratio of AD04580 to AD04585 administered to a subject in need thereof is about 4:1. In some embodiments, the ratio of AD04580 to AD04585 administered to a subject in need thereof is about 5:1. In some embodiments, the ratio of AD04580 to AD04585 administered to a subject in need thereof is about 1:1. In some embodiments, the ratio of AD04580 to AD04585 administered to a subject in need thereof is about 1:2. In some embodiments, about 1 mg/kg (mpk) of AD04580 and about 1 mg/kg of AD04585 are administered to a subject in need thereof. In some embodiments, about 1.5 mg/kg of AD04580 and about 1.5 mg/kg of AD04585 are administered to a subject in need thereof. In some embodiments, between about 0.05 and about 5 mg/kg of AD04580 and between about 0.05 and about 5 mg/kg of AD04585 are administered to a subject in need thereof.

In some embodiments, AD04580 and AD04585 are administered in a combined amount of about 25-400 mg. In some embodiments, AD04580 and AD04585 are administered in a combined amount of about 50 mg, about 75 mg, about 100 mg, or about 125 mg per dose administration. In some embodiments, AD04580 and AD04585 are administered in a combined amount of about 40 mg, about 100 mg, or about 200 mg per dose administration. In some embodiments, AD04580 and AD04585 are administered in a combined amount of about 40 mg per dose administration. In some embodiments, AD04580 and AD04585 are administered in a combined amount of about 100 mg per dose administration. In some embodiments, AD04580 and AD04585 are administered in a combined amount of about 200 mg per dose administration. In some embodiments, AD04580 and AD04585 are administered in about 1-18 week intervals. In some embodiments, AD04580 and AD04585 are administered in about 1-6 month intervals. In some embodiments, AD04580 and AD04585 are administered in about 4-week intervals or 1-month intervals. In some embodiments, AD04580 and AD04585 are administered in about 7-day, 14-day, 21-day or 28-day intervals. In some embodiments, AD04580 and AD04585 are administered in 28-day intervals or about 28-day intervals. In some embodiments, AD04580 and AD04585 are administered in a combined amount of about 40 mg, about 100 mg or about 200 mg per dose administration in 28-day intervals or about 28-day intervals (i.e., Q4W). In some embodiments, AD04580 and AD04585 are administered in a combined amount of about 40 mg per dose administration in 28-day intervals or about 28-day intervals (i.e., Q4W). In some embodiments, AD04580 and AD04585 are administered in a combined amount of about 100 mg per dose administration in 28-day intervals or about 28-day intervals (i.e., Q4W). In some embodiments, AD04580 and AD04585 are administered in a combined amount of about 200 mg per dose administration in 28-day intervals or about 28-day intervals (i.e., Q4W).

In some embodiments, AD04580 and AD04585 are administered for a duration of about 1-12 months. In some embodiments, AD04580 and AD04585 are administered for a duration of about 3 months. In some embodiments, AD04580 and AD04585 are administered in a combined amount of about 25-400 mg per dose administration and in the ratio of about 2:1, about 3:1, about 1:1, about 4:1, about 5:1 or about 1:2. In some embodiments, AD04580 and AD04585 are administered in a combined amount of about 100 mg per dose administration and in the ratio of about 2:1.

In some embodiments, AD04580 is administered in an amount of about 3-650 mg per dose administration, and AD04585 is administered in an amount of about 2-325 mg per dose administration. In some embodiments, AD04580 is administered in an amount of about 35-265 mg per dose administration. In some embodiments, AD04580 is administered in an amount of about 50-75 mg per dose administration. In some embodiments, AD04585 is administered in an amount of about 20-125 mg per dose administration. In some embodiments, AD04585 is administered in the amount of about 25-50 mg per dose administration.

In some embodiments, AD04580 and AD04585 are administered in a combined amount of about 1-10 mg/kg per dose administration. In some embodiments, AD04580 and AD04585 are administered in a combined amount of about 1-5 mg/kg per dose administration. In some embodiments, AD04580 and AD04585 are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, AD04585 is administered in an amount of about 0.3-5 mg/kg per dose administration, and AD04580 is administered in an amount of about 0.6-7 mg/kg per dose administration. In some embodiments, AD04585 is administered in an amount of about 0.5-2.5 mg/kg per dose administration. In some embodiments, AD04585 is administered in an amount of about 0.3-1.5 mg/kg per dose administration. In some embodiments, AD04580 is administered in an amount of about 0.6-5 mg/kg per dose administration. In some embodiments, AD04580 is administered in an amount of about 1-2.5 mg/kg per dose administration.

In some embodiments, AD04580 and AD04585 are administered at a combined dose of 25-400 mg per dose administration. In an embodiment, AD04580 and AD04585 are administered at a combined dose of 25-400 mg, and AD04580 is administered with AD04585 at a ratio of 1:1. In an embodiment, the dose of each of AD04580 and AD04585 is in an amount of about 12 mg for a combined dose of about 25 mg. In an embodiment, the dose of each of AD04580 and AD04585 is in an amount of about 17 mg for a combined dose of about 35 mg. In an embodiment, the dose of each of AD04580 and AD04585 is in an amount of about 20 mg for a combined dose of about 40 mg. In an embodiment, the dose of each of AD04580 and AD04585 is in an amount of about 25 mg for a combined dose of about 50 mg. In an embodiment, the dose of each of AD04580 and AD04585 is in an amount of about 50 mg for a combined dose of about 100 mg. In an embodiment, the dose of each of AD04580 and AD04585 is in an amount of about 100 mg for a combined dose of about 200 mg. In an embodiment, the dose of each of AD04580 and AD04585 is in an amount of about 150 mg for a combined dose of about 300 mg. In an embodiment, the dose of each of AD04580 and AD04585 is in an amount of about 200 mg for a combined dose of about 400 mg. In some embodiments, AD04580 and AD04585 are administered in about 7-day, about 14-day, about 21-day or about 28-day intervals. In some embodiments, AD04580 and AD04585 are administered in 28-day intervals or about 28-day intervals (i.e., Q4W). In an embodiment, the dose of each of AD04580 and AD04585 is in an amount of about 20 mg for a combined dose of about 40 mg, or in an amount of about 50 mg for a combined dose of about 100 mg, or in an amount of about 100 mg for a combined dose of about 200 mg, and AD04580 and AD04585 are administered in 28-day intervals or about 28-day intervals (i.e., Q4W). In an embodiment, the dose of each of AD04580 and AD04585 is in an amount of about 20 mg for a combined dose of about 40 mg and AD04580 and AD04585 are administered in 28-day intervals or about 28-day intervals (i.e., Q4W). In an embodiment, the dose of each of AD04580 and AD04585 is in an amount of about 50 mg for a combined dose of about 100 mg and AD04580 and AD04585 are administered in 28-day intervals or about 28-day intervals (i.e., Q4W). In an embodiment, the dose of each of AD04580 and AD04585 is in an amount of about 100 mg for a combined dose of about 200 mg and AD04580 and AD04585 are administered in 28-day intervals or about 28-day intervals (i.e., Q4W).

In an embodiment, AD04580 and AD04585 are administered at a combined dose of 25-400 mg per dose, and AD04585 is administered with AD04580 at a ratio of 1:2. In an embodiment, the dose of AD04580 is in an amount of about 16 mg, and the dose of AD04585 is in an amount of about 8 mg for a combined dose of about 25 mg. In an embodiment, the dose of AD04585 is in an amount of about 12 mg, and the dose of the AD04580 is in an amount of about 24 mg for a combined dose of about 35 mg. In an embodiment, the dose of AD04585 is in an amount of about 13 mg, and the dose of the AD04580 is in an amount of about 27 mg for a combined dose of about 40 mg. In an embodiment, the dose of AD04580 is in an amount of about 33 mg, and the dose of AD04585 is in an amount of about 17 mg for a combined dose of about 50 mg. In an embodiment, the dose of AD04585 is in an amount of about 35 mg, and the dose of AD04580 is in an amount of about 65 mg for a combined dose of about 100 mg. In an embodiment, the dose of AD04585 is in an amount of about 67 mg, and the dose of AD04580 is in an amount of about 133 mg for a combined dose of about 200 mg. In an embodiment, the dose of the AD04585 is in an amount of about 100 mg, and the dose of AD04580 is in an amount of about 200 mg for a combined dose of about 300 mg. In an embodiment, the dose of AD04585 is in an amount of about 135 mg, and the dose of AD04580 is in an amount of about 270 mg for a combined dose of about 400 mg. In some embodiments, AD04580 and AD04585 are administered in about 7-day, about 14-day, about 21-day or about 28-day intervals. In some embodiments, AD04580 and AD04585 are administered in 28-day intervals or about 28-day intervals (i.e., Q4W). In an embodiment, the dose of AD04585 is in an amount of about 13 mg, and the dose of the AD04580 is in an amount of about 27 mg for a combined dose of about 40 mg, and AD04580 and AD04585 are administered in 28-day intervals or about 28-day intervals (i.e., Q4W). In an embodiment, the dose of AD04585 is in an amount of about 35 mg, and the dose of AD04580 is in an amount of about 65 mg for a combined dose of about 100 mg, and AD04580 and AD04585 are administered in 28-day intervals or about 28-day intervals (i.e., Q4W). In an embodiment, the dose of AD04585 is in an amount of about 67 mg, and the dose of AD04580 is in an amount of about 133 mg for a combined dose of about 200 mg, and AD04580 and AD04585 are administered in 28-day intervals or about 28-day intervals (i.e., Q4W).

In an embodiment, AD04580 and AD04585 are administered at a combined dose of 25-400 mg per dose, AD04585 is administered with AD04580 at a ratio of 1:3. In an embodiment, the dose of AD04580 is in an amount of about 18 mg, and the dose of AD04585 is in an amount of about 6 mg for a combined dose of about 25 mg. In an embodiment, the dose of AD04585 is in an amount of about 9 mg, and the dose of AD04580 is in an amount of about 27 mg for a combined dose of about 35 mg. In an embodiment, the dose of AD04585 is in an amount of about 10 mg, and the dose of AD04580 is in an amount of about 30 mg for a combined dose of about 40 mg. In an embodiment, the dose of AD04580 is in an amount of about 36 mg, and the dose of AD04585 is in an amount of about 12 mg for a combined dose of about 50 mg. In an embodiment, the dose of AD04585 is in an amount of about 25 mg, and the dose of AD04580 is in an amount of about 75 mg for a combined dose of about 100 mg. In an embodiment, the dose of AD04585 is in an amount of about 50 mg, and the dose of AD04580 is in an amount of about 150 mg for a combined dose of about 200 mg. In an embodiment, the dose of AD04585 is in an amount of about 75 mg, and the dose of AD04580 is in an amount of about 225 mg for a combined dose of about 300 mg. In an embodiment, the dose of AD04585 is in an amount of about 100 mg, and the dose of AD04580 is in an amount of about 300 mg for a combined dose of about 400 mg. In some embodiments, AD04580 and AD04585 are administered in about 7-day, about 14-day, about 21-day or about 28-day intervals. In some embodiments, AD04580 and AD04585 are administered in 28-day intervals or about 28-day intervals (i.e., Q4W). In an embodiment, the dose of AD04585 is in an amount of about 10 mg, and the dose of AD04580 is in an amount of about 30 mg for a combined dose of about 40 mg, and AD04580 and AD04585 are administered in 28-day intervals or about 28-day intervals (i.e., Q4W). In an embodiment, the dose of AD04585 is in an amount of about 25 mg, and the dose of AD04580 is in an amount of about 75 mg for a combined dose of about 100 mg, and AD04580 and AD04585 are administered in 28-day intervals or about 28-day intervals (i.e., Q4W). In an embodiment, the dose of AD04585 is in an amount of about 50 mg, and the dose of AD04580 is in an amount of about 150 mg for a combined dose of about 200 mg, and AD04580 and AD04585 are administered in 28-day intervals or about 28-day intervals (i.e., Q4W).

Provided herein is a method for inhibiting expression of a Hepatitis B Virus gene in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO: 111, SEQ ID NO:126, SEQ ID NO:127, SEQ ID NO:128, SEQ ID NO:171, SEQ ID NO: 175, SEQ ID NO: 179 and SEQ ID NO: 180, and
   (ii) a sense strand comprising a nucleotide sequence of any one of the following: SEQ ID NO:229, SEQ ID NO: 235, SEQ ID NO:252, SEQ ID NO:253, SEQ ID NO:273, SEQ ID NO: 307, SEQ ID NO:302 and SEQ ID NO:319, and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 50-400 mg per month.

Also provided herein is A method for treating a disease, disorder, or condition associated with an infection caused by Hepatitis B Virus in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO:126, SEQ ID NO:127, SEQ ID NO:128, SEQ ID NO:171, SEQ ID NO: 179 and SEQ ID NO: 180, and
   (ii) a sense strand comprising a nucleotide sequence of any one of the following: SEQ ID NO:229, SEQ ID NO:252, SEQ ID NO:253, SEQ ID NO:273, SEQ ID NO:302 and SEQ ID NO:319, and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in an amount of about 50-400 mg per month.

In some embodiments, the first and the second HBV RNAi agents are administered in a ratio of about 1:1, 2:1, 3:1, 4:1 or 5:1. In some embodiments, the first and the second HBV RNAi agents are administered in a ratio of about 2:1.

In some embodiments, the first and the second HBV RNAi agents are administered in a combined amount of about 25-75 mg per dose administration and in the ratio of about 2:1, about 3:1, about 1:1, about 4:1, about 5:1 or about 1:2. In some embodiments, the first and the second HBV RNAi agents are administered in a combined amount of about 50-125 mg per dose administration and in the ratio of about 2:1, about 3:1, about 1:1, about 4:1, about 5:1 or about 1:2. In some embodiments, the first and the second HBV RNAi agents are administered in a combined amount of about 75-150 mg per dose administration and in the ratio of about 2:1, about 3:1, about 1:1, about 4:1, about 5:1 or about 1:2. In some embodiments, the first and the second HBV RNAi agents are administered in a combined amount of about 100-200 mg per dose administration and in the ratio of about 2:1, about 3:1, about 1:1, about 4:1, about 5:1 or about 1:2. In some embodiments, the first and the second HBV RNAi agents are administered in a combined amount of about 150-250 mg per dose administration and in the ratio of about 2:1, about 3:1, about 1:1, about 4:1, about 5:1 or about 1:2. In some embodiments, the first and the second HBV RNAi agents are administered in a combined amount of about 200-300 mg per dose administration and in the ratio of about 2:1, about 3:1, about 1:1, about 4:1, about 5:1 or about 1:2. In some embodiments, the first and the second HBV RNAi agents are administered in a combined amount of about 300-400 mg per dose administration and in the ratio of about 2:1, about 3:1, about 1:1, about 4:1, about 5:1 or about 1:2. In some embodiments, the first and the second HBV RNAi agents are administered in a combined amount of about 50-100 mg per dose administration and in the ratio of about 2:1, about 3:1, about 1:1, about 4:1, about 5:1 or about 1:2. In some embodiments, the first and the second HBV RNAi agents are administered in a combined amount of about 25-400 mg per dose administration and in the ratio of about 2:1. In some embodiments, the first and the second HBV RNAi agents are administered in a combined amount of about 25-75 mg per dose administration and in the ratio of about 2:1. In some embodiments, the first and the second HBV RNAi agents are administered in a combined amount of about 50-125 mg per dose administration and in the ratio of about 2:1. In some embodiments, the first and the second HBV RNAi agents are administered in a combined amount of about 75-150 mg per dose administration and in the ratio of about 2:1. In some embodiments, the first and the second HBV RNAi agents are administered in a combined amount of about 100-200 mg per dose administration and in the ratio of about 2:1. In some embodiments, the first and the second HBV RNAi agents are administered in a combined amount of about 125-225 mg per dose administration and in the ratio of about 2:1. In some embodiments, the first and the second HBV RNAi agents are administered in a combined amount of about 150-250 mg per dose administration and in the ratio of about 2:1. In some embodiments, the first and the second HBV RNAi agents are administered in a combined amount of about 200-300 mg per dose administration and in the ratio of about 2:1. In some embodiments, the first and the second HBV RNAi agents are administered in a combined amount of about 300-400 mg per dose administration and in the ratio of about 2:1. In some embodiments, the first and the second HBV RNAi agents are administered in a combined amount of about 35 mg per dose administration and in the ratio of about 2:1. In some embodiments, the first and the second HBV RNAi agents are administered in a combined amount of about 40 mg per dose administration and in the ratio of about 2:1. In some embodiments, the first and the second HBV RNAi agents are administered in a combined amount of about 100 mg per dose administration and in the ratio of about 2:1. In some embodiments, the first and the second HBV RNAi agents are administered in a combined amount of about 200 mg per dose administration and in the ratio of about 2:1. In some embodiments, the first and the second HBV RNAi agents are administered in a combined amount of about 300 mg per dose administration and in the ratio of about 2:1. In some embodiments, the first and the second HBV RNAi agents are administered in a combined amount of about 400 mg per dose administration and in the ratio of about 2:1.

In some embodiments, the first RNAi agent is administered in the amount of about 3-650 mg per dose administration, and the second RNAi agent is administered in the amount of about 2-325 mg per dose administration. In some embodiments, the first RNAi agent is administered in the amount of about 35-265 mg per dose administration. In some embodiments, the first RNAi agent is administered in the amount of about 50-75 mg per dose administration. In some embodiments, the second RNAi agent is administered in the amount of about 20-125 mg per dose administration. In some embodiments, the second RNAi agent is administered in the amount of about 25-50 mg per dose administration.

In some embodiments, the first and the second HBV RNAi agents are administered in a combined amount of about 1-10 mg/kg per dose administration. In some embodiments, the first and the second HBV RNAi agents are administered in a combined amount of about 1-5 mg/kg per dose administration. In some embodiments, the first and the second HBV RNAi agents are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, the first RNAi agent is administered in the amount of about 0.6-7 mg/kg per dose administration, and the second RNAi agent is administered in the amount of about 0.3-5 mg/kg per dose administration. In some embodiments, the second RNAi agent is administered in the amount of about 0.5-2.5 mg/kg per dose administration. In some embodiments, the second RNAi agent is administered in the amount of about 0.3-1.5 mg/kg per dose administration. In some embodiments, the first RNAi agent is administered in the amount of about 0.6-5 mg/kg per dose administration. In some embodiments, the first RNAi agent is administered in the amount of about 1-2.5 mg/kg per dose administration.

In some embodiments, an HBV RNAi agent disclosed herein consists of or comprises AD05070 linked to (NAG37)s shown as a sodium salt having the structure represented by the following:

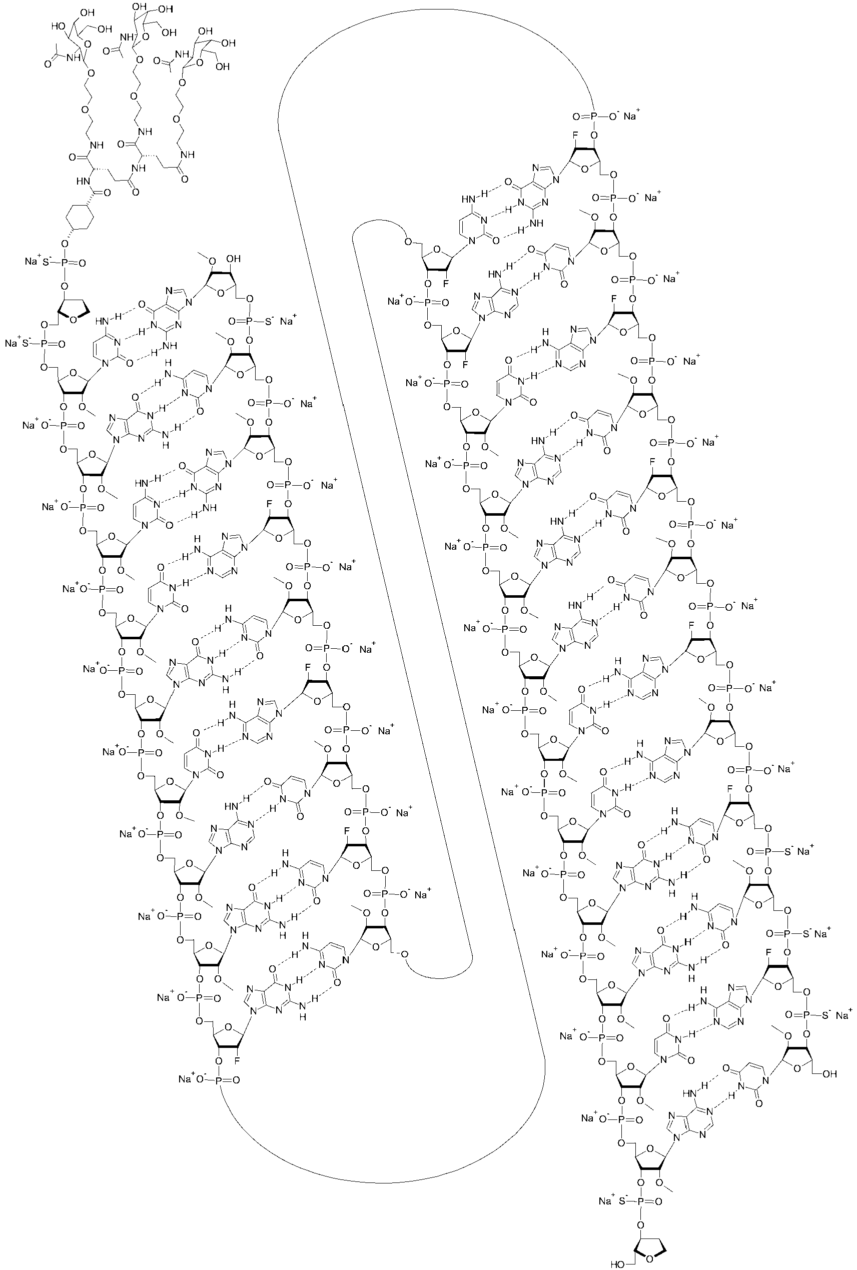

In some embodiments, an HBV RNAi agent disclosed herein consists of or comprises AD05070 linked to (NAG25)s shown as a sodium salt having the structure represented by the following:

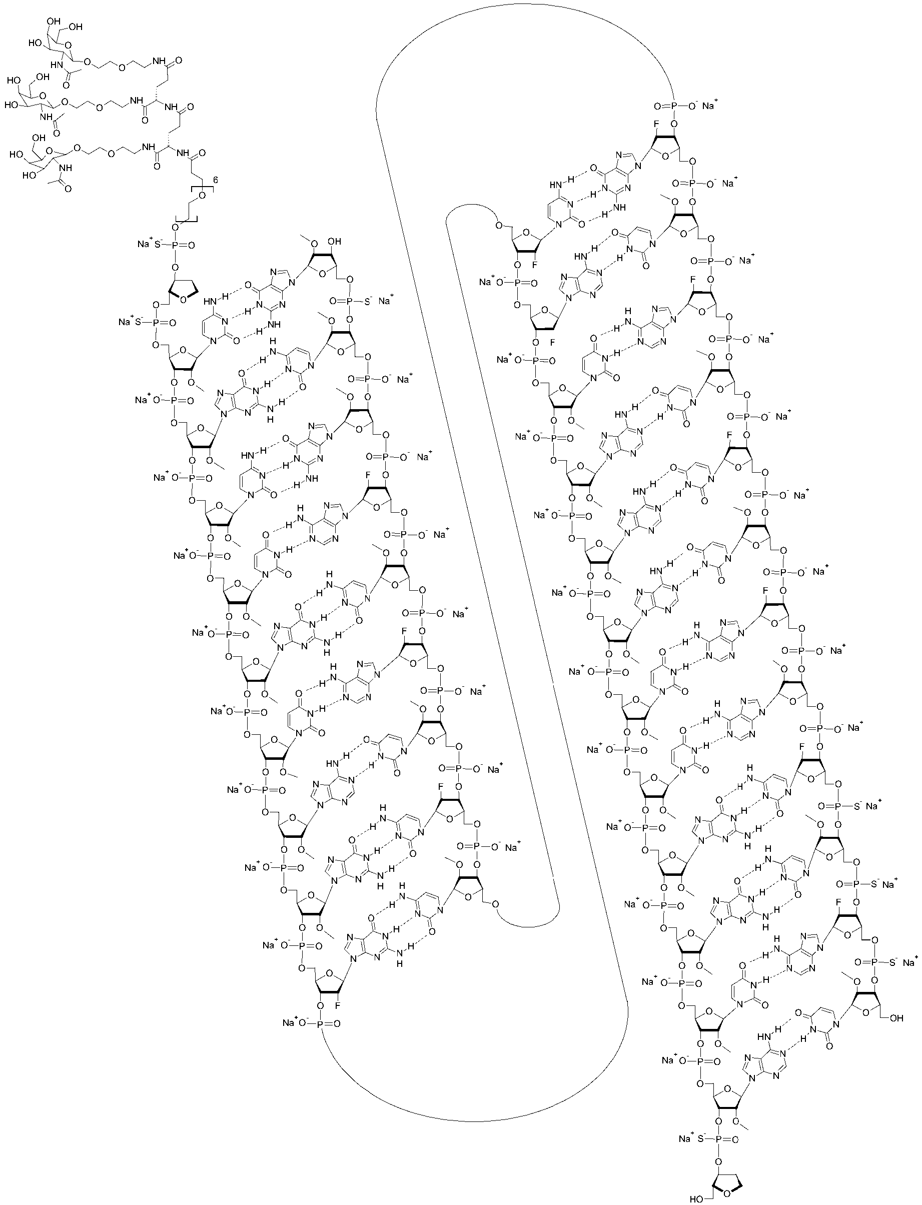

In some embodiments, an HBV RNAi agent disclosed herein consists of or comprises AD05070 linked to (NAG37)s shown as a free acid having the structure represented by the following:

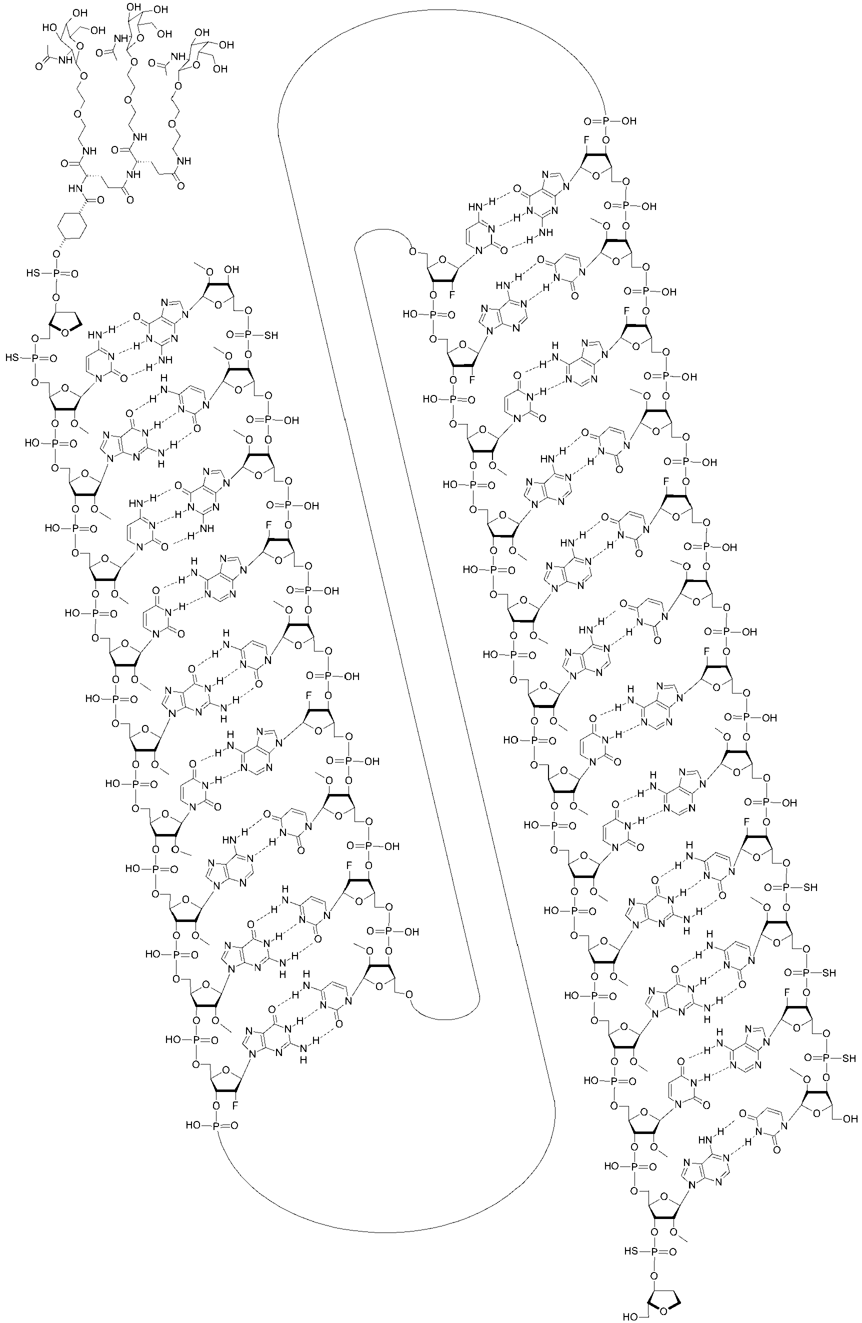

In some embodiments, an HBV RNAi agent disclosed herein consists of or comprises AD04580 linked to (NAG31)s shown as a sodium salt having the structure represented by the following:

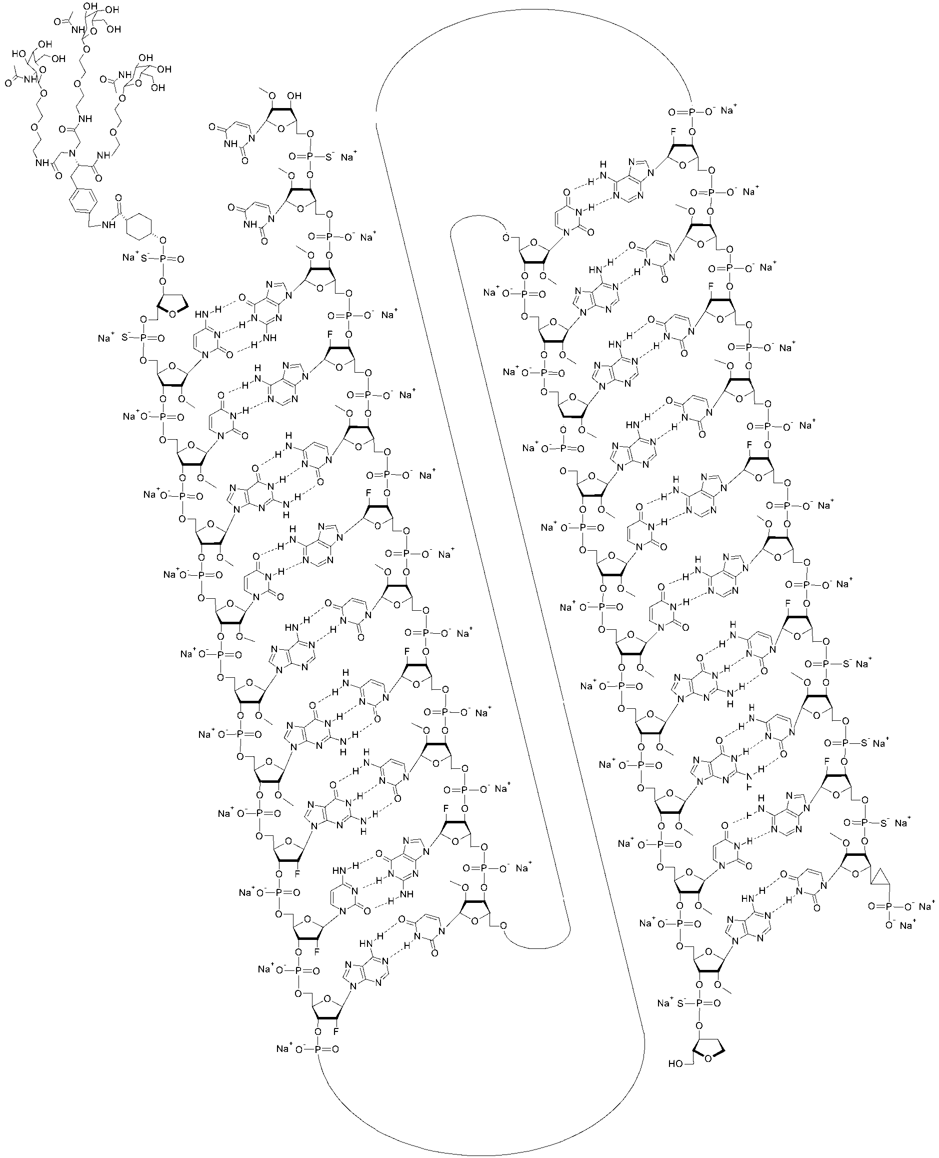

In some embodiments, an HBV RNAi agent disclosed herein consists of or comprises AD04585 linked to (NAG25)s shown as a sodium salt having the structure represented by the following:

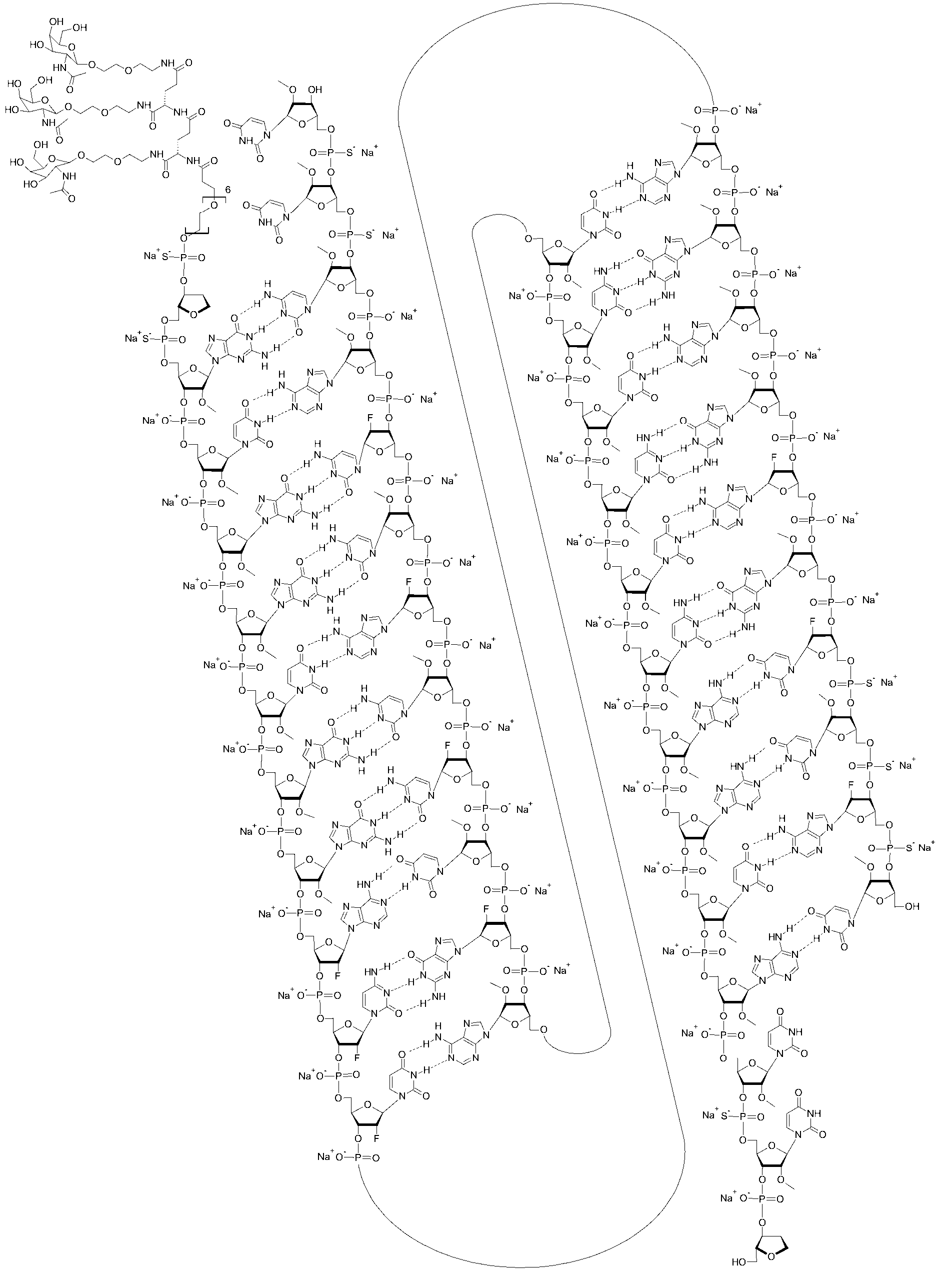

In some embodiments, an HBV RNAi agent disclosed herein consists of or comprises AD04872 linked to (NAG37)s shown as a sodium salt having the structure represented by the following:

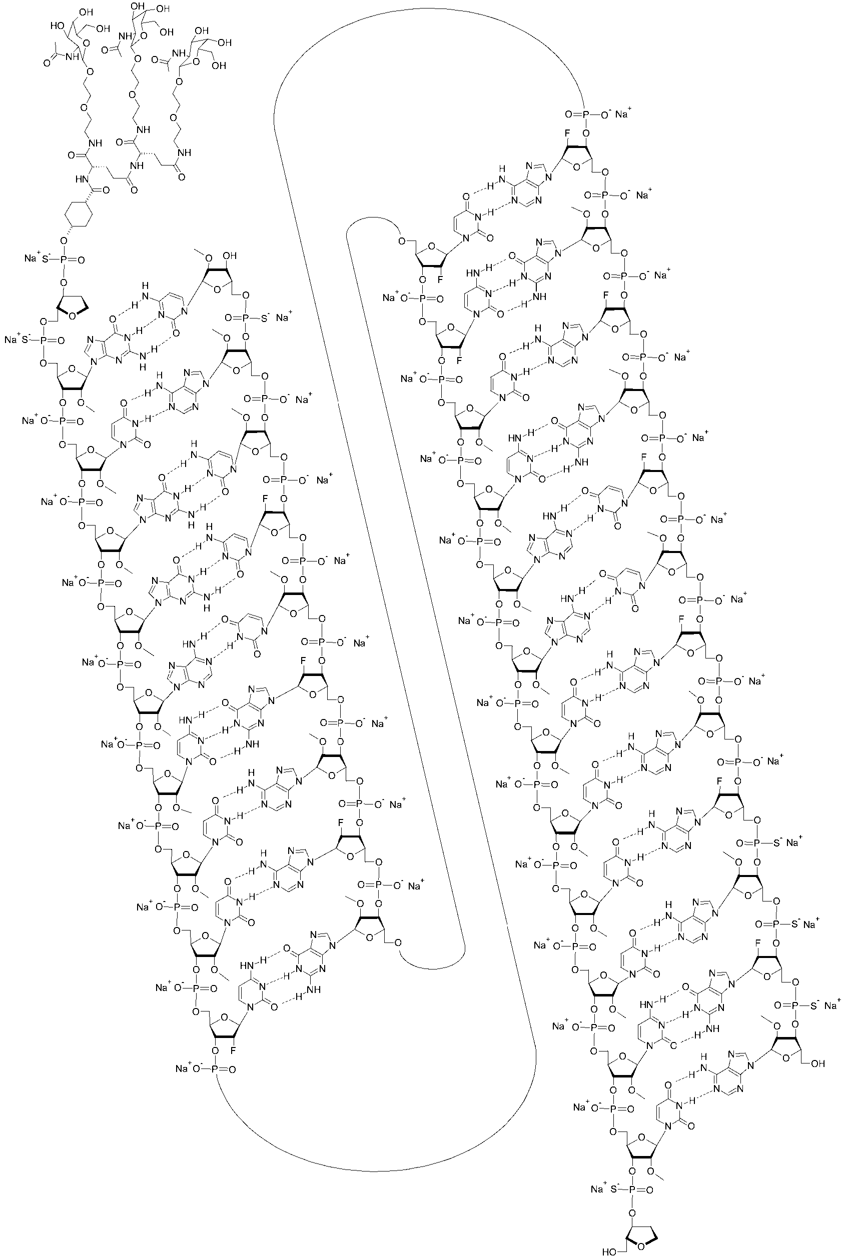

In some embodiments, an HBV RNAi agent disclosed herein consists of or comprises AD04872 linked to (NAG25)s shown as a sodium salt having the structure represented by the following:

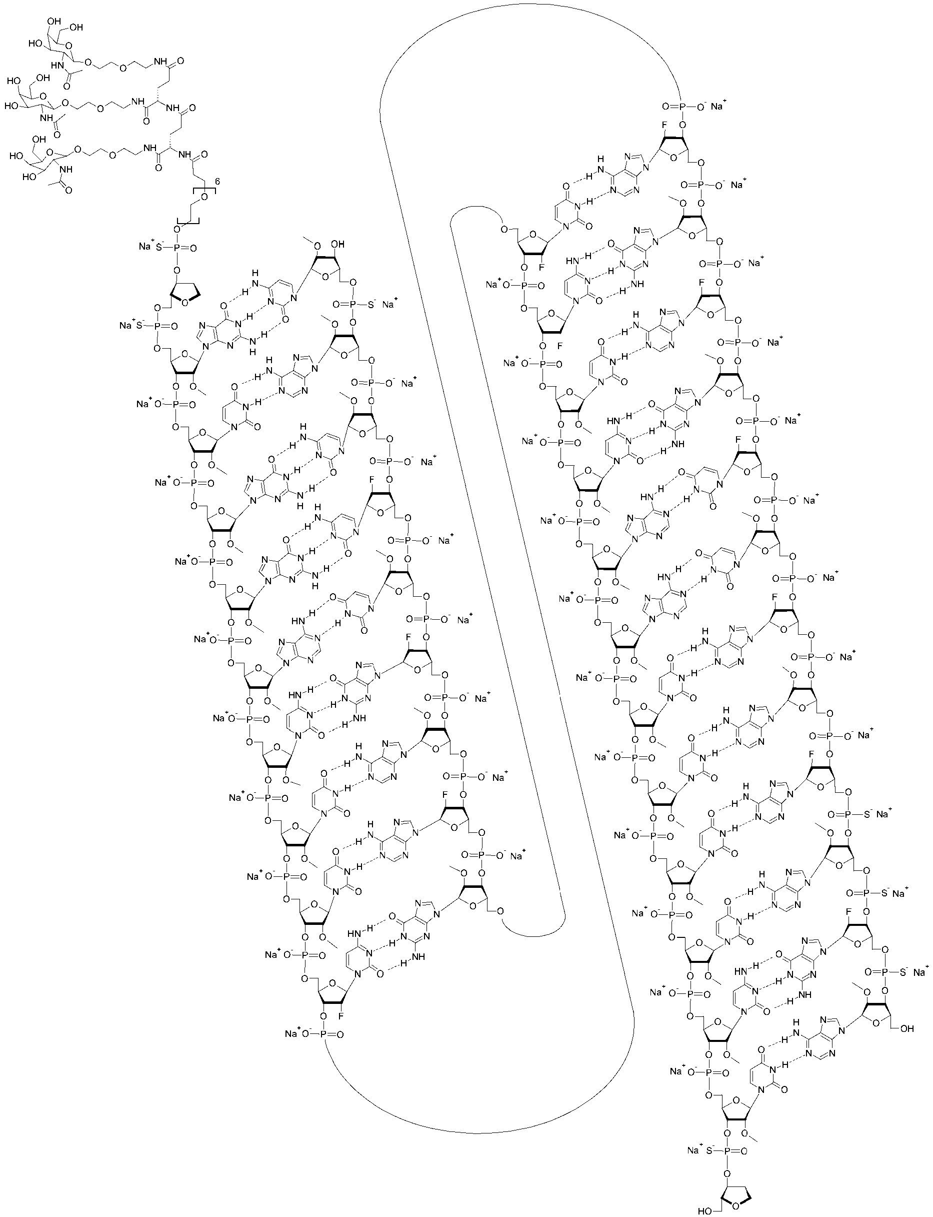

In some embodiments, an HBV RNAi agent disclosed herein consists of or comprises AD04872 linked to (NAG37)s shown as a free acid having the structure represented by the following:

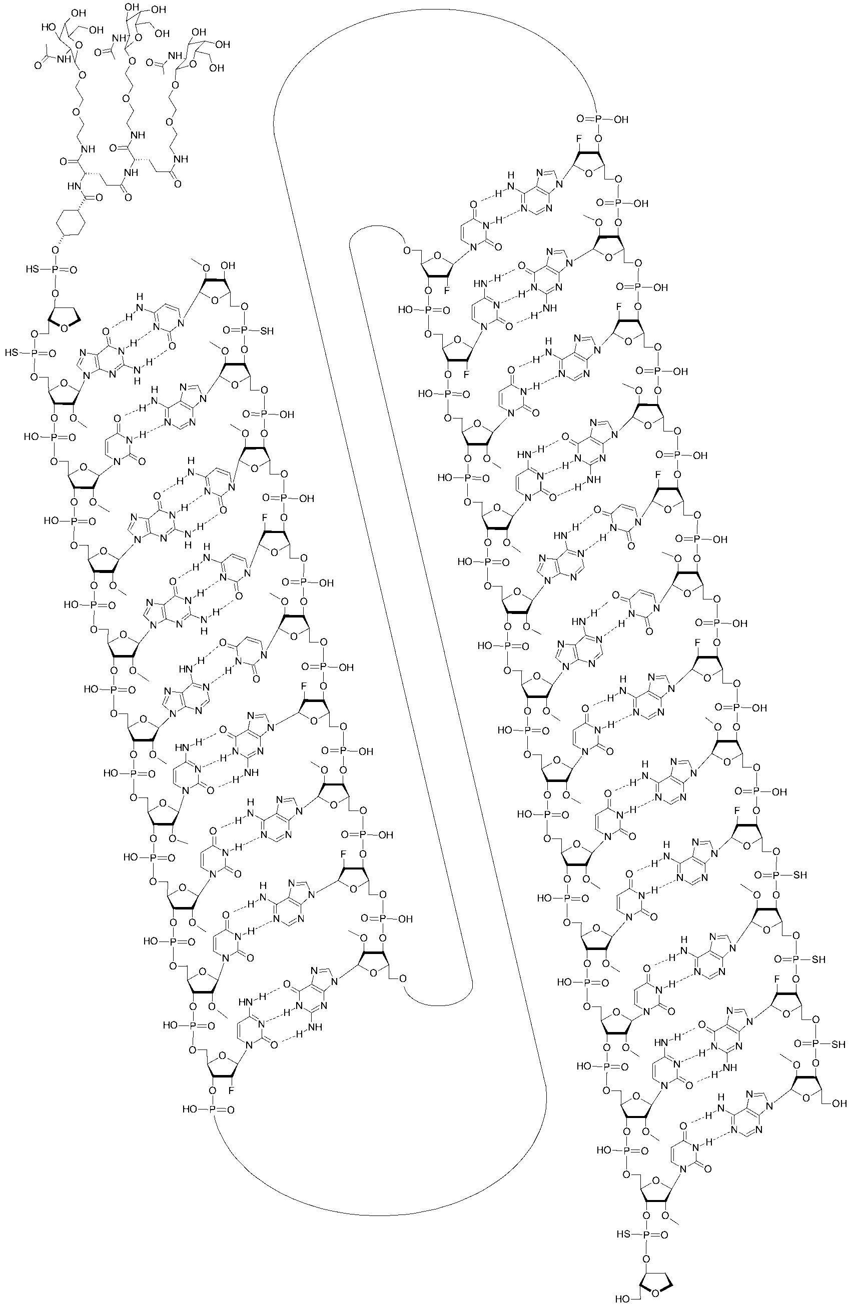

In some embodiments, the described HBV RNAi agent(s) are optionally combined with one or more additional (i.e., second, third, etc.) therapeutics. A second therapeutic can be another HBV RNAi agent (e.g., a HBV RNAi agent which targets a different sequence within an HBV genome). An additional therapeutic can also be a small molecule drug, antibody, antibody fragment, and/or vaccine. The HBV RNAi agents, with or without the one or more additional therapeutics, can be combined with one or more excipients to form pharmaceutical compositions.

In some embodiments, the described HBV RNAi agent(s) are optionally combined with one or more additional therapeutics, wherein the additional therapeutic is a nucleoside inhibitor or nucleotide inhibitor. In some embodiments, the described HBV RNAi agent(s) are optionally combined with one or more additional therapeutics, wherein the additional therapeutic entecavir, tenofovir, tenofovir alafenamide, tenofovir disoproxil, lamivudine, or another antiviral therapeutic. In some embodiments the nucleoside inhibitor is entecavir or tenofovir. In some embodiments, entecavir is administered in the amount of 0.5-1 mg daily. In some embodiments, tenofovir is administered in the amount of 300 mg once daily.

In some embodiments, the described HBV RNAi agent(s) are optionally combined with one or more additional therapeutics, wherein the additional therapeutic is an interferon. In some embodiments, the described HBV RNAi agent(s) are optionally combined with one or more additional therapeutics, wherein the additional therapeutic is interferon-alpha. In some embodiments, the described HBV RNAi agent(s) are optionally combined with one or more HBV additional therapeutics, wherein the additional therapeutic is an HBV vaccine.

In some embodiments, the described HBV RNAi agent(s) are optionally combined with one or more additional therapeutics in a single dosage form (i.e., a cocktail included in a single injection). In some embodiments, the described HBV RNAi agent(s) may be administered separately from one or more optional additional therapeutics. In some embodiments, the described HBV RNAi agent(s) are administered to a subject in need thereof via subcutaneous injection, and the one or more optional additional therapeutics are administered orally, which together provide for a treatment regimen for diseases and conditions associated with HBV infection. In some embodiments, the described HBV RNAi agent(s) are administered to a subject in need thereof via subcutaneous injection, and the one or more optional additional therapeutics are administered via a separate subcutaneous injection.

In some embodiments, disclosed herein are compositions for delivering an HBV RNAi agent to a liver cell in vivo, the composition including an HBV RNAi agent conjugated or linked to a targeting group. In some embodiments, the targeting group is an asialoglycoprotein receptor ligand. In some embodiments, compositions for delivering an HBV RNAi agent to a liver cell in vivo are described, the composition including an HBV RNAi agent linked to an N-acetyl-galactosamine targeting ligand.

In some embodiments, one or more of the described HBV RNAi agents are administered to a mammal in a pharmaceutically acceptable carrier or diluent. In some embodiments, the mammal is a human.

The use of Hepatitis B Virus RNAi agent(s) provides methods for therapeutic and/or prophylactic treatment of diseases/disorders which are associated with HBV infection. The described HBV RNAi agents mediate RNA interference to inhibit the expression of one or more genes necessary for replication and/or pathogenesis of Hepatitis B Virus. In particular, for example, HBV RNAi agents may inhibit viral polymerase, core protein, surface antigen, e-antigen and/or the X protein, in a cell, tissue or mammal. HBV RNAi agents can be used to treat HBV infection. HBV RNAi agents can also be used to treat or prevent chronic liver diseases/disorders, inflammations, fibrotic conditions and proliferative disorders, like cancers, associated with HBV infection. In some embodiments, the methods further comprise treatment of Hepatitis D Virus (HDV) in the subject. Such methods comprise administration of HBV RNAi agent to a human being or animal infected with HBV. Further, compositions for delivery of HBV RNAi agents to liver cells in vivo are described.

In another aspect, described herein are methods for therapeutic and/or prophylactic treatment of diseases/disorders which are associated with HBV infection or inhibition of expression of one or more HBV genes comprising administering a pharmaceutical composition comprising one or more HBV RNAi agents can be administered in a number of ways depending upon whether local or systemic treatment is desired. Administration can be, but is not limited to, intravenous, intraarterial, subcutaneous, intraperitoneal, subdermal (e.g., via an implanted device), and intraparenchymal administration. In some embodiments, the pharmaceutical compositions described herein are administered by subcutaneous injection.

The described HBV RNAi agents and/or compositions can be used in methods for therapeutic treatment of HBV infection or disease or conditions caused by HBV infection. Such methods include administration of an HBV RNAi agent as described herein to a subject, e.g., a human or animal subject.

As used herein, the terms "oligonucleotide" and "polynucleotide" mean a polymer of linked nucleosides each of which can be independently modified or unmodified.

As used herein, an "RNAi agent" or "RNAi trigger" means a composition that contains an RNA or RNA-like (e.g., chemically modified RNA) oligonucleotide molecule that is capable of degrading or inhibiting translation of messenger RNA (mRNA) transcripts of a target mRNA in a sequence specific manner. As used herein, RNAi agents may operate through the RNA interference mechanism (i.e., inducing RNA interference through interaction with the RNA interference pathway machinery (RNA-induced silencing complex or RISC) of mammalian cells), or by any alternative mechanism(s) or pathway(s). While it is believed that RNAi agents, as that term is used herein, operate primarily through the RNA interference mechanism, the disclosed RNAi agents are not bound by or limited to any particular pathway or mechanism of action. RNAi agents disclosed herein are comprised of a sense strand and an antisense strand, and include, but are not limited to: short interfering RNAs (siRNAs), double-stranded RNAs (dsRNA), micro RNAs (miRNAs), short hairpin RNAs (shRNA), and dicer substrates. The antisense strand of the RNAi agents described herein is at least partially complementary to the mRNA being targeted. RNAi agents may be comprised of modified nucleotides and/or one or more non-phosphodiester linkages.

As used herein, the terms "silence," "reduce," "inhibit," "down-regulate," or "knockdown" when referring to expression of a given gene, mean that the expression of the gene, as measured by the level of RNA transcribed from the gene or the level of polypeptide, protein or protein subunit translated from the mRNA in a cell, group of cells, tissue, organ, or subject in which the gene is transcribed, is reduced when the cell, group of cells, tissue, organ, or subject is treated with oligomeric compounds, such as RNAi agents, described herein as compared to a second cell, group of cells, tissue, organ, or subject that has not or have not been so treated.

As used herein, the term "sequence" or "nucleotide sequence" mean a succession or order of nucleobases or nucleotides, described with a succession of letters using standard nomenclature.

As used herein, a "nucleotide base," or "nucleobase" is a heterocyclic pyrimidine or purine compound, which is a standard constituent of all nucleic acids, and includes the bases that form the nucleotides adenine (A), guanine (G), cytosine (C), thymine (T), and uracil (U). A nucleobase may further be modified to include, without limitation, universal bases, hydrophobic bases, promiscuous bases, size-expanded bases, and fluorinated bases.

As used herein, and unless otherwise indicated, the term "complementary," when used to describe a first nucleotide sequence (e.g., RNAi agent sense strand or targeted mRNA) in relation to a second nucleotide sequence (e.g., RNAi agent antisense strand or a single-stranded antisense oligonucleotide), means the ability of an oligonucleotide or polynucleotide including the first nucleotide sequence to hybridize (form base pair hydrogen bonds under mammalian physiological conditions (or similar conditions in vitro)) and form a duplex or double helical structure under certain conditions with an oligonucleotide or polynucleotide including the second nucleotide sequence. Complementary sequences include Watson-Crick base pairs or non-Watson-Crick base pairs and include natural or modified nucleotides or nucleotide mimics, at least to the extent that the above hybridization requirements are fulfilled. Sequence identity or complementarity is independent of modification. For example, a and Af are complementary to U (or T) and identical to A for the purposes of determining identity or complementarity.

As used herein, "perfectly complementary" or "fully complementary" means that all (100%) of the bases in a contiguous sequence of a first polynucleotide will hybridize with the same number of bases in a contiguous sequence of a second polynucleotide. The contiguous sequence may comprise all or a part of a first or second nucleotide sequence.

As used herein, "partially complementary" means that in a hybridized pair of nucleobase sequences, at least 70%, but not all, of the bases in a contiguous sequence of a first polynucleotide will hybridize with the same number of bases in a contiguous sequence of a second polynucleotide.

As used herein, "subject," "patient," or "individual" includes a mammal, such as a human or other animal, and typically is human. In some embodiments, the subject, e.g., patient, to whom the therapeutic agents and compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent, a dog, a cat, a farm animal, such as a cow or a horse, etc.

As used herein, "substantially complementary" means that in a hybridized pair of nucleobase sequences, at least about 85%, but not all, of the bases in a contiguous sequence of a first polynucleotide will hybridize with the same number of bases in a contiguous sequence of a second polynucleotide. The terms "complementary," "fully complementary," and "substantially complementary" herein may be used with respect to the base matching between the sense strand and the antisense strand of a double-stranded RNAi agent, between the antisense strand of an RNAi agent and a sequence of a target mRNA, or between a single-stranded antisense oligonucleotide and a sequence of a target mRNA.

As used herein, the term "substantially identical" or" substantially identity" as applied to nucleic acid sequence means that a nucleic acid sequence comprises a sequence that has at least about 85% sequence identity or more, preferably at least 90%, at least 95%, or at least 99%, compared to a reference sequence. Percentage of sequence identity is determined by comparing two optimally aligned sequences over a comparison window. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity. The inventions disclosed herein encompasses nucleotide sequences substantially identical to those disclosed herein, e.g., in Tables 2, 3, and 4. In some embodiments, the sequences disclosed herein are exactly identical, or at least about 85%, 86%, 87%, 88%, 89%, 90%, 91%, 92%, 93%, 94%, 95%, 96%, 97%, 98%, or 99% percent identical to those disclosed herein, e.g., in Tables 1, 2, 3 and 4.

As used herein, the terms "treat," "treatment," and the like, mean the methods or steps taken to provide relief from or alleviation of the number, severity, and/or frequency of one or more symptoms of a disease or condition in a subject.

As used herein, the phrase "introducing into a cell," when referring to an oligomeric compound, means functionally delivering the oligomeric compound into a cell. The phrase "functional delivery," means that delivering the oligomeric compound to the cell in a manner that enables the oligomeric compound to have the expected biological activity, e.g., sequence-specific inhibition of gene expression.

Unless stated otherwise, use of the symbol 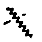 as used herein means that any group or groups may be linked thereto that is in accordance with the scope of the inventions described herein.

As used herein, the term "isomers" refers to compounds that have identical molecular formulae, but that differ in the nature or the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images are termed "enantiomers," or sometimes optical isomers. A carbon atom bonded to four non-identical substituents is termed a "chiral center."

As used herein, unless specifically identified in a structure as having a particular conformation, for each structure in which asymmetric centers are present and thus give rise to enantiomers, diastereomers, or other stereoisomeric configurations, each structure disclosed herein is intended to represent all such possible isomers, including their optically pure and racemic forms. For example, the structures disclosed herein are intended to cover mixtures of diastereomers as well as single stereoisomers.

As used in a claim herein, the phrase "consisting of" excludes any element, step, or ingredient not specified in the claim. When used in a claim herein, the phrase "consisting essentially of" limits the scope of a claim to the specified materials or steps and those that do not materially affect the basic and novel characteristic(s) of the claimed invention.

The person of ordinary skill in the art would readily understand and appreciate that the compounds and compositions disclosed herein may have certain atoms (e.g., N, O, or S atoms) in a protonated or deprotonated state, depending upon the environment in which the compound or composition is placed. Accordingly, as used herein, the structures disclosed herein envisage that certain functional groups, such as, for example, OH, SH, or NH, may be protonated or deprotonated. The disclosure herein is intended to cover the disclosed compounds and compositions regardless of their state of protonation based on the environment (such as pH), as would be readily understood by the person of ordinary skill in the art.

Unless otherwise defined, all technical and scientific terms used herein have the same meaning as commonly understood by one of ordinary skill in the art. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### BRIEF DESCRIPTION OF THE DRAWINGS

A better understanding of features and advantages of the present disclosure will be obtained by reference to the following detailed description, which sets forth illustrative embodiments of the disclosure, and the accompanying drawings.
**FIG. 1** shows the mean log HBsAg change from day 1 for CHB patients from different cohorts (c2b, c3b, c4b, c5b, c8 and c9 of Example 19)
**FIG. 2** shows individual changes in HBV DNA for CHB patients from different cohorts (c2b, c8 and c9 of Example 19). Solid line indicates HBeAg positive patients and dashed line indicates HBeAg negative patients.
**FIG. 3** shows individual changes in HBV DNA for CHB patients from different cohorts (c1b, c1c, c4b, c5b, c7, c9, c10 and c11 of Example 19).
**FIG. 4** shows individual changes in Log HBV RNA for CHB patients from cohort 10 of Example 19.
**FIG. 5** shows individual changes in Log HBV RNA for CHB patients from cohort 7 of Example 19.
**FIG. 6** shows individual changes in Log HBV RNA for CHB patients from different cohorts (c2b, c3b, c4b, c5b, c8 and c9 of Example 19). Solid line indicates HBeAg positive patients and dashed line indicates HBeAg negative patients.
**FIG. 7** shows individual changes in HBeAg for CHB patients from different cohorts (c2b, c4b, c5b, c8 and c9 of Example 19).
**FIG. 8** shows individual changes in HBeAg for CHB patients from different cohorts (c1b, c3b and c10 of Example 19).
**FIG. 9** shows individual changes in HBcrAg for CHB patients from different cohorts (c2b, c3b, c4b, c5b, c8 and c9 of Example 19). Solid line indicates HBeAg positive patients and dashed line indicates HBeAg negative patients.
**FIG. 10** shows individual changes in HBcrAg for CHB patients from cohort 6 of Example 19.
**FIG. 11** shows individual changes in HBcrAg for CHB patients from cohort 7 of Example 19.
**FIG. 12** shows individual changes in HBcrAg for CHB patients from cohort 10 of Example 19.
**FIG. 13** shows the NADIR log HBsAg reduction for HBeAg positive patients and HBeAg negative patients.
**FIG. 14** shows the mean log HBsAg change from day 1 for CHB patients from different cohorts (c2b, c3b, c4b, c5b, c8 and c9 of Example 19).
**FIG. 15** shows the mean change for antigen HBsAg from day 1 for CHB patients receiving three doses of HBV RNAi agents (AD04872 and AD05070) via subcutaneous injections in 28-day intervals (cohorts 1b, 1c, 2b, 3b, 4b, 5b, 8 and 9 of Example 20).
**FIG. 16** shows the mean changes for antigen HBsAg and HBeAg from day 1 for HBsAg-positive CHB patients receiving three doses of HBV RNAi agents (AD04872 and AD05070) via subcutaneous injections in 28-day intervals (cohorts 1b, 1c, 2b, 3b, 4b, 5b, 8 and 9 of Example 20).
**FIG. 17** shows the mean HBsAg changes from Day 1 to Day 113 for CHB patients in cohorts 1b, 1c, 2b, 3b, 4b and 5b of Example 21, respectively. The patients were given three subcutaneous doses of the RNAi component in the amount of: 25 mg (cohort 1b), 50 mg (cohort 1c), 100 mg (cohort 2b), 200 mg (cohort 3b), 300 mg (cohort 4b) or 400 mg (cohort 5b) on Day 1, 29 and 57. All patients were given NA daily.
**FIG. 18** shows the mean and individual HBsAg changes on Day 113 from Day 1 for all patients in cohorts 1b, 1c, 2b, 3b, 4b and 5b of Example 21. Dots with an arrow indicate HBeAg positive patients. Dots without an arrow indicate HBeAg negative patients.
**FIGS. 19A-19D** show the individual changes in HBV DNA, HBV RNA, HBeAg and HBcrAg from Day 1 to Day 113 for CHB patients in cohorts 1b, 1c, 2b, 3b, 4b and 5b of Example 21, respectively. The patients were given three subcutaneous doses of the RNAi component in the amount of: 25 mg (cohort 1b), 50 mg (cohort 1c), 100 mg (cohort 2b), 200 mg (cohort 3b), 300 mg (cohort 4b) or 400 mg (cohort 5b) on Day 1, 29 and 57. And all patients were given NA daily. Each line represents one individual CHB patient.
**FIG. 20** shows the mean change for antigen HBsAg from day 1 for CHB patients receiving three doses of HBV RNAi agents (AD04872 and AD05070) via subcutaneous injections in 28-day intervals (cohorts 1b, 1c, 2b, 3b, 4b, 5b, 8 and 9 of Example 20; until day 932).
**FIG. 21** shows the mean changes for antigen HBsAg and HBeAg from day 1 for HBsAg-positive CHB patients receiving three doses of HBV RNAi agents (AD04872 and AD05070) via subcutaneous injections in 28-day intervals (cohorts 1b, 1c, 2b, 3b, 4b, 5b, 8 and 9 of Example 20; until day 392).
**FIG. 22** shows sustained suppression of HBsAg in a subset of patients ~9 months after the last RNAi dose (mean 1.74); cf. example 20: 8 CHB patients/cohort (NA experienced or naive; HBeAg +ve or -ve) received 3 subcutaneous JNJ-3989 doses (days 1, 27, 57) of 100, 200, 300 (n=16) or 400mg; patients started/continued with an NA on day 1 and continued throughout the study.

### DETAILED DESCRIPTION

Described herein are methods of treating and/or preventing symptoms or diseases associated with an infection caused by Hepatitis B Virus (HBV) in a subject in need thereof or inhibiting expression of one or more Hepatitis B Virus genes. Particularly, described herein are methods of treating or preventing an HBV-associated disease or symptoms in a subject or inhibiting expression of one or more HBV genes comprising administering RNAi agents that inhibit expression of Hepatitis B Virus (HBV) (referred to herein as HBV RNAi agents or HBV RNAi triggers). Each HBV RNAi agent comprises a sense strand and an antisense strand. The sense strand and the antisense strand each can be 16 to 30 nucleotides in length. In some embodiments, the sense and antisense strands each can be 17 to 26 nucleotides in length. The sense and antisense strands can be either the same length or they can be different lengths. In some embodiments, the sense and antisense strands are each independently 17 to 26 nucleotides in length. In some embodiments, the sense and antisense strands are each independently 17-21 nucleotides in length. In some embodiments, both the sense and antisense strands are each 21-26 nucleotides in length. In some embodiments, the sense strand is about 19 nucleotides in length while the antisense strand is about 21 nucleotides in length. In some embodiments, the sense strand is about 21 nucleotides in length while the antisense strand is about 23 nucleotides in length. In some embodiments, both the sense and antisense strands are each 26 nucleotides in length. In some embodiments, the RNAi agent sense and antisense strands are each independently 17, 18, 19, 20, 21, 22, 23, 24, 25, or 26 nucleotides in length. In some embodiments, a double-stranded RNAi agent has a duplex length of about 16, 17, 18, 19, 20, 21, 22, 23 or 24 nucleotides. This region of perfect or substantial complementarity between the sense strand and the antisense strand is typically 15-25 (e.g., 15, 16, 17, 18, 19, 20, 21, 22, 23, 24, or 25) nucleotides in length and occurs at or near the 5' end of the antisense strand (e.g., this region may be separated from the 5' end of the antisense strand by 0, 1, 2, 3, or 4 nucleotides that are not perfectly or substantially complementary).

The sense strand and antisense strand each contain a core stretch sequence that is 16 to 23 nucleobases in length. An antisense strand core stretch sequence is 100% (perfectly) complementary or at least about 85% (substantially) complementary to a nucleotide sequence (sometimes referred to, e.g., as a target sequence) present in the HBV mRNA target. A sense strand core stretch sequence is 100% (perfectly) complementary or at least about 85% (substantially) complementary to a core stretch sequence in the antisense strand, and thus the sense strand core stretch sequence is perfectly identical or at least about 85% identical to a nucleotide sequence (target sequence) present in the HBV mRNA target. A sense strand core stretch sequence can be the same length as a corresponding antisense core sequence or it can be a different length. In some embodiments, the antisense strand core stretch sequence is 16, 17, 18, 19, 20, 21, 22, or 23 nucleotides in length. In some embodiments, the sense strand core stretch sequence is 16, 17, 18, 19, 20, 21, 22, or 23 nucleotides in length.

Examples of sense and antisense strand nucleotide sequences used in forming HBV RNAi agents are provided in Tables 3 and 4. Examples of RNAi agent duplexes, that include the nucleotide sequences in Tables 3 and 4, are provided in Table 5.

The HBV RNAi agent sense and antisense strands anneal to form a duplex. A sense strand and an antisense strand of an HBV RNAi agent may be partially, substantially, or fully complementary to each other. Within the complementary duplex region, the sense strand core stretch sequence is at least about 85% complementary or 100% complementary to the antisense core stretch sequence. In some embodiments, the sense strand core stretch sequence contains a sequence of at least 16, at least 17, at least 18, at least 19, at least 20, or at least 21 nucleotides that is at least about 85% or 100% complementary to a corresponding 16, 17, 18, 19, 20, or 21 nucleotide sequence of the antisense strand core stretch sequence (i.e., the sense strand and antisense core stretch sequences of an HBV RNAi agent have a region of at least 16, at least 17, at least 18, at least 19, at least 20, or at least 21 nucleotides that is at least 85% base paired or 100% base paired.).

In some embodiments, the antisense strand of an HBV RNAi agent disclosed herein differs by 0, 1, 2, or 3 nucleotides from any of the antisense strand sequences in Table 2 or Table 3. In some embodiments, the sense strand of an HBV RNAi agent disclosed herein differs by 0, 1, 2, or 3 nucleotides from any of the sense strand sequences in Table 2 or Table 4.

The length of the HBV RNAi agent sense and antisense strands described herein are independently 16 to 30 nucleotides in length. In some embodiments, the sense and antisense strands are independently 17 to 26 nucleotides in length. In some embodiments, the sense and antisense strands are 19-26 nucleotides in length. In some embodiments, the described RNAi agent sense and antisense strands are independently 17, 18, 19, 20, 21, 22, 23, 24, 25, or 26 nucleotides in length. The sense and antisense strands can be either the same length or they can be different lengths. In some embodiments, a sense strand and an antisense strand are each 26 nucleotides in length. In some embodiments, a sense strand is 23 nucleotides in length and an antisense strand is 21 nucleotides in length. In some embodiments, a sense strand is 22 nucleotides in length and an antisense strand is 21 nucleotides in length. In some embodiments, a sense strand is 21 nucleotides in length and an antisense strand is 21 nucleotides in length. In some embodiments, a sense strand is 19 nucleotides in length and an antisense strand is 21 nucleotides in length.

The sense strand and/or the antisense strand may optionally and independently contain an additional 1, 2, 3, 4, 5, or 6 nucleotides (extension) at the 3' end, the 5' end, or both the 3' and 5' ends of the core sequences. The antisense strand additional nucleotides, if present, may or may not be complementary to the corresponding sequence in an HBV mRNA. The sense strand additional nucleotides, if present, may or may not be identical to the corresponding sequence in an HBV mRNA. The antisense strand additional nucleotides, if present, may or may not be complementary to the corresponding sense strand's additional nucleotides, if present.

As used herein, an extension comprises 1, 2, 3, 4, 5, or 6 nucleotides at the 5' and/or 3' end of the sense strand core stretch sequence and/or antisense strand core stretch sequence. The extension nucleotides on a sense strand may or may not be complementary to nucleotides, either core stretch sequence nucleotides or extension nucleotides, in the corresponding antisense strand. Conversely, the extension nucleotides on an antisense strand may or may not be complementary to nucleotides, either core stretch sequence nucleotides or extension nucleotides, in the corresponding sense strand. In some embodiments, both the sense strand and the antisense strand of an RNAi agent contain 3' and 5' extensions. In some embodiments, one or more of the 3' extension nucleotides of one strand base pairs with one or more 5' extension nucleotides of the other strand. In other embodiments, one or more of 3' extension nucleotides of one strand do not base pair with one or more 5' extension nucleotides of the other strand. In some embodiments, an HBV RNAi agent has an antisense strand having a 3' extension and a sense strand having a 5' extension.

In some embodiments, an HBV RNAi agent comprises an antisense strand having a 3' extension of 1, 2, 3, 4, 5, or 6 nucleotides in length. In other embodiments, an HBV RNAi agent comprises an antisense strand having a 3' extension of 1, 2, or 3 nucleotides in length. In some embodiments, one or more of the antisense strand extension nucleotides comprise uracil or thymidine nucleotides or nucleotides which are complementary to a corresponding HBV mRNA sequence. In some embodiments, a 3' antisense strand extension includes or consists of, but is not limited to: AUA, UGCUU, CUG, UG, UGCC, CUGCC, CGU, CUU, UGCCUA, CUGCCU, UGCCU, UGAUU, GCCUAU, T, TT, U, UU (each listed 5' to 3').

In some embodiments, the 3' end of the antisense strand may include additional abasic nucleosides (Ab). In some embodiments, Ab or AbAb may be added to the 3' end of the antisense strand.

In some embodiments, an HBV RNAi agent comprises an antisense strand having a 5' extension of 1, 2, 3, 4, or 5 nucleotides in length. In other embodiments, an HBV RNAi agent comprises an antisense strand having a 5' extension of 1 or 2 nucleotides in length. In some embodiments, one or more of the antisense strand extension nucleotides comprises uracil or thymidine nucleotides or nucleotides which are complementary to a corresponding HBV mRNA sequence. In some embodiments, the 5' antisense strand extension includes or consists of, but is no limited to, UA, TU, U, T, UU, TT, CUC (each listed 5' to 3'). An antisense strand may have any of the 3' extensions described above in combination with any of the 5' antisense strand extensions described, if present.

In some embodiments, an HBV RNAi agent comprises a sense strand having a 3' extension of 1, 2, 3, 4, or 5 nucleotides in length. In some embodiments, one or more of the sense strand extension nucleotides comprises adenosine, uracil, or thymidine nucleotides, AT dinucleotide, or nucleotides which correspond to nucleotides in the HBV mRNA sequence. In some embodiments, the 3' sense strand extension includes or consists of, but is not limited to: T, UT, TT, UU, UUT, TTT, or TTTT (each listed 5' to 3').

In some embodiments, the 3' end of the sense strand may include additional abasic nucleosides. In some embodiments, UUAb, UAb, or Ab may be added to the 3' end of the sense strand. In some embodiments, the one or more abasic nucleosides added to the 3' end of the sense strand may be inverted (invAb). In some embodiments, one or more inverted abasic nucleosides may be inserted between the targeting ligand and the nucleobase sequence of the sense strand of the RNAi agent. In some embodiments, the inclusion of one or more inverted abasic nucleosides at or near the terminal end or terminal ends of the sense strand of an RNAi agent may allow for enhanced activity or other desired properties of an RNAi agent.

In some embodiments, an HBV RNAi agent comprises a sense strand having a 5' extension of 1, 2, 3, 4, 5, or 6 nucleotides in length. In some embodiments, one or more of the sense strand extension nucleotides comprise uracil or adenosine nucleotides or nucleotides which correspond to nucleotides in the HBV mRNA sequence. In some embodiments, the sense strand 5' extension can be, but is not limited to: CA, AUAGGC, AUAGG, AUAG, AUA, A, AA, AC, GCA, GGCA, GGC, UAUCA, UAUC, UCA, UAU, U, UU (each listed 5' to 3'). A sense strand may have a 3' extension and/or a 5' extension.

In some embodiments, the 5' end of the sense strand may include an additional abasic nucleoside (Ab) or nucleosides (AbAb). In some embodiments, the one or more abasic nucleosides added to the 5' end of the sense strand may be inverted (invAb). In some embodiments, one or more inverted abasic nucleosides may be inserted between the targeting ligand and the nucleobase sequence of the sense strand of the RNAi agent. In some embodiments, the inclusion of one or more inverted abasic nucleosides at or near the terminal end or terminal ends of the sense strand of an RNAi agent may allow for enhanced activity or other desired properties of an RNAi agent.

Examples of nucleotide sequences used in forming HBV RNAi agents are provided in Tables 3 and 4. In some embodiments, an HBV RNAi agent antisense strand includes a nucleotide sequence of any of the sequences in Table 3. In some embodiments, an HBV RNAi agent antisense strand includes the sequence of nucleotides 1-17, 2-15, 2-17, 1-18, 2-18, 1-19, 2-19, 1-20, 2-20, 1-21, 2-21, 1-22, 2-22, 1-23, 2-23, 1-24, 2-24, 1-25, 2-25, 1-26, or 2-26 of any of the sequences in Table 3. In some embodiments, an HBV RNAi agent sense strand includes the nucleotide sequence of any of the sequences in Table 4. In some embodiments, an HBV RNAi agent sense strand includes the sequence of nucleotides 1-18, 1-19, 1-20, 1-21, 1-22, 1-23, 1-24, 1-25, 1-26, 2-19, 2-20, 2-21, 2-22, 2-23, 2-24, 2-25, 2-26, 3-20, 3-21, 3-22, 3-23, 3-24, 3-25, 3-26, 4-21, 4-22, 4-23, 4-24, 4-25, 4-26, 5-22, 5-23, 5-24, 5-25, 5-26, 6-23, 6-24, 6-25, 6-26, 7-24, 7-25, 7-25, 8-25, 8-26 of any of the sequences in Table 4.

In some embodiments, the sense and antisense strands of the RNAi agents described herein contain the same number of nucleotides. In some embodiments, the sense and antisense strands of the RNAi agents described herein contain different numbers of nucleotides. In some embodiments, the sense strand 5' end and the antisense strand 3' end of an RNAi agent form a blunt end. In some embodiments, the sense strand 3' end and the antisense strand 5' end of an RNAi agent form a blunt end. In some embodiments, both ends of an RNAi agent form blunt ends. In some embodiments, neither end of an RNAi agent is blunt-ended. As used herein a blunt end refers to an end of a double stranded RNAi agent in which the terminal nucleotides of the two annealed strands are complementary (form a complementary base-pair). In some embodiments, the sense strand 5' end and the antisense strand 3' end of an RNAi agent form a frayed end. In some embodiments, the sense strand 3' end and the antisense strand 5' end of an RNAi agent form a frayed end. In some embodiments, both ends of an RNAi agent form a frayed end. In some embodiments, neither end of an RNAi agent is a frayed end. As used herein a frayed end refers to an end of a double stranded RNAi agent in which the terminal nucleotides of the two annealed strands from a pair (i.e. do not form an overhang) but are not complementary (i.e. form a non-complementary pair). As used herein, an overhang is a stretch of one or more unpaired nucleotides at the end of one strand of a double stranded RNAi agent. The unpaired nucleotides may be on the sense strand or the antisense strand, creating either 3' or 5' overhangs. In some embodiments, the RNAi agent contains: a blunt end and a frayed end, a blunt end and 5' overhang end, a blunt end and a 3' overhang end, a frayed end and a 5' overhang end, a frayed end and a 3' overhang end, two 5' overhang ends, two 3' overhang ends, a 5' overhang end and a 3' overhang end, two frayed ends, or two blunt ends.

A nucleotide base (or nucleobase) is a heterocyclic pyrimidine or purine compound which is a constituent of all nucleic acids and includes adenine (A), guanine (G), cytosine (C), thymine (T), and uracil (U). As used herein, the term "nucleotide" can include a modified nucleotide (such as, for example, a nucleotide mimic, abasic site (Ab), or a surrogate replacement moiety). Modified nucleotides, when used in various polynucleotide or oligonucleotide constructs, may preserve activity of the compound in cells while at the same time increasing the serum stability of these compounds, and can also minimize the possibility of activating interferon activity in humans upon administering of the polynucleotide or oligonucleotide construct.

In some embodiments, an HBV RNAi agent is prepared or provided as a salt, mixed salt, or a free-acid. In some embodiments, an HBV RNAi agent is prepared as a sodium salt. Such forms are within the scope of the inventions disclosed herein.

### Modified Nucleotides

In some embodiments, an HBV RNAi agent contains one or more modified nucleotides. As used herein, a "modified nucleotide" is a nucleotide other than a ribonucleotide (2'-hydroxyl nucleotide). In some embodiments, at least 50% (e.g., at least 60%, at least 70%, at least 80%, at least 90%, at least 95%, at least 97%, at least 98%, at least 99%, or 100%) of the nucleotides are modified nucleotides. As used herein, modified nucleotides include, but are not limited to, deoxyribonucleotides, nucleotide mimics, abasic nucleotides (represented herein as Ab), 2'-modified nucleotides, 3' to 3' linkages (inverted) nucleotides (represented herein as invdN, invN, invn, invAb), non-natural base-comprising nucleotides, bridged nucleotides, peptide nucleic acids (PNAs), 2',3'-seco nucleotide mimics (unlocked nucleobase analogues, represented herein as NUNA or NUNA), locked nucleotides (represented herein as NLNA or NLNA), 3'-O-methoxy (2' internucleoside linked) nucleotides (represented herein as 3'-OMen), 2'-F-Arabino nucleotides (represented herein as NfANA or NfANA), 5'-Me, 2'-fluoro nucleotide (represented herein as 5Me-Nf), morpholino nucleotides, vinyl phosphonate deoxyribonucleotides (represented herein as vpdN), vinyl phosphonate containing nucleotides, and cyclopropyl phosphonate containing nucleotides (cPrpN). 2'-modified nucleotides (i.e. a nucleotide with a group other than a hydroxyl group at the 2' position of the five-membered sugar ring) include, but are not limited to, 2'-O-methyl nucleotides (represented herein as a lower case letter 'n' in a nucleotide sequence), 2'-deoxy-2'-fluoro nucleotides (represented herein as Nf, also represented herein as 2'-fluoro nucleotide), 2'-deoxy nucleotides (represented herein as dN), 2'-methoxyethyl (2'-O-2-methoxylethyl) nucleotides (represented herein as NM or 2'-MOE), 2'-amino nucleotides, and 2'-alkyl nucleotides. It is not necessary for all positions in a given compound to be uniformly modified. Conversely, more than one modification may be incorporated in a single HBV RNAi agent or even in a single nucleotide thereof. The HBV RNAi agent sense strands and antisense strands may be synthesized and/or modified by methods known in the art. Modification at one nucleotide is independent of modification at another nucleotide.

Modified nucleobases include synthetic and natural nucleobases, such as 5-substituted pyrimidines, 6-azapyrimidines and N-2, N-6 and O-6 substituted purines, (e.g., 2-aminopropyladenine, 5-propynyluracil, or 5-propynylcytosine), 5-methylcytosine (5-me-C), 5-hydroxymethyl cytosine, xanthine, hypoxanthine, 2-aminoadenine, 6-alkyl (e.g., 6-methyl, 6-ethyl, 6-isopropyl, or 6-n-butyl) derivatives of adenine and guanine, 2-alkyl (e.g., 2-methyl, 2-ethyl, 2-isopropyl, or 2-n-butyl) and other alkyl derivatives of adenine and guanine, 2-thiouracil, 2-thiothymine, 2-thiocytosine, 5-halouracil, cytosine, 5-propynyl uracil, 5-propynyl cytosine, 6-azo uracil, 6-azo cytosine, 6-azo thymine, 5-uracil (pseudouracil), 4-thiouracil, 8-halo, 8-amino, 8-sulfhydryl, 8-thioalkyl, 8-hydroxyl and other 8-substituted adenines and guanines, 5-halo (e.g., 5-bromo), 5-trifluoromethyl, and other 5-substituted uracils and cytosines, 7-methylguanine and 7-methyladenine, 8-azaguanine and 8-azaadenine, 7-deazaguanine, 7-deazaadenine, 3-deazaguanine, and 3-deazaadenine.

In some embodiments, all or substantially all of the nucleotides of an RNAi agent are modified nucleotides. As used herein, an RNAi agent wherein substantially all of the nucleotides present are modified nucleotides is an RNAi agent having four or fewer (i.e., 0, 1, 2, 3, or 4) nucleotides in both the sense strand and the antisense strand being ribonucleotides. As used herein, a sense strand wherein substantially all of the nucleotides present are modified nucleotides is a sense strand having two or fewer (i.e., 0, 1, or 2) nucleotides in the sense strand being ribonucleotides. As used herein, an antisense sense strand wherein substantially all of the nucleotides present are modified nucleotides is an antisense strand having two or fewer (i.e., 0, 1, or 2) nucleotides in the sense strand being ribonucleotides. In some embodiments, one or more nucleotides of an RNAi agent is a ribonucleotide.

### Modified Internucleoside Linkages

In some embodiments, one or more nucleotides of an HBV RNAi agent are linked by non-standard linkages or backbones (i.e., modified internucleoside linkages or modified backbones). In some embodiments, a modified internucleoside linkage is a non-phosphate-containing covalent internucleoside linkage. Modified internucleoside linkages or backbones include, but are not limited to, 5'-phosphorothioate groups (represented herein as a lower case "s"), chiral phosphorothioates, thiophosphates, phosphorodithioates, phosphotriesters, aminoalkyl-phosphotriesters, alkyl phosphonates (e.g., methyl phosphonates or 3'-alkylene phosphonates), chiral phosphonates, phosphinates, phosphoramidates (e.g., 3'-amino phosphoramidate, aminoalkylphosphoramidates, or thionophosphoramidates), thionoalkyl-phosphonates, thionoalkylphosphotriesters, morpholino linkages, boranophosphates having normal 3'-5' linkages, 2'-5' linked analogs of boranophosphates, or boranophosphates having inverted polarity wherein the adjacent pairs of nucleoside units are linked 3'-5' to 5'-3' or 2'-5' to 5'-2'. In some embodiments, a modified internucleoside linkage or backbone lacks a phosphorus atom. Modified internucleoside linkages lacking a phosphorus atom include, but are not limited to, short chain alkyl or cycloalkyl inter-sugar linkages, mixed heteroatom and alkyl or cycloalkyl inter-sugar linkages, or one or more short chain heteroatomic or heterocyclic inter-sugar linkages. In some embodiments, modified internucleoside backbones include, but are not limited to, siloxane backbones, sulfide backbones, sulfoxide backbones, sulfone backbones, formacetyl and thioformacetyl backbones, methylene formacetyl and thioformacetyl backbones, alkene-containing backbones, sulfamate backbones, methyleneimino and methylenehydrazino backbones, sulfonate and sulfonamide backbones, amide backbones, and other backbones having mixed N, O, S, and CH2 components.

In some embodiments, a sense strand of an HBV RNAi agent can contain 1, 2, 3, 4, 5, or 6 phosphorothioate linkages, an antisense strand of an HBV RNAi agent can contain 1, 2, 3, 4, 5, or 6 phosphorothioate linkages, or both the sense strand and the antisense strand independently can contain 1, 2, 3, 4, 5, or 6 phosphorothioate linkages. In some embodiments, a sense strand of an HBV RNAi agent can contain 1, 2, 3, or 4 phosphorothioate linkages, an antisense strand of an HBV RNAi agent can contain 1, 2, 3, or 4 phosphorothioate linkages, or both the sense strand and the antisense strand independently can contain 1, 2, 3, or 4 phosphorothioate linkages.

In some embodiments, an HBV RNAi agent sense strand contains at least two phosphorothioate internucleoside linkages. In some embodiments, the at least two phosphorothioate internucleoside linkages are between the nucleotides at positions 1-3 from the 3' end of the sense strand. In some embodiments, the at least two phosphorothioate internucleoside linkages are between the nucleotides at positions 1-3, 2-4, 3-5, 4-6, 4-5, or 6-8 from the 5' end of the sense strand. In some embodiments, an HBV RNAi agent antisense strand contains four phosphorothioate internucleoside linkages. In some embodiments, the four phosphorothioate internucleoside linkages are between the nucleotides at positions 1-3 from the 5' end of the sense strand and between the nucleotides at positions 19-21, 20-22, 21-23, 22-24, 23-25, or 24-26 from the 5' end. In some embodiments, an HBV RNAi agent contains at least two phosphorothioate internucleoside linkages in the sense strand and three or four phosphorothioate internucleoside linkages in the antisense strand.

In some embodiments, an HBV RNAi agent contains one or more modified nucleotides and one or more modified internucleoside linkages. In some embodiments, a 2'-modified nucleoside is combined with modified internucleoside linkage.

### HBV RNAi Agents

In some embodiments, the HBV RNAi agents disclosed herein target an HBV gene at or near the positions of the HBV genome shown in the following Table 1. In some embodiments, the antisense strand of an HBV RNAi agent disclosed herein includes a core stretch sequence that is fully, substantially, or at least partially complementary to a target HBV 19-mer sequence disclosed in Table 1.

**Table 1. Example 19-mer HBV cDNA target sequences for HBV RNAi agents (taken from Hepatitis B virus (subtype ADW2), genotype A, complete genome GenBank AM282986.1 (SEQ ID NO:1)).**

| **SEQ ID No.** | **HBV 19-mer Target Sequences (5' → 3')** | **Genome Position of SEQ ID NO: 1** | **Region of HBV Gene Targeted** |
|---|---|---|---|
| 2 | GTGGTGGACTTCTCTCAAT | 256-274 | S ORF |
| 3 | TGGTGGACTTCTCTCAATT | 257-275 | S ORF |
| 4 | GGACTTCTCTCAATTTTCT | 261-279 | S ORF |
| 5 | GCTGTAGGCATAAATTGGT | 1780-1798 | X ORF |
| 6 | CTGTAGGCATAAATTGGTC | 1781-1799 | X ORF |

In some embodiments, an HBV RNAi agent includes an antisense strand wherein position 19 of the antisense strand (5'→3') is capable of forming a base pair with position 1 of a 19-mer target sequence disclosed in Table 1. In some embodiments, an HBV RNAi agent includes an antisense strand wherein position 1 of the antisense strand (5' → 3') is capable of forming a base pair with position 19 of the 19-mer target sequence disclosed in Table 1.

In some embodiments, an HBV RNAi agent includes an antisense strand wherein position 2 of the antisense strand (5' → 3') is capable of forming a base pair with position 18 of the 19-mer target sequence disclosed in Table 1. In some embodiments, an HBV RNAi agent includes an antisense strand wherein positions 2 through 18 of the antisense strand (5' → 3') are capable of forming base pairs with each of the respective complementary bases located at positions 18 through 2 of the 19-mer target sequence disclosed in Table 1.

In some embodiments, the HBV RNAi agents include core 19-mer nucleotide sequences shown in the following Table 2.

**Table 2. HBV RNAi agent antisense strand and sense strand core stretch sequences (N=any nucleotide).**

| **SEQ ID NO:** | **Antisense Sequence (5' → 3')** | **SEQ ID NO:** | **Sense Sequence (5' → 3')** | **Genome Position of SEQ ID NO: 1** |
|---|---|---|---|---|
| | **(19-mer)** | | **(19-mer)** | |
| 7 | AUUGAGAGAAGUCCACCAC | 34 | GUGGUGGACUUCUCUCAAU | 256-274 |
| 8 | UUUGAGAGAAGUCCACCAC | 35 | GUGGUGGACUUCUCUCAAA | 256-274 |
| 9 | AUUGAGAGAAGUCCACCAN | 36 | NUGGUGGACUUCUCUCAAU | 256-274 |
| 10 | UUUGAGAGAAGUCCACCAN | 37 | NUGGUGGACUUCUCUCAAA | 256-274 |
| 11 | NUUGAGAGAAGUCCACCAN | 38 | NUGGUGGACUUCUCUCAAN | 256-274 |
| 12 | AAUUGAGAGAAGUCCACCA | 39 | UGGUGGACUUCUCUCAAUU | 257-275 |
| 13 | UAUUGAGAGAAGUCCACCA | 40 | UGGUGGACUUCUCUCAAUA | 257-275 |
| 14 | AAUUGAGAGAAGUCCACCN | 41 | NGGUGGACUUCUCUCAAUU | 257-275 |
| 15 | UAUUGAGAGAAGUCCACCN | 42 | NGGUGGACUUCUCUCAAUA | 257-275 |
| 16 | NAUUGAGAGAAGUCCACCN | 43 | NGGUGGACUUCUCUCAAUN | 257-275 |
| 17 | AGAAAAUUGAGAGAAGUCC | 44 | GGACUUCUCUCAAUUUUCU | 261-279 |
| 18 | UGAAAAUUGAGAGAAGUCC | 45 | GGACUUCUCUCAAUUUUCA | 261-279 |
| 19 | AGAAAAUUGAGAGAAGUCN | 46 | NGACUUCUCUCAAUUUUCU | 261-279 |
| 20 | UGAAAAUUGAGAGAAGUCN | 47 | NGACUUCUCUCAAUUUUCA | 261-279 |
| 21 | NGAAAAUUGAGAGAAGUCN | 48 | NGACUUCUCUCAAUUUUCN | 261-279 |
| 22 | ACCAAUUUAUGCCUACAGC | 49 | GCUGUAGGCAUAAAUUGGU | 1780-1798 |
| 23 | UCCAAUUUAUGCCUACAGC | 50 | GCUGUAGGCAUAAAUUGGA | 1780-1798 |
| 24 | ACCAAUUUAUGCCUACAGN | 51 | NCUGUAGGCAUAAAUUGGU | 1780-1798 |
| 25 | UCCAAUUUAUGCCUACAGN | 52 | NCUGUAGGCAUAAAUUGGA | 1780-1798 |
| 26 | NCCAAUUUAUGCCUACAGN | 53 | NCUGUAGGCAUAAAUUGGN | 1780-1798 |
| 27 | GACCAAUUUAUGCCUACAG | 54 | CUGUAGGCAUAAAUUGGUC | 1781-1799 |
| 28 | AACCAAUUUAUGCCUACAG | 55 | CUGUAGGCAUAAAUUGGUU | 1781-1799 |
| 29 | UACCAAUUUAUGCCUACAG | 56 | CUGUAGGCAUAAAUUGGUA | 1781-1799 |
| 30 | GACCAAUUUAUGCCUACAN | 57 | NUGUAGGCAUAAAUUGGUC | 1781-1799 |
| 31 | AACCAAUUUAUGCCUACAN | 58 | NUGUAGGCAUAAAUUGGUU | 1781-1799 |
| 32 | UACCAAUUUAUGCCUACAN | 59 | NUGUAGGCAUAAAUUGGUA | 1781-1799 |
| 33 | NACCAAUUUAUGCCUACAN | 60 | NUGUAGGCAUAAAUUGGUN | 1781-1799 |

The HBV RNAi agent sense strands and antisense strands that comprise or consist of the nucleotide sequences in Table 2 can be modified nucleotides or unmodified nucleotides. In some embodiments, the HBV RNAi agents having the sense and antisense strand sequences that comprise or consist of the nucleotide sequences in Table 2 are all or substantially all modified nucleotides.

In some embodiments, the antisense strand of an HBV RNAi agent disclosed herein differs by 0, 1, 2, or 3 nucleotides from any of the antisense strand sequences in Table 2. In some embodiments, the sense strand of an HBV RNAi agent disclosed herein differs by 0, 1, 2, or 3 nucleotides from any of the sense strand sequences in Table 2.

Modified HBV RNAi agent antisense strand sequences, as well as their underlying unmodified sequences, are provided in Table 3. Modified HBV RNAi agent sense strands, as well as their underlying unmodified sequences, are provided in Table 4. In forming HBV RNAi agents, each of the nucleotides in each of the unmodified sequences listed in Tables 3 and 4 may be a modified nucleotide.

As used herein (including in Tables 3 and 4), the following notations are used to indicate modified nucleotides, targeting groups, and linking groups. As the person of ordinary skill in the art would readily understand, unless otherwise indicated by the sequence, that when present in an oligonucleotide, the monomers are mutually linked by 5'-3'-phosphodiester bonds:
- A: = adenosine-3'-phosphate;
- C: = cytidine-3'-phosphate;
- G: = guanosine-3'-phosphate;
- U: = uridine-3'-phosphate
- n: = any 2'-OMe modified nucleotide
- a: = 2'-O-methyladenosine-3'-phosphate
- as: = 2'-O-methyladenosine-3'-phosphorothioate
- c: = 2'-O-methylcytidine-3'-phosphate
- cs: = 2'-O-methylcytidine-3'-phosphorothioate
- g: = 2'-O-methylguanosine-3'-phosphate
- gs: = 2'-O-methylguanosine-3'-phosphorothioate
- t: = 2'-O-methyl-5-methyluridine-3'-phosphate
- ts: = 2'-O-methyl-5-methyluridine-3'-phosphorothioate
- u: = 2'-O-methyluridine-3'-phosphate
- us: = 2'-O-methyluridine-3'-phosphorothioate
- Nf: = any 2'-fluoro modified nucleotide
- Af: = 2'-fluoroadenosine-3'-phosphate
- Afs: = 2'-fluoroadenosine-3'-phosporothioate
- Cf: = 2'-fluorocytidine-3'-phosphate
- Cfs: = 2'-fluorocytidine-3'-phosphorothioate
- Gf: = 2'-fluoroguanosine-3'-phosphate
- Gfs: = 2'-fluoroguanosine-3'-phosphorothioate
- Tf: = 2'-fluoro-5'-methyluridine-3'-phosphate
- Tfs: = 2'-fluoro-5'-methyluridine-3'-phosphorothioate
- Uf: = 2'-fluorouridine-3'-phosphate
- Ufs: = 2'-fluorouridine-3'-phosphorothioate
- dN: = any 2'-deoxyribonucleotide
- dT: = 2'-deoxythymidine-3'-phosphate
- N_{UNA}: = 2',3'-seco nucleotide mimics (unlocked nucleobase analogs)
- N_{LNA}: = locked nucleotide
- Nf_{ANA}: = 2'-F-Arabino nucleotide
- NM: = 2'-methoxyethyl nucleotide
- AM: = 2'-methoxyethyladenosine-3'-phosphate
- AMs: = 2'-methoxyethyladenosine-3'-phosphorothioate
- TM: = 2'-methoxyethylthymidine-3'-phosphate
- TMs: = 2'-methoxyethylthymidine-3'-phosphorothioate
- R: = ribitol
- (invdN): = any inverted deoxyribonucleotide (3'-3' linked nucleotide)
- (invAb): = inverted (3'-3' linked) abasic deoxyribonucleotide, see Table 6
- (invAb)s: = inverted (3'-3' linked) abasic deoxyribonucleotide-5'-phosphorothioate, see Table 6
- (invn): = any inverted 2'-OMe nucleotide (3'-3' linked nucleotide)
- s: = phosphorothioate linkage
- vpdN: = vinyl phosphonate deoxyribonucleotide
- (5Me-Nf): = 5'-Me, 2'-fluoro nucleotide
- cPrp: = cyclopropyl phosphonate, see Table 6
- epTcPr: = see Table 6
- epTM: = see Table 6

The person or ordinary skill in the art would readily understand that the terminal nucleotide at the 3' end of a given oligonucleotide sequence would typically have a hydroxyl (-OH) group at the respective 3' position of the given monomer instead of a phosphate moiety ex vivo. Thus, for example, as shown above in the structure representation of AD05070, above, the "g" modified nucleotide on the terminal 3' end of the antisense strand of AM06606-AS has a hydroxyl group positioned at its 3' position. Unless expressly indicated otherwise herein, such understandings of the person of ordinary skill in the art are used when describing the HBV RNAi agents and compositions of HBV RNAi agents disclosed herein.

Targeting groups and linking groups include the following, for which their chemical structures are provided below in Table 6: (PAZ), (NAG13), (NAG13)s, (NAG18), (NAG18)s, (NAG24), (NAG24)s, (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), (NAG39)s. Each sense strand and/or antisense strand can have any targeting groups or linking groups listed above, as well as other targeting or linking groups, conjugated to the 5' and/or 3' end of the sequence.

**Table 3. HBV RNAi Agent antisense strand sequences.**

| AS Strand ID | Modified sequence (5' → 3') | SEQ ID NO. | Unmodified sequence (5' → 3') | SEQ ID NO. |
|---|---|---|---|---|
| AM03508-AS | usAfscCfaAfuUfuAfuGfcCfuAfcAfgGfccsusuAu | 61 | UACCAAUUUAUGCCUACAGGCCUUAU | 149 |
| AM04441-AS | usAfscCfaAfuUfuAfuGfcCfuAfcAfgGfcscsu | 62 | UACCAAUUUAUGCCUACAGGCCU | 150 |
| AM04442-AS | usAfscsCfaAfuUfuAfuGfcCfuAfcAfgGfccsu | 63 | UACCAAUUUAUGCCUACAGGCCU | 150 |
| AM04443-AS | usAfscsCfaAfuUfuAfuGfcCfuAfcAfgGfsc | 64 | UACCAAUUUAUGCCUACAGGC | 151 |
| AM04661-AS | usGfsugaAfgCfGfaaguGfcAfcacsusu | 65 | UGUGAAGCGAAGUGCACACUU | 152 |
| AM04768-AS | usAfscCfaAfuUfuAfuGfcCfuAfcAfgCfcsusccgc | 66 | UACCAAUUUAUGCCUACAGCCUCCGC | 153 |
| AM04769-AS | vpusAfscCfaAfuUfuAfuGfcCfuAfcAfgCfcsusccgc | 67 | UACCAAUUUAUGCCUACAGCCUCCGC | 153 |
| AM05011-AS | usAfscsCfaAfuUfuAfuGfcCfuAfcAfgusu | 68 | UACCAAUUUAUGCCUACAGUU | 154 |
| AM05012-AS | usAfscsCfaAfuUfuAfuGfcCfuAfcAfggsc | 69 | UACCAAUUUAUGCCUACAGGC | 151 |
| AM05013-AS | vpusAfscsCfaAfuUfuAfuGfcCfuAfcAfgGfsc | 70 | UACCAAUUUAUGCCUACAGGC | *151* |
| AM05014-AS | vpusAfscsCfaAfuUfuAfuGfcCfuAfcAfgusu | 71 | UACCAAUUUAUGCCUACAGUU | 154 |
| AM05052-AS | asUfsusGfaGfaGfaAfgUfcCfaCfcAfcGfsa | 72 | AUUGAGAGAAGUCCACCACGA | 155 |
| AM05053-AS | asUfsusGfaGfaGfaAfgUfcCfaCfcAfcgsa | 73 | AUUGAGAGAAGUCCACCACGA | 155 |
| AM05054-AS | asUfsusGfaGfaGfaAfgUfcCfaCfcAfcusu | 74 | AUUGAGAGAAGUCCACCACUU | 156 |
| AM05055-AS | vpusUfsusGfaGfaGfaAfgUfcCfaCfcAfcGfsa | 75 | UUUGAGAGAAGUCCACCACGA | 157 |
| AM05056-AS | asAfsusUfgAfgAfgAfaGfuCfcAfcCfaCfsg | 76 | AAUUGAGAGAAGUCCACCACG | 158 |
| AM05057-AS | asAfsusUfgAfgAfgAfaGfuCfcAfcCfacsg | 77 | AAUUGAGAGAAGUCCACCACG | 158 |
| AM05058-AS | asAfsusUfgAfgAfgAfaGfuCfcAfcCfausu | 78 | AAUUGAGAGAAGUCCACCAUU | 159 |
| AM05060-AS | vpusAfsusUfgAfgAfgAfaGfuCfcAfcCfaCfsg | 79 | UAUUGAGAGAAGUCCACCACG | 160 |
| AM05351-AS | usAfscsCfaAfuUfuAfuGfcCfuAfcAfgGfsu | 80 | UACCAAUUUAUGCCUACAGGU | 161 |
| AM05608-AS | usAfscCfaAfuUfuAfuGfcCfuAfcAfgsusu | 81 | UACCAAUUUAUGCCUACAGUU | 154 |
| AM05609-AS | usAfscsCfaAfuUfuAfuGfcCfuAfcAfgcsc | 82 | UACCAAUUUAUGCCUACAGCC | 162 |
| AM05610-AS | usAfscsCfaAfuUfuAfuGfcCfuAfcAfgccusu | 83 | UACCAAUUUAUGCCUACAGCCUU | 163 |
| AM05611-AS | usAfscsCfaAfuUfuAfuGfcCfuAfcAfgccusc | 84 | UACCAAUUUAUGCCUACAGCCUC | 164 |
| AM05612-AS | usAfscscaauUfuAfuGfcCfuacagcsc | 85 | UACCAAUUUAUGCCUACAGCC | 162 |
| AM05613-AS | usAfscscaauUfuAfuGfcCfuacagccusu | 86 | UACCAAUUUAUGCCUACAGCCUU | 163 |
| AM05614-AS | usAfscscaauUfuAfuGfcCfuacagccusc | 87 | UACCAAUUUAUGCCUACAGCCUC | 164 |
| AM05618-AS | asUfsusgagaGfaAfgUfcCfaccacusu | 88 | AUUGAGAGAAGUCCACCACUU | 156 |
| AM05621-AS | usUfsusGfaGfaGfaAfgUfcCfaCfcAfcusu | 89 | UUUGAGAGAAGUCCACCACUU | 165 |
| AM05623-AS | asUfsusGfaGfaGfaAfgUfcCfaCfcAfcggusu | 90 | AUUGAGAGAAGUCCACCACGGUU | 166 |
| AM05626-AS | asUfsusgagaGfaAfgUfcCfaccacggusu | 91 | AUUGAGAGAAGUCCACCACGGUU | 166 |
| AM05628-AS | asUfsusGfaGfaGfaAfgUfcCfaCfcAfcgagsu | 92 | AUUGAGAGAAGUCCACCACGAGU | 167 |
| AM05631-AS | usAfsusUfgAfgAfgAfaGfuCfcAfcCfaCfsg | 93 | UAUUGAGAGAAGUCCACCACG | 160 |
| AM05632-AS | usAfsusugagAfgAfaGfuCfcaccacsg | 94 | UAUUGAGAGAAGUCCACCACG | 160 |
| AM05633-AS | usAfsusUfgAfgAfgAfaGfuCfcAfcCfaCfgusu | 95 | UAUUGAGAGAAGUCCACCACGUU | 168 |
| AM05634-AS | usAfsusugagAfgAfaGfuCfcaccacgasg | 96 | UAUUGAGAGAAGUCCACCACGAG | 169 |
| AM05635-AS | usAfsusUfgAfgAfgAfaGfuCfcAfcCfaCfgasg | 97 | UAUUGAGAGAAGUCCACCACGAG | 169 |
| AM05637-AS | usAfsusUfgAfgAfgAfaGfuCfcAfcCfaCfgsa | 98 | UAUUGAGAGAAGUCCACCACGA | 170 |
| AM05638-AS | usAfsusugagAfgAfaGfuCfcaccacgsa | 99 | UAUUGAGAGAAGUCCACCACGA | 170 |
| AM05747-AS | asGfsasAfaAfuugagAfgAfaGfuCfcAfsc | 100 | AGAAAAUUGAGAGAAGUCCAC | 171 |
| AM05849-AS | usAfscsCfaAfuuuauGfcCfuAfcAfgusu | 101 | UACCAAUUUAUGCCUACAGUU | 154 |
| AM05850-AS | usAfscsCfaAfuuuauGfcCfuAfcAfgcsc | 102 | UACCAAUUUAUGCCUACAGCC | 162 |
| AM05851-AS | usAfscsCfaAfuuuauGfcCfuAfcAfgcusu | 103 | UACCAAUUUAUGCCUACAGCUU | 172 |
| AM05852-AS | usAfscsCfaAfuuuauGfcCfuAfcAfgccsu | 104 | UACCAAUUUAUGCCUACAGCCU | 173 |
| AM05853-AS | usAfscsCfaAfuuuauGfcCfuAfcAfgccusu | *105* | UACCAAUUUAUGCCUACAGCCUU | 163 |
| AM05854-AS | usAfscsCfaAfuuuauGfcCfuAfcAfgccusc | 106 | UACCAAUUUAUGCCUACAGCCUC | 164 |
| AM05855-AS | cPrpusAfscsCfaAfuUfuAfuGfcCfuAfcAfgusu | 107 | UACCAAUUUAUGCCUACAGUU | 154 |
| AM05860-AS | cPrpusAfsusUfgAfgAfgAfaGfuCfcAfcCfaCfsg | 108 | UAUUGAGAGAAGUCCACCACG | 160 |
| AM05862-AS | usAfsusUfgAfgagaaGfuCfcAfcCfausu | 109 | UAUUGAGAGAAGUCCACCAUU | 174 |
| AM05863-AS | usAfsusUfgAfgagaaGfuCfcAfcCfacsg | 110 | UAUUGAGAGAAGUCCACCACG | 160 |
| AM05864-AS | usAfsusUfgAfgagaaGfuCfcAfcCfacsusu | 111 | UAUUGAGAGAAGUCCACCACUU | 175 |
| AM05865-AS | usAfsusUfgAfgagaaGfuCfcAfcCfacsgsa | 112 | UAUUGAGAGAAGUCCACCACGA | 170 |
| AM05867-AS | vpusAfsusUfgAfgagaaGfuCfcAfcCfaCfsg | 113 | UAUUGAGAGAAGUCCACCACG | 160 |
| AM05873-AS | usUfsusGfaGfagaagUfcCfaCfcAfcusu | 114 | UUUGAGAGAAGUCCACCACUU | 165 |
| AM05874-AS | usUfsusGfaGfagaagUfcCfaCfcAfcgsa | 115 | UUUGAGAGAAGUCCACCACGA | 157 |
| AM05875-AS | usUfsusGfaGfagaagUfcCfaCfcAfcgusu | 116 | UUUGAGAGAAGUCCACCACGUU | 176 |
| AM05876-AS | usUfsusGfaGfagaagUfcCfaCfcAfcgasg | 117 | UUUGAGAGAAGUCCACCACGAG | 177 |
| AM05877-AS | cPrpusUfsusGfaGfaGfaAfgUfcCfaCfcAfcusu | 118 | UUUGAGAGAAGUCCACCACUU | 165 |
| AM06074-AS | cPrpusAfsusUfgAfgagaaGfuCfcAfcCfacsusu | 119 | UAUUGAGAGAAGUCCACCACUU | 175 |
| AM06142-AS | usAfsusUfgAfgagaaGfuCfcAfcCfacusu | 120 | UAUUGAGAGAAGUCCACCACUU | 175 |
| AM06143-AS | usAfsusUfgAfgagaaGfuCfcAfcCfacgusu | 121 | UAUUGAGAGAAGUCCACCACGUU | 168 |
| AM06144-AS | usAfsusUfgAfgagaaGfuCfcAfcCfacuus(invAb) | 122 | UAUUGAGAGAAGUCCACCACUU | 175 |
| AM06145-AS | usAfsusUfgAfgagaaGfuCfcAfcCfacgasg | 123 | UAUUGAGAGAAGUCCACCACGAG | 169 |
| AM06222-AS | usAfsusUfgAfgAfgAfaGfuCfcAfcCfacusu | 124 | UAUUGAGAGAAGUCCACCACUU | 175 |
| AM06281-AS | asGfsasAfaAfuUfgAfgAfgAfaGfuCfcusu | 125 | AGAAAAUUGAGAGAAGUCCUU | 178 |
| AM06282-AS | asGfsasAfaAfuUfgAfgAfgAfaGfuCfcasc | 126 | AGAAAAUUGAGAGAAGUCCAC | 171 |
| AM06283-AS | asGfsasAfaAfuUfgAfgAfgAfaGfuCfcacusu | 127 | AGAAAAUUGAGAGAAGUCCACUU | 179 |
| AM06284-AS | asGfsasAfaAfuUfgAfgAfgAfaGfuCfcacsc | 128 | AGAAAAUUGAGAGAAGUCCACC | 180 |
| AM06285-AS | usGfsasAfaAfuUfgAfgAfgAfaGfuCfcusu | 129 | UGAAAAUUGAGAGAAGUCCUU | 152 |
| AM06286-AS | usGfsasAfaAfuUfgAfgAfgAfaGfuCfcasc | 130 | UGAAAAUUGAGAGAAGUCCAC | 181 |
| AM06299-AS | asCfscsAfaUfuUfaUfgCfcUfaCfaGfcusu | 131 | ACCAAUUUAUGCCUACAGCUU | 182 |
| AM06300-AS | asCfscsAfaUfuUfaUfgCfcUfaCfaGfccusu | 132 | ACCAAUUUAUGCCUACAGCCUU | 183 |
| AM06301-AS | asCfscsAfaUfuUfaUfgCfcUfaCfaGfccusc | 133 | ACCAAUUUAUGCCUACAGCCUC | 184 |
| AM06302-AS | usCfscsAfaU fuU faU fgCfc U faCfaGfcusu | 134 | UCCAAUUUAUGCCUACAGCUU | 185 |
| AM06303-AS | usCfscsAfaUfuUfaUfgCfcUfaCfaGfccusu | 135 | UCCAAUUUAUGCCUACAGCCUU | 186 |
| AM06463-AS | cPrpusAfscsCfaAfuUfuAfuGfcCfuAfcAfgcsc | 136 | UACCAAUUUAUGCCUACAGCC | 162 |
| AM06464-AS | usAfscsCfaAfuUfuAfuGfcCfuAfcAfgscsc | 137 | UACCAAUUUAUGCCUACAGCC | 162 |
| AM06465-AS | cPrpusAfscsCfaAfuUfuAfuGfcCfuAfcAfgscsc | 138 | UACCAAUUUAUGCCUACAGCC | 162 |
| AM06604-AS | usAfscsCfaAfuUfuAfuGfcCfuAfcAfgcsu | 139 | UACCAAUUUAUGCCUACAGCU | 187 |
| AM06606-AS | usAfscsCfaAfuUfuAfuGfcCfuAfcAfgcsg | 140 | UACCAAUUUAUGCCUACAGCG | 188 |
| AM06608-AS | asAfsesCfaAfuUfuAfuGfcCfuAfcAfgcsc | 141 | AACCAAUUUAUGCCUACAGCC | 189 |
| AM06611-AS | usAfscsCfaAfuUfUfAfuGfcCfuAfcAfgusu | 142 | UACCAAUUUAUGCCUACAGUU | 154 |
| AM06612-AS | usAfscsCfaAfuUfuAfuGfcCfuAfcAfgCfsc | 143 | UACCAAUUUAUGCCUACAGCC | 162 |
| AM06614-AS | asCfscAfaUfuUfaUfgCfcUfaCfaGfcCfsu | 144 | ACCAAUUUAUGCCUACAGCCU | 190 |
| AM06616-AS | usCfscAfaUfuUfaUfgCfcUfaCfaGfcCfsu | 145 | UCCAAUUUAUGCCUACAGCCU | 191 |
| AM06618-AS | asCfscAfaUfuUfaUfgCfcUfaCfaGfccsg | 146 | ACCAAUUUAUGCCUACAGCCG | 192 |
| AM06620-AS | usCfscAfaUfuUfaUfgCfcUfaCfaGfccsg | 147 | UCCAAUUUAUGCCUACAGCCG | 193 |
| AM06751-AS | usAfscsCfaAfuUfuAfuGfcCfuAfcAfggsg | 148 | UACCAAUUUAUGCCUACAGGG | 194 |

**Table 4. HBV RNAi agent sense strand sequences.**

| Strand ID | Modified sequence (5' → 3') | SEQ ID NO. | Unmodified sequence (5' → 3') | SEQ ID NO. |
|---|---|---|---|---|
| AM04444-SS | (NAG25)uusgsccuguagGfCfAfuaaauugguaus(invdT) | 195 | UUGCCUGUAGGCAUAAAUUGGUAUT | 275 |
| AM04445-SS | (NAG25)uauausgsccuguagGfCfAfuaaauuggu(invdA) | 196 | UAUAUGCCUGUAGGCAUAAAUUGGUA | 276 |
| AM04767-SS | (NAG25)gcggagsgcuguagGfCfAfuaaauuggTM(invdA) | 197 | GCGGAGGCUGUAGGCAUAAAUUGGTA | 277 |
| AM05010-SS | (NAG25)scsuguagGfCfAfuaaauugguauus(invAb) | 198 | CUGUAGGCAUAAAUUGGUAUU | 278 |
| AM05015-SS | (NAG25)sgsccuguagGfCfAfuaaauugguas(invAb) | 199 | GCCUGUAGGCAUAAAUUGGUA | 279 |
| AM05016-SS | (NAG25)sgsccuguagGfCfAfuaaauuggus(invdA) | 200 | GCCUGUAGGCAUAAAUUGGUA | 279 |
| AM05017-SS | (NAG25)sgsccuguagGfCfAfuaaauugguAMs(invAb) | 201 | GCCUGUAGGCAUAAAUUGGUA | 279 |
| AM05018-SS | (NAG25)sgsccuguagGfCfAfuaaauuggTMAMs(invAb) | 202 | GCCUGUAGGCAUAAAUUGGTA | 280 |
| AM05019-SS | (NAG25)sasacuguagGfCfAfuaaauugguas(invAb) | 203 | AACUGUAGGCAUAAAUUGGUA | 281 |
| AM05034-SS | (NAG25)suscguggugGfAfCfuucucucaaus(invAb) | 204 | UCGUGGUGGACUUCUCUCAAU | 282 |
| AM05046-SS | (NAG25)sasaguggugGfAfCfuucucucaaus(invAb) | 205 | AAGUGGUGGACUUCUCUCAAU | 283 |
| AM05047-SS | (NAG25)suscguggugGfAfCfuucucucaAMTMs(invAb) | 206 | UCGUGGUGGACUUCUCUCAAT | 284 |
| AM05048-SS | (NAG25)scsgugguggAfCfUfucucucaauus(invAb) | 207 | CGUGGUGGACUUCUCUCAAUU | 285 |
| AM05049-SS | (NAG25)sasaugguggAfCfUfucucucaauus(invAb) | 208 | AAUGGUGGACUUCUCUCAAUU | 286 |
| AM05050-SS | (NAG25)scsgugguggAfCfUfucucucaaTMTMs(invAb) | 209 | CGUGGUGGACUUCUCUCAATT | 287 |
| AM05051-SS | (NAG25)sgsgacuucuCfUfCfaauuuucuaas(invAb) | 210 | GGACUUCUCUCAAUUUUCUAA | 288 |
| AM05063-SS | (NAG25)scsgugguggAfCfUfucucucaauas(invAb) | 211 | CGUGGUGGACUUCUCUCAAUA | 289 |
| AM05064-SS | (NAG25)suscguggugGfAfCfuucucucaaas(invAb) | 212 | UCGUGGUGGACUUCUCUCAAA | 290 |
| AM05346-SS | (NAG31)sasccuguagGfCfAfuaaauugguas(invAb) | 213 | ACCUGUAGGCAUAAAUUGGUA | 291 |
| AM05347-SS | (NAG31)s(invAb)scuguagGfCfAfuaaauugguas(invAb) | 214 | CUGUAGGCAUAAAUUGGUA | 292 |
| AM05606-SS | (NAG25)s(invAb)scuguagGfCfAfuaaauugguas(invAb) | 215 | CUGUAGGCAUAAAUUGGUA | 292 |
| AM05607-SS | (NAG37)s(invAb)scuguagGfCfAfuaaauugguas(invAb) | 216 | CUGUAGGCAUAAAUUGGUA | 292 |
| AM05615-SS | (NAG25)s(invAb)sacuguagGfCfAfuaaauugguas(invAb) | 217 | ACUGUAGGCAUAAAUUGGUA | 293 |
| AM05616-SS | (NAG25)sgsgcuguagGfCfAfuaaauugguas(invAb) | 218 | GGCUGUAGGCAUAAAUUGGUA | 294 |
| AM05617-SS | (NAG37)sasaguggugGfAfCfuucucucaaus(invAb) | 219 | AAGUGGUGGACUUCUCUCAAU | 283 |
| AM05620-SS | (NAG25)sasaguggugGfAfCfuucucucaaas(invAb) | 220 | AAGUGGUGGACUUCUCUCAAA | 295 |
| AM05622-SS | (NAG25)scscguggugGfAfCfuucucucaaus(invAb) | 221 | CCGUGGUGGACUUCUCUCAAU | 296 |
| AM05624-SS | (NAG25)s(invAb)sccguggugGfAfCfuucucucaaus(invAb) | 222 | CCGUGGUGGACUUCUCUCAAU | 296 |
| AM05627-SS | (NAG25)scsucguggugGfAfCfuucucucaaus(invAb) | 223 | CUCGUGGUGGACUUCUCUCAAU | 297 |
| AM05629-SS | (NAG25)s(invAb)sguggugGfAfCfuucucucaaus(invAb) | 224 | GUGGUGGACUUCUCUCAAU | 298 |
| AM05630-SS | (NAG25)s(invAb)sguggugGfAfCfuucucucaauusu(invAb) | 225 | GUGGUGGACUUCUCUCAAUUU | 299 |
| AM05636-SS | (NAG25)suscgugguggAfCfUfucucucaauus(invAb) | 226 | UCGUGGUGGACUUCUCUCAAUU | 300 |
| AM05639-SS | (NAG25)s(invAb)sugguggAfCfUfucucucaauus(invAb) | 227 | UGGUGGACUUCUCUCAAUU | 301 |
| AM05640-SS | (NAG37)s(invAb)sugguggAfCfUfucucucaauus(invAb) | 228 | UGGUGGACUUCUCUCAAUU | 301 |
| AM05746-SS | (NAG25)sgsuggacuuCfUfCfucaauuuucus(invAb) | 229 | GUGGACUUCUCUCAAUUUUCU | 302 |
| AM05856-SS | (NAG25)s(invAb)scuguagGfCfAfuaaauugguausu(invAb) | 230 | CUGUAGGCAUAAAUUGGUAUU | 278 |
| AM05857-SS | (NAG25)s(invAb)sgcuguagGfCfAfuaaauugguausu(invAb) | 231 | GCUGUAGGCAUAAAUUGGUAUU | 303 |
| AM05858-SS | (NAG25)s(invAb)sggcuguagGfCfAfuaaauugguausu(invAb) | 232 | GGCUGUAGGCAUAAAUUGGUAUU | 304 |
| AM05859-SS | (NAG25)s(invAb)saacuguagGfCfAfuaaauugguausu(invAb) | 233 | AACUGUAGGCAUAAAUUGGUAUU | *305* |
| AM05868-SS | (NAG25)s(invAb)ugguggAfCfUfucucucaauausu(invAb) | 234 | UGGUGGACUUCUCUCAAUAUU | 306 |
| AM05869-SS | (NAG25)s(invAb)sgugguggAfCfUfucucucaauausu(invAb) | 235 | GUGGUGGACUUCUCUCAAUAUU | 307 |
| AM05870-SS | (NAG25)sasaugguggAfCfUfucucucaauausu(invAb) | 236 | AAUGGUGGACUUCUCUCAAUAUU | 308 |
| AM05871-SS | (NAG25)scsgugguggAfCfUfucucucaauausu(invAb) | 237 | CGUGGUGGACUUCUCUCAAUAUU | 309 |
| AM05872-SS | (NAG31)scsgugguggAfCfUfucucucaauas(invAb) | 238 | CGUGGUGGACUUCUCUCAAUA | 289 |
| AM05879-SS | (NAG25)s(invAb)saaguggugGfAfCfuucucucaaus(invAb) | 239 | AAGUGGUGGACUUCUCUCAAU | 283 |
| AM05880-SS | (NAG25)s(invAb)sguggugGfAfCfuucucucaaausu(invAb) | 240 | GUGGUGGACUUCUCUCAAAUU | 310 |
| AM05881-SS | (NAG25)s(invAb)scguggugGfAfCfuucucucaaausu(invAb) | 241 | CGUGGUGGACUUCUCUCAAAUU | 311 |
| AM05882-SS | (NAG25)sasaguggugGfAfCfuucucucaaausu(invAb) | 242 | AAGUGGUGGACUUCUCUCAAAUU | 312 |
| AM05883-SS | (NAG25)suscguggugGfAfCfuucucucaaausu(invAb) | 243 | UCGUGGUGGACUUCUCUCAAAUU | 313 |
| AM06146-SS | (NAG37)s(invAb)sgugguggAfCfUfucucucaauausu(invAb) | 244 | GUGGUGGACUUCUCUCAAUAUU | 307 |
| AM06147-SS | (NAG37)s(invAb)scgugguggAfCfUfucucucaauausu(invAb) | 245 | CGUGGUGGACUUCUCUCAAUAUU | 309 |
| AM06148-SS | (NAG37)s(invAb)scucgugguggAfCfUfucucucaauas(invAb) | 246 | CUCGUGGUGGACUUCUCUCAAUA | 314 |
| AM06149-SS | (NAG37)s(invAb)scucgugguggAfCfUfucucucaauausu(invAb) | 247 | CUCGUGGUGGACUUCUCUCAAUAUU | 315 |
| AM06150-SS | (NAG37)s(invAb)sggcuguagGfCfAfuaaauugguas(invAb) | 248 | GGCUGUAGGCAUAAAUUGGUA | 294 |
| AM06151-SS | (NAG37)s(invAb)sgaggcuguagGfCfAfuaaauugguas(invAb) | 249 | GAGGCUGUAGGCAUAAAUUGGUA | 316 |
| AM06152-SS | (NAG37)s(invAb)sgaggcuguagGfCfAfuaaauugguausu(invAb) | 250 | GAGGCUGUAGGCAUAAAUUGGUAUU | 317 |
| AM06287-SS | (NAG37)s(invAb)sggacuuCfUfCfucaauuuucus(invAb) | 251 | GGACUUCUCUCAAUUUUCU | 318 |
| AM06288-SS | (NAG37)s(invAb)sguggacuuCfUfCfucaauuuucus(invAb) | 252 | GUGGACUUCUCUCAAUUUUCU | 302 |
| AM06289-SS | (NAG37)s(invAb)sgguggacuuCfUfCfucaauuuucus(invAb) | 253 | GGUGGACUUCUCUCAAUUUUCU | 319 |
| AM06290-SS | (NAG37)s(invAb)sggacuuCfUfCfucaauuuucas(invAb) | 254 | GGACUUCUCUCAAUUUUCA | 320 |
| AM06291-SS | (NAG37)s(invAb)sguggacuuCfUfCfucaauuuucas(invAb) | 255 | GUGGACUUCUCUCAAUUUUCA | 321 |
| AM06304-SS | (NAG37)s(invAb)sgcuguaGfGfCfauaaauuggus(invAb) | 256 | GCUGUAGGCAUAAAUUGGU | 322 |
| AM06305-SS | (NAG37)s(invAb)sggcuguaGfGfCfauaaauuggus(invAb) | 257 | GGCUGUAGGCAUAAAUUGGU | 323 |
| AM06306-SS | (NAG37)s(invAb)sgaggcuguaGfGfCfauaaauuggus(invAb) | 258 | GAGGCUGUAGGCAUAAAUUGGU | 324 |
| AM06307-SS | (NAG37)s(invAb)sgcuguaGfGfCfauaaauuggas(invAb) | 259 | GCUGUAGGCAUAAAUUGGA | 325 |
| AM06308-SS | (NAG37)s(invAb)sggcuguaGfGfCfauaaauuggas(invAb) | 260 | GGCUGUAGGCAUAAAUUGGA | 326 |
| AM06603-SS | (NAG37)s(invAb)sagcuguagGfCfAfuaaauugguas(invAb) | 261 | AGCUGUAGGCAUAAAUUGGUA | 327 |
| AM06605-SS | (NAG37)s(invAb)scgcuguagGfCfAfuaaauugguas(invAb) | 262 | CGCUGUAGGCAUAAAUUGGUA | 328 |
| AM06607-SS | (NAG37)s(invAb)sggcuguagGfCfAfuaaauugguus(invAb) | 263 | GGCUGUAGGCAUAAAUUGGUU | 329 |
| AM06609-SS | (NAG37)s(invAb)scuguagGfCfAfuaaauugguasuus(invAb) | 264 | CUGUAGGCAUAAAUUGGUAUU | 278 |
| AM06610-SS | (NAG37)s(invAb)scuGfuAfgGfCfAfuAfaAfuUfgGfuasuus(invAb) | 265 | CUGUAGGCAUAAAUUGGUAUU | 278 |
| AM06613-SS | (NAG37)s(invAb)saggcuguaGfGfCfauaaauuggus(invAb) | 266 | AGGCUGUAGGCAUAAAUUGGU | 330 |
| AM06615-SS | (NAG37)s(invAb)saggcuguaGfGfCfauaaauuggas(invAb) | 267 | AGGCUGUAGGCAUAAAUUGGA | 331 |
| AM06617-SS | (NAG37)s(invAb)scggcuguaGfGfCfauaaauuggus(invAb) | 268 | CGGCUGUAGGCAUAAAUUGGU | 332 |
| AM06619-SS | (NAG37)s(invAb)scggcuguaGfGfCfauaaauuggas(invAb) | 269 | CGGCUGUAGGCAUAAAUUGGA | 333 |
| AM06750-SS | (NAG37)s(invAb)scccuguagGfCfAfuaaauugguas(invAb) | 270 | CCCUGUAGGCAUAAAUUGGUA | 334 |
| AM06752-SS | (NAG37)csgcuguagGfCfAfuaaauugguas(invAb) | 271 | CGCUGUAGGCAUAAAUUGGUA | 328 |
| AM06753-SS | (NAG37)csccuguagGfCfAfuaaauugguas(invAb) | 272 | CCCUGUAGGCAUAAAUUGGUA | 334 |
| AM06776-SS | (NAG25)s(invAb)sguggacuuCfUfCfucaauuuucus(invAb) | 273 | GUGGACUUCUCUCAAUUUUCU | 302 |
| AM06777-SS | (NAG25)s(invAb)scgcuguagGfCfAfuaaauugguas(invAb) | 274 | CGCUGUAGGCAUAAAUUGGUA | 328 |

The HBV RNAi agents described herein are formed by annealing an antisense strand with a sense strand. A sense strand containing a sequence listed in Table 4 can be hybridized to any antisense strand containing a sequence listed in Table 3, provided the two sequences have a region of at least about 85% complementarity over a contiguous 16, 17, 18, 19, 20, or 21 nucleotide sequence.

In some embodiments, the antisense strand of an HBV RNAi agent disclosed herein differs by 0, 1, 2, or 3 nucleotides from any of the antisense strand sequences in Table 3. In some embodiments, the sense strand of an HBV RNAi agent disclosed herein differs by 0, 1, 2, or 3 nucleotides from any of the sense strand sequences in Table 4.

In some embodiments, an HBV RNAi agent antisense strand comprises a nucleotide sequence of any of the sequences in Table 3. In some embodiments, an HBV RNAi agent antisense strand comprises the sequence of nucleotides (from 5' end → 3' end) 1-17, 2-17, 1-18, 2-18, 1-19, 2-19, 1-20, 2-20, 1-21, 2-21, 1-22, 2-22, 1-23, 2-23, 1-24, 2-24, 1-25, 2-25, 1-26, or 2-26 of any of the sequences in Table 3.

In some embodiments, an HBV RNAi agent sense strand comprises the nucleotide sequence of any of the sequences in Table 4. In some embodiments, an HBV RNAi agent sense strand comprises the sequence of nucleotides (from 5' end → 3' end) 1-17, 2-17, 3-17, 4-17, 1-18, 2-18, 3-18, 4-18, 1-19, 2-19, 3-19, 4-19, 1-20, 2-20, 3-20, 4-20, 1-21, 2-21, 3-21, 4-21, 1-22, 2-22, 3-22, 4-22, 1-23, 2-23, 3-23, 4-23, 1-24, 2-24, 3-24, 4-24, 1-25, 2-25, 3-25, 4-25, 1-26, 2-26, 3-26, or 4-26 of any of the sequences in Table 4.

For the HBV RNAi agents disclosed herein, the nucleotide at position 1 of the antisense strand (from 5' end → 3' end) can be perfectly complementary to an HBV gene, or can be non-complementary to an HBV gene. In some embodiments, the nucleotide at position 1 of the antisense strand (from 5' end → 3' end) is a U, A, or dT. In some embodiments, the nucleotide at position 1 of the antisense strand (from 5' end → 3' end) forms an A:U or U:A base pair with the sense strand.

In some embodiments, an HBV RNAi agent antisense strand comprises the sequence of nucleotides (from 5' end → 3' end) 2-18 or 2-19 of any of the antisense strand sequences in Table 3. In some embodiments, an HBV RNAi sense strand comprises the sequence of nucleotides (from 5' end → 3' end) 1-17 or 1-18 of any of the sense strand sequences in Table 4.

In some embodiments, an HBV RNAi agent includes (i) an antisense strand comprising the sequence of nucleotides (from 5' end → 3' end) 2-18 or 2-19 of any of the antisense strand sequences in Table 3, and (ii) a sense strand comprising the sequence of nucleotides (from 5' end → 3' end) 1-17 or 1-18 of any of the sense strand sequences in Table 4.

A sense strand containing a sequence listed in Table 4 can be hybridized to any antisense strand containing a sequence listed in Table 3 provided the two sequences have a region of at least about 85% complementarity over a contiguous 16, 17, 18, 19, 20, or 21 nucleotide sequence. Representative sequence pairings are exemplified by the Duplex ID Nos. shown in Table 5.

In some embodiments, an HBV RNAi agent comprises of any of the Duplex ID Nos. presented herein. In some embodiments, an HBV RNAi agent consists of any of the Duplex ID Nos. presented herein. In some embodiments, an HBV RNAi agent comprises the sense strand and/or the antisense strand nucleotide sequences of any of the Duplex ID Nos. presented herein. In some embodiments, an HBV RNAi agent comprises the sense strand and antisense strand nucleotide sequences of any of the Duplex ID Nos. presented herein and a targeting group and/or linking group wherein the targeting group and/or linking group is covalently linked (i.e. conjugated) to the sense strand or the antisense strand. In some embodiments, an HBV RNAi agent comprises the sense strand and antisense strand modified nucleotide sequences of any of the Duplex ID Nos. presented herein. In some embodiments, an HBV RNAi agent comprises the sense strand and antisense strand modified nucleotide sequences of any of the Duplex ID Nos. presented herein and a targeting group and/or linking group wherein the targeting group and/or linking group is covalently linked to the sense strand or the antisense strand.

In some embodiments, an HBV RNAi agent comprises an antisense strand and a sense strand having the nucleotide sequences of any of the antisense strand/sense strand duplexes of Table 5, and further comprises an asialoglycoprotein receptor ligand targeting group.

In some embodiments, an HBV RNAi agent comprises an antisense strand and a sense strand having the nucleotide sequences of any of the antisense strand and/or sense strand nucleotide sequences of any of the duplexes of Table 5, and further comprises a targeting group selected from the group consisting of (PAZ), (NAG13), (NAG13)s, (NAG18), (NAG18)s, (NAG24), (NAG24)s, (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s.

In some embodiments, an HBV RNAi agent comprises an antisense strand and a sense strand having the modified nucleotide sequences of any of the antisense strand and/or sense strand nucleotide sequences of any of the duplexes of Table 5.

In some embodiments, an HBV RNAi agent comprises an antisense strand and a sense strand having the modified nucleotide sequences of any of the antisense strand and/or sense strand nucleotide sequences of any of the duplexes of Table 5, and further comprises an asialoglycoprotein receptor ligand targeting group.

In some embodiments, an HBV RNAi agent comprises any of the duplexes of Table 5.

In some embodiments, an HBV RNAi agent consists of any of the duplexes of Table 5.

**Table 5. Examples of HBV RNAi agent duplexes.**

| **Duplex ID** | **Antisense Strand ID** | **Sense Strand ID** |
|---|---|---|
| AD03498 | AM03508-AS | AM04445-SS |
| AD03499 | AM04441-AS | AM04444-SS |
| AD03500 | AM04442-AS | AM04444-SS |
| AD03501 | AM04443-AS | AM04444-SS |
| AD03738 | AM04768-AS | AM04767-SS |
| AD03739 | AM04769-AS | AM04767-SS |
| AD03967 | AM04443-AS | AM05010-SS |
| AD03968 | AM05011-AS | AM05010-SS |
| AD03969 | AM04443-AS | AM05015-SS |
| AD03970 | AM05011-AS | AM05019-SS |
| AD03971 | AM05012-AS | AM05015-SS |
| AD03972 | AM04443-AS | AM05016-SS |
| AD03973 | AM04443-AS | AM05017-SS |
| AD03974 | AM04443-AS | AM05018-SS |
| AD03975 | AM05013-AS | AM05015-SS |
| AD03976 | AM05014-AS | AM05019-SS |
| AD03977 | AM05013-AS | AM05017-SS |
| AD03978 | AM05013-AS | AM04444-SS |
| AD04001 | AM05052-AS | AM05034-SS |
| AD04002 | AM05053-AS | AM05034-SS |
| AD04003 | AM05054-AS | AM05046-SS |
| AD04004 | AM05052-AS | AM05047-SS |
| AD04005 | AM05055-AS | AM05064-SS |
| AD04006 | AM05056-AS | AM05048-SS |
| AD04007 | AM05057-AS | AM05048-SS |
| AD04008 | AM05058-AS | AM05049-SS |
| AD04009 | AM05056-AS | AM05050-SS |
| AD04010 | AM05060-AS | AM05063-SS |
| AD04176 | AM05351-AS | AM05346-SS |
| AD04177 | AM04443-AS | AM05347-SS |
| AD04178 | AM05011-AS | AM05347-SS |
| AD04412 | AM05011-AS | AM05606-SS |
| AD04413 | AM05011-AS | AM05607-SS |
| AD04414 | AM05608-AS | AM05606-SS |
| AD04415 | AM05011-AS | AM05615-SS |
| AD04416 | AM05609-AS | AM05616-SS |
| AD04417 | AM05610-AS | AM05616-SS |
| AD04418 | AM05611-AS | AM05616-SS |
| AD04419 | AM05612-AS | AM05616-SS |
| AD04420 | AM05613-AS | AM05616-SS |
| AD04421 | AM05614-AS | AM05616-SS |
| AD04422 | AM05054-AS | AM05617-SS |
| AD04423 | AM05618-AS | AM05046-SS |
| AD04425 | AM05621-AS | AM05620-SS |
| AD04426 | AM05623-AS | AM05622-SS |
| AD04427 | AM05623-AS | AM05624-SS |
| AD04428 | AM05626-AS | AM05622-SS |
| AD04429 | AM05626-AS | AM05624-SS |
| AD04430 | AM05628-AS | AM05627-SS |
| AD04431 | AM05054-AS | AM05629-SS |
| AD04432 | AM05054-AS | AM05630-SS |
| AD04433 | AM05631-AS | AM05048-SS |
| AD04434 | AM05632-AS | AM05048-SS |
| AD04435 | AM05633-AS | AM05048-SS |
| AD04436 | AM05635-AS | AM05048-SS |
| AD04437 | AM05634-AS | AM05048-SS |
| AD04438 | AM05637-AS | AM05636-SS |
| AD04439 | AM05638-AS | AM05636-SS |
| AD04440 | AM05058-AS | AM05639-SS |
| AD04441 | AM05057-AS | AM05639-SS |
| AD04442 | AM05057-AS | AM05640-SS |
| AD04511 | AM05747-AS | AM05746-SS |
| AD04570 | AM05011-AS | AM05856-SS |
| AD04571 | AM05849-AS | AM05856-SS |
| AD04572 | AM05850-AS | AM05856-SS |
| AD04573 | AM05851-AS | AM05857-SS |
| AD04574 | AM05852-AS | AM05857-SS |
| AD04575 | AM05853-AS | AM05858-SS |
| AD04576 | AM05854-AS | AM05858-SS |
| AD04577 | AM05011-AS | AM05859-SS |
| AD04578 | AM05850-AS | AM05858-SS |
| AD04579 | AM05014-AS | AM05347-SS |
| AD04580 | AM05855-AS | AM05347-SS |
| AD04581 | AM05860-AS | AM05063-SS |
| AD04583 | AM05862-AS | AM05868-SS |
| AD04584 | AM05863-AS | AM05868-SS |
| AD04585 | AM05864-AS | AM05869-SS |
| AD04586 | AM05865-AS | AM05869-SS |
| AD04587 | AM05862-AS | AM05870-SS |
| AD04588 | AM05863-AS | AM05871-SS |
| AD04590 | AM05867-AS | AM05063-SS |
| AD04591 | AM05860-AS | AM05872-SS |
| AD04592 | AM05054-AS | AM05879-SS |
| AD04593 | AM05873-AS | AM05880-SS |
| AD04594 | AM05874-AS | AM05880-SS |
| AD04595 | AM05875-AS | AM05881-SS |
| AD04596 | AM05876-AS | AM05881-SS |
| AD04597 | AM05873-AS | AM05882-SS |
| AD04598 | AM05874-AS | AM05883-SS |
| AD04599 | AM05877-AS | AM05620-SS |
| AD04734 | AM06074-AS | AM05869-SS |
| AD04771 | AM06142-AS | AM06146-SS |
| AD04772 | AM06143-AS | AM06147-SS |
| AD04773 | AM06144-AS | AM06146-SS |
| AD04774 | AM06145-AS | AM06148-SS |
| AD04775 | AM06145-AS | AM06149-SS |
| AD04776 | AM05850-AS | AM06150-SS |
| AD04777 | AM05854-AS | AM06151-SS |
| AD04778 | AM05854-AS | AM06152-SS |
| AD04822 | AM06222-AS | AM06146-SS |
| AD04823 | AM05609-AS | AM06150-SS |
| AD04871 | AM06281-AS | AM06287-SS |
| AD04872 | AM06282-AS | AM06288-SS |
| AD04873 | AM06283-AS | AM06288-SS |
| AD04874 | AM06284-AS | AM06289-SS |
| AD04875 | AM06285-AS | AM06290-SS |
| AD04876 | AM06286-AS | AM06291-SS |
| AD04881 | AM06299-AS | AM06304-SS |
| AD04882 | AM06300-AS | AM06305-SS |
| AD04883 | AM06301-AS | AM06306-SS |
| AD04884 | AM06302-AS | AM06307-SS |
| AD04885 | AM06303-AS | AM06308-SS |
| AD04962 | AM05864-AS | AM06146-SS |
| AD04963 | AM05855-AS | AM05607-SS |
| AD04981 | AM06463-AS | AM06150-SS |
| AD04982 | AM06464-AS | AM06150-SS |
| AD04983 | AM06465-AS | AM06150-SS |
| AD05069 | AM06604-AS | AM06603-SS |
| AD05070 | AM06606-AS | AM06605-SS |
| AD05071 | AM06608-AS | AM06607-SS |
| AD05072 | AM05011-AS | AM06609-SS |
| AD05073 | AM06611-AS | AM06610-SS |
| AD05074 | AM06612-AS | AM06150-SS |
| AD05075 | AM06614-AS | AM06613-SS |
| AD05076 | AM06616-AS | AM06615-SS |
| AD05077 | AM06618-AS | AM06617-SS |
| AD05078 | AM06620-AS | AM06619-SS |
| AD05147 | AM06751-AS | AM06750-SS |
| AD05148 | AM06606-AS | AM06752-SS |
| AD05149 | AM06751-AS | AM06753-SS |
| AD05164 | AM06282-AS | AM06776-SS |
| AD05165 | AM06606-AS | AM06777-SS |

In some embodiments, an HBV RNAi agent is prepared or provided as a salt, mixed salt, or a free-acid. The RNAi agents described herein, upon delivery to a cell expressing an HBV gene, inhibit or knockdown expression of one or more HBV genes *in vivo.*

### Targeting Groups, Linking Groups, and Delivery Vehicles

In some embodiments, an HBV RNAi agent is conjugated to one or more non-nucleotide groups including, but not limited to a targeting group, linking group, delivery polymer, or a delivery vehicle. The non-nucleotide group can enhance targeting, delivery or attachment of the RNAi agent. Examples of targeting groups and linking groups are provided in Table 6. The non-nucleotide group can be covalently linked to the 3' and/or 5' end of either the sense strand and/or the antisense strand. In some embodiments, an HBV RNAi agent contains a non-nucleotide group linked to the 3' and/or 5' end of the sense strand. In some embodiments, a non-nucleotide group is linked to the 5' end of an HBV RNAi agent sense strand. A non-nucleotide group may be linked directly or indirectly to the RNAi agent via a linker/linking group. In some embodiments, a non-nucleotide group is linked to the RNAi agent via a labile, cleavable, or reversible bond or linker.

In some embodiments, a non-nucleotide group enhances the pharmacokinetic or biodistribution properties of an RNAi agent or conjugate to which it is attached to improve cell- or tissue-specific distribution and cell-specific uptake of the conjugate. In some embodiments, a non-nucleotide group enhances endocytosis of the RNAi agent.

Targeting groups or targeting moieties enhance the pharmacokinetic or biodistribution properties of a conjugate to which they are attached to improve cell-specific distribution and cell-specific uptake of the conjugate. A targeting group can be monovalent, divalent, trivalent, tetravalent, or have higher valency. Representative targeting groups include, without limitation, compounds with affinity to cell surface molecule, cell receptor ligands, hapten, antibodies, monoclonal antibodies, antibody fragments, and antibody mimics with affinity to cell surface molecules. In some embodiments, a targeting group is linked to an RNAi agent using a linker, such as a PEG linker or one, two, or three abasic and/or ribitol (abasic ribose) groups. In some embodiments, a targeting group comprises a galactose derivative cluster.

The HBV RNAi agents described herein may be synthesized having a reactive group, such as an amine group, at the 5'-terminus. The reactive group may be used to subsequently attach a targeting moiety using methods typical in the art.

In some embodiments, a targeting group comprises an asialoglycoprotein receptor ligand. In some embodiments, an asialoglycoprotein receptor ligand includes or consists of one or more galactose derivatives. As used herein, the term galactose derivative includes both galactose and derivatives of galactose having affinity for the asialoglycoprotein receptor that is equal to or greater than that of galactose. Galactose derivatives include, but are not limited to: galactose, galactosamine, N-formylgalactosamine, N-acetyl-galactosamine, N-propionyl-galactosamine, N-n-butanoyl-galactosamine, and N-iso-butanoylgalactos-amine (see for example: Iobst, S.T. and Drickamer, K. J.B.C. 1996, 271, 6686). Galactose derivatives, and clusters of galactose derivatives, that are useful for in vivo targeting of oligonucleotides and other molecules to the liver are known in the art (see, for example, Baenziger and Fiete, 1980, Cell, 22, 611-620; Connolly et al., 1982, J. Biol. Chem., 257, 939-945). Galactose derivatives have been used to target molecules to hepatocytes in vivo through their binding to the asialoglycoprotein receptor (ASGPr) expressed on the surface of hepatocytes. Binding of ASGPr ligands to the ASGPr(s) facilitates cell-specific targeting to hepatocytes and endocytosis of the molecule into hepatocytes. ASGPr ligands can be monomeric (e.g., having a single galactose derivative) or multimeric (e.g., having multiple galactose derivatives). The galactose derivative or galactose derivative cluster may be attached to the 3' or 5' end of the RNAi polynucleotide using methods known in the art. The preparation of targeting groups, such as galactose derivative clusters, is described in, for example, U.S. Patent Application Serial Nos. 15/452,324 and 15/452,423, the contents of both of which are incorporated herein in their entirety.

As used herein, a galactose derivative cluster comprises a molecule having two to four terminal galactose derivatives. A terminal galactose derivative is attached to a molecule through its C-1 carbon. In some embodiments, the galactose derivative cluster is a galactose derivative trimer (also referred to as tri-antennary galactose derivative or tri-valent galactose derivative). In some embodiments, the galactose derivative cluster comprises N-acetyl-galactosamines. In some embodiments, the galactose derivative cluster comprises three N-acetyl-galactosamines. In some embodiments, the galactose derivative cluster is a galactose derivative tetramer (also referred to as tetra-antennary galactose derivative or tetra-valent galactose derivative). In some embodiments, the galactose derivative cluster comprises four N-acetyl-galactosamines.

As used herein, a galactose derivative trimer contains three galactose derivatives, each linked to a central branch point. As used herein, a galactose derivative tetramer contains four galactose derivatives, each linked to a central branch point. The galactose derivatives can be attached to the central branch point through the C-1 carbons of the saccharides. In some embodiments, the galactose derivatives are linked to the branch point via linkers or spacers. In some embodiments, the linker or spacer is a flexible hydrophilic spacer, such as a PEG group (see, for example, U.S. Patent No. 5,885,968; Biessen et al. J. Med. Chem. 1995 Vol. 39 p. 1538-1546). In some embodiments, the PEG spacer is a PEG3 spacer. The branch point can be any small molecule which permits attachment of three galactose derivatives and further permits attachment of the branch point to the RNAi agent. An example of branch point group is a di-lysine or di-glutamate. Attachment of the branch point to the RNAi agent can occur through a linker or spacer. In some embodiments, the linker or spacer comprises a flexible hydrophilic spacer, such as, but not limited to, a PEG spacer. In some embodiments, the linker comprises a rigid linker, such as a cyclic group. In some embodiments, a galactose derivative comprises or consists of N-acetyl-galactosamine. In some embodiments, the galactose derivative cluster is comprised of a galactose derivative tetramer, which can be, for example, an N-acetyl-galactosamine tetramer.

In some embodiments, pharmaceutical compositions for delivering an HBV RNAi agent to a liver cell *in vivo* are described. Such pharmaceutical compositions can include, for example, an HBV RNAi agent conjugated to a galactose derivative cluster. In some embodiments, the galactose derivative cluster is comprised of a galactose derivative trimer, which can be, for example, an N-acetyl-galactosamine trimer, or galactose derivative tetramer, which can be, for example, an N-acetyl-galactosamine tetramer.

Targeting groups include, but are not limited to, (PAZ), (NAG13), (NAG13)s, (NAG18), (NAG18)s, (NAG24), (NAG24)s, (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), and (NAG39)s. Other targeting groups, including galactose cluster targeting ligands, are known in the art.

In some embodiments, a linking group is conjugated to the RNAi agent. The linking group facilitates covalent linkage of the agent to a targeting group or delivery polymer or delivery vehicle. The linking group can be linked to the 3' or the 5' end of the RNAi agent sense strand or antisense strand. In some embodiments, the linking group is linked to the RNAi agent sense strand. In some embodiments, the linking group is conjugated to the 5' or 3' end of an RNAi agent sense strand. In some embodiments, a linking group is conjugated to the 5' end of an RNAi agent sense strand. Examples of linking groups, include, but are not limited to: reactive groups such a primary amines and alkynes, alkyl groups, abasic nucleosides, ribitol (abasic ribose), and/or PEG groups.

A linker or linking group is a connection between two atoms that links one chemical group (such as an RNAi agent) or segment of interest to another chemical group (such as a targeting group or delivery polymer) or segment of interest via one or more covalent bonds. A labile linkage contains a labile bond. A linkage may optionally include a spacer that increases the distance between the two joined atoms. A spacer may further add flexibility and/or length to the linkage. Spacers may include, but are not be limited to, alkyl groups, alkenyl groups, alkynyl groups, aryl groups, aralkyl groups, aralkenyl groups, and aralkynyl groups; each of which can contain one or more heteroatoms, heterocycles, amino acids, nucleotides, and saccharides. Spacer groups are well known in the art and the preceding list is not meant to limit the scope of the description.

Any of the HBV RNAi agent nucleotide sequences listed in Tables 3 and 4, whether modified or unmodified, may contain 3' or 5' targeting group and/or linking group. Any of the HBV RNAi agent sequences listed in Table 3 and 4 which contain a 3' or 5' targeting group and/or linking group, may alternatively contain no 3' or 5' targeting group and/or linking group, or may contain a different 3' or 5' targeting group and/or linking group including, but not limited to, those depicted in Table 3. Any of the HBV RNAi agent duplexes listed in Table 5, whether modified or unmodified, may further comprise a targeting group and/or linking group, including, but not limited to, those depicted in Table 3, and the targeting group or linking group may be attached to the 3' or 5' terminus of either the sense strand or the antisense strand of the HBV RNAi agent duplex.

Examples of targeting groups and linking groups are provided in Table 6. Table 4 provides several embodiments of HBV RNAi agent sense strands having a targeting group or linking group linked to the 5' or 3' end.

**Table 6. Structures representing various modified nucleotides, targeting groups, and linking groups.**

| | |
|---|---|
| 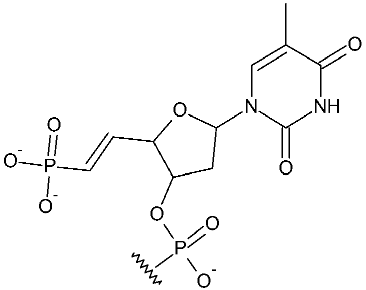 vpdT | 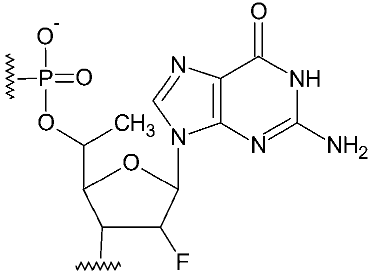 5Me-Gf |
| 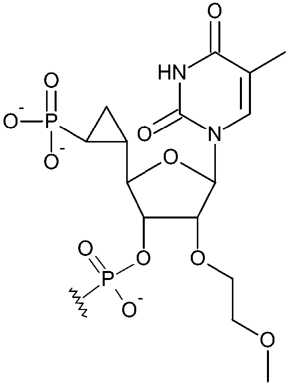 cPrpTM | 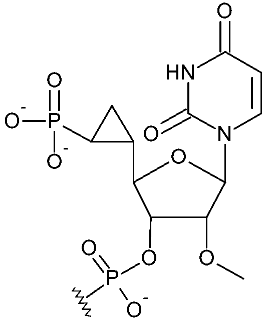 cPrpu |
| 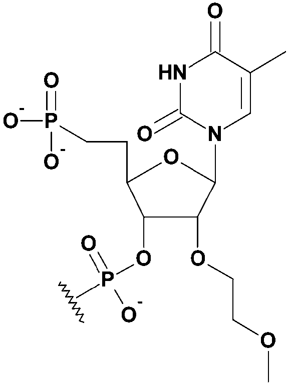 epTM | 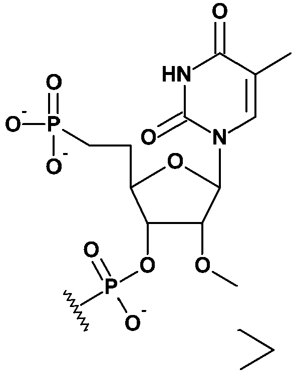 epTcPr |
| When positioned internally on oligonucleotide: | |
| linkage towards 5' end of oligonucleotide | |
| 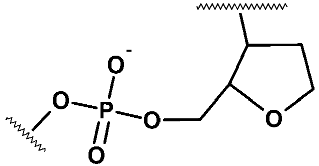 linkage towards 3' end of oligonucleotide (invAb) | |
| When positioned internally on oligonucleotide: | |
| linkage towards 5' end of oligonucleotide | |
| 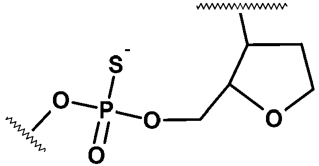 linkage towards 3' end of oligonucleotide (invAb)s | |
| When positioned at the 3' terminal end of oligonucleotide: | |
| linkage towards 5' end of oligonucleotide | |
| 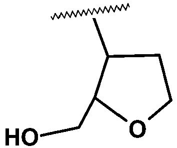 (invAb) | |
| 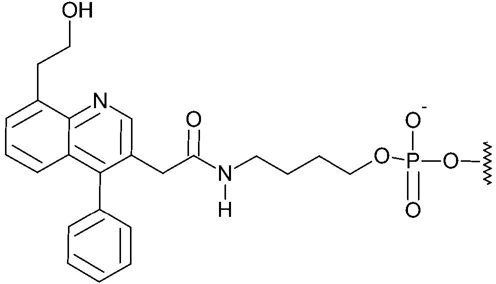 (PAZ) | |
| 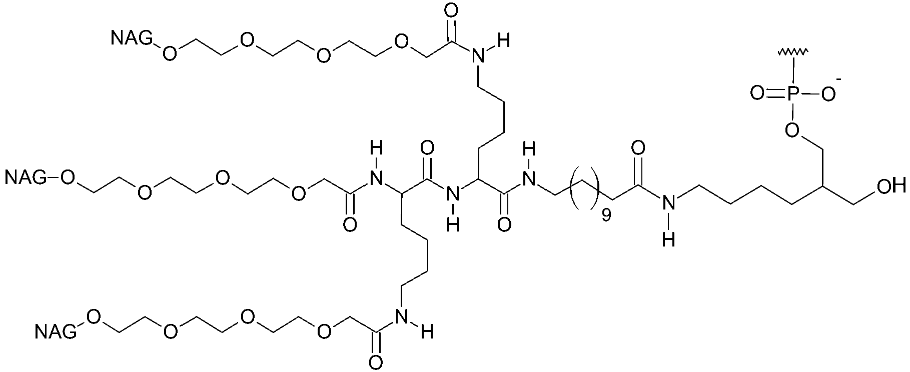 (NAG13) | |
| 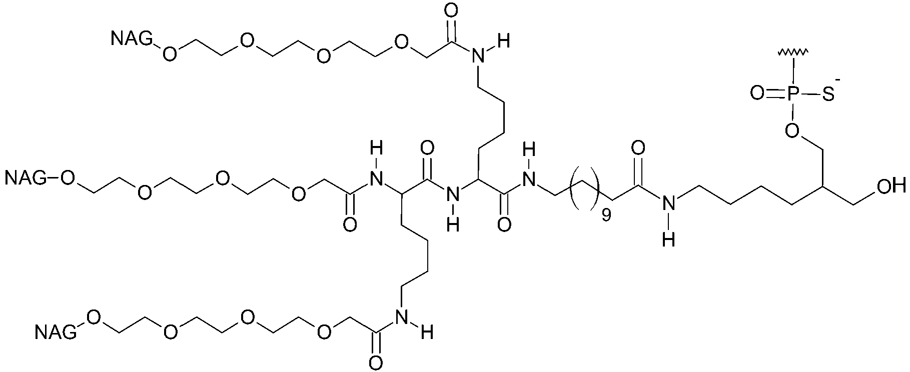 (NAG13)s | |
| 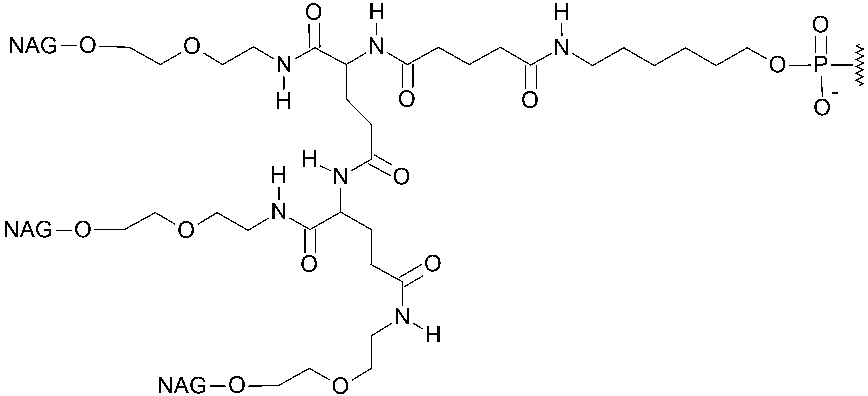 (NAG18) | |
| 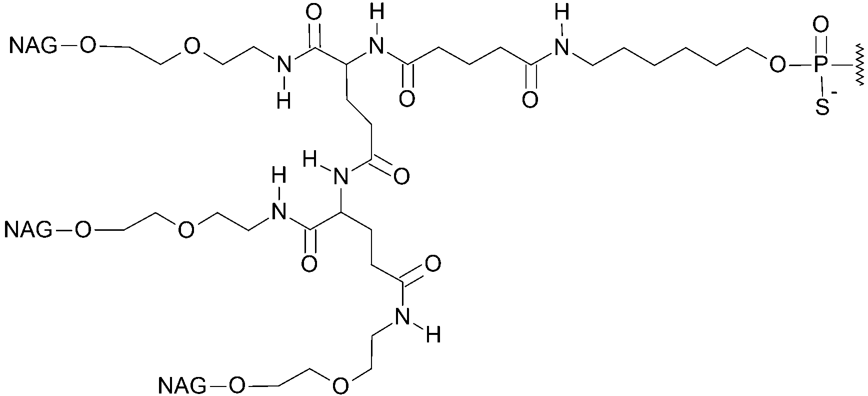 (NAG 18)s | |
| 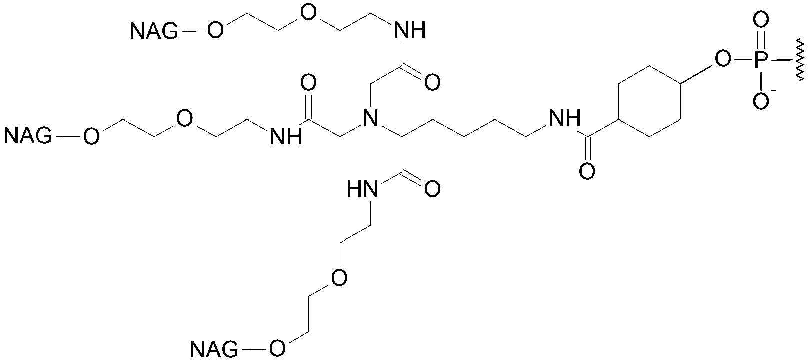 (NAG24) | |
| 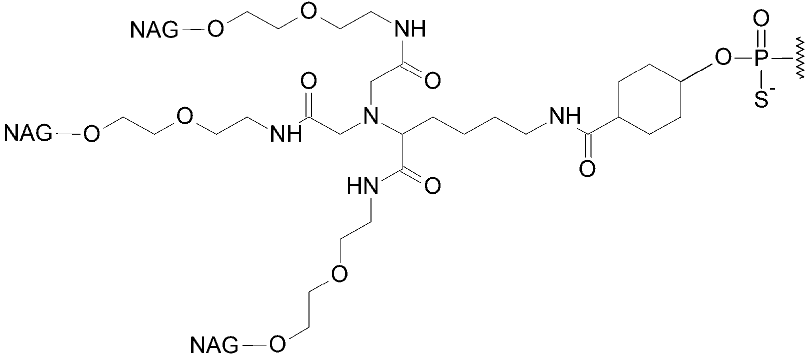 (NAG24)s | |
| 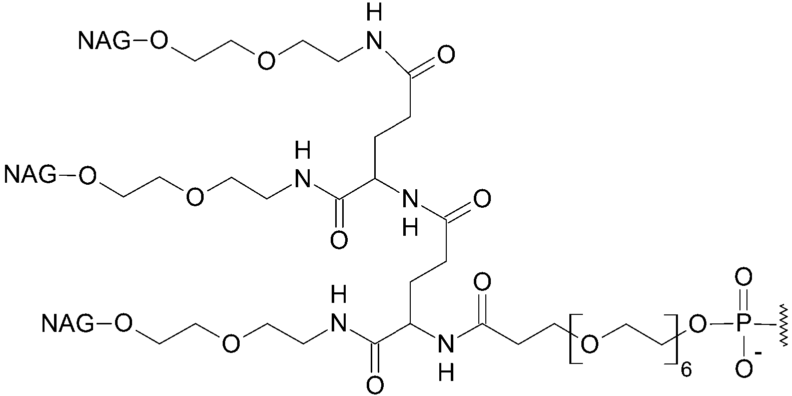 (NAG25) | |
| 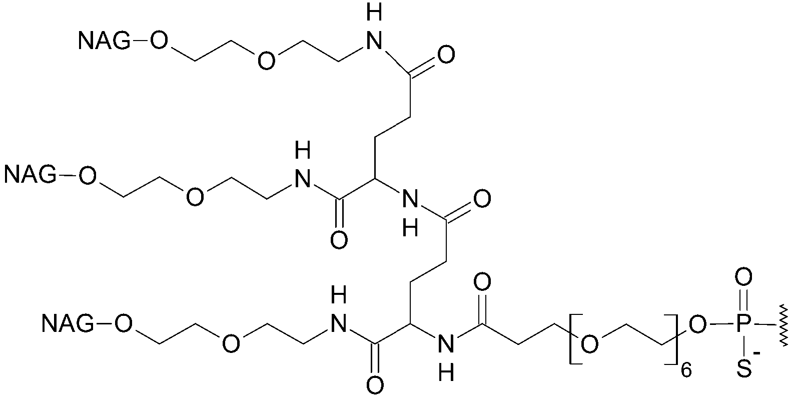 (NAG25)s | |
| 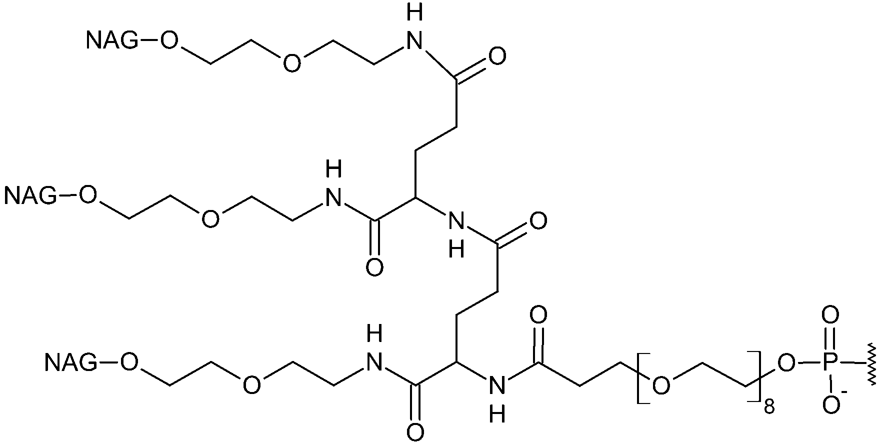 (NAG26) | |
| 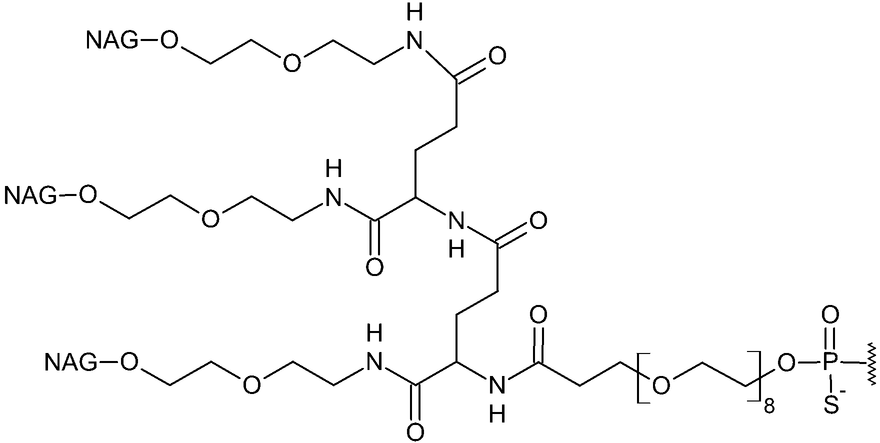 (NAG26)s | |
| 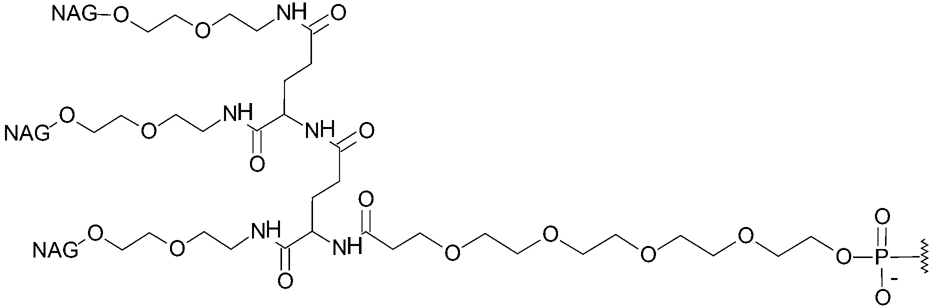 (NAG27) | |
| 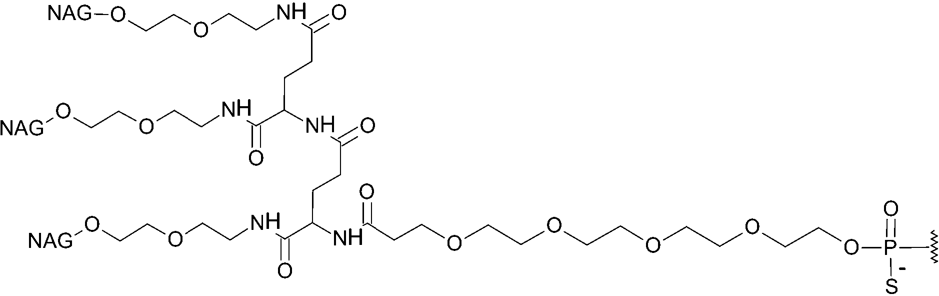 (NAG27)s | |
| 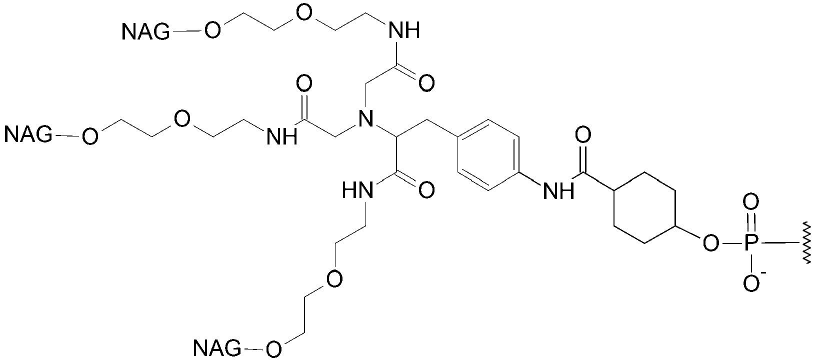 (NAG28) | |
| 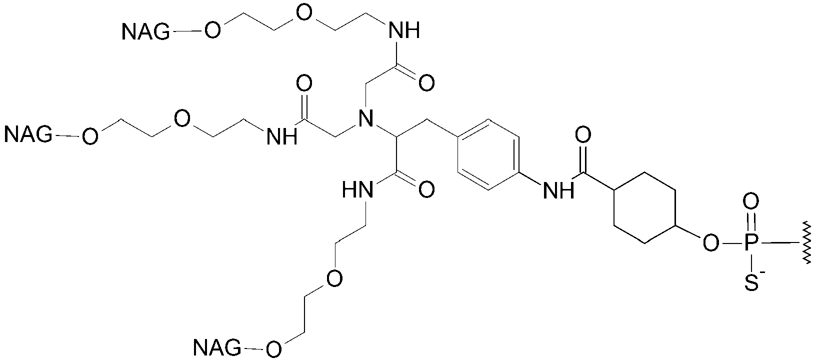 (NAG28)s | |
| 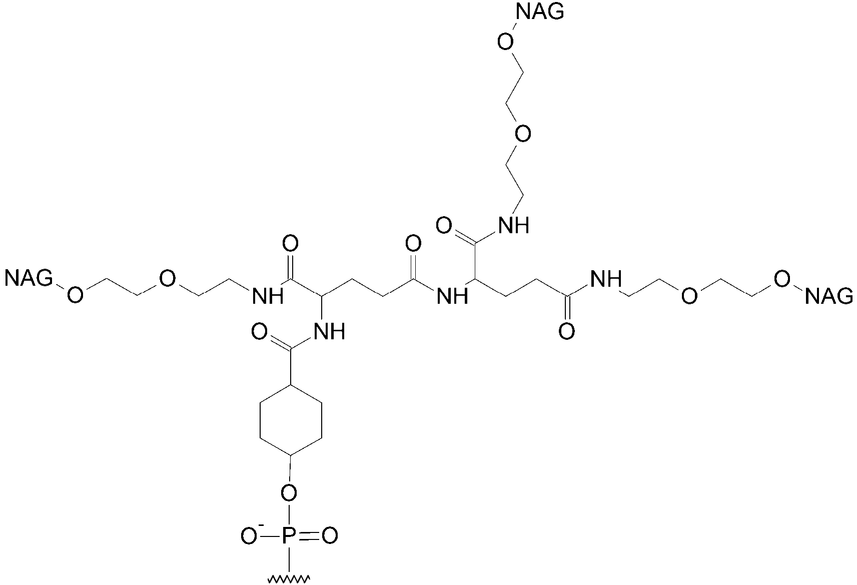 (NAG29) | |
| 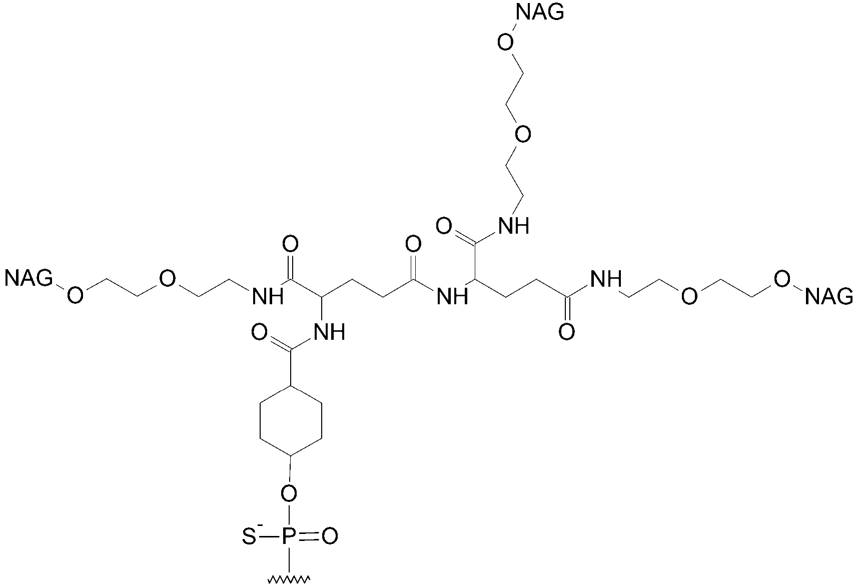 (NAG29)s | |
| 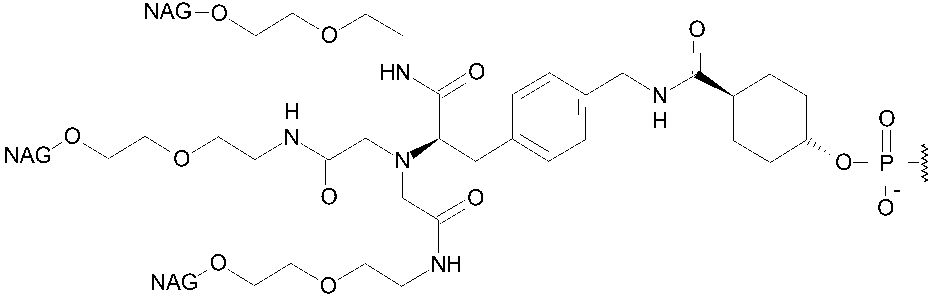 (NAG30) | |
| 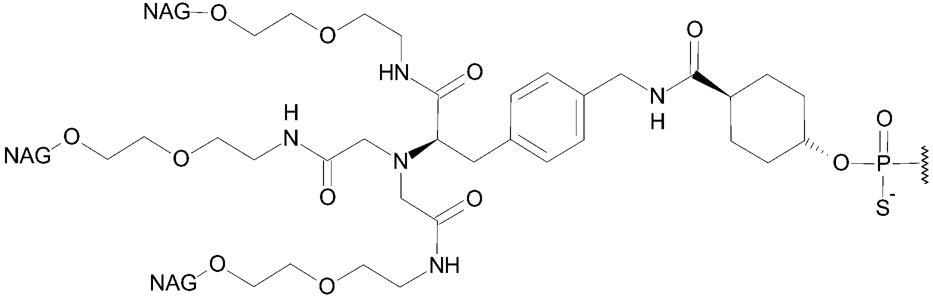 (NAG30)s | |
| 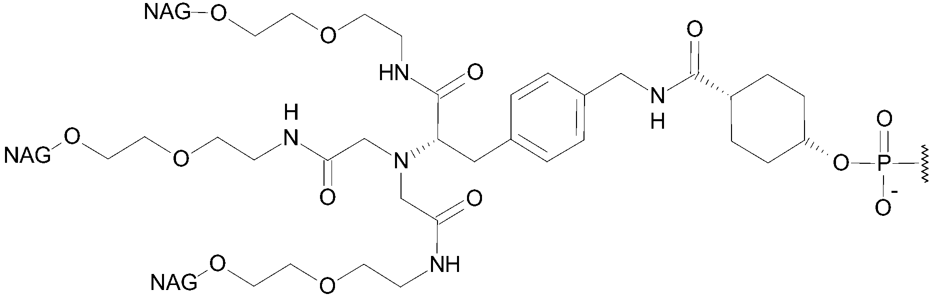 (NAG31) | |
| 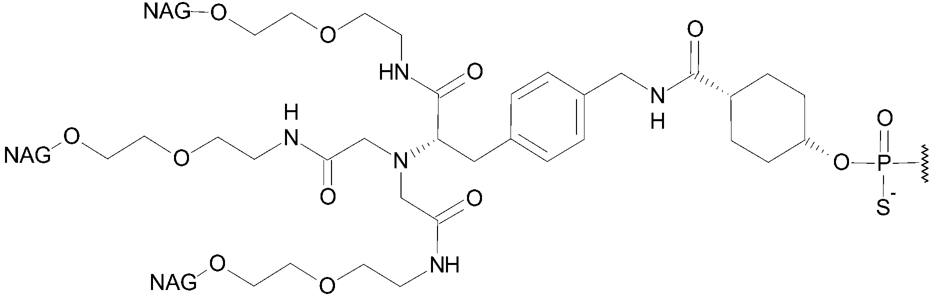 (NAG31)s | |
| 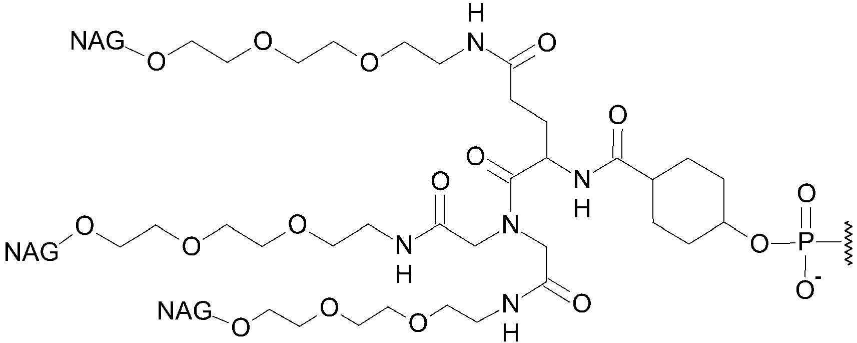 (NAG32) | |
| 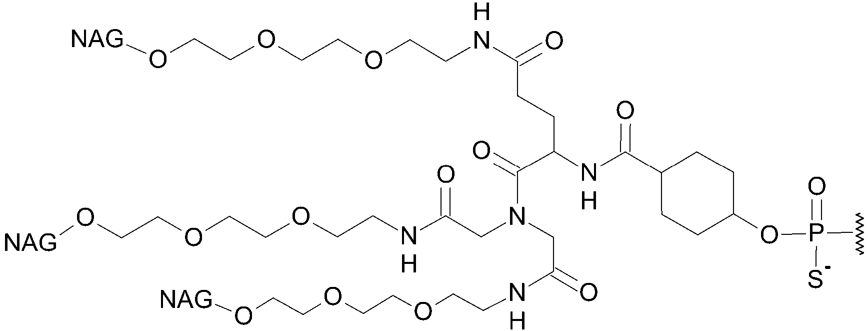 (NAG32)s | |
| 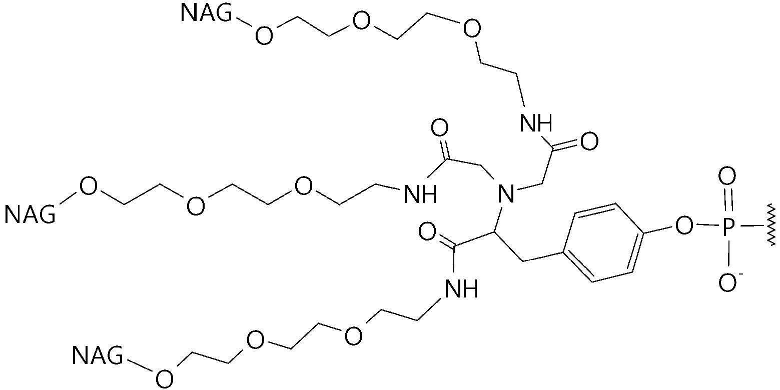 (NAG33) | |
| 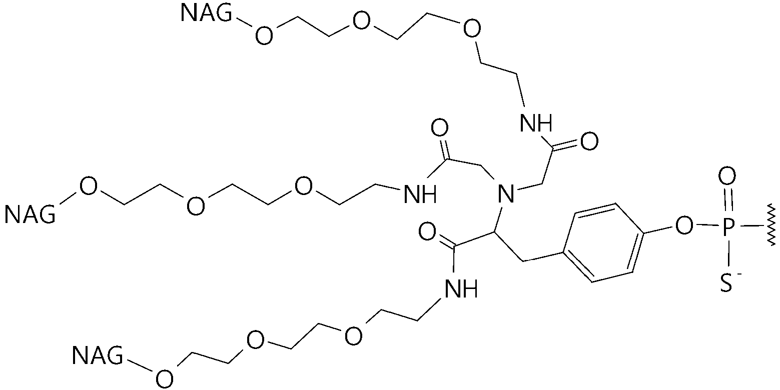 (NAG33)s | |
| 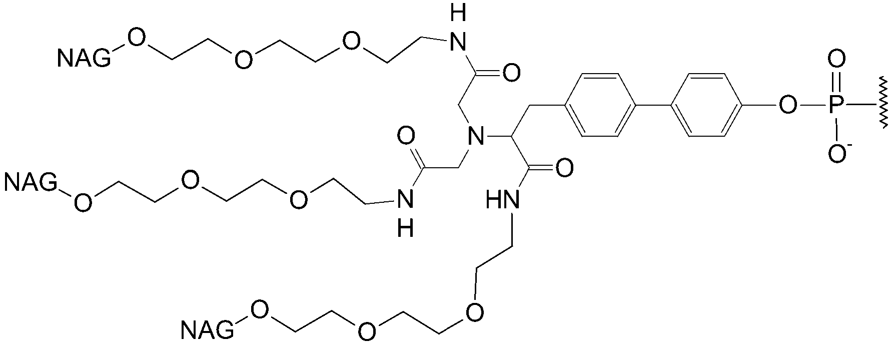 (NAG34) | |
| 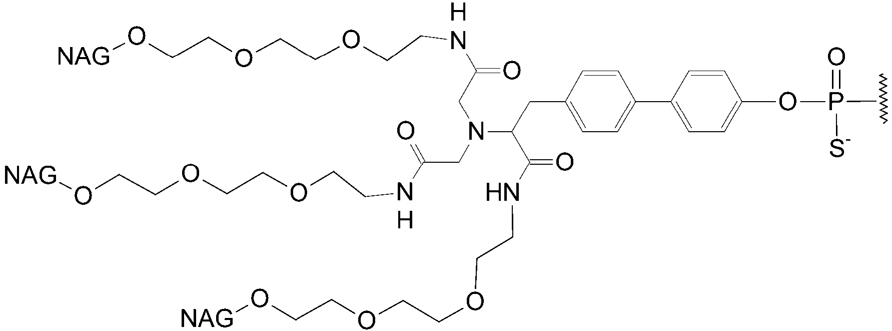 (NAG34)s | |
| 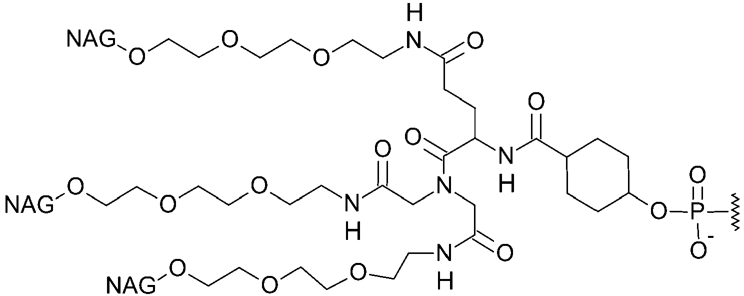 (NAG35) | |
| 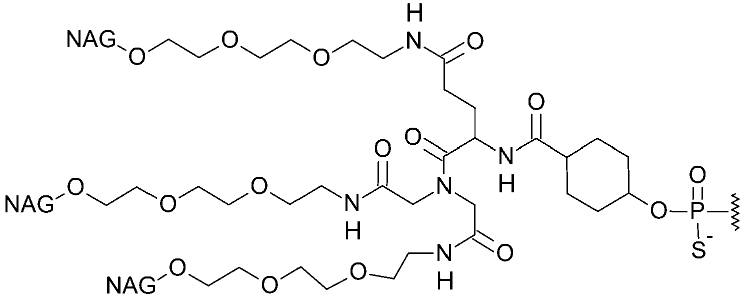 (NAG35)s | |
| 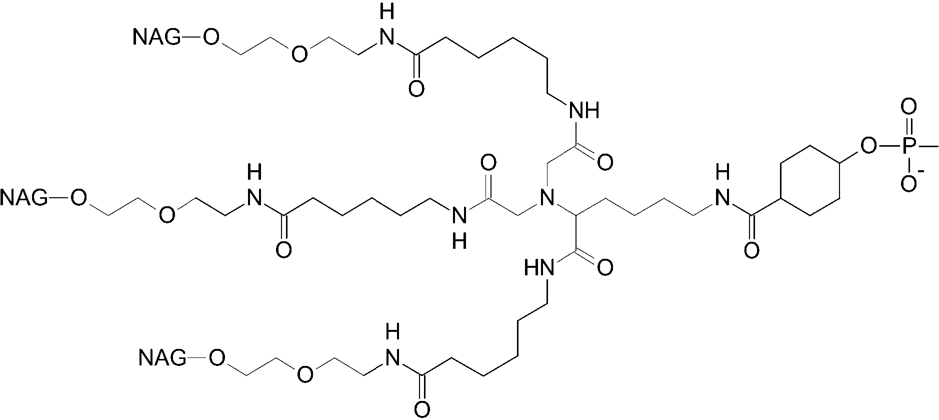 (NAG36) | |
| 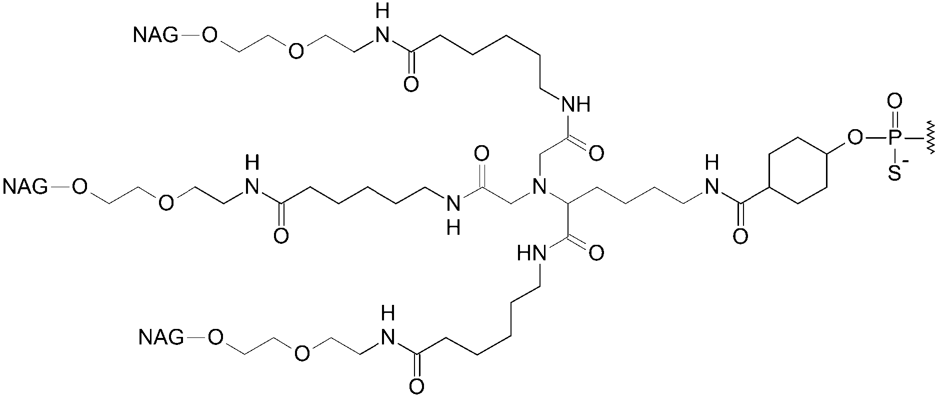 (NAG36)s | |
| 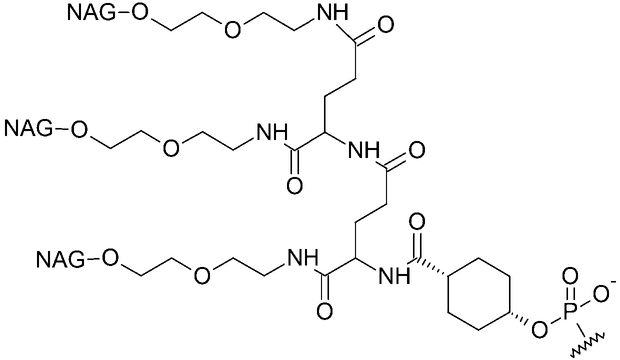 (NAG37) | |
| 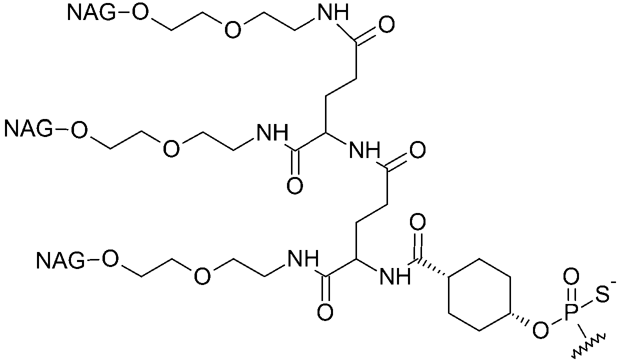 (NAG37)s | |
| 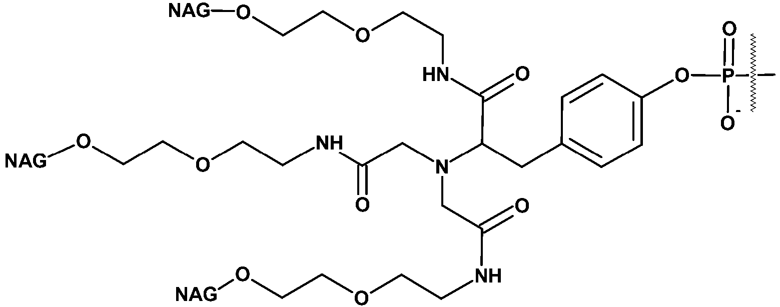 (NAG 38) | |
| 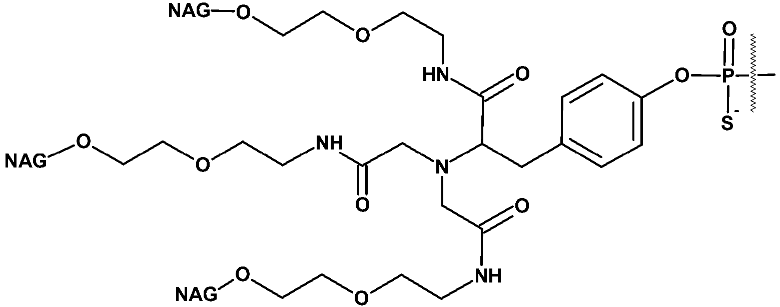 (NAG 38)s | |
| 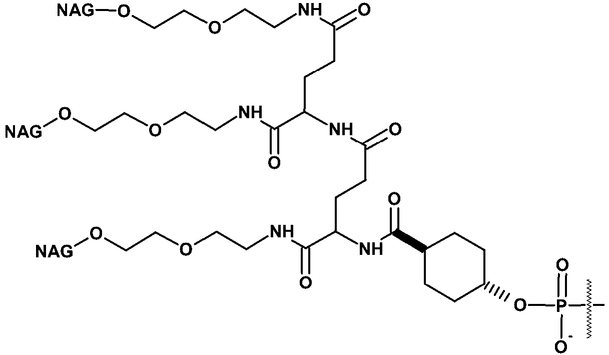 (NAG39) | |
| 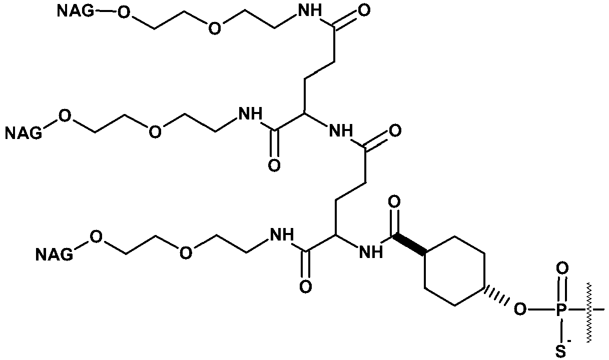 (NAG39)s | |

In each of the above structures in Table 6, NAG comprises an N-acetyl-galactosamine or another ASGPr ligand, as would be understood by a person of ordinary skill in the art to be attached in view of the structures above and description provided herein. For example, in some embodiments, NAG in the structures provided in Table 6 is represented by the following structure:

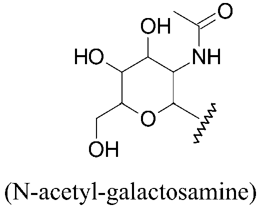

Each (NAGx) may be attached to an HBV RNAi agent via a phosphate group (as in (NAG25), (NAG30), and (NAG31)), or a phosphorothioate group, (as is (NAG25)s, (NAG29)s, (NAG30)s, (NAG31)s, or (NAG37)s), or another linking group.

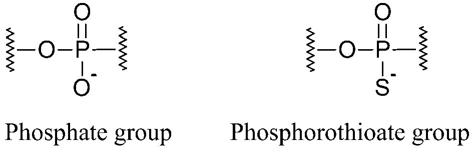

Other linking groups known in the art may be used.

### Delivery Vehicles

In another aspect, the methods described herein further comprises a delivery vehicle. In some embodiments, the delivery vehicle may be used to deliver an RNAi agent to a cell or tissue. A delivery vehicle is a compound that improves delivery of the RNAi agent to a cell or tissue. A delivery vehicle can include, or consist of, but is not limited to: a polymer, such as an amphipathic polymer, a membrane active polymer, a peptide, a melittin peptide, a melittin-like peptide (MLP), a lipid, a reversibly modified polymer or peptide, or a reversibly modified membrane active polyamine.

In some embodiments, the RNAi agents can be combined with lipids, nanoparticles, polymers, liposomes, micelles, DPCs or other delivery systems available in the art. The RNAi agents can also be chemically conjugated to targeting groups, lipids (including, but not limited to cholesterol and cholesteryl derivatives), nanoparticles, polymers, liposomes, micelles, DPCs (see, for example WO 2000/053722, WO 2008/0022309, WO 2011/104169, and WO 2012/083185, WO 2013/032829, WO 2013/158141, each of which is incorporated herein by reference), or other delivery systems available in the art.

### Pharmaceutical Compositions and Formulations

In another aspect, methods described herein comprise one or more HBV RNAi agents, wherein the one or more HBV agents are prepared as pharmaceutical compositions or formulations. In some embodiments, pharmaceutical compositions include at least one HBV RNAi agent. These pharmaceutical compositions are particularly useful in the inhibition of the expression of the target mRNA in a target cell, a group of cells, a tissue, or an organism. The pharmaceutical compositions can be used to treat a subject having a disease or disorder that would benefit from reduction in the level of the target mRNA, or inhibition in expression of the target gene. The pharmaceutical compositions can be used to treat a subject at risk of developing a disease or disorder that would benefit from reduction of the level of the target mRNA or an inhibition in expression the target gene. In one embodiment, the method includes administering an HBV RNAi agent linked to a targeting ligand as described herein, to a subject to be treated. In some embodiments, one or more pharmaceutically acceptable excipients (including vehicles, carriers, diluents, and/or delivery polymers) are added to the pharmaceutical compositions including an HBV RNAi agent, thereby forming a pharmaceutical formulation suitable for *in vivo* delivery to a human.

The pharmaceutical compositions that include an HBV RNAi agent and methods disclosed herein may decrease the level of the target mRNA in a cell, group of cells, group of cells, tissue, or subject, including: administering to the subject a therapeutically effective amount of a herein described HBV RNAi agent, thereby inhibiting the expression of a target mRNA in the subject.

In some embodiments, the described pharmaceutical compositions including an HBV RNAi agent are used for treating or managing clinical presentations associated with HBV infection. In some embodiments, a therapeutically or prophylactically effective amount of one or more of pharmaceutical compositions is administered to a subject in need of such treatment, prevention or management. In some embodiments, administration of any of the disclosed HBV RNAi agents can be used to decrease the number, severity, and/or frequency of symptoms of a disease in a subject.

The described pharmaceutical compositions including an HBV RNAi agent can be used to treat at least one symptom in a subject having a disease or disorder that would benefit from reduction or inhibition in expression of HBV mRNA. In some embodiments, the subject is administered a therapeutically effective amount of one or more pharmaceutical compositions including an HBV RNAi agent thereby treating the symptom. In other embodiments, the subject is administered a prophylactically effective amount of one or more HBV RNAi agents, thereby preventing the at least one symptom.

The route of administration is the path by which an HBV RNAi agent is brought into contact with the body. In general, methods of administering drugs and nucleic acids for treatment of a mammal are well known in the art and can be applied to administration of the compositions described herein. The HBV RNAi agents disclosed herein can be administered via any suitable route in a preparation appropriately tailored to the particular route. Thus, herein described pharmaceutical compositions can be administered by injection, for example, intravenously, intramuscularly, intracutaneously, subcutaneously, intraarticularly, or intraperitoneally. In some embodiments, there herein described pharmaceutical compositions via subcutaneous injection.

The pharmaceutical compositions including an HBV RNAi agent described herein can be delivered to a cell, group of cells, tumor, tissue, or subject using oligonucleotide delivery technologies known in the art. In general, any suitable method recognized in the art for delivering a nucleic acid molecule (in vitro or in vivo) can be adapted for use with a herein described compositions. For example, delivery can be by local administration, (e.g., direct injection, implantation, or topical administering), systemic administration, or subcutaneous, intravenous, intraperitoneal, or parenteral routes, including intracranial (e.g., intraventricular, intraparenchymal and intrathecal), intramuscular, transdermal, airway (aerosol), nasal, oral, rectal, or topical (including buccal and sublingual) administration. In certain embodiments, the compositions are administered by subcutaneous or intravenous infusion or injection.

Accordingly, in some embodiments, the herein described pharmaceutical compositions may comprise one or more pharmaceutically acceptable excipients. In some embodiments, the pharmaceutical compositions described herein can be formulated for administration to a subject.

As used herein, a pharmaceutical composition or medicament includes a pharmacologically effective amount of at least one of the described therapeutic compounds and one or more pharmaceutically acceptable excipients. Pharmaceutically acceptable excipients (excipients) are substances other than the Active Pharmaceutical ingredient (API, therapeutic product, e.g., HBV RNAi agent) that are intentionally included in the drug delivery system. Excipients do not exert or are not intended to exert a therapeutic effect at the intended dosage. Excipients may act to a) aid in processing of the drug delivery system during manufacture, b) protect, support or enhance stability, bioavailability or patient acceptability of the API, c) assist in product identification, and/or d) enhance any other attribute of the overall safety, effectiveness, of delivery of the API during storage or use. A pharmaceutically acceptable excipient may or may not be an inert substance.

Excipients include, but are not limited to: absorption enhancers, anti-adherents, anti-foaming agents, anti-oxidants, binders, buffering agents, carriers, coating agents, colors, delivery enhancers, delivery polymers, dextran, dextrose, diluents, disintegrants, emulsifiers, extenders, fillers, flavors, glidants, humectants, lubricants, oils, polymers, preservatives, saline, salts, solvents, sugars, suspending agents, sustained release matrices, sweeteners, thickening agents, tonicity agents, vehicles, water-repelling agents, and wetting agents.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor ELTM (BASF, Parsippany, NJ) or phosphate buffered saline. It should be stable under the conditions of manufacture and storage and should be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filter sterilization. Generally, dispersions are prepared by incorporating the active compound into a sterile vehicle which contains a basic dispersion medium and the required other ingredients from those enumerated above. In the case of sterile powders for the preparation of sterile injectable solutions, methods of preparation include vacuum drying and freeze-drying which yields a powder of the active ingredient plus any additional desired ingredient from a previously sterile-filtered solution thereof.

Formulations suitable for intra-articular administration can be in the form of a sterile aqueous preparation of the drug that can be in microcrystalline form, for example, in the form of an aqueous microcrystalline suspension. Liposomal formulations or biodegradable polymer systems can also be used to present the drug for both intra-articular and ophthalmic administration.

The active compounds can be prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions can also be used as pharmaceutically acceptable carriers. These can be prepared according to methods known to those skilled in the art, for example, as described in U.S. Patent No. 4,522,811.

The HBV RNAi agents can be formulated in compositions in dosage unit form for ease of administration and uniformity of dosage. Dosage unit form refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the therapeutic effect to be achieved, and the limitations inherent in the art of compounding such an active compound for the treatment of individuals.

A pharmaceutical composition can contain other additional components commonly found in pharmaceutical compositions. Such additional components include, but are not limited to: anti-pruritics, astringents, local anesthetics, or anti-inflammatory agents (e.g., antihistamine, diphenhydramine, etc.). It is also envisioned that cells, tissues or isolated organs that express or comprise the herein defined RNAi agents may be used as "pharmaceutical compositions." As used herein, "pharmacologically effective amount," "therapeutically effective amount," or simply "effective amount" refers to that amount of an RNAi agent to produce a pharmacological, therapeutic or preventive result.

Generally, an effective amount of an active compound will be in the range of from about 0.1 to about 100 mg/kg of body weight/day, e.g., from about 1.0 to about 50 mg/kg of body weight/day. In some embodiments, an effective amount of an active compound will be in the range of from about 0.25 to about 5 mg/kg of body weight per dose. In some embodiments, an effective amount of an active compound will be in the range of 25-400 mg per 1-18 weeks or 1-6 months. In some embodiments, an effective amount of an active compound will be in the range of 50-125 mg per 4 weeks or per one month. In some embodiments, an effective amount of an active compound will be in the range of 40-200 mg per 4 weeks or per one month. In some embodiments, an effective amount of an active compound will be 40 mg per 4 weeks or per one month. In some embodiments, an effective amount of an active compound will be 100 mg per 4 weeks or per one month. In some embodiments, an effective amount of an active compound will be 200 mg per 4 weeks or per one month. In some embodiments, an effective amount of an active ingredient will be in the range of from about 0.5 to about 3 mg/kg of body weight per dose. In some embodiments, an effective amount of an active ingredient will be in the range of from about 25-400 mg per dose. In some embodiments, an effective amount of an active ingredient will be in the range of from about 50-125 mg per dose. In some embodiments, an effective amount of an active ingredient will be about 35 mg, 50 mg, 100 mg, 200 mg, 300 mg or 400 mg per dose. In some embodiments, an effective amount of an active ingredient will be about 40 mg, 100 mg, or 200 mg per dose. The amount administered will also likely depend on such variables as the overall health status of the patient, the relative biological efficacy of the compound delivered, the formulation of the drug, the presence and types of excipients in the formulation, and the route of administration. Also, it is to be understood that the initial dosage administered can be increased beyond the above upper level in order to rapidly achieve the desired blood-level or tissue level, or the initial dosage can be smaller than the optimum.

The one or more (e.g., at least two) HBV RNAi agents described herein can be formulated into one single composition or separate individual compositions. In some embodiments, the HBV RNAi agents in separate individual compositions can be formulated with the same or different excipients and carriers. In some embodiments, the HBV RNAi agents in separate individual compositions agents can be administered through same or different administration routes. In some embodiments, the HBV RNAi agents are administered subcutaneously.

For treatment of disease or for formation of a medicament or composition for treatment of a disease, the pharmaceutical compositions described herein including an HBV RNAi agent can be combined with an excipient or with a second therapeutic agent or treatment including, but not limited to: a second or other RNAi agent, a small molecule drug, an antibody, an antibody fragment, and/or a vaccine.

The described HBV RNAi agents, when added to pharmaceutically acceptable excipients or adjuvants, can be packaged into kits, containers, packs, or dispensers. The pharmaceutical compositions described herein may be packaged in pre-filled syringes or vials.

### Methods of Treatment and Inhibition of Expression

Described herein are methods for treating a subject (e.g., a human or mammal) having a disease or disorder that would benefit from administration of the compound comprising administering one or more HBV RNAi agents described herein to the subject. In some embodiments, the subject (e.g., a human) has a disease or disorder that would benefit from reduction or inhibition in expression of HBV mRNA. The subject is administered a therapeutically effective amount of any one or more RNAi agents. The subject can be a human, patient, or human patient. The subject may be an adult, adolescent, child, or infant. Also described herein are methods for treating a subject in need thereof comprising administering a pharmaceutical composition described herein, wherein the pharmaceutical composition comprises an HBV RNAi agent. Such methods include administration of a pharmaceutical composition described herein to a human being or animal.

In some embodiments, the symptoms and diseases associated with an HBV infection include but are not limited to chronic liver diseases/disorders, inflammations, fibrotic conditions, proliferative disorders (including cancers, such as hepatocellular carcinoma), Hepatitis D Virus (HDV) infection, and acute HBV infection. In some embodiments, the symptoms and diseases are associated with chronic HBV infection and/or HDV infection. In some embodiments, the subject has been tested for HBV infection for at least 6-12 months. In some embodiments, the subject has a diagnosis of HBeAg positive or HBeAg negative chronic HBV infection. In some embodiments the subject has a diagnosis of HBeAg positive or HBeAg negative chronic HBV infection for at least 6 months. In some embodiments, the symptom or disease associated with the HBV infection is a chronic liver disease or disorder, liver inflammation, liver fibrotic condition, a proliferative hepatocellular disorder, hepatocellular carcinoma, Hepatitis D virus infection, acute HBV infection, chronic hepatitis B or chronic HBV infection. In some embodiments, the symptom or disease associated with the HBV infection is chronic HBV infection or chronic hepatitis B.

In some embodiments, the subject is infected with HBV. In some embodiments, the described methods are used to treat at least one symptom in a subject having a HBV infection. The subject is administered a therapeutically effective amount of any one or more of the described RNAi agents.

In some embodiments, the subject has both a HBV infection and a HDV infection. In some embodiments, the methods described herein are used to treat a subject infected with both HBV and HDV. In some embodiments, the described methods are used to treat at least one symptom in a subject having a HBV or a HDV infection. The subject is administered a therapeutically effective amount of any one or more of the described RNAi agents.

In some embodiments, the methods described herein are used to treat or manage a clinical presentation wherein a subject infected with HBV. The subject is administered a therapeutically or effective amount of one or more of the HBV RNAi agents or HBV RNAi agent-containing compositions described herein. In some embodiments, the method comprises administering a composition comprising an HBV RNAi agent described herein to a subject to be treated.

In some embodiments, the method comprises administering one or more HBV RNAi agents described herein. In some embodiments, the one or more HBV RNAi agents are administered in the amount of about 5-1000 mg per dose administration. In some embodiments, the one or more HBV RNAi agents are administered in the amount of about 5-25 mg, about 25-50 mg, about 50-75 mg, about 75-100 mg, about 100-125 mg, about 125-150 mg, about 150-175 mg, about 175-200 mg, about 200-225 mg, about 225-250 mg, about 250-275 mg, about 275-300 mg, about 300-325 mg, about 325-350 mg, about 350-375 mg, about 375-400 mg, about 400-425 mg, about 425-450 mg, about 450-475 mg, about 475-500 mg, about 500-550 mg, about 550-600 mg, about 600-650 mg, about 650-700 mg, about 700-750 mg, about 750-800 mg, about 800-850 mg, about 850-900 mg, about 900-950 mg, about 950-1000 mg, about 5-50 mg, about 50-100 mg, about 100-200 mg, about 200-300 mg, about 300-400 mg, about 400-500 mg, about 500-750 mg, about 750-1000 mg, 25-100 mg, 50-150 mg, 100-200 mg, 150-250 mg, 200-300 mg, 300-400 mg, 25-200 mg, or 200-400 mg per dose administration. In some embodiments, the one or more HBV RNAi agents are administered in the amount of about 25mg, about 50 mg, about 75 mg, about 100 mg, about 125 mg, about 150 mg, about 175 mg, about 200 mg, about 250 mg, about 300 mg, about 350 mg, about 400 mg, about 450 mg, about 500 mg, about 600 mg, about 700 mg, about 800 mg, about 900 mg, or about 1000 mg per dose administration. In some embodiments, the one or more HBV RNAi agents are administered in the amount of about 25mg, about 50 mg, about 75 mg, about 100 mg, or about 125 mg per dose administration. In some embodiments, the one or more HBV RNAi agents are administered in the amount of about 35mg, about 50 mg, about 100 mg, about 200 mg, about 300 mg, or about 400 mg per dose administration. In some embodiments, the one or more HBV RNAi agents are administered in the amount of about 40 mg, about 100 mg, or about 200 mg per dose administration.

In some embodiments, the method comprises administering a combination of at least two HBV RNAi agents, wherein each HBV RNAi agent is designed to target a different HBV transcript (for example, a composition that includes two HBV RNAi agents, wherein the first HBV RNAi agent includes an antisense strand that is at least partially complementary to a nucleotide sequence located in the S ORF of an HBV gene, while the second HBV RNAi agent includes an antisense strand that is at least partially complementary to a nucleotide sequence located in the X ORF of an HBV gene).

In some embodiments, the at least two HBV RNAi agents are administered in the amount of about 5-1000 mg per dose administration. In some embodiments, the at least two HBV RNAi agents are administered in the amount of about 5-25 mg, about 25-50 mg, about 50-75 mg, about 75-100 mg, about 100-125 mg, about 125-150 mg, about 150-175 mg, about 175-200 mg, about 200-225 mg, about 225-250 mg, about 250-275 mg, about 275-300 mg, about 300-325 mg, about 325-350 mg, about 350-375 mg, about 375-400 mg, about 400-425 mg, about 425-450 mg, about 450-475 mg, about 475-500 mg, about 500-550 mg, about 550-600 mg, about 600-650 mg, about 650-700 mg, about 700-750 mg, about 750-800 mg, about 800-850 mg, about 850-900 mg, about 900-950 mg, about 950-1000 mg, about 5-50 mg, about 50-100 mg, about 100-200 mg, about 200-300 mg, about 300-400 mg, about 400-500 mg, about 500-750 mg, about 750-1000 mg, 25-100 mg, 50-150 mg, 100-200 mg, 150-250 mg, 200-300 mg, 300-400 mg, 25-200 mg, or 200-400 mg per dose administration. In some embodiments, the at least two HBV RNAi agents are administered in the amount of about 25mg, about 50 mg, about 75 mg, about 100 mg, about 125 mg, about 150 mg, about 175 mg, about 200 mg, about 250 mg, about 300 mg, about 350 mg, about 400 mg, about 450 mg, about 500 mg, about 600 mg, about 700 mg, about 800 mg, about 900 mg, or about 1000 mg per dose administration. In some embodiments, the at least two HBV RNAi agents are administered in the amount of about 25mg, about 50 mg, about 75 mg, about 100 mg, or about 125 mg per dose administration. In some embodiments, the at least two HBV RNAi agents are administered in the amount of about 35mg, about 50 mg, about 100 mg, about 200 mg, about 300 mg, or about 400 mg per dose administration. In some embodiments, the at least two HBV RNAi agents are administered in the amount of about 40 mg, about 100 mg, or about 200 mg per dose administration.

In some embodiments, the at least two HBV RNAi agents are administered in a combined amount of about 1-10 mg/kg per dose administration. In some embodiments, the at least two HBV RNAi agents are administered in a combined amount of about 1-5 mg/kg per dose administration. In some embodiments, the at least two HBV RNAi agents are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, the at least two HBV RNAi agents are administered simultaneously. In some embodiments, the at least two HBV RNAi agents are administered sequentially. In some embodiments, the at least two HBV RNAi agents are administered as a single composition. In some embodiments, the at least two HBV RNAi agents are administered as two or more individual compositions.

In some embodiments, described herein are methods of treating or preventing an HBV-associated disease or symptoms in a subject or inhibiting expression of one or more HBV genes comprising administering a composition that comprises a combination of one HBV RNAi agent that targets the S ORF of an HBV RNA (i.e., having an antisense strand that targets the S transcripts (S, pre-S1, and pre-S2), the pregenomic RNA (core and polymerase), and the pre-core transcripts (HBeAg) of an HBV genome), and one HBV RNAi agent that targets the X ORF of an HBV RNA (i.e., having an antisense strand that targets the X transcript of an HBV genome, the S transcripts (S, pre-S1, and pre-S2), the pregenomic RNA (core and polymerase), and the pre-core transcripts (HBeAg) of an HBV genome). In some embodiments, the composition described herein comprises at least one HBV RNAi agent that contains a sequence that targets the S ORF of an HBV gene, and a second HBV RNAi agent that contains a sequence that targets the X ORF of an HBV gene.

In some embodiments, the two HBV RNAi agents are administered in a ratio of about 1:1, 2:1, 3:1, 4:1 or 5:1. In some embodiments, the two HBV RNAi agents are administered in a ratio of about 2:1.

In some embodiments, the two HBV RNAi agents are administered in a combined amount of about 5-1000 mg per dose administration and in the ratio of about 2:1, about 3:1, about 1:1, about 4:1, about 5:1 or about 1:2. In some embodiments, the two HBV RNAi agents are administered in a combined amount of about 5-125 mg per dose administration and in the ratio of about 2:1, about 3:1, about 1:1, about 4:1, about 5:1 or about 1:2. In some embodiments, the two HBV RNAi agents are administered in a combined amount of about 25-150 mg per dose administration and in the ratio of about 2:1, about 3:1, about 1:1, about 4:1, about 5:1 or about 1:2. In some embodiments, the two HBV RNAi agents are administered in a combined amount of about 50-200 mg per dose administration and in the ratio of about 2:1, about 3:1, about 1:1, about 4:1, about 5:1 or about 1:2. In some embodiments, the two HBV RNAi agents are administered in a combined amount of about 100-250 mg per dose administration and in the ratio of about 2:1, about 3:1, about 1:1, about 4:1, about 5:1 or about 1:2. In some embodiments, the two HBV RNAi agents are administered in a combined amount of about 200-300 mg per dose administration and in the ratio of about 2:1, about 3:1, about 1:1, about 4:1, about 5:1 or about 1:2. In some embodiments, the two HBV RNAi agents are administered in a combined amount of about 300-400 mg per dose administration and in the ratio of about 2:1, about 3:1, about 1:1, about 4:1, about 5:1 or about 1:2. In some embodiments, the two HBV RNAi agents are administered in a combined amount of about 400-500 mg per dose administration and in the ratio of about 2:1, about 3:1, about 1:1, about 4:1, about 5:1 or about 1:2. In some embodiments, the two HBV RNAi agents are administered in a combined amount of about 500-750 mg per dose administration and in the ratio of about 2:1, about 3:1, about 1:1, about 4:1, about 5:1 or about 1:2. In some embodiments, the two HBV RNAi agents are administered in a combined amount of about 750-1000 mg per dose administration and in the ratio of about 2:1, about 3:1, about 1:1, about 4:1, about 5:1 or about 1:2. In some embodiments, the two HBV RNAi agents are administered in a combined amount of about 25-125 mg per dose administration and in the ratio of about 2:1, about 3:1, about 1:1, about 4:1, about 5:1 or about 1:2. In some embodiments, the two HBV RNAi agents are administered in a combined amount of about 5-1000 mg per dose administration and in the ratio of about 2:1. In some embodiments, the two HBV RNAi agents are administered in a combined amount of about 25-75 mg per dose administration and in the ratio of about 2:1. In some embodiments, the two HBV RNAi agents are administered in a combined amount of about 50-125 mg per dose administration and in the ratio of about 2:1. In some embodiments, the two HBV RNAi agents are administered in a combined amount of about 75-150 mg per dose administration and in the ratio of about 2:1. In some embodiments, the two HBV RNAi agents are administered in a combined amount of about 100-200 mg per dose administration and in the ratio of about 2:1. In some embodiments, the two HBV RNAi agents are administered in a combined amount of about 200-300 mg per dose administration and in the ratio of about 2:1. In some embodiments, the two HBV RNAi agents are administered in a combined amount of about 300-400 mg per dose administration and in the ratio of about 2:1. In some embodiments, the two HBV RNAi agents are administered in a combined amount of about 400-500 mg per dose administration and in the ratio of about 2:1. In some embodiments, the two HBV RNAi agents are administered in a combined amount of about 500-750 mg per dose administration and in the ratio of about 2:1. In some embodiments, the two HBV RNAi agents are administered in a combined amount of about 750-1000 mg per dose administration and in the ratio of about 2:1. In some embodiments, the two HBV RNAi agents are administered in a combined amount of about 35 mg per dose administration and in the ratio of about 2:1. In some embodiments, the two HBV RNAi agents are administered in a combined amount of about 40 mg per dose administration and in the ratio of about 2:1. In some embodiments, the two HBV RNAi agents are administered in a combined amount of about 100 mg per dose administration and in the ratio of about 2:1. In some embodiments, the two HBV RNAi agents are administered in a combined amount of about 200 mg per dose administration and in the ratio of about 2:1. In some embodiments, the two HBV RNAi agents are administered in a combined amount of about 300 mg per dose administration and in the ratio of about 2:1. In some embodiments, the two HBV RNAi agents are administered in a combined amount of about 400 mg per dose administration and in the ratio of about 2:1.In another aspect, methods described here comprise administering one or more HBV RNAi agents (e.g., two or more) in 1-18 week intervals or 1-6 month intervals. In some embodiments, the HBV RNAi agents are administered in about 1-18 week intervals. In some embodiments, the HBV RNAi agents are administered in about 1-week intervals, about 2-week intervals, about 3-week intervals, about 4-week intervals, about 5-week intervals, about 6-week intervals, about 7-week intervals, about 8-week intervals, about 9-week intervals, about 10-week intervals, about 11-week intervals, about 12-week intervals, about 13-week intervals, about 14-week intervals, about 15-week intervals, about 16-week intervals, about 17-week intervals, or about 18-week intervals. In some embodiments, the HBV RNAi agents are administered in about 1-6 month intervals. In some embodiments, the HBV RNAi agents are administered in about 1- month intervals, about 2-month intervals, about 3- month intervals, about 4- month intervals, about 5- month intervals, or about 6-month intervals. In some embodiments, the HBV RNAi agents are administered in about 4-week intervals or 1-month intervals. In some embodiments, the HBV RNAi agents are administered in about 7-day, 14-day, 21-day or 28-day intervals. In some embodiments, the HBV RNAi agents are administered in about 28-day intervals (i.e., Q4W).

In another aspect, methods described here comprise administering one or more HBV RNAi agents (*e.g.,* two or more) for a duration of about 1-12 months or 1-18 weeks. In some embodiments, the RNAi agents are administered for a duration of about 1-12 months. In some embodiments, the HBV RNAi agents are administered for a duration of at least about 1 month, at least about 2 months, at least about 3 months, at least about 4 months, at least about 5 months, at least about 6 months, at least about 7 months, at least about 8 months, at least about 9 months, at least about 10 months, at least about 11 months or at least about 12 months. In some embodiments, the HBV RNAi agents are administered for a duration of about 1-18 weeks. In some embodiments, the HBV RNAi agents are administered for a duration of at least about 1 week, at least about 2 weeks, at least about 3 weeks, at least about 4 weeks, at least about 5 weeks, at least about 6 weeks, at least about 7 weeks, at least about 8 weeks, at least about 9 weeks, at least about 10 weeks, at least about 11 weeks, at least about 12 weeks, at least about 13 weeks, at least about 14 weeks, at least about 15 weeks, at least about 16 weeks, at least about 17 weeks, or at least about 18 weeks. In some embodiments, the HBV RNAi agents are administered for a duration of about 12 weeks or 3 months.

In some embodiments, the first RNAi agent is administered in an amount of about 3-650 mg per dose administration, and the second RNAi agent is administered in an amount of about 2-325 mg per dose administration. In some embodiments, the first RNAi agent is administered in an amount of about 35-265 mg per dose administration. In some embodiments, the first RNAi agent is administered in an amount of about 50-75 mg per dose administration. In some embodiments, the second RNAi agent is administered in an amount of about 20-125 mg per dose administration. In some embodiments, the second RNAi agent is administered in an amount of about 25-50 mg per dose administration.

In some embodiments, two RNAi agents are administered at a combined dose of 25-400 mg per dose administration. In an embodiment, two RNAi agents are administered at a combined dose of 25-400 mg, and the first RNAi agent is administered with the second RNAi agent at a ratio of 1:1. In an embodiment, the dose of each of the first and second RNAi agents is in an amount of about 12 mg for a combined dose of about 25 mg. In an embodiment, the dose of each of the first and second RNAi agents is in an amount of about 17 mg for a combined dose of about 35 mg. In an embodiment, the dose of each of the first and second RNAi agents is in an amount of about 25 mg for a combined dose of about 50 mg. In an embodiment, the dose of each of the first and second RNAi agents is in an amount of about 50 mg for a combined dose of about 100 mg. In an embodiment, the dose of each of the first and second RNAi agents is in an amount of about 100 mg for a combined dose of about 200 mg. In an embodiment, the dose of each of the first and second RNAi agents is in an amount of about 150 mg for a combined dose of about 300 mg. In an embodiment, the dose of each of the first and second RNAi agents is in an amount of about 200 mg for a combined dose of about 400 mg.

In an embodiment, two RNAi agents are administered at a combined dose of 25-400 mg per dose, and the first RNAi agent is administered with the second RNAi agent at a ratio of 2:1. In an embodiment, the dose of the first RNAi agent is in an amount of about 16 mg, and the dose of the second RNAi agent is in an amount of about 8 mg for a combined dose of about 25 mg. In an embodiment, the dose of the first RNAi agent is in an amount of about 24 mg, and the dose of the second RNAi agent is in an amount of about 12 mg for a combined dose of about 35 mg. In an embodiment, the dose of the first RNAi agent is in an amount of about 33 mg, and the dose of the second RNAi agent is in an amount of about 17 mg for a combined dose of about 50 mg. In an embodiment, the dose of the first RNAi agent is in an amount of about 65 mg, and the dose of the second RNAi agent is in an amount of about 35 mg for a combined dose of about 100 mg. In an embodiment, the dose of the first RNAi agent is in an amount of about 133 mg, and the dose of the second RNAi agent is in an amount of about 67 mg for a combined dose of about 200 mg. In an embodiment, the dose of the first RNAi agent is in an amount of about 200 mg, and the dose of the second RNAi agent is in an amount of about 100 mg for a combined dose of about 300 mg. In an embodiment, the dose of the first RNAi agent is in an amount of about 270 mg, and the dose of the second RNAi agent is in an amount of about 135 mg for a combined dose of about 400 mg.

In an embodiment, two RNAi agents are administered at a combined dose of 25-400 mg per dose, the first RNAi agent is administered with the second RNAi agent at a ratio of 3:1. In an embodiment, the dose of the first RNAi agent is in an amount of about 18 mg, and the dose of the second RNAi agent is in an amount of about 6 mg for a combined dose of about 25 mg. In an embodiment, the dose of the first RNAi agent is in an amount of about 27 mg, and the dose of the second RNAi agent is in an amount of about 9 mg for a combined dose of about 35 mg. In an embodiment, the dose of the first RNAi agent is in an amount of about 36 mg, and the dose of the second RNAi agent is in an amount of about 12 mg for a combined dose of about 50 mg. In an embodiment, the dose of the first RNAi agent is in an amount of about 75 mg, and the dose of the second RNAi agent is in an amount of about 25 mg for a combined dose of about 100 mg. In an embodiment, the dose of the first RNAi agent is in an amount of about 150 mg, and the dose of the second RNAi agent is in an amount of about 50 mg for a combined dose of about 200 mg. In an embodiment, the dose of the first RNAi agent is in an amount of about 225 mg, and the dose of the second RNAi agent is in an amount of about 75 mg for a combined dose of about 300 mg. In an embodiment, the dose of the first RNAi agent is in an amount of about 300 mg, and the dose of the second RNAi agent is in an amount of about 100 mg for a combined dose of about 400 mg.

In some embodiments, the two HBV RNAi agents are administered in a combined amount of about 1-10 mg/kg per dose administration. In some embodiments, the two HBV RNAi agents are administered in a combined amount of about 1-5 mg/kg per dose administration. In some embodiments, the two HBV RNAi agents are administered in a combined amount of about 1-1.5 mg/kg, about 1.5-2.0 mg/kg, about 2.0-2.5 mg/kg, about 2.5-3.0 mg/kg, about 3.0-3.5 mg/kg, about 3.5-4.0 mg/kg, about 4.0-4.5 mg/kg, about 4.5-5.0 mg/kg, about 5.0-5.5 mg/kg, about 5.5-6.0 mg/kg, about 6.0-6.5 mg/kg, about 6.5-7.0 mg/kg, about 7.0-7.5 mg/kg, about 7.5-8.0 mg/kg, about 8.0-8.5 mg/kg, about 8.5-9.0 mg/kg, about 9.0-9.5 mg/kg, about 9.5-10 mg/kg, about 1-2.5 mg/kg, about 2.5-5.0 mg/kg, about 5.0-7.5 mg/kg, about 7.5-10 mg/kg, about 1-5.0 mg/kg, or about 5.0-10 mg/kg per dose administration.

In some embodiments, the first RNAi agent is administered in an amount of about 0.6-7 mg/kg per dose administration, and the second RNAi agent is administered in an amount of about 0.3-5 mg/kg per dose administration. In some embodiments, the second RNAi agent is administered in an amount of about 0.5-2.5 mg/kg per dose administration. In some embodiments, the second RNAi agent is administered in an amount of about 0.3-1.5 mg/kg per dose administration. In some embodiments, the first RNAi agent is administered in an amount of about 0.6-5 mg/kg per dose administration. In some embodiments, the first RNAi agent is administered in an amount of about 1-2.5 mg/kg per dose administration.

In some embodiments, the gene expression level and/or mRNA level of an HBV gene in a subject to whom a described HBV RNAi agent is administered is reduced by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or greater than 99% relative to the subject prior to being administered the HBV RNAi agent or to a subject not receiving the HBV RNAi agent. The gene expression level and/or mRNA level in the subject may be reduced in a cell, group of cells, and/or tissue of the subject. In some embodiments, the expressed protein level of an HBV gene in a subject to whom a described HBV RNAi agent has been administered is reduced by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or greater than 99% relative to the subject prior to being administered the HBV RNAi agent or to a subject not receiving the HBV RNAi agent. The protein level in the subject may be reduced in a cell, group of cells, tissue, blood, and/or other fluid of the subject. For example, in some embodiments, the amount or level of Hepatitis B surface antigen (HBsAg) in a subject to whom a described HBV RNAi agent has been administered is reduced by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or greater than 99% relative to the subject prior to being administered the HBV RNAi agent or to a subject not receiving the HBV RNAi agent. In some embodiments, the amount or level of Hepatitis B e-antigen (HBeAg) in a subject to whom a described HBV RNAi agent has been administered is reduced by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or greater than 99% relative to the subject prior to being administered the HBV RNAi agent or to a subject not receiving the HBV RNAi agent. In some embodiments, the amount or level of serum HBV DNA in a subject to whom a described HBV RNAi agent has been administered is reduced by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or greater than 99% relative to the subject prior to being administered the HBV RNAi agent or to a subject not receiving the HBV RNAi agent. A reduction in the presence of serum HBV DNA, HBV gene expression, HBV mRNA, or HBV protein amounts or levels may be assessed by methods known in the art. Reduction or decrease in HBV mRNA amount or level, expressed protein amount or level, and/or serum HBV DNA amount or level, are collectively referred to herein as a reduction or decrease in HBV or inhibiting or reducing the expression of HBV.

### Cells and Tissues and non-Human organisms

Cells, tissues, and non-human organisms that include at least one of the HBV RNAi agents described herein is contemplated. The cell, tissue, or non-human organism is made by delivering the RNAi agent to the cell, tissue, or non-human organism.

The above provided embodiments and items are now illustrated with the following, non-limiting examples.

### EXAMPLES

### Example 1. Synthesis of HBV V RNAi agents.

HBV RNAi agent duplexes shown in Table 5 were synthesized in accordance with the following:
*A. Synthesis.* The sense and antisense strands of the HBV RNAi agents were synthesized according to phosphoramidite technology on solid phase used in oligonucleotide synthesis. Depending on the scale, either a MerMade96E^{®} (Bioautomation), a MerMade12^{®} (Bioautomation), or an OP Pilot 100 (GE Healthcare) was used. Syntheses were performed on a solid support made of controlled pore glass (CPG, 500 Å or 600Å, obtained from Prime Synthesis, Aston, PA, USA). All RNA and 2'-modified phosphoramidites were purchased from Thermo Fisher Scientific (Milwaukee, WI, USA). Specifically, the following 2'-O-methyl phosphoramidites were used: (5'-O-dimethoxytrityl-N⁶-(benzoyl)-2'-O-methyl-adenosine-3'-O-(2-cyanoethyl-N,N-diisopropylamino) phosphoramidite, 5'-O-dimethoxy-trityl-N⁴-(acetyl)-2'-O-methyl-cytidine-3'-O-(2-cyanoethyl-N,N-diisopropyl-amino) phosphoramidite, (5'-O-dimethoxytrityl-N²-(isobutyryl)-2'-O-methyl-guanosine-3'-O-(2-cyanoethyl-N,N-diisopropylamino) phosphoramidite, and 5'-O-dimethoxytrityl-2'-O-methyl-uridine-3'-O-(2-cyanoethyl-N,N-diisopropylamino) phosphoramidite. The 2'-deoxy-2'-fluoro-phosphoramidites carried the same protecting groups as the 2'-O-methyl amidites. The abasic (3'-O-dimethoxytrityl-2'-deoxyribose-5'-O-(2-cyanoethyl-N,N-diisopropylamino) phosphoramidites were purchased from ChemGenes (Wilmington, MA, USA). Targeting ligand containing phosphoramidites were dissolved in anhydrous dichloromethane or anhydrous acetonitrile (50 mM), while all other amidites were dissolved in anhydrous acetonitrile (50 mM) and molecular sieves (3Å) were added. 5-Benzylthio-1H-tetrazole (BTT, 250 mM in acetonitrile) or 5-Ethylthio-1H-tetrazole (ETT, 250 mM in acetonitrile) was used as activator solution. Coupling times were 12 min (RNA), 15 min (targeting ligand), 90 sec (2'OMe), and 60 sec (2'F). In order to introduce phosphorothioate linkages, a 100 mM solution of 3-phenyl 1,2,4-dithiazoline-5-one (POS, obtained from PolyOrg, Inc., Leominster, MA, USA) in anhydrous Acetonitrile was employed.
*B. Cleavage and deprotection of support bound oligomer.* After finalization of the solid phase synthesis, the dried solid support was treated with a 1:1 volume solution of 40 wt. % methylamine in water and 28% ammonium hydroxide solution (Aldrich) for 1.5 hours at 30°C. The solution was evaporated and the solid residue was reconstituted in water (see below).
*C. Purification.* Crude oligomers were purified by anionic exchange HPLC using a TSKgel SuperQ-5PW 13µm column and Shimadzu LC-8 system. Buffer A was 20 mM Tris, 5 mM EDTA, pH 9.0 and contained 20% Acetonitrile and buffer B was the same as buffer A with the addition of 1.5 M sodium chloride. UV traces at 260 nm were recorded. Appropriate fractions were pooled then run on size exclusion HPLC using a GE Healthcare XK 26/40 column packed with Sephadex G-25 fine with a running buffer of filtered DI water or 100mM ammonium bicarbonate, pH 6.7 and 20% Acetonitrile.
*D. Annealing.* Complementary strands were mixed by combining equimolar RNA solutions (sense and antisense) in 1×Phosphate-Buffered Saline (Corning, Cellgro) to form the RNAi agents. Some RNAi agents were lyophilized and stored at -15 to -25°C. Duplex concentration was determined by measuring the solution absorbance on a UV-Vis spectrometer in 1× Phosphate-Buffered Saline. The solution absorbance at 260 nm was then multiplied by a conversion factor and the dilution factor to determine the duplex concentration. Unless otherwise stated, all conversion factor was 0.037 mg/(mL·cm). For some experiments, a conversion factor was calculated from an experimentally determined extinction coefficient.

### Example 2. pHBV model mice.

Six to eight-week-old female NOD.CB17-Prkdscid/NcrCrl (NOD-SCID) mice were transiently transfected *in vivo* with MC-HBV1.3 by hydrodynamic tail vein injection (Yang PL et al. "Hydrodynamic injection of viral DNA: a mouse model of acute hepatitis B virus infection," PNAS USA 2002 Vol. 99: p. 13825-13830), administered 30 to 45 days prior to administration of an HBV RNAi agent or control. MC-HBV1.3 is a plasmid-derived minicircle that contains the same terminally redundant human hepatitis B virus sequence HBV1.3 as in plasmid pHBV1.3 and in the HBV1.3.32 transgenic mice (GenBank accession #V01460) (Guidotti LG et al., "High-level hepatitis B virus replication in transgenic mice," J Virol 1995 Vol. 69, p6158-6169.). 5 or 10 µg MC-HBV1.3 in Ringer's Solution in a total volume of 10% of the animal's body weight was injected into mice via tail vein to create pHBV model of chronic HBV infection. The solution was injected through a 27-gauge needle in 5-7 seconds as previously described (Zhang G et al., "High levels of foreign gene expression in hepatocytes after tail vein injection of naked plasmid DNA." Human Gene Therapy 1999 Vol. 10, p1735-1737.). At pre-dose (either day 1 pre-dose, day -1, or day -2), Hepatitis B surface antigen (HBsAg) HBsAg expression levels in serum were measured by ELISA and the mice were grouped according to average HBsAg expression levels.

*Analyses:* At various times, before and after administration of HBV RNAi agents, serum HBsAg, serum HBeAg, serum HBV DNA, or liver HBV RNA may be measured. HBV expression levels were normalized to pre-administration expression levels and to control mice injected with phosphate buffered saline ("PBS").
*i) Serum collection:* Mice were anesthetized with 2-3% isoflurane and blood samples were collected from the submandibular area into serum separation tubes (Sarstedt AG & Co., Nümbrecht, Germany). Blood was allowed to coagulate at ambient temperature for 20 min. The tubes were centrifuged at 8,000 ×g for 3 min to separate the serum and stored at 4°C.
*ii) Serum Hepatitis B surface antigen (HBsAg) levels:* Serum was collected and diluted 10 to 8000-fold in PBS containing 5% nonfat dry milk. Secondary HBsAg standards diluted in the nonfat milk solution were prepared from serum of ICR mice (Harlan Sprague Dawley) that had been transfected with 10 µg HBsAg-expressing plasmid pRc/CMV-HBs (Aldevron, Fargo, ND). HBsAg levels were determined with a GS HBsAg EIA 3.0 kit (Bio-Rad Laboratories, Inc., Redmond, WA) as described by the manufacturer. Recombinant HBsAg protein, ayw subtype, also diluted in nonfat milk in PBS, was used as a primary standard (Aldevron).

HBsAg expression for each animal was normalized to the control group of mice injected with PBS in order to account for the non-treatment related decline in expression of MC-HBV1.3. First, the HBsAg level for each animal at a time point was divided by the pre-treatment level of expression in that animal in order to determine the ratio of expression "normalized to pre-treatment". Expression at a specific time point was then normalized to the control group by dividing the "normalized to pre-treatment" ratio for an individual animal by the average "normalized to pre-treatment" ratio of all mice in the normal PBS control group.

*iii) Serum Hepatitis B e-antigen (HBeAg) levels:* HBeAg analysis was performed with the HBeAg enzyme linked immunosorbent assay (ELISA) as described by the manufacturer (DiaSorin) using serum diluted 4- to 20-fold in 5% nonfat dry milk. The amount of antigen was determined in the linear range of the assay and quantitated against HBeAg protein standards (Fitzgerald Industries International, catalog # 30-AH18, Acton, MA).

HBeAg expression for each animal was normalized to the control group of mice injected with PBS in order to account for the non-treatment related decline in expression of MC-HBV1.3. For evaluation of HBeAg in serum, HBeAg is analyzed from pooled group or subgroup serum samples. First, the HBeAg level for each pooled group or subgroup was divided by the pre-treatment level of expression in the same group or subgroup in order to determine the ratio of expression "normalized to pre-treatment". Expression at a specific time point was then normalized to the control group by dividing the "normalized to pre-treatment" ratio for a group or subgroup by the average "normalized to pre-treatment" ratio of all samples from the normal PBS control group.

*iv) Serum HBV DNA levels:* Equal volumes of serum from mice in a group or subgroup were pooled to a final volume of 100 µL. DNA was isolated from serum samples using the QIAamp MinElute Virus Spin Kit (Qiagen, Valencia, CA) following the manufacturer's instructions. Sterile 0.9% saline was added to each sample to a final volume of 200 µL. Serum samples were added to tubes containing buffer and protease. Carrier RNA was added to aid in the isolation of small amounts of DNA. 1 ng of pHCR/UbC-SEAP plasmid DNA (Wooddell CI, et al. "Long-term RNA interference from optimized siRNA expression constructs in adult mice." Biochem Biophys Res Commun (2005) 334, 117-127) was added as a recovery control. After incubating 15 min at 56°C, nucleic acids were precipitated from the lysates with ethanol and the entire solution applied to a column. After washing, the samples were eluted into a volume of 50 µL Buffer AVE.

The number of copies of HBV genomes in DNA isolated from the pHBV mouse model serum was determined by qPCR. Plasmid pSEAP-HBV353-777, encoding a short segment of the HBV genome within the S gene (bases 353-777 of GenBank accession #V01460), was used to create a six log standard curve. Samples with recovery of DNA below 2 standard deviations from the average, based on detection of pHCR/UbC-SEAP were omitted. TaqMan chemistry-based primers and probes with fluor/ZEN/IBFQ are utilized.

qPCR assays were performed on a 7500 Fast or StepOne Plus Real-Time PCR system (Life Technologies). For evaluation of HBV DNA in serum, DNA was isolated from singlet or duplicate purification steps from pooled group serum samples. Quantitations of HBV DNA and recovery control plasmid were determined by qPCR reactions performed in triplicate. The probes to quantitate HBV and pHCR/UbC-SEAP were included in each reaction.

### Example 3. HBV RNAi agents in pHBV model mice.

The pHBV mouse model described in Example 2, above, was used. At day 1, each mouse was administered a single subcutaneous injection of 200 µl containing 2 mg/kg (mpk) of an HBV RNAi agent formulated in phosphate buffered saline ("PBS"), or 200 µl of phosphate buffered saline without an HBV RNAi agent, to be used as a control. Each of the HBV RNAi agents included N-acetyl-galactosamine targeting ligands conjugated to the 5'-terminal end of the sense strand, as shown in Tables 4 and 5. The HBV RNAi agents tested included those having the duplex numbers shown in Table 7, below. The injections were performed between the skin and muscle (i.e. subcutaneous injections) into the loose skin over the neck and shoulder area. Three (3) mice in each group were tested (n=3).

Serum was collected on day 8, day 15, day 22, and day 29, and serum Hepatitis B surface antigen (HBsAg) levels were determined pursuant to the procedure set forth in Example 2, above. Data from the experiment is shown in the following Table:

**Table 7. Average HBsAg levels normalized to pre-treatment and PBS control in pHBV mice following administration of HBV RNAi agents from Example 3 (standard deviation reflected as (+/-)).**

| **Group** | **Day 8** | **Day 15** | **Day 22** | **Day 29** |
|---|---|---|---|---|
| PBS | 1.000 ± 0.185 | 1.000 ± 0.288 | 1.000 ± 0.540 | 1.000 ± 0.326 |
| AD04178 | 0.164 ± 0.043 | 0.206 ± 0.044 | 0.293 ± 0.050 | 0.348 ± 0.099 |
| AD04579 | 0.083 ± 0.028 | 0.099 ± 0.022 | 0.112 ± 0.022 | 0.138 ± 0.056 |
| AD04580 | 0.048 ± 0.007 | 0.073 ± 0.012 | 0.085 ± 0.012 | 0.126 ± 0.014 |
| AD04570 | 0.241 ± 0.076 | 0.294 ± 0.071 | 0.276 ± 0.068 | 0.474 ± 0.092 |
| AD04572 | 0.190 ± 0.040 | 0.279 ± 0.011 | 0.323 ± 0.049 | 0.441 ± 0.046 |
| AD04573 | 0.333 ± 0.143 | 0.505 ± 0.106 | 0.361 ± 0.060 | 0.444 ± 0.068 |
| AD04574 | 0.291 ± 0.032 | 0.650 ± 0.056 | 0.388 ± 0.048 | 0.485 ± 0.070 |
| AD04575 | 0.397 ± 0.189 | 0.514 ± 0.234 | 0.574 ± 0.204 | 0.689 ± 0.207 |
| AD04419 | 0.262 ± 0.038 | 0.174 ± 0.042 | 0.258 ± 0.064 | 0.311 ± 0.089 |
| AD04578 | 0.210 ± 0.056 | 0.235 ± 0.033 | 0.298 ± 0.035 | 0.336 ± 0.049 |

RNAi agents AD04178, AD04579, AD04580, AD04570, AD04572, AD04573, AD04574, AD04575, AD04419, and AD04578 were each designed to have antisense strand sequences at least partially complementary to the X open reading frame at positions 1781-1789 of the HBV genome shown in Tables 1 and 2, above. Each of the HBV RNAi agents showed substantial reduction in HBsAg as compared to the PBS control across all measured time points. For example, AD04580 showed greater than 95% reduction in s-antigen levels at day 8 (0.048 ± 0.007 HBsAg level) when normalized to pre-treatment and PBS control.

Additionally, serum HBV DNA levels were determined for the PBS, AD04579, and AD04580 groups from serum samples collected on days 8, 15, 22, 29, 36, 43 and 50, pursuant to the procedure set forth in Example 2, above. Serum from each group was pooled and then DNA was isolated from the serum in duplicate isolations. Data are presented in the following Table:

**Table 8. Average Serum HBV DNA levels normalized to pre-treatment and PBS control in pHBV mice following administration of HBV RNAi agents from Example 3 (standard deviation reflected as (+/-)).**

| **Group** | **Day 8** | **Day 15** | **Day 22** | **Day 29** |
|---|---|---|---|---|
| PBS | 1.0000 ±0.1185 | 1.0000 ± 0.0591 | 1.0000 ± 0.0322 | 1.0000 ± 0.0597 |
| AD04579 | 0.1541 ±0.0070 | 0.1776 ± 0.0027 | 0.1810 ± 0.0450 | 0.3738 ± 0.0302 |
| AD04580 | 0.0921 ±0.0253 | 0.0869 ± 0.0117 | 0.1444 ± 0.0755 | 0.0950 ± 0.0026 |
| | | | | |

| **Group** | **Day 36** | **Day 43** | **Day 50** | |
|---|---|---|---|---|
| PBS | 1.0000 ± 0.1625 | 1.0000 ± 0.0055 | 1.0000 ± 0.1484 | |
| AD04579 | 0.9670 ± 0.1247 | 0.7643 ± 0.1334 | 0.6299 ± 0.1319 | |
| AD04580 | 0.4949 ± 0.0096 | 0.4350 ± 0.0344 | 0.6819 ± 0.0266 | |

The data in Table 8 indicate that both RNAi agents examined provided a substantial reduction in HBV DNA levels compared to the PBS group, with AD04580 achieving slightly greater than 1 log knockdown at nadir (e.g., 0.0869 ± 0.0117 average serum DNA level at day 15).

### Example 4. HB V RNAi agents in pHBV model mice.

The pHBV mouse model described in Example 2, above, was used. At day 1, each mouse was given a single subcutaneous administration of 200 µl containing 2 mg/kg (mpk) of an HBV RNAi agent formulated in phosphate buffered saline, or 200 µl of phosphate buffered saline without an HBV RNAi agent to be used as a control. Each of the HBV RNAi agents included N-acetyl-galactosamine targeting ligands conjugated to the 5'-terminal end of the sense strand, as shown in Tables 4 and 5. The HBV RNAi agents administered included those listed in Table 9, below. The injections were performed between the skin and muscle (i.e. subcutaneous injections) into the loose skin over the neck and shoulder area. Three (3) mice in each group were tested (n=3).

Serum was collected on day 8, day 15, day 22, and day 29, and serum Hepatitis B surface antigen (HBsAg) levels were determined pursuant to the procedure set forth in Example 2, above. Data from the experiment is shown in the following Table:

**Table 9. Average HBsAg levels normalized to pre-treatment and PBS control in pHBV mice following administration of HBV RNAi agents from Example 4 (standard deviation reflected as (+/-)).**

| **Group** | **Day 8** | **Day 15** | **Day 22** | **Day 29** |
|---|---|---|---|---|
| PBS | 1.000 ± 0.085 | 1.000 ± 0.235 | 1.000 ± 0.171 | 1.000 ± 0.099 |
| AD04010 | 0.229 ± 0.141 | 0.165 ± 0.091 | 0.142 ± 0.085 | 0.116 ± 0.076 |
| AD04581 | 0.379 ± 0.042 | 0.221 ± 0.066 | 0.135 ± 0.040 | 0.112 ± 0.050 |
| AD04591 | 0.285 ± 0.101 | 0.145 ± 0.064 | 0.086 ± 0.024 | 0.081 ± 0.026 |
| AD04434 | 0.295 ± 0.041 | 0.191 ± 0.008 | 0.147 ± 0.016 | 0.187 ± 0.049 |
| AD04583 | 0.488 ± 0.018 | 0.545 ± 0.037 | 0.511 ± 0.086 | 0.663 ± 0.112 |
| AD04584 | 0.392 ± 0.136 | 0.337 ± 0.073 | 0.364 ± 0.075 | 0.515 ± 0.155 |
| AD04585 | 0.099 ± 0.016 | 0.042 ± 0.014 | 0.030 ± 0.009 | 0.044 ± 0.014 |
| AD04586 | 0.222 ± 0.056 | 0.107 ± 0.034 | 0.074 ± 0.016 | 0.106 ± 0.039 |
| AD04588 | 0.255 ± 0.065 | 0.205 ± 0.021 | 0.185 ± 0.021 | 0.207 ± 0.024 |
| AD04438 | 0.265 ± 0.106 | 0.113 ± 0.045 | 0.091 ± 0.031 | 0.130 ± 0.038 |

RNAi agents AD04010, AD04581, AD04591, AD04434, AD04583, AD04584, AD04585, AD04586, AD04588, and AD04438 were designed to have antisense strand sequences that are at least partially complementary to the S open reading frame at positions 257-275 of the HBV genome, as shown in Tables 1 and 2. The HBV RNAi agents shown in Table 9, directly above, each showed substantial reduction in HBsAg as compared to the PBS control across all measured time points. For example, AD04585 exhibited approximately a 90% reduction of HBsAg at day 8, a 95% reduction at day 15, a 97% reduction at day 22, and a 95% reduction at day 29.

Additionally, serum HBV DNA levels were determined for the PBS, AD04585 groups from serum samples collected on days 8, 15, 22, 29, 36, 43 and 50, pursuant to the procedure set forth in Example 2, above. Serum from each group was pooled and then DNA was isolated from the serum in duplicate isolations. Data are presented in the following Table:

**Table 10. Average Serum HBV DNA levels normalized to pre-treatment and PBS control in pHBV mice following administration of HBV RNAi agents from Example 4 (standard deviation reflected as (+/-)).**

| **Group** | **Day 8** | **Day 15** | **Day 22** | **Day 29** |
|---|---|---|---|---|
| PBS | 1.000 ± 0.248 | 1.000 ± 0.089 | 1.000 ± 0.195 | 1.000 ± 0.180 |
| AD04585 | 0.901 ± 0.183 | 0.225 ± 0.003 | 0.187 ± 0.023 | 0.191 ± 0.004 |
| | | | | |

| **Group** | **Day 36** | **Day 43** | **Day 50** | |
|---|---|---|---|---|
| PBS | 1.000 ± 0.018 | 1.000 ± 0.033 | 1.000 ± 0.778 | |
| AD04585 | 0.209 ± 0.017 | 0.171 ± 0.019 | 0.305 ± 0.010 | |

The data in Table 10 indicate that HBV RNAi agent AD04585 provided a reduction in HBV DNA levels compared to the PBS group.

### Example 5. Dose response and combinations of HBV RNAi Agents in pHBV model mice.

The pHBV mouse model described in Example 2, above, was used. The mice were divided into various groups including those set forth in Table 11, below, and the mice were given 200 µl subcutaneous injections pursuant to the dosing regimen set forth in Table 11:

**Table 11. Dosing groups of pHBV mice for Example 5.**

| Group | RNAi Agent and Dose | Dosing Regimen |
|---|---|---|
| A | PBS (no RNAi agent) | Single injection on day 1 |
| B | 3.0 mg/kg AD04585 | Single injection on day 1 |
| C | 3.0 mg/kg AD04585 | Injection on day 1, day 8, and day 15 (i.e., three weekly injections) |
| D | 3.0 mg/kg AD04580 | Single injection on day 1 |
| E | 3.0 mg/kg AD04580 | Injection on day 1, day 8, and day 15 (i.e., three weekly injections) |
| F | 1.0 mg/kg AD4585 ± 1.0 mg/kg AD04580 | Injection on day 1, and another injection on day 22 |
| G | 1.0 mg/kg AD4585 ± 1.0 mg/kg AD04580 | Injection on day 1, day 8, day 15, and day 43 |
| H | 1.5 mg/kg AD4585 ± 1.5 mg/kg AD04580 | Injection on day 1, day 22, and day 43 |
| I | 1.5 mg/kg AD4585 ± 1.5 mg/kg AD04580 | Injection on day 1, day 8, day 15, and day 43 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 11. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected on day 8, day 15, day 22, day 29, day 36, day 43, day 50, and day 57, and serum Hepatitis B surface antigen (HBsAg) levels were determined pursuant to the procedure set forth in Example 2, above. Data from the experiment is shown in the following Table:

**Table 12. Average HBsAg levels normalized to pre-treatment and PBS control in pHBV mice following administration of HBV RNAi agents from Example 5 (standard deviation reflected as (+/-)).**

| **Group** | **Day 8** | **Day 15** | **Day 22** | **Day 29** |
|---|---|---|---|---|
| A | 1.000 ± 0.162 | 1.000 ± 0.138 | 1.000 ± 0.083 | 1.000 ± 0.204 |
| B | 0.060 ± 0.015 | 0.010 ± 0.003 | 0.006 ± 0.002 | 0.007 ± 0.002 |
| C | 0.087 ± 0.014 | 0.004 ± 0.001 | 0.001 ± 0.0003 | 0.0002 ± 0.0001 |
| D | 0.026 ± 0.009 | 0.035 ± 0.013 | 0.037 ± 0.014 | 0.046 ± 0.006 |
| E | 0.023 ± 0.005 | 0.002 ± 0.001 | 0.001 ± 0.0003 | 0.001 ± 0.0004 |
| F | 0.063 ± 0.046 | 0.083 ± 0.051 | 0.086 ± 0.016 | 0.027 ± 0.006 |
| G | 0.062 ± 0.011 | 0.022 ± 0.008 | 0.009 ± 0.003 | 0.008 ± 0.002 |
| H | 0.055 ± 0.015 | 0.062 ± 0.002 | 0.072 ± 0.013 | 0.011 ± 0.001 |
| I | 0.031 ± 0.006 | 0.008 ± 0.001 | 0.003 ± 0.0004 | 0.003 ± 0.0003 |
| | | | | |

| **Group** | **Day 36** | **Day 43** | **Day 50** | **Day 57** |
|---|---|---|---|---|
| A | 1.000 ± 0.211 | 1.000 ± 0.189 | 1.000 ± 0.179 | 1.000 ± 0.062 |
| B | 0.013 ± 0.005 | 0.027 ± 0.004 | 0.026 ± 0.004 | 0.057 ± 0.012 |
| C | 0.001 ± 0.0002 | 0.002 ± 0.001 | 0.008 ± 0.004 | 0.020 ± 0.015 |
| D | 0.116 ± 0.019 | 0.214 ± 0.056 | 0.263 ± 0.046 | 0.404 ± 0.030 |
| E | 0.003 ± 0.0001 | 0.007 ± 0.001 | 0.012 ± 0.002 | 0.033 ± 0.011 |
| F | 0.029 ± 0.003 | 0.065 ± 0.005 | 0.064 ± 0.004 | 0.161 ± 0.033 |
| G | 0.014 ± 0.008 | 0.039 ± 0.011 | 0.018 ± 0.008 | 0.046 ± 0.008 |
| H | 0.017 ± 0.005 | 0.039 ± 0.008 | 0.007 ± 0.001 | 0.013 ± 0.003 |
| I | 0.007 ± 0.001 | 0.020 ± 0.002 | 0.005 ± 0.001 | 0.011 ± 0.002 |

HBV RNAi agents AD04580 and AD04585 each individually showed a reduction in HBsAg as compared to the PBS control across all measured time points. Furthermore, combination treatment of AD04585 and AD04580, which as noted in the Examples above target different regions of the HBV genome, also showed reduction in HBsAg as compared to the PBS control across all measured time points.

Additionally, serum HBV DNA levels were determined for each of the groups in Table 11 from serum samples collected on days 8, 15, 22, 29, and 36, pursuant to the procedure set forth in Example 2, above. Serum from each group was pooled and then DNA was isolated from the serum in duplicate reactions. Data are presented in the following Table:

**Table 13. Average Serum HBV DNA levels normalized to pre-treatment and PBS control in pHBV mice following administration of HBV RNAi agents from Example 5 (standard deviation reflected as (+/-)).**

| **Group** | **Day 8** | **Day 15** | **Day 22** | **Day 29** |
|---|---|---|---|---|
| A | 1.000 ± 0.063 | 1.000 ± 0.059 | 1.000 ± 0.372 | 1.000 ± 0.237 |
| B | 0.267 ± 0.003 | 0.043 ± 0.016 | 0.038 ± 0.008 | 0.044 ± 0.004 |
| C | 0.236 ± 0.016 | 0.023 ± 0.001 | 0.004 ± 0.001 | 0.002 ± 0.000 |
| D | 0.058 ± 0.016 | 0.085 ± 0.017 | 0.252 ± 0.071 | 0.217 ± 0.009 |
| E | 0.056 ± 0.002 | 0.0009 ± 0.0004 | 0.0005 ± 0.0002 | 0.003 ± 0.002 |
| F | 0.298 ± 0.013 | 0.351 ± 0.032 | 0.823 ± 0.127 | 0.217 ± 0.007 |
| G | 0.276 ± 0.035 | 0.112 ± 0.020 | 0.061 ± 0.002 | 0.073 ± 0.002 |
| H | 0.232 ± 0.012 | 0.213 ± 0.028 | 0.403 ± 0.047 | 0.079 ± 0.005 |
| I | 0.092 ± 0.026 | 0.055 ± 0.000 | 0.002 ± 0.003 | 0.010 ± 0.004 |
| | | | | |

| **Group** | **Day 36** | | | |
|---|---|---|---|---|
| A | 1.000 ± 0.024 | | | |
| B | 0.046 ± 0.007 | | | |
| C | 0.003 ± 0.000 | | | |
| D | 0.319 ± 0.034 | | | |
| E | 0.002 ± 0.000 | | | |
| F | 0.122 ± 0.004 | | | |
| G | 0.047 ± 0.006 | | | |
| H | 0.056 ± 0.003 | | | |
| I | 0.021 ± 0.007 | | | |

The data in Table 13 indicate that the RNAi agents examined, both individually and in combination, provided a reduction in HBV DNA levels compared to the PBS group. Re-dosing or increasing the dose amount yielded additional HBV DNA reductions.

### Example 6. HBV RNAi agents in pHBV mice: dose response and combination studies.

The pHBV mouse model described in Example 2, above, was used. Mice were divided into various groups as set forth in Table 14, below, and each mouse was administered a single 200 µl subcutaneous injection pursuant to the dosing regimen set forth in Table 14:

**Table 14. Dosing groups of pHBV mice for Example 6.**

| Group | RNAi Agent and Dose | Dosing Regimen |
|---|---|---|
| A | PBS (no RNAi agent) | Single injection on day 1 |
| B | 4.0 mg/kg AD04981 | Single injection on day 1 |
| C | 1.0 mg/kg AD04981 | Single injection on day 1 |
| D | 2.0 mg/kg AD04981 | Single injection on day 1 |
| E | 1.0 mg/kg AD04963 | Single injection on day 1 |
| F | 2.0 mg/kg AD04963 | Single injection on day 1 |
| G | 3.0 mg/kg AD04872 | Single injection on day 1 |
| H | 3.0 mg/kg AD04872 ± 1.0 mg/kg AD04981 | Single injection on day 1 |
| I | 3.0 mg/kg AD04872 ± 1.0 mg/kg AD04963 | Single injection on day 1 |
| J | 3.0 mg/kg AD04872 ± | Single injection on day 1 |
| | 2.0 mg/kg AD04981 | |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 14. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected on day -1 prior to administration, and then on day 8, day 15, day 22, day 29, and day 36, and serum HBsAg levels were determined pursuant to the procedure set forth in Example 2, above. Data from the experiment is shown in the following Table 15, with Average HBsAg reflecting the normalized average value of HBsAg:

**Table 15. Average HBsAg levels normalized to pre-treatment and PBS control in pHBV mice following administration of HBV RNAi agents from Example 6 (standard deviation reflected as (+/-)).**

| **Group** | **Day 8** | **Day 15** | **Day 22** |
|---|---|---|---|
| A | 1.000 ± 0.068 | 1.000 ± 0.183 | 1.000 ± 0.181 |
| B | 0.085 ± 0.020 | 0.068 ± 0.005 | 0.089 ± 0.014 |
| C | 0.283 ± 0.039 | 0.343 ± 0.055 | 0.436 ± 0.004 |
| D | 0.161 ± 0.052 | 0.137 ± 0.036 | 0.190 ± 0.068 |
| E | 0.182 ± 0.040 | 0.233 ± 0.023 | 0.436 ± 0.029 |
| F | 0.078 ± 0.024 | 0.093 ± 0.015 | 0.167 ± 0.028 |
| G | 0.066 ± 0.030 | 0.013 ± 0.002 | 0.010 ± 0.002 |
| H | 0.033 ± 0.012 | 0.016 ± 0.005 | 0.020 ± 0.005 |
| I | 0.040 ± 0.011 | 0.028 ± 0.003 | 0.032 ± 0.007 |
| J | 0.035 ± 0.010 | 0.019 ± 0.002 | 0.021 ± 0.001 |
| | | | |

| **Group** | **Day 29** | **Day 36** | |
|---|---|---|---|
| A | 1.000 ± 0.032 | 1.000 ± 0.141 | |
| B | 0.148 ± 0.016 | 0.194 ± 0.047 | |
| C | 0.622 ± 0.041 | 0.741 ± 0.132 | |
| D | 0.234 ± 0.055 | 0.280 ± 0.071 | |
| E | 0.623 ± 0.116 | 0.782 ± 0.114 | |
| F | 0.259 ± 0.014 | 0.368 ± 0.068 | |
| G | 0.010 ± 0.003 | 0.009 ± 0.004 | |
| H | 0.022 ± 0.005 | 0.024 ± 0.009 | |
| I | 0.065 ± 0.014 | 0.087 ± 0.015 | |
| J | 0.031 ± 0.0001 | 0.044 ± 0.002 | |

The HBV RNAi agents tested showed a reduction in HBsAg as compared to the PBS control across all measured time points. Furthermore, combination treatment of AD04872 (which includes an antisense strand sequence that is at least partially complementary to the S ORF at positions 261-279 of the HBV genome, as shown in Tables 1 and 2) and either AD04981 or AD04963 (both of which include antisense strand sequences that are at least partially complementary to the X ORF at positions 1781-1799 of the HBV genome, as shown in Tables 1 and 2), which are shown in Groups H, I, and J of Example 6, illustrate that combination treatment of two RNAi agents targeting, one which targets in the S ORF, and the other which targets in the X ORF of the HBV genome, similarly showed reduction in HBsAg compared to the PBS control across all measured time points.

Additionally, Serum Hepatitis B e-antigen (HBeAg) levels were also assessed. Samples from the mice in each respective group were first pooled, and the resulting serum samples were assayed in singlet. Data from the experiment is shown in the following Table:

**Table 16. Average HBeAg levels normalized to pre-treatment and PBS control in pHBV mice following administration of HBV RNAi agents from Example 6.**

| **Group** | **Day 8** | **Day 15** | **Day 22** | **Day 29** | **Day 36** |
|---|---|---|---|---|---|
| A | 1.000 | 1.000 | 1.000 | 0.183 | 1.000 |
| B | 0.138 | 0.180 | 0.274 | 0.005 | 0.089 |
| C | 0.316 | 0.376 | 0.588 | 0.055 | 0.436 |
| D | 0.167 | 0.250 | 0.262 | 0.036 | 0.190 |
| E | 0.301 | 0.327 | 0.447 | 0.023 | 0.436 |
| F | 0.167 | 0.172 | 0.305 | 0.015 | 0.167 |
| G | 0.275 | 0.135 | 0.158 | 0.002 | 0.010 |
| H | 0.080 | 0.053 | 0.094 | 0.005 | 0.020 |
| I | 0.165 | 0.124 | 0.185 | 0.003 | 0.032 |
| J | 0.120 | 0.057 | 0.101 | 0.002 | 0.021 |

As shown in Table 16, the combination AD04872 (which targets the S ORF of the HBV genome) with either AD04981 or AD04963 (both of which target the X ORF of the HBV genome), showed a further reduction in HBeAg levels relative to administering AD04872 alone.

### Example 7. HBV RNAi Agents in pHBV mice: additional dose response and combination studies.

The pHBV mouse model described in Example 2, above, was used. Mice were divided into various groups as set forth in Table 17, below, and each mouse was administered a single 200 µl subcutaneous injection pursuant to the dosing regimen set forth in Table 17:

**Table 17. Dosing groups of pHBV mice for Example 7.**

| **Group** | **RNAi Agent and Dose** | **Dosing Regimen** |
|---|---|---|
| A | PBS (no RNAi agent) | Single injection on day 1 |
| B | 4.0 mg/kg AD04776 | Single injection on day 1 |
| C | 1.0 mg/kg AD04982 | Single injection on day 1 |
| D | 2.0 mg/kg AD04982 | Single injection on day 1 |
| E | 1.0 mg/kg AD04776 | Single injection on day 1 |
| F | 2.0 mg/kg AD04776 | Single injection on day 1 |
| G | 3.0 mg/kg AD04872 | Single injection on day 1 |
| H | 3.0 mg/kg AD04872 + 1.0 mg/kg AD04982 | Single injection on day 1 |
| I | 3.0 mg/kg AD04872 + 2.0 mg/kg AD04982 | Single injection on day 1 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 17. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Four (4) mice in each group were tested on day -1 and day 8 (n=4), and then one mouse per group was euthanized for histological evaluation. Three (3) mice in each group were tested at day 22 and day 29 (n=3).

Serum was collected on day -1 prior to administration, and then on day 8, day 15, day 22, and day 29, and serum Hepatitis B surface antigen (HBsAg) levels were determined pursuant to the procedure set forth in Example 2, above. Data from the experiment is shown in the following Table 18:

**Table 18. Average HBsAg levels normalized to pre-treatment (day -1) and PBS control in pHBV mice following administration of HBV RNAi agents from Example 7 (standard deviation reflected as (+/-)).**

| **Group** | **Day 8** | **Day 15** | **Day 22** | **Day 29** |
|---|---|---|---|---|
| A | 1.000 ± 0.347 | 1.000 ± 0.278 | 1.000 ± 0.194 | 1.000 ± 0.318 |
| B | 0.117 ± 0.069 | 0.085 ± 0.039 | 0.148 ± 0.045 | 0.198 ± 0.049 |
| C | 0.519 ± 0.058 | 0.375 ± 0.012 | 0.422 ± 0.046 | 0.525 ± 0.037 |
| D | 0.342 ± 0.062 | 0.255 ± 0.046 | 0.272 ± 0.122 | 0.314 ± 0.068 |
| E | 0.279 ± 0.057 | 0.245 ± 0.032 | 0.374 ± 0.121 | 0.304 ± 0.035 |
| F | 0.224 ± 0.018 | 0.161 ± 0.009 | 0.310 ± 0.016 | 0.482 ± 0.053 |
| G | 0.029 ± 0.010 | 0.005 ± 0.001 | 0.004 ± 0.001 | 0.006 ± 0.001 |
| H | 0.016 ± 0.005 | 0.004 ± 0.001 | 0.010 ± 0.006 | 0.015 ± 0.008 |
| I | 0.026 ± 0.012 | 0.008 ± 0.001 | 0.010 ± 0.002 | 0.015 ± 0.005 |

The HBV RNAi agents tested showed a reduction in HBsAg as compared to the PBS control across all measured time points.

Additionally, Serum Hepatitis B e-antigen (HBeAg) levels were also assessed. Samples from the mice in each respective group were first pooled, and the resulting serum samples were assayed in singlet. Data from the experiment is shown in the following Table:

**Table 19. Average HBeAg levels normalized to pre-treatment and PBS control in pHBV mice following administration of HBV RNAi agents from Example 7.**

| **Group** | **Day 8** | **Day 15** | **Day 22** | **Day 29** | **Day 36** |
|---|---|---|---|---|---|
| A | 1.000 | 1.000 | 1.000 | 1.000 | 1.000 |
| B | 0.193 | 0.213 | 0.260 | 0.307 | 0.464 |
| C | 0.471 | 0.424 | 0.562 | 0.513 | 0.705 |
| D | 0.335 | 0.310 | 0.411 | 0.442 | 0.500 |
| E | 0.381 | 0.368 | 0.355 | 0.564 | 0.483 |
| F | 0.275 | 0.255 | 0.370 | 0.495 | 0.449 |
| G | 0.323 | 0.218 | 0.205 | 0.250 | 0.190 |
| H | 0.124 | 0.102 | 0.099 | 0.156 | 0.156 |
| I | 0.081 | 0.059 | 0.045 | 0.063 | 0.086 |

**Table 19-1. Average HBeAg fold knockdown normalized to pre-treatment and PBS control in pHBV mice following administration of HBV RNAi agents from Example 7.**

| **Group** | **Day 8 (Fold KD)** | **Day 15 (Fold KD)** | **Day 22 (Fold KD)** | **Day 29 (Fold KD)** | **Day 36 (Fold KD)** |
|---|---|---|---|---|---|
| A | 1.0 | 1.0 | 1.0 | 1.0 | 1.0 |
| B | 5.2 | 4.7 | 3.8 | 3.3 | 2.2 |
| C | 2.1 | 2.4 | 1.8 | 2.0 | 1.4 |
| D | 3.0 | 3.2 | 2.4 | 2.3 | 2.0 |
| E | 2.6 | 2.7 | 2.8 | 1.8 | 2.1 |
| F | 3.6 | 3.9 | 2.7 | 2.0 | 2.2 |
| G | 3.1 | 4.6 | 4.9 | 4.0 | 5.3 |
| H | 8.1 | 9.8 | 10.1 | 6.4 | 6.4 |
| I | 12.3 | 17.0 | 22.3 | 15.7 | 11.6 |

Table 19-1 reflects the fold knockdown ratio of HBeAg compared to control, which is calculated as normalized HBeAg level of the control (PBS) group/normalized HBeAg level of the respected RNAi agent(s) group (i.e., 1.000/HBeAg level). The data in Table 19-1 indicate that the combination of AD04872 (which, as noted above, includes an antisense strand sequence that is at least partially complementary to the S ORF at positions 261-279 of the HBV genome) with AD04982 (which includes an antisense strand sequence that is at least partially complementary to the X ORF at positions 1781-1799 of the HBV genome), showed a further reduction in HBeAg levels relative to administering the individual RNAi agents alone *(See, e.g.,* Tables 19 and 19-1 for Groups H and I). Further, the data from this Example also show that the combination of AD04872 with AD04982 resulted in fold decrease of HBeAg greater than the sum of the fold decrease of HBeAg in AD04872 and AD04982 administered individually. For example, Group I (which is the administration of 3.0 mg/kg AD04872 + 2.0 mg/kg AD04982) resulted in a fold decrease of HBeAg at day 15 of 17.0, which is greater than the sum of the fold decrease for Group G (3.0 mg/kg AD04872) of 4.6 plus the fold decrease for Group D (2.0 mg/kg AD04982) of 3.2.

Further, serum HBV DNA levels were determined for each of the groups in Table 17 from serum samples collected on days -1, 8, 15, 22, 29, and 36, pursuant to the procedure set forth in Example 2, above. Serum HBV DNA was isolated from each animal at each time point. Data are presented in the following Table:

**Table 20. Average Serum HBV DNA levels normalized to pre-treatment and PBS control in pHBV mice following administration of HBV RNAi agents from Example 7 (standard deviation reflected as (+/-)).**

| **Group** | **Day 8** | **Day 15** | **Day 22** | **Day 29** |
|---|---|---|---|---|
| A | 1.000 ± 0.493 | 1.000 ± 0.358 | 1.000 ± 0.424 | 1.000 ± 0.387 |
| B | 0.224 ± 0.150 | 0.263 ± 0.185 | 0.335 ± 0.204 | 0.449 ± 0.108 |
| C | 0.358 ± 0.207 | 0.428 ± 0.073 | 0.433 ± 0.220 | 0.474 ± 0.090 |
| D | 0.516 ± 0.163 | 0.523 ± 0.264 | 0.244 ± 0.123 | 0.241 ± 0.085 |
| E | 0.601 ± 0.388 | 0.319 ± 0.125 | 0.279 ± 0.138 | 0.506 ± 0.525 |
| F | 0.363 ± 0.128 | 0.374 ± 0.197 | 0.275 ± 0.146 | 0.385 ± 0.141 |
| G | 0.071 ± 0.032 | 0.022 ± 0.009 | 0.015 ± 0.015 | 0.025 ± 0.005 |
| H | 0.069 ± 0.070 | 0.018 ± 0.014 | 0.019 ± 0.020 | 0.022 ± 0.001 |
| I | 0.044 ± 0.024 | 0.033 ± 0.016 | 0.017 ± 0.012 | 0.022 ± 0.014 |
| | | | | |

| **Group** | **Day 36** | | | |
|---|---|---|---|---|
| A | 1.000 ± 0.326 | | | |
| B | 0.603 ± 0.068 | | | |
| C | 0.509 ± 0.163 | | | |
| D | 0.543 ± 0.079 | | | |
| E | 0.444 ± 0.407 | | | |
| F | 0.721 ± 0.043 | | | |
| G | 0.058 ± 0.030 | | | |
| H | 0.047 ± 0.021 | | | |
| I | 0.058 ± 0.051 | | | |

The data in Table 20 indicate that the RNAi agents examined, both individually and in combination, provided a reduction in HBV DNA levels compared to the PBS group, and further show that the combination of AD04872 (which targets the S ORF) and AD04982 (which targets the X ORF) reduces serum HBV DNA to a similar degree as an equal amount of AD04872 alone.

### Example 8. HBV RNAi Agents in pHBV mice: further dose response and combination studies.

The pHBV mouse model described in Example 2, above, was used. Mice were divided into various groups as set forth in Table 21, below, and each mouse was administered a single 200 µl subcutaneous injection pursuant to the dosing regimen set forth in Table 21:

**Table 21. Dosing groups of pHBV mice for Example 8.**

| **Group** | **RNAi Agent and Dose** | **Dosing Regimen** | **Number of Animals (n)** |
|---|---|---|---|
| 1 | PBS (no RNAi agent) | Single injection on day 1 | 4 |
| 2A | 4.0 mg/kg AD04872 + 1.0 mg/kg AD05070 | Single injection on day 1 | 4 |
| 2B | 4.0 mg/kg AD04872 + 1.0 mg/kg AD05070 | Single injection on day 1 | 4 |
| 3A | 3.3 mg/kg AD04872 + 1.7 mg/kg AD05070 | Single injection on day 1 | 4 |
| 3B | 3.3 mg/kg AD04872 + 1.7 mg/kg AD05070 | Single injection on day 1 | 4 |
| 4A | 3.2 mg/kg AD04872 + 0.8 mg/kg AD05070 | Single injection on day 1 | 4 |
| 4B | 3.2 mg/kg AD04872 + 0.8 mg/kg AD05070 | Single injection on day 1 | 4 |
| 5A | 2.7 mg/kg AD04872 + 1.3 mg/kg AD05070 | Single injection on day 1 | 4 |
| 5B | 2.7 mg/kg AD04872 + 1.3 mg/kg AD05070 | Single injection on day 1 | 4 |
| 6A | 4.0 mg/kg AD05070 | Single injection on day 1 | 4 |
| 6B | 4.0 mg/kg AD05070 | Single injection on day 1 | 4 |
| 7A | 1.7 mg/kg AD05070 | Single injection on day 1 | 4 |
| 7B | 1.7 mg/kg AD05070 | Single injection on day 1 | 4 |
| 8A | 0.8 mg/kg AD05070 | Single injection on day 1 | 4 |
| 8B | 0.8 mg/kg AD05070 | Single injection on day 1 | 4 |
| 9 | 1.7 mg/kg AD05148 | Single injection on day 1 | 4 |
| 10 | 2.7 mg/kg AD04872 | Single injection on day 1 | 3 |
| 11 | 1.7 mg/kg AD05147 | Single injection on day 1 | 3 |
| 12 | 4.0 mg/kg AD04872 | Single injection on day 1 | 3 |
| 13 | 1.7 mg/kg AD05149 | Single injection on day 1 | 3 |

Additionally, the mice are scheduled to be euthanized pursuant to the following schedule:
- Day 11: Euthanize 2 mice from groups 2A, 3A, 4A, 5A, 6A, 7A and 8A, and euthanize one mouse from group 9.
- Day 14: Euthanize 2 mice from groups 2A, 3A, 4A, 5A, 6A, 7A, and 8A.
- Day 21: Euthanize 2 mice from groups 2B, 3B, 4B, 5B, 6B, 7B, and 8B.
- Day 28: Euthanize 2 mice from groups 1, 2B, 3B, 4B, 5B, 6B, 7B, and 8B, and all mice (4) from groups 10 and 12.

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 21. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. As shown in Table 14 above, four (4) mice in each group were tested (n=4), except for groups 10, 11, 12 and 13, in which three mice were tested (n=3).

Serum was collected on day -1 prior to administration, and on days 8, 14, 21 and 28, and serum Hepatitis B surface antigen (HBsAg) levels were determined pursuant to the procedure set forth in Example 2, above. Data from the experiment is shown in the following Table:

**Table 22. Average HBsAg levels normalized to pre-treatment and PBS control in pHBV mice following administration of HBV RNAi agents from Example 8 (standard deviation reflected as (+/-)).**

| **Group Number** | **Day 8** | **Day 14** | **Day 21** | **Day 28** |
|---|---|---|---|---|
| 1 | 1.000 ± 0.089 | 1.000 ± 0.087 | 1.000 ± 0.132 | 1.000 ± 0.138 |
| 2A | 0.009 ± 0.003 | 0.005 ± 0.001 | | |
| 2B | 0.006 ± 0.003 | 0.002 ± 0.001 | 0.004 ± 0.001 | 0.005 ± 0.001 |
| 3A | 0.032 ± 0.021 | 0.009 ± 0.004 | | |
| 3B | 0.028 ± 0.027 | 0.008 ± 0.006 | 0.012 ± 0.005 | 0.015 ± 0.005 |
| 4A | 0.036 ± 0.020 | 0.012 ± 0.006 | | |
| 4B | 0.029 ± 0.025 | 0.010 ± 0.008 | 0.015 ± 0.005 | 0.022 ± 0.004 |
| 5A | 0.027 ± 0.014 | 0.008 ± 0.002 | | |
| 5B | 0.027 ± 0.013 | 0.007 ± 0.003 | 0.019 ± 0.004 | 0.031 ± 0.005 |
| 6A | 0.058 ± 0.035 | 0.069 ± 0.039 | | |
| 6B | 0.117 ± 0.058 | 0.079 ± 0.047 | 0.145 ± 0.082 | 0.135 ± 0.061 |
| 7A | 0.189 ± 0.100 | 0.084 ± 0.029 | | |
| 7B | 0.099 ± 0.010 | 0.147 ± 0.025 | 0.267 ± 0.048 | 0.345 ± 0.063 |
| 8A | 0.355 ± 0.099 | 0.366 ± 0.069 | | |
| 8B | 0.271 ± 0.058 | 0.334 ± 0.060 | 0.464 ± 0.055 | 0.624 ± 0.053 |
| 9 | 0.239 ± 0.148 | 0.179 ± 0.127 | 0.309 ± 0.213 | 0.345 ± 0.225 |
| 10 | 0.018 ± 0.009 | 0.005 ± 0.003 | 0.005 ± 0.002 | 0.007 ± 0.003 |
| 11 | 0.129 ± 0.068 | 0.138 ± 0.060 | 0.239 ± 0.092 | 0.315 ± 0.119 |
| 12 | 0.033 ± 0.022 | 0.002 ± 0.001 | 0.002 ± 0.001 | 0.002 ± 0.0004 |
| 13 | 0.200 ± 0.093 | 0.239 ± 0.114 | 0.367 ± 0.123 | 0.477 ± 0.125 |

The HBV RNAi agents tested, both alone and in combination, showed a substantial reduction in HBsAg as compared to the PBS control across all measured time points.

### Example 9. RNAi agent delivery.

The pHBV mouse model described in Example 2, above, was used. At day 1, each mouse was administered a single subcutaneous injection of 200 µl containing 10 mg/kg (mpk) of an HBV RNAi agent formulated in phosphate buffered saline, or 200 µl of phosphate buffered saline without an HBV RNAi agent, to be used as a control. The HBV RNAi agents tested included those having the duplex numbers shown in Table 23, below, which each included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected prior to administration, and then on day 8, day 15, day 22, and day 29, and serum Hepatitis B surface antigen (HBsAg) levels were determined pursuant to the procedure set forth in Example 2, above. Data from the experiment is shown in the following Table:

**Table 23. Average HBsAg levels normalized to pre-treatment and PBS control in pHBV mice following administration of HBV RNAi agents from Example 9 (standard deviation reflected as (+/-)).**

| RNAi agent | HBsAg in serum at nadir (norm. fraction) | %KD at nadir | Day of nadir |
|---|---|---|---|
| PBS | 1.000 | N/A | N/A |
| AD03498 | 0.087 ± 0.016 | 91.3% | 8 |
| AD03499 | 0.069 ± 0.011 | 93.1% | 15 |
| AD03500 | 0.095 ±0.031 | 90.5% | 8 |
| AD03501 | 0.046 ± 0.020 | 95.4% | 15 |

Each of the HBV RNAi agents shown in Table 23, above, included an antisense strand sequence that is at least partially complementary to the X ORF at positions 1781-1799 of the HBV genome. Each of the RNAi agents showed a significant knockdown compared to PBS control.

### Example 10. HBV RNAi Agents in pHB V mice: further combination studies.

The pHBV mouse model described in Example 2, above, was used. Mice were divided into various groups as set forth in Table 24, below, and each mouse was administered a single 200 µl subcutaneous injection pursuant to the dosing regimen set forth in Table 24:

**Table 24. Dosing groups of pHBV mice for Example 10.**

| **Group** | **RNAi Agent and Dose** | **Dosing Regimen** |
|---|---|---|
| A | PBS Group I (no RNAi agent) | Single injection on day 1 and day 22 |
| B | PBS Group II (no RNAi agent) | Single injection on day 1 and day 22 |
| C | 3.0 mg/kg AD04585 | Single injection on day 1, day 22, day 50, and day 64 |
| D | 3.0 mg/kg AD04771 | Single injection on day 1 and day 22 |
| E | 3.0 mg/kg AD04580 | Single injection on day 1, day 22, day 50, and day 64 |
| F | 3.0 mg/kg AD04776 | Single injection on day 1 and day 22 |
| G | 1.5 mg/kg AD04585 + 1.5 mg/kg AD04580 | Single injection on day 1, day 22, day 50, and day 64 |
| H | 1.5 mg/kg AD04771 + 1.5 mg/kg AD04776 | Single injection on day 1 and day 22 |
| I | 2.0 mg/kg AD04771 + 1.0 mg/kg AD04776 | Single injection on day 1 and day 22 |
| J | 2.25 mg/kg AD04771 + 0.75 mg/kg AD04776 | Single injection on day 1 and day 22 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 24. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected prior to administration, and then on day -1, day 8, day 15, day 22, day 29, day 36, day 43, day 50, day 57, and day 64. Serum Hepatitis B surface antigen (HBsAg) levels were determined pursuant to the procedure set forth in Example 2, above. Data from the experiment is shown in the following:

**Table 25. Average HBsAg levels normalized to pre-treatment and PBS control (Group A used as control) in pHBV mice following administration of HBV RNAi agents from Example 10 (standard deviation reflected as (+/-)).**

| **Group** | **Day 8** | **Day 15** | **Day 22** |
|---|---|---|---|
| A | 1.000 ± 0.146 | 1.000 ± 0.095 | 1.000 ± 0.202 |
| B | 0.931 ± 0.161 | 1.091 ± 0.156 | 1.132 ± 0.259 |
| C | 0.071 ± 0.050 | 0.031 ± 0.022 | 0.024 ± 0.013 |
| D | 0.134 ± 0.035 | 0.130 ± 0.024 | 0.119 ± 0.028 |
| E | 0.015 ± 0.001 | 0.041 ± 0.012 | 0.087 ± 0.015 |
| F | 0.197 ± 0.081 | 0.308 ± 0.138 | 0.476 ± 0.156 |
| G | 0.029 ± 0.015 | 0.069 ± 0.029 | 0.094 ± 0.016 |
| H | 0.191 ± 0.057 | 0.315 ± 0.094 | 0.420 ± 0.126 |
| I | 0.153 ± 0.050 | 0.194 ± 0.076 | 0.233 ± 0.116 |
| J | 0.155 ± 0.059 | 0.177 ± 0.067 | 0.316 ± 0.117 |
| | | | |

| **Group** | **Day 29** | **Day 36** | **Day 43** |
|---|---|---|---|
| A | 1.000 ± 0.182 | 1.000 ± 0.287 | 1.000 ± 0.298 |
| B | 1.417 ± 0.414 | 1.166 ± 0.248 | |
| C | 0.007 ± 0.005 | 0.004 ± 0.003 | 0.006 ± 0.001 |
| D | 0.048 ± 0.023 | 0.036 ± 0.020 | 0.052 ± 0.027 |
| E | 0.014 ± 0.006 | 0.021 ± 0.011 | 0.026 ± 0.011 |
| F | 0.246 ± 0.081 | 0.244 ± 0.097 | 0.179 ± 0.061 |
| G | 0.023 ± 0.009 | 0.027 ± 0.009 | 0.037 ± 0.013 |
| H | 0.200 ± 0.080 | 0.185 ± 0.081 | 0.194 ± 0.055 |
| I | 0.141 ± 0.082 | 0.133 ± 0.051 | 0.151 ± 0.082 |
| J | 0.133 ± 0.064 | 0.102 ± 0.039 | 0.129 ± 0.050 |
| | | | |

| **Group** | **Day 50** | **Day 57** | **Day 64** |
|---|---|---|---|
| A | 1.000 ± 0.296 | 1.000 ± 0.394 | 1.000 ± 0.395 |
| B | | | |
| C | 0.015 ± 0.0001 | 0.002 ± 0.001 | 0.004 ± 0.001 |
| D | | | |
| E | 0.052 ± 0.015 | 0.009 ± 0.002 | 0.018 ± 0.007 |
| F | | | |
| G | 0.076 ± 0.020 | 0.012 ± 0.003 | 0.020 ± 0.007 |
| H | | | |
| I | | | |
| J | | | |

HBV RNAi agents AD04585 and AD04771 were designed to have antisense strand sequences that are at least partially complementary to the S open reading frame at positions 257-275 of the HBV genome, as shown in Tables 1 and 2. HBV RNAi agents AD04580 and AD04776 were designed to have antisense strand sequences that are at least partially complementary to the X open reading frame at positions 1781-1799 of the HBV genome, as shown in Tables 1 and 2 The HBV RNAi agents tested, both alone and in combination, showed a reduction in HBsAg as compared to the PBS control across all measured time points. Each subsequent dose further reduced the nadir of HBsAg reduction.

Additionally, serum HBV DNA levels were determined for Group C (3.0 mg/kg AD04585), Group E (3.0 mg/kg AD04580), and Group G (1.5 mg/kg AD04585 + 1.5 mg/kg AD04580) in Table 24, from serum samples collected on days -1, 8, 15, 22, 29, and 36, 43 and 50 pursuant to the procedure set forth in Example 2, above. Serum HBV DNA was isolated for each animal at each of these time points. Data are presented in the following Table:

**Table 26. Average Serum HBV DNA levels normalized to pre-treatment and PBS controls (both PBS groups A and B) in pHBV mice following administration of HBV RNAi agents from Example 10 (standard deviation reflected as (+/-)).**

| **Group** | **Day 8** | **Day 15** | **Day 22** | **Day 29** |
|---|---|---|---|---|
| A/B (PBS) | 1.000 ± 0.316 | 1.000 ± 0.427 | 1.000 ± 0.428 | 1.000 ± 0.475 |
| C | 0.172 ± 0.151 | 0.142 ± 0.079 | 0.252 ± 0.132 | 0.072 ± 0.086 |
| E | 0.024 ± 0.015 | 0.042 ± 0.037 | 0.449 ± 0.184 | 0.053 ± 0.048 |
| G | 0.093 ± 0.053 | 0.083 ± 0.037 | 0.370 ± 0.153 | 0.211 ± 0.060 |
| | | | | |

| **Group** | **Day 36** | **Day 43** | **Day 50** | |
|---|---|---|---|---|
| A/B (PBS) | 1.000 ± 0.623 | 1.000 ± 0.532 | 1.000 ± 0.532 | |
| C | 0.044 ± 0.020 | 0.104 ± 0.033 | 0.156 ± 0.016 | |
| E | 0.012 ± 0.004 | 0.061 ± 0.031 | 0.161 ± 0.019 | |
| G | 0.048 ± 0.022 | 0.147 ± 0.010 | 0.295 ± 0.041 | |

The data in Table 26 indicate that the HBV RNAi agents examined, both individually and in combination, provided a reduction in HBV DNA levels compared to the PBS group.

### Example 11. HBV RNAi Agents in pHBV mice: combination studies.

The pHBV mouse model described in Example 2, above, was used. Mice were divided into various groups as set forth in Table 27, below, and each mouse was administered a single 200 µl subcutaneous injection pursuant to the dosing regimen set forth in Table 27:

**Table 27. Dosing groups of pHBV mice for Example 11.**

| Group | RNAi Agent and Dose | Dosing Regimen |
|---|---|---|
| A | PBS (no RNAi agent) | Single injection on day 1 |
| B | 3.0 mg/kg AD04962 | Single injection on day 1 |
| C | 3.0 mg/kg AD04963 | Single injection on day 1 |
| D | 1.5 mg/kg AD04962 + 1.5 mg/kg AD04963 | Single injection on day 1 |
| E | 2.0 mg/kg AD04962 + 1.0 mg/kg AD04963 | Single injection on day 1 |
| F | 2.25 mg/kg AD04962 + 0.75 mg/kg AD04963 | Single injection on day 1 |
| G | 1.5 mg/kg AD04962 + 1.5 mg/kg AD04963 | Single injection on day 1 |
| H | 3.0 mg/kg AD04962 + 3.0 mg/kg AD04963 | Single injection on day 1 |
| I | 1.5 mg/kg AD04962 + 1.5 mg/kg AD04963 | Single injection on day 1 |
| J | 4.5 mg/kg AD04962 + 4.5 mg/kg AD04963 | Single injection on day 1 |
| K | 3.0 mg/kg AD04872 | Single injection on day 1 |
| L | 3.0 mg/kg AD04882 | Single injection on day 1 |
| M | 3.0 mg/kg AD04885 | Single injection on day 1 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 24. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected on day -1 prior to administration, and then on day 8, day 15, day 22, day 29, and day 36 (except for Group L (AD04882) and Group M (AD04885), and serum Hepatitis B surface antigen (HBsAg) levels were determined pursuant to the procedure set forth in Example 2, above. Data from the experiment is shown in the following Table:

**Table 28. Average HBsAg normalized to pre-treatment and PBS control in pHBV mice following administration of HBV RNAi agents from Example 11 (standard deviation reflected as (+/-)).**

| **Group** | **Day 8** | **Day 15** | **Day 22** |
|---|---|---|---|
| A | 1.000 ± 0.048 | 1.000 ± 0.144 | 1.000 ± 0.083 |
| B | 0.125 ± 0.025 | 0.083 ± 0.014 | 0.063 ± 0.016 |
| C | 0.019 ± 0.005 | 0.035 ± 0.008 | 0.052 ± 0.009 |
| D | 0.054 ± 0.013 | 0.079 ± 0.009 | 0.108 ± 0.021 |
| E | 0.099 ± 0.025 | 0.098 ± 0.053 | 0.142 ± 0.050 |
| F | 0.070 ± 0.015 | 0.103 ± 0.036 | 0.140 ± 0.020 |
| G | 0.041 ± 0.021 | 0.012 ± 0.008 | 0.021 ± 0.013 |
| H | 0.020 ± 0.006 | 0.044 ± 0.010 | 0.062 ± 0.019 |
| I | 0.077 ± 0.017 | 0.019 ± 0.004 | 0.004 ± 0.001 |
| J | 0.012 ± 0.002 | 0.021 ± 0.001 | 0.032 ± 0.002 |
| K | 0.045 ± 0.014 | 0.013 ± 0.005 | 0.008 ± 0.005 |
| L | 0.106 ± 0.020 | 0.176 ± 0.044 | 0.215 ± 0.082 |
| M | 0.275 ± 0.029 | 0.378 ± 0.080 | 0.572 ± 0.043 |
| | | | |

| **Group** | **Day 29** | **Day 36** | |
|---|---|---|---|
| A | 1.000 ± 0.209 | 1.000 ± 0.270 | |
| B | | 0.079 ± 0.020 | 0.096 ± 0.007 |
| C | | 0.087 ± 0.014 | 0.164 ± 0.026 |
| D | | 0.176 ± 0.014 | 0.292 ± 0.030 |
| E | | 0.223 ± 0.082 | 0.373 ± 0.150 |
| F | | 0.213 ± 0.020 | 0.328 ± 0.034 |
| G | | 0.031 ± 0.013 | 0.078 ± 0.064 |
| H | | 0.97 ± 0.028 | 0.160 ± 0.060 |
| I | | 0.008 ± 0.001 | 0.002 ± 0.0003 |
| J | | 0.044 ± 0.008 | 0.069 ± 0.009 |
| K | | 0.011 ± 0.007 | 0.011 ± 0.009 |
| L | | 0.299 ± 0.009 | |
| M | | 0.792 ± 0.057 | |

RNAi agent AD04962 was designed to have an antisense strand sequence that is at least partially complementary to the S open reading frame at positions 257-275 of the HBV genome, as shown in Tables 1 and 2. RNAi agent AD04872 was designed to have an antisense strand sequence that is at least partially complementary to the S open reading frame at positions 261-279 of the HBV genome, as shown in Tables 1 and 2. RNAi agent AD04963 was designed to have an antisense strand sequence that is at least partially complementary to the X open reading frame at positions 1781-1799 of the HBV genome, as shown in Tables 1 and 2. RNAi agents AD04882 and AD04885 were designed to have antisense strand sequences that are at least partially complementary to the X open reading frame at positions 1780-1798 of the HBV genome, as shown in Tables 1 and 2. The HBV RNAi agents shown in Table 9, directly above, each showed a reduction in HBsAg as compared to the PBS control across all measured timepoints, both individually and in combination. Re-dosing yielded additional HBsAg reduction.

Additionally, Serum Hepatitis B e-antigen (HBeAg) levels were also assessed for all groups except Groups L and M. Samples from the mice in each respective group were first pooled, and the resulting serum samples were assayed in singlet. Data from the experiment is shown in the following Table:

**Table 29. Average HBeAg levels normalized to pre-treatment and PBS control in pHBV mice following administration of HBV RNAi agents from Example 11.**

| **Group** | **Day 8** | **Day 22** | **Day 29** | **Day 36** |
|---|---|---|---|---|
| A | 1.000 | 1.000 | 1.000 | 1.000 |
| B | 0.425 | 0.291 | 0.371 | 0.365 |
| C | 0.152 | 0.170 | 0.328 | 0.356 |
| D | 0.266 | 0.249 | 0.456 | 0.440 |
| E | 0.278 | 0.295 | 0.589 | 0.561 |
| F | 0.306 | 0.291 | 0.718 | 0.522 |
| G | 0.183 | 0.138 | 0.291 | 0.249 |
| H | 0.091 | 0.131 | 0.315 | 0.238 |
| I | 0.183 | 0.052 | 0.069 | 0.036 |
| J | 0.089 | 0.114 | 0.190 | 0.236 |
| K | 0.458 | 0.172 | 0.322 | 0.207 |

Further, serum HBV DNA levels were determined for each of the groups in Table 27 from serum samples collected on days 8, 15, 22, and 29, pursuant to the procedure set forth in Example 2, above. Serum HBV DNA was isolated from each animal at each time point. Data are presented in the following Table:

**Table 30. Average Serum HBV DNA levels normalized to pre-treatment and PBS control in pHBV mice following administration of HBV RNAi agents from Example 7 (standard deviation reflected as (+/-)).**

| **Group** | **Day 8** | **Day 15** | **Day 22** | **Day 29** |
|---|---|---|---|---|
| A | 1.000 ± 0.232 | 1.000 ± 0.463 | **1.000** ± 0.272 | 1.000 ± 0.205 |
| B | 0.577 ± 0.219 | 0.222 ± 0.064 | 0.196 ± 0.055 | 0.261 ± 0.117 |
| C | 0.165 ± 0.051 | 0.070 ± 0.042 | 0.142 ± 0.105 | 0.228 ± 0.174 |
| D | 0.343 ± 0.125 | 0.307 ± 0.091 | 0.300 ± 0.092 | 0.356 ± 0.032 |
| E | 0.262 ± 0.033 | 0.216 ± 0.018 | 0.227 ± 0.028 | 0.279 ± 0.090 |
| F | 0.320 ± 0.134 | 0.332 ± 0.208 | 0.344 ± 0.209 | 0.338 ± 0.211 |
| G | 0.231 ± 0.036 | 0.034 ± 0.024 | 0.069 ± 0.039 | 0.077 ± 0.020 |
| H | 0.229 ± 0.101 | 0.155 ± 0.121 | 0.148 ± 0.079 | 0.215 ± 0.035 |
| I | 0.281 ± 0.129 | 0.109 ± 0.071 | 0.023 ± 0.019 | 0.011 ± 0.009 |
| J | 0.078 ± 0.050 | 0.061 ± 0.020 | 0.074 ± 0.029 | 0.056 ± 0.030 |
| K | 0.314 ± 0.064 | 0.119 ± 0.043 | 0.076 ± 0.067 | 0.078 ± 0.095 |
| L | 0.295 ± 0.077 | 0.305 ± 0.101 | 0.213 ± 0.088 | 0.186 ± 0.084 |
| M | 0.515 ± 0.247 | 0.505 ± 0.293 | 0.488 ± 0.318 | 0.478 ± 0.267 |

The data in Table 30 indicate that the RNAi agents examined, both individually and in combination, provided a reduction in HBV DNA levels compared to the PBS group. Re-dosing yielded addition reduction of HBV DNA.

### Example 12. HBV RNAi Agents in pHBV mice.

The pHBV mouse model described in Example 2, above, was used. Mice were divided into various groups as set forth in Table 31, below, and each mouse was administered a single 200 µl subcutaneous injection pursuant to the dosing regimen set forth in Table 31:

**Table 31. Dosing groups of pHBV mice for Example 12.**

| Group | RNAi Agent and Dose | Dosing Regimen |
|---|---|---|
| A | PBS (no RNAi agent) | Single injection on day 1 |
| B | 2.0 mg/kg AD04871 | Single injection on day 1 |
| C | 2.0 mg/kg AD04872 | Single injection on day 1 |
| D | 2.0 mg/kg AD04874 | Single injection on day 1 |
| E | 2.0 mg/kg AD04875 | Single injection on day 1 |
| F | 2.0 mg/kg AD04876 | Single injection on day 1 |
| G | 2.0 mg/kg AD04881 | Single injection on day 1 |
| H | 2.0 mg/kg AD04883 | Single injection on day 1 |
| I | 2.0 mg/kg AD04884 | Single injection on day 1 |

Each mouse was given a subcutaneous administration of 200 µl containing the amount of HBV RNAi agent formulated in phosphate buffered saline, or 200 µl of phosphate buffered saline without an HBV RNAi agent, as set forth in Table 24. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected prior to administration, and then on day 8, day 15, and day 22. Group A (PBS), Group B (2.0 mg/kg AD04871), Group C (2.0 mg/kg AD04872), Group D (2.0 mg/kg AD04874), Group E (2.0 mg/kg AD04875), and Group F (2.0 mg/kg AD04876) also had serum collected on day 29, day 36, day 43, and day 50. Serum Hepatitis B surface antigen (HBsAg) levels were determined pursuant to the procedure set forth in Example 2, above. Data from the experiment is shown in the following Table:

**Table 32. Average HBsAg normalized to pre-treatment and PBS control in pHBV mice following administration of HBV RNAi agents from Example 12 (standard deviation reflected as (+/-)).**

| **Group** | **Day 8** | **Day 15** | **Day 22** | **Day 29** |
|---|---|---|---|---|
| A | 1.000 ± 0.132 | 1.000 ± 0.089 | 1.000 ± 0.080 | 1.000 ± 0.098 |
| B | 0.102 ± 0.034 | 0.041 ± 0.021 | 0.049 ± 0.033 | 0.048 ± 0.031 |
| C | 0.153 ± 0.064 | 0.064 ± 0.032 | 0.063 ± 0.034 | 0.042 ± 0.017 |
| D | 0.123 ± 0.022 | 0.049 ± 0.017 | 0.039 ± 0.010 | 0.023 ± 0.001 |
| E | 0.190 ± 0.075 | 0.094 ± 0.038 | 0.107 ± 0.061 | 0.081 ± 0.051 |
| F | 0.190 ± 0.031 | 0.076 ± 0.035 | 0.084 ± 0.038 | 0.049 ± 0.024 |
| G | 0.159 ± 0.047 | 0.216 ± 0.057 | 0.235 ± 0.151 | |
| H | 0.508 ± 0.078 | 0.666 ± 0.131 | 0.543 ± 0.048 | |
| I | 0.279 ± 0.087 | 0.357 ± 0.078 | 0.614 ± 0.156 | |
| | | | | |

| **Group** | **Day 36** | **Day 43** | **Day 50** | |
|---|---|---|---|---|
| A | 1.000 ± 0.065 | 1.000 ± 0.242 | 1.000 ± 0.224 | |
| B | 0.054 ± 0.038 | 0.064 ± 0.030 | 0.092 ± 0.025 | |
| C | 0.049 ± 0.017 | 0.054 ± 0.015 | 0.085 ± 0.010 | |
| D | 0.037 ± 0.004 | 0.037 ± 0.010 | 0.065 ± 0.012 | |
| E | 0.126 ± 0.077 | 0.125 ± 0.063 | 0.170 ± 0.079 | |
| F | 0.089 ± 0.044 | 0.082 ± 0.034 | 0.115 ± 0.028 | |
| G | | | | |
| H | | | | |
| I | | | | |

HBV RNAi agents AD04871, AD04872, AD04874, AD04875, and AD04876 were each designed to have antisense strand sequences that are at least partially complementary to the S open reading frame at positions 261-279 of the HBV genome, as shown in Tables 1 and 2. Each of these HBV RNAi agents should a substantial reduction in HBsAg compared to PBS control. For example, a single 2 mg/kg dose of each of AD04871 (Group B), AD04872 (Group C) and AD04874 (Group D), and AD04876 (Group F), exhibited a greater than 90% reduction in HBsAg for each of the timepoints measured from day 15 through day 43 compared to control. HBV RNAi agents AD04881, AD04883, AD04884 were each designed to have antisense strand sequences that are at least partially complementary to the X open reading frame at positions 1780-1798 of the HBV genome, as shown in Tables 1 and 2.

### Example 13. Dose response and combinations of HBVV RNAi Agents in X Region Knockout model mice.

As an alternative means in assessing the effects of the combination of an RNAi agent that includes an antisense strand sequence that is at least partially complementary to a region located in the S ORF of an HBV mRNA, and a second RNAi agent that includes an antisense strand sequence that is at least partially complementary to a region located in the X ORF of an HBV mRNA, a plasmid was generated that included the HBV genome with a knockout of the binding site for HBV RNAi agents that target positions 1780 and 1781, as shown in Tables 1 and 2 (hereinafter referred to as X Region Knockout mice). This model was generated by mutating ten (10) bases in the pHBV1.3 plasmid within the binding site of these RNAi agents. The remainder of the HBV mRNA, including the S-region, remained functional. Thus, in this HBV mouse model, inclusion of an HBV RNAi agent having an antisense strand that targets positions 1780 and 1781 of the HBV genome disclosed herein is expected to be ineffective in silencing expression.

The mice were divided into various groups including those set forth in Table 33, below, and the mice were given 200 µl subcutaneous injections pursuant to the dosing regimen set forth in the following Table:

**Table 33. Dosing groups of X Region Knockout mice for Example 13.**

| Group | RNAi Agent and Dose | Dosing Regimen | **Number of Animals (n)** |
|---|---|---|---|
| 1 | PBS (no RNAi agent) | Single injection on day 1 | 4 |
| 2 | 2.0 mg/kg AD04585 + 1.0 mg/kg AD04963 | Single injection on day 1 | 4 |
| 3 | 2.0 mg/kg AD04872 + 1.0 mg/kg AD04963 | Single injection on day 1 | 4 |
| 4 | 2.5 mg/kg AD04585 + 0.5 mg/kg AD04963 | Single injection on day 1 | 4 |
| 5 | 2.5 mg/kg AD04872 + 0.5 mg/kg AD04963 | Single injection on day 1 | 4 |
| 6 | 3.0 mg/kg AD04963 | Single injection on day 15 | 1 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 33. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected on day 5, day 8, day 15, day 22, and day 29 and serum Hepatitis B surface antigen (HBsAg) levels were determined pursuant to the procedure set forth in Example 2, above. Serum was also collected for Groups 1 through 5 on days 36 and 43. Data from the experiment is shown in the following Table 34:

**Table 34. Average HBsAg normalized to pre-treatment and PBS control in X Region Knockout mice following administration of HBV RNAi agents from Example 13 (standard deviation reflected as (+/-)).**

| **Group** | **Day 8** | **Day 15** | **Day 22** |
|---|---|---|---|
| 1 | 1.000 ± 0.186 | 1.000 ± 0.165 | 1.000 ± 0.132 |
| 2 | 0.061 ± 0.034 | 0.041 ± 0.035 | 0.030 ± 0.015 |
| 3 | 0.020 ± 0.011 | 0.007 ± 0.003 | 0.003 ± 0.002 |
| 4 | 0.063 ± 0.039 | 0.022 ± 0.011 | 0.029 ± 0.013 |
| 5 | 0.027 ± 0.014 | 0.003 ± 0.003 | 0.001 ± 0.001 |
| 6 | 0.948 | 1.360 | 1.652 |
| | | | |

| | **Day 29** | **Day 36** | **Day 43** |
|---|---|---|---|
| 1 | 1.000 ± 0.059 | 1.000 ± 0.044 | 1.000 ± 0.045 |
| 2 | 0.051 ± 0.029 | 0.062 ± 0.029 | |
| 3 | 0.004 ± 0.003 | 0.008 ± 0.003 | 0.018 ± 0.007 |
| 4 | 0.040 ± 0.022 | 0.061 ± 0.030 | |
| 5 | 0.002 ± 0.001 | 0.003 ± 0.002 | 0.014 ± 0.006 |
| 6 | 1.831 | | |

As expected, Group 6, which was a single dose of 3.0 mg/kg of HBV RNAi agent AD04963 and includes an antisense strand that is at least partially complementary to the X open reading frame at positions 1781-1799 of the HBV genome, was unable to provide knockdown of HBsAg. Additionally, each of Groups 2 through 5 provided substantial knockdown of HBsAg compared to PBS control, with both Group 3 and Group 5 exhibiting a greater than 2 log reduction in HBsAg at nadir (day 22).

### Example 14. Dose response and combinations of HBV RNAi Agents in X Region Knockout model mice.

The X Region Knockout mouse model described in Example 13, above, was used. Mice were divided into various groups including those set forth in Table 31, below, and each mouse was administered a single 200 µl subcutaneous injection pursuant to the dosing regimen set forth in Table 35:

**Table 35. Dosing groups of X Region Knockout mice for Example 14.**

| **Group** | **RNAi Agent and Dose** | **Dosing Regimen** |
|---|---|---|
| 1 | PBS (no RNAi agent) | Single injection on day 1 |
| 2 | 2.0 mg/kg AD04872 | Single injection on day 1 |
| 3 | 2.0 mg/kg AD04872 + 0.7 mg/kg AD05070 | Single injection on day 1 |
| 4 | 2.0 mg/kg AD04872 + 1.0 mg/kg AD05070 | Single injection on day 1 |
| 5 | 2.0 mg/kg AD04872 + 2.0 mg/kg AD05070 | Single injection on day 1 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 35. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group shown in Table 35 were tested (n=3).

Serum was collected on day 1 (pre-dose), day 8, day 15, day 22, and day 29, and serum Hepatitis B surface antigen (HBsAg) levels were determined pursuant to the procedure set forth in Example 2, above. Data from the experiment is shown in the following Table:

**Table 36. Average HBsAg levels normalized to pre-treatment and PBS control in X Region Knockout mice from Example 14.**

| **Group** | **Day 8** | **Day 15** | **Day 22** | **Day 29** |
|---|---|---|---|---|
| 1 | 1.000 ± 0.120 | 1.000 ± 0.255 | 1.000 ± 0.224 | 1.000 ± 0.143 |
| 2 | 0.104 ± 0.104 | 0.009 ± 0.009 | 0.005 ± 0.004 | 0.005 ± 0.003 |
| 3 | 0.076 ± 0.041 | 0.010 ± 0.009 | 0.006 ± 0.005 | 0.005 ± 0.005 |
| 4 | 0.036 ± 0.008 | 0.002 ± 0.001 | 0.001 ± 0.001 | 0.002 ± 0.001 |
| 5 | 0.019 ± 0.017 | 0.003 ± 0.002 | 0.003 ± 0.001 | 0.004 ± 0.000 |

Table 36 shows that HBV RNAi agent AD04872 administered alone, and the combination of AD04872 (which includes an antisense strand that is at least partially complementary to the S open reading from at positions 261-279 of the HBV genome) and AD05070 (which includes an antisense strand that is at least partially complementary to the X open reading frame at positions 1781-1799 of the HBV genome), provided significant knockdown of HBsAg compared to PBS control across each of the time points measured.. Addition of 0.7 mg/kg to 2 mg/kg HBV RNAi agent AD05070 for which there was a mutated target site in this X Region Knockout model did not diminish the activity of the 2 mg/kg HBV RNAi agent AD04872.

Additionally, serum HBV DNA levels were determined from serum samples collected on days 8, 15, and 22 pursuant to the procedure set forth in Example 2, above. Serum from each group was pooled and then DNA was isolated from the serum in singlet. Data are presented in the following Table:

**Table 37. Average Serum HBV DNA levels normalized to pre-treatment and PBS controls in X Region Knockout mice following administration of HBV RNAi agents from Example 14 (standard deviation reflected as (+/-)).**

| **Group** | **Day 8** | **Day 15** | **Day 22** |
|---|---|---|---|
| 1 | 1.000 ± 0.007 | 1.000 ± 0.011 | 1.000 ± 0.066 |
| 2 | 0.225 ± 0.019 | 0.022 ± 0.001 | 0.036 ± 0.001 |
| 3 | 0.151 ± 0.002 | 0.029 ± 0.001 | 0.042 ± 0.003 |
| 4 | 0.140 ± 0.006 | 0.016 ± 0.000 | 0.018 ± 0.000 |
| 5 | 0.069 ± 0.002 | 0.018 ± 0.003 | 0.043 ± 0.002 |

Addition of 0.7 mg/kg to 2 mg/kg HBV RNAi agent AD05070 for which there was a mutated target site in this X Region Knockout model did not diminish the activity of the 2 mg/kg HBV RNAi agent AD04872.

### Example 15. HBV RNAi agents in pHBV mice.

The pHBV mouse model described in Example 2, above, was used. Mice were divided into various groups including those set forth in Table 38, below, and each mouse was administered a single 200 µl subcutaneous injection pursuant to the dosing regimen set forth in Table 38:

**Table 38. Dosing groups of pHBV mice for Example 15.**

| Group | RNAi Agent and Dose | Dosing Regimen |
|---|---|---|
| 1 | PBS (no RNAi agent) | Single injection on day 1 |
| 2 | 2.0 mg/kg AD04776 | Single injection on day 1 |
| 3 | 2.0 mg/kg AD05069 | Single injection on day 1 |
| 4 | 2.0 mg/kg AD05070 | Single injection on day 1 |
| 5 | 2.0 mg/kg AD05071 | Single injection on day 1 |
| 6 | 2.0 mg/kg AD05073 | Single injection on day 1 |
| 7 | 2.0 mg/kg AD05074 | Single injection on day 1 |
| 8 | 2.0 mg/kg AD05075 | Single injection on day 1 |
| 9 | 2.0 mg/kg AD05076 | Single injection on day 1 |
| 10 | 2.0 mg/kg AD05077 | Single injection on day 1 |
| 11 | 2.0 mg/kg AD05078 | Single injection on day 1 |
| 12 | 3.0 mg/kg AD04872 + | Single injection on day 1 |
| | 1.0 mg/kg AD04776 | |
| 13 | 3.0 mg/kg AD04872 + 1.0 mg/kg AD05069 | Single injection on day 1 |
| 14 | 3.0 mg/kg AD04872 + 1.0 mg/kg AD05070 | Single injection on day 1 |
| 15 | 3.0 mg/kg AD04872 + 1.0 mg/kg AD05071 | Single injection on day 1 |
| 16 | 3.0 mg/kg AD04872 + 1.0 mg/kg AD05073 | Single injection on day 1 |
| 17 | 3.0 mg/kg AD04872 + 1.0 mg/kg AD05074 | Single injection on day 1 |
| 18 | 3.0 mg/kg AD04872 + 1.0 mg/kg AD05075 | Single injection on day 1 |
| 19 | 3.0 mg/kg AD04872 + 1.0 mg/kg AD05076 | Single injection on day 1 |
| 20 | 3.0 mg/kg AD04872 + 1.0 mg/kg AD05077 | Single injection on day 1 |
| 21 | 3.0 mg/kg AD04872 + 1.0 mg/kg AD05078 | Single injection on day 1 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 38. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected on day -1 prior to administration, and then on day 8, day 15, day 22, day 29, day 36, day 43, and day 50. Serum Hepatitis B surface antigen (HBsAg) levels were determined pursuant to the procedure set forth in Example 2, above. Data from the experiment is shown in the following Table 39, with Average HBsAg reflecting the normalized average value of HBsAg:

**Table 39. Average HBsAg normalized to pre-treatment and PBS control in pHBV mice following administration of HBV RNAi agents from Example 15.**

| **Group** | **Day 8** | **Day 15** | **Day 22** | **Day 29** |
|---|---|---|---|---|
| 1 | 1.000 ± 0.119 | 1.000 ± 0.047 | 1.000 ± 0.080 | 1.000 ± 0.027 |
| 2 | 0.339 ± 0.076 | 0.414 ± 0.126 | 0.385 ± 0.067 | 0.450 ± 0.075 |
| 3 | 0.240 ± 0.096 | 0.361 ± 0.078 | 0.446 ± 0.073 | 0.508 ± 0.114 |
| 4 | 0.081 ± 0.026 | 0.127 ± 0.031 | 0.223 ± 0.057 | 0.330 ± 0.112 |
| 5 | 0.452 ± 0.020 | 0.431 ± 0.126 | 0.373 ± 0.079 | 0.383 ± 0.080 |
| 6 | 0.375 ± 0.181 | 0.632 ± 0.192 | 0.463 ± 0.117 | 0.567 ± 0.159 |
| 7 | 0.325 ± 0.032 | 0.438 ± 0.125 | 0.393 ± 0.056 | 0.443 ± 0.096 |
| 8 | 0.155 ± 0.031 | 0.322 ± 0.019 | 0.333 ± 0.077 | 0.463 ± 0.043 |
| 9 | 0.245 ± 0.063 | 0.467 ± 0.090 | 0.477 ± 0.045 | 0.562 ± 0.049 |
| 10 | 0.120 ± 0.062 | 0.173 ± 0.029 | 0.289 ± 0.019 | 0.331 ± 0.042 |
| 11 | 0.128 ± 0.042 | 0.172 ± 0.046 | 0.179 ± 0.015 | 0.215 ± 0.049 |
| 12 | 0.040 ± 0.015 | 0.014 ± 0.004 | 0.014 ± 0.006 | 0.015 ± 0.004 |
| 13 | 0.050 ± 0.020 | 0.015 ± 0.011 | 0.017 ± 0.008 | 0.022 ± 0.009 |
| 14 | 0.020 ± 0.011 | 0.011 ± 0.006 | 0.015 ± 0.006 | 0.023 ± 0.004 |
| 15 | 0.043 ± 0.005 | 0.013 ± 0.005 | 0.010 ± 0.002 | 0.011 ± 0.004 |
| 16 | 0.021 ± 0.017 | 0.008 ± 0.004 | 0.012 ± 0.003 | 0.011 ± 0.001 |
| 17 | 0.032 ± 0.011 | 0.009 ± 0.003 | 0.007 ± 0.002 | 0.008 ± 0.0003 |
| 18 | 0.023 ± 0.014 | 0.010 ± 0.006 | 0.009 ± 0.006 | 0.009 ± 0.004 |
| 19 | 0.025 ± 0.006 | 0.010 ± 0.004 | 0.009 ± 0.002 | 0.010 ± 0.003 |
| 20 | 0.061 ± 0.013 | 0.027 ± 0.006 | 0.020 ± 0.003 | 0.029 ± 0.006 |
| 21 | 0.061 ± 0.050 | 0.013 ± 0.010 | 0.012 ± 0.005 | 0.018 ± 0.006 |
| | | | | |

| **Group** | **Day 36** | **Day 43** | **Day 50** | |
|---|---|---|---|---|
| 1 | 1.000 ± 0.031 | 1.000 ± 0.114 | 1.000 ± 0.112 | |
| 2 | 0.617 ± 0.116 | 0.643 ± 0.154 | 0.665 ± 0.199 | |
| 3 | 0.638 ± 0.067 | 0.743 ± 0.015 | 0.792 ± 0.115 | |
| 4 | 0.472 ± 0.121 | 0.515 ± 0.126 | 0.689 ± 0.167 | |
| 5 | 0.591 ± 0.159 | 0.604 ± 0.086 | 0.709 ± 0.115 | |
| 6 | 0.717 ± 0.136 | 0.686 ± 0.194 | 0.781 ± 0.301 | |
| 7 | 0.586 ± 0.069 | 0.775 ± 0.143 | 0.747 ± 0.095 | |
| 8 | 0.666 ± 0.066 | 0.803 ± 0.096 | 0.856 ± 0.180 | |
| 9 | 0.801 ± 0.047 | 0.667 ± 0.055 | 0.765 ± 0.208 | |
| 10 | 0.640 ± 0.059 | 0.667 ± 0.034 | 0.742 ± 0.133 | |
| 11 | 0.429 ± 0.063 | 0.383 ± 0.005 | 0.497 ± 0.060 | |
| 12 | 0.037 ± 0.013 | 0.044 ± 0.012 | 0.056 ± 0.014 | |
| 13 | 0.046 ± 0.011 | 0.055 ± 0.010 | 0.070 ± 0.010 | |
| 14 | 0.054 ± 0.016 | 0.070 ± 0.018 | 0.096 ± 0.012 | |
| 15 | 0.029 ± 0.011 | 0.032 ± 0.015 | 0.051 ± 0.020 | |
| 16 | 0.033 ± 0.005 | 0.038 ± 0.007 | 0.062 ± 0.004 | |
| 17 | 0.021 ± 0.002 | 0.031 ± 0.004 | 0.061 ± 0.005 | |
| 18 | 0.034 ± 0.014 | 0.047 ± 0.016 | 0.079 ± 0.017 | |
| 19 | 0.028 ± 0.005 | 0.037 ± 0.006 | 0.060 ± 0.011 | |
| 20 | 0.070 ± 0.009 | 0.063 ± 0.018 | 0.097 ± 0.018 | |
| 21 | 0.040 ± 0.012 | 0.066 ± 0.007 | 0.120 ± 0.036 | |

RNAi agents AD04776, AD05069, AD05070, AD05071, AD05073, and AD05074 were each designed to have an antisense strand sequence that is at least partially complementary to the X open reading frame at positions 1781-1799 of the HBV genome, as shown in Tables 1 and 2. RNAi agents AD05075, AD05076, AD05077, and AD05078 were each designed to have antisense strand sequences that are at least partially complementary to the X open reading frame at positions 1780-1798 of the HBV genome, as shown in Tables 1 and 2.

Table 39 shows that HBV RNAi agents AD04776, AD05069, AD05070, AD05071, AD05073, and AD05074 administered alone or their combination with AD04872 (which includes an antisense strand that is at least partially complementary to the S open reading from at positions 261-279 of the HBV genome) provided significant knockdown of HBsAg compared to PBS control across each of the time points measured.

### Example 16. HBV RNAi Agents in pHBV mice: dose response and combination studies.

The pHBV mouse model described in Example 2, above, was used. Mice were divided into various groups as set forth in Table 40, below, and each mouse was administered a single 200 µl subcutaneous injection pursuant to the dosing regimen set forth in Table 40:

**Table 40. Dosing groups of pHBV mice for Example 16.**

| **Group** | **RNAi Agent and Dose** | **Dosing Regimen** |
|---|---|---|
| 1 | PBS (no RNAi agent) | Single injection on day 1 |
| 2 | 3.2 mg/kg AD04872 | Single injection on day 1 |
| 3 | 3.2 mg/kg AD04872 | Single injection on day 1 and day 22 |
| 4 | 3.0 mg/kg AD04872 + 0.8 mg/kg AD05070 | Single injection on day 1 |
| 5 | 3.0 mg/kg AD04872 + 0.8 mg/kg AD05070 | Single injection on day 1 and day 22 |
| 6 | 3.0 mg/kg AD04872 + 1.0 mg/kg AD05070 | Single injection on day 1 |
| 7 | 3.0 mg/kg AD04872 + 1.0 mg/kg AD05070 | Single injection on day 1 and day 22 |
| 8 | 2.7 mg/kg AD04872 + 1.3 mg/kg AD05070 | Single injection on day 1 |
| 9 | 2.7 mg/kg AD04872 + 1.3 mg/kg AD05070 | Single injection on day 1 and day 22 |
| 10 | 2.0 mg/kg AD04872 + 2.0 mg/kg AD04776 | Single injection on day 1 and day 22 |
| 11 | 0.8 mg/kg AD05070 | Single injection on day 1 and day 22 |
| 12 | 1.3 mg/kg AD05070 | Single injection on day 1 and day 22 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 40. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Six (6) mice in each group were tested (n=6).

Serum was collected prior to administration, and then on day 8, day 15, day 22, and day 29, and serum Hepatitis B surface antigen (HBsAg) levels were determined pursuant to the procedure set forth in Example 2, above. Data from the experiment is shown in the following Table 41:

**Table 41. Average HBsAg levels normalized to pre-treatment and PBS control in pHBV mice following administration of HBV RNAi agents from Example 16 (standard deviation reflected as (+/-)).**

| **Group** | **Day 8** | **Day 15** | **Day 22** | **Day 29** |
|---|---|---|---|---|
| 1 | 1.000 ± 0.117 | 1.000 ± 0.213 | 1.000 ± 0.169 | 1.000 ± 0.130 |
| 2 | 0.050 ± 0.018 | 0.015 ± 0.007 | 0.011 ± 0.005 | 0.009 ± 0.006 |
| 3 | 0.051 ± 0.037 | 0.014 ± 0.011 | 0.010 ± 0.006 | 0.002 ± 0.001 |
| 4 | 0.029 ± 0.018 | 0.010 ± 0.006 | 0.011 ± 0.006 | 0.010 ± 0.005 |
| 5 | 0.022 ± 0.003 | 0.007 ± 0.001 | 0.009 ± 0.003 | 0.001 ± 0.001 |
| 6 | 0.027 ± 0.012 | 0.007 ± 0.004 | 0.008 ± 0.005 | 0.011 ± 0.005 |
| 7 | 0.028 ± 0.012 | 0.010 ± 0.005 | 0.009 ± 0.005 | 0.001 ± 0.000 |
| 8 | 0.033 ± 0.016 | 0.016 ± 0.008 | 0.020 ± 0.009 | 0.021 ± 0.011 |
| 9 | 0.034 ± 0.025 | 0.015 ± 0.011 | 0.018 ± 0.013 | 0.003 ± 0.002 |
| 10 | 0.038 ± 0.021 | 0.015 ± 0.005 | 0.019 ± 0.004 | 0.003 ± 0.001 |
| 11 | 0.446 ± 0.143 | 0.376 ± 0.120 | 0.474 ± 0.149 | 0.338 ± 0.123 |
| 12 | 0.307 ± 0.111 | 0.257 ± 0.122 | 0.236 ± 0.057 | 0.138 ± 0.031 |

The HBV RNAi agents tested, both individually and in combination, showed a reduction in HBsAg as compared to the PBS control across all measured time points. HBsAg expression was further reduced in all groups that were re-dosed on day 22.

Additionally, Serum Hepatitis B e-antigen (HBeAg) levels were also assessed. For the day 8 measurement, the serum samples for all six mice in each group were pooled, and the resulting samples were assayed in singlet. For the day -1, day 15, day 22, and day 29 measurements, the six mice from each group were paired within each group and their respective serum samples were pooled, forming three subgroups for each group. The serum samples for each of the three subgroups for each group were then assayed. Data from the experiment is shown in the following Table 42:

**Table 42. Average HBeAg levels normalized to pre-treatment and PBS control in pHBV mice following administration of HBV RNAi agents from Example 16 (standard deviation for days 15, 22, and 29 reflected as (+/-)).**

| **Group** | **Day 8** | **Day 15** | **Day 22** | **Day 29** |
|---|---|---|---|---|
| 1 | 1.000 | 1.000 ±0.011 | 1.000 ±0.170 | 1.000 ±0.173 |
| 2 | 0.510 | 0.308 ± 0.031 | 0.217 ± 0.021 | 0.226 ± 0.035 |
| 3 | 0.488 | 0.301 ± 0.065 | 0.283 ± 0.081 | 0.147 ± 0.030 |
| 4 | 0.213 | 0.216 ± 0.067 | 0.192 ± 0.029 | **0.141** ± 0.048 |
| 5 | 0.192 | 0.211 ± 0.053 | 0.216 ± 0.088 | 0.047 ± 0.016 |
| 6 | 0.176 | 0.163 ± 0.022 | 0.238 ± 0.069 | 0.117 ± 0.011 |
| 7 | 0.165 | 0.175 ± 0.046 | 0.215 ± 0.061 | 0.028 ± 0.012 |
| 8 | 0.128 | 0.166 ± 0.065 | 0.386 ± 0.284 | 0.167 ± 0.118 |
| 9 | 0.172 | 0.171 ± 0.037 | 0.244 ± 0.052 | 0.032 ± 0.010 |
| 10 | 0.180 | 0.211 ± 0.012 | 0.283 ± 0.034 | 0.034 ± 0.001 |
| 11 | 0.634 | 0.594 ± 0.082 | 0.840 ± 0.152 | 0.271 ± 0.029 |
| 12 | 0.486 | 0.441 ± 0.066 | 0.804 ± 0.096 | 0.214 ± 0.039 |

The HBV RNAi agents tested, both individually and in combination, showed a reduction in HBeAg as compared to the saline control across all measured time points. HBeAg expression was further reduced in all groups that were re-dosed on day 22.

Further, serum HBV DNA levels were determined for each of the groups in Table 40 from serum samples collected on days -1, 8, 15, and 22, pursuant to the procedure set forth in Example 2, above. Serum from each pair of mice was pooled and then DNA was isolated from each serum pool in a single isolation. Data are presented in the following Table:

**Table 43. Average Serum HBV DNA levels normalized to pre-treatment and PBS control in pHBV mice following administration of HBV RNAi agents from Example 16 (standard deviation reflected as (+/-)).**

| **Group** | **Day 8** | **Day 15** | **Day 22** |
|---|---|---|---|
| 1 | 1.000 ± 0.122 | 1.000 ± 0.299 | 1.000 ± 0.241 |
| 2 | 0.312 ±0.016 | 0.126 ± 0.008 | 0.087 ± 0.018 |
| 3 | 0.264 ± 0.065 | 0.081 ± 0.023 | 0.073 ± 0.028 |
| 4 | 0.321 ± 0.254 | 0.120 ± 0.066 | 0.134 ± 0.101 |
| 5 | 0.319 ± 0.081 | 0.108 ± 0.038 | 0.098 ± 0.051 |
| 6 | 0.260 ± 0.095 | 0.068 ± 0.010 | 0.076 ± 0.031 |
| 7 | 0.170 ± 0.028 | 0.082 ± 0.013 | 0.062 ± 0.018 |
| 8 | 0.188 ± 0.020 | 0.192 ± 0.160 | 0.307 ± 0.309 |
| 9 | 0.242 ± 0.003 | 0.100 ± 0.042 | 0.075 ± 0.028 |
| 10 | 0.322 ± 0.028 | 0.159 ± 0.025 | 0.086 ± 0.016 |
| 11 | 1.124 ± 0.142 | 0.742 ± 0.127 | 0.807 ± 0.192 |
| 12 | 1.004 ± 0.144 | 0.541 ± 0.340 | 0.569 ± 0.060 |

The HBV RNAi agents tested, both individually and in combination, showed a reduction in serum HBV DNA as compared to the saline control across all measured time points except in groups 11 and 12 that had no reduction in serum HBV DNA at Day 8.

### Example 17. HBV RNAi Agents in pHBV mice.

The pHBV mouse model described in Example 2, above, was used. Mice were divided into various groups as set forth in Table 44, below, and each mouse was administered a single 200 µl subcutaneous injection pursuant to the dosing regimen set forth in Table 44:

**Table 44. Dosing groups of pHBV mice for Example 17.**

| **Group** | **RNAi Agent and Dose** | **Dosing Regimen** |
|---|---|---|
| 1 | PBS (no RNAi agent) | Single injection on day 1 |
| 2 | 5 mg/kg AD04585 + 1 mg/kg AD04963 | Single injection on day 1 |
| 3 | 5 mg/kg AD04872 + 1 mg/kg AD04963 | Single injection on day 1 |
| 4 | 5 mg/kg AD04585 + 1 mg/kg AD04963 | Single injection on day 1 and day 8 |
| 5 | 5 mg/kg AD04872 + 1 mg/kg AD04963 | Single injection on day 1 and day 8 |
| 6 | 2.5 mg/kg AD04585 + 0.5 mg/kg AD04963 | Single injection on day 1 |
| 7 | 2.0 mg/kg AD04585 + 1.0 mg/kg AD04963 | Single injection on day 1 |
| 8 | 2.5 mg/kg AD04872 + 0.5 mg/kg AD04963 | Single injection on day 1 |
| 9 | 2.0 mg/kg AD04872 + 1.0 mg/kg AD04963 | Single injection on day 1 |
| 10 | 5 mg/kg AD04872 + 1 mg/kg AD04981 | Single injection on day 1 |
| 11 | 2.5 mg/kg AD04872 + 0.5 mg/kg AD04981 | Single injection on day 1 and day 8 |
| 12 | 2.5 mg/kg AD04872 + 0.5 mg/kg AD04981 | Single injection on day 1 |
| 13 | 2 mg/kg AD04872 + 1 mg/kg AD04981 | Single injection on day 1 |
| 14 | 2.5 mg/kg AD04585 + 0.5 mg/kg AD04981 | Single injection on day 1 |
| 15 | 2 mg/kg AD04585 + 1 mg/kg AD04981 | Single injection on day 1 |
| 16 | 0.5 mg/kg AD04981 | Single injection on day 1 |

Each mouse was given a subcutaneous administration of 200 µl containing the amount of HBV RNAi agent(s) formulated in phosphate buffered saline, or 200 µl of phosphate buffered saline without an HBV RNAi agent, as set forth in Table 44. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3).

Serum was collected prior to administration, and then on day 8, day 14, day 21, and day 29 and day 36, and serum Hepatitis B surface antigen (HBsAg) levels were determined pursuant to the procedure set forth in Example 2, above. Data from the experiment is shown in the following Table 45:

**Table 45. Average HBsAg levels normalized to pre-treatment and PBS control in pHBV mice following administration of HBV RNAi agents from Example 17 (standard deviation reflected as (+/-)).**

| **Group** | **Day 8** | **Day 14** | **Day 21** | **Day 29** |
|---|---|---|---|---|
| 1 | 1.000 ± 0.068 | 1.000 ± 0.125 | 1.000 ± 0.152 | 1.000 ± 0.110 |
| 2 | 0.058 ± 0.033 | 0.059 ± 0.022 | 0.085 ± 0.023 | 0.158 ± 0.021 |
| 3 | 0.025 ± 0.009 | 0.014 ± 0.006 | 0.015 ± 0.008 | 0.026 ± 0.015 |
| 4 | 0.032 ± 0.007 | 0.005 ± 0.001 | 0.006 ± 0.002 | 0.014 ± 0.002 |
| 5 | 0.024 ± 0.009 | 0.003 ± 0.001 | 0.001 ± 0.0004 | 0.001 ± 0.0005 |
| 6 | 0.063 ± 0.020 | 0.077 ± 0.013 | 0.131 ± 0.011 | 0.214 ± 0.026 |
| 7 | 0.041 ± 0.018 | 0.059 ± 0.017 | 0.091 ± 0.016 | 0.140 ± 0.045 |
| 8 | 0.070 ± 0.008 | 0.046 ± 0.016 | 0.043 ± 0.009 | 0.055 ± 0.012 |
| 9 | 0.043 ± 0.006 | 0.027 ± 0.003 | 0.064 ± 0.017 | 0.064 ± 0.014 |
| 10 | 0.015 ± 0.008 | 0.005 ± 0.003 | 0.005 ± 0.003 | 0.005 ± 0.003 |
| 11 | 0.047 ± 0.014 | 0.005 ± 0.003 | 0.003 ± 0.002 | 0.003 ± 0.003 |
| 12 | 0.062 ± 0.006 | 0.025 ± 0.007 | 0.027 ± 0.005 | 0.033 ± 0.005 |
| 13 | 0.092 ± 0.029 | 0.050 ± 0.021 | 0.050 ± 0.022 | 0.054 ± 0.0019 |
| 14 | 0.310 ± 0.180 | 0.056 ± 0.010 | 0.081 ± 0.010 | 0.112 ± 0.0018 |
| 15 | 0.304 ± 0.044 | 0.083 ± 0.021 | 0.115 ± 0.013 | 0.165 ± 0.025 |
| 16 | 1.667 ± 0.217 | 0.416 ± 0.163 | 0.341 ± 0.179 | 0.511 ± 0.0011 |
| | | | | |

| **Group** | **Day 36** | | | |
|---|---|---|---|---|
| 1 | 1.000 ± 0.225 | | | |
| 2 | | | | |
| 3 | 0.049 ± 0.019 | | | |
| 4 | | | | |
| 5 | 0.004 ± 0.0004 | | | |
| 6 | | | | |
| 7 | | | | |
| 8 | | | 0.081 ± 0.010 | |
| 9 | | | 0.108 ± 0.026 | |
| 10 | | | 0.009 ± 0.004 | |
| 11 | | | 0.005 ± 0.003 | |
| 12 | | | 0.060 ± 0.014 | |
| 13 | | | 0.094 ± 0.027 | |
| 14 | | | | |
| 15 | | | | |
| 16 | | | 0.634 ± 0.005 | |

The HBV RNAi agent combinations tested showed a reduction in HBsAg as compared to the saline control across all measured time points. Combinations containing AD04872 showed greater reductions than the equivalent combinations with AD04585 in place of AD04872.

Additionally, serum HBV DNA levels were determined for serum samples collected on days 8, 14, 21, and 29 pursuant to the procedure set forth in Example 2, above. Serum HBV DNA was isolated from each animal at each time point. Data are presented in the following Table 46:

**Table 46. Average Serum HBV DNA levels normalized to pre-treatment and PBS control in pHBV mice following administration of HBV RNAi agents from Example 17 (standard deviation reflected as (+/-)).**

| **Group** | **Day 8** | **Day 14** | **Day 21** | **Day 29** |
|---|---|---|---|---|
| 1 | 1.000 ± 0.280 | 1.000 ± 0.269 | 1.000 ± 0.418 | 1.000 ± 0.383 |
| 2 | 0.136 ± 0.068 | 0.192 ± 0.071 | 0.173 ± 0.032 | 0.292 ± 0.039 |
| 3 | 0.097 ± 0.034 | 0.068 ± 0.016 | 0.076 ± 0.034 | 0.131 ± 0.061 |
| 4 | 0.061 ± 0.039 | 0.002 ± 0.001 | 0.003 ± 0.001 | 0.019 ± 0.013 |
| 5 | 0.068 ± 0.025 | 0.003 ± 0.002 | 0.0009 ± 0.0003 | 0.0009 ± 0.0003 |
| 6 | 0.354 ± 0.299 | 0.345 ± 0.187 | 0.522 ± 0.234 | 0.509 ± 0.106 |
| 7 | 0.103 ± 0.064 | 0.291 ± 0.025 | 0.203 ± 0.043 | 0.203 ± 0.015 |
| 8 | 0.336 ± 0.142 | 0.185 ± 0.071 | 0.183 ± 0.065 | 0.162 ± 0.064 |
| 9 | 0.198 ± 0.055 | 0.093 ± 0.023 | 0.118 ± 0.054 | 0.143 ± 0.032 |
| 10 | 0.122 ± 0.071 | 0.024 ± 0.026 | 0.023 ± 0.020 | 0.014 ± 0.017 |
| 11 | 0.160 ± 0.069 | 0.016 ± 0.023 | 0.003 ± 0.001 | 0.005 ± 0.004 |
| 12 | 0.158 ± 0.039 | 0.120 ± 0.044 | 0.100 ± 0.049 | 0.091 ± 0.034 |
| 13 | 0.190 ± 0.038 | 0.169 ± 0.025 | 0.066 ± 0.015 | 0.081 ± 0.015 |
| 14 | 0.434 ± 0.136 | 0.318 ± 0.104 | 0.144 ± 0.094 | 0.240 ± 0.029 |
| 15 | 0.358 ± 0.185 | 0.287 ± 0.108 | 0.279 ± 0.080 | 0.303 ± 0.038 |
| 16 | 0.713 ± 0.085 | 0.674 ± 0.140 | 0.496 ± 0.128 | 0.590 ± 0.093 |

The HBV RNAi agent combinations tested showed a reduction in serum HBV DNA as compared to the saline control across all measured time points. Combinations containing AD04872 showed greater reductions than the equivalent combinations with AD04585 in place of AD04872. These greater reductions were observed at Day 22 and Day 29.

### Example 18. HBV RNAi Agents in a HBV-infected Humanized Mouse Model.

For this study, Male FRG^{®} (genotype Fah -/-/ Rag2 -/-/ Il2rg -/- triple knockout mice on a C57BL/6 background (Yecuris) were transplanted with human hepatocytes when they were 1-2 months old. The human hepatocytes were allowed to repopulate the liver for approximately 6 months with periodic NTBC treatment to discourage growth of mouse hepatocytes. At 9 months of age the mice were given an intravenous inoculation of 4 x 10⁸ genomes/kg HBV genotype C, which infected the human hepatocytes. After 2-3 months, serum HBV DNA levels reached a plateau indicating the human hepatocytes were maximally infected (mouse hepatocytes cannot be infected by HBV). Mice were one year old at the start of treatment with HBV RNAi agents, thus nearing the end of their life span.

Pre-treatment serum samples were taken on day -10 and day -3. Beginning on day 1, each mouse was administered an oral daily gavage with 0.01 mg/kg Entecavir dissolved in water to inhibit HBV replication. Daily dosing of Entecavir continued until the day mice were euthanized. Entecavir administration was expected to reduce serum HBV DNA in chronically infected human patients, but not reduce HBsAg.

Mice were divided into various groups including those set forth in Table 47, below:

**Table 47. Dosing groups of HBV-infected FRG humanized model mice for Example 18.**

| **Group** | **RNAi Agent and Dose** | **Dosing Regimen** | **Terminal Day** |
|---|---|---|---|
| A- mouse 1 | PBS (no RNAi agent) | Single injection on day 1 | Euthanized day 21 (unhealthy animal) |
| A- mouse 2 | PBS (no RNAi agent) | Single injection on day 1 and day 29 | Euthanized day 36 |
| B- mouse 1 | 4.0 mg/kg AD04872 + 2.0 mg/kg AD05070 | Single injection on day 1 and day 29 | Euthanized day 36 |
| B- mouse 2 | 4.0 mg/kg AD04872 + 2.0 mg/kg AD05070 | Single injection on day 1 and day 29 | Euthanized day 40 |
| C- mouse 1 | 4.5 mg/kg AD04872 + 1.5 mg/kg AD05070 | Single injection on day 1 | Euthanized day 15 |
| C- mouse 2 | 4.5 mg/kg AD04872 + 1.5 mg/kg AD05070 | Single injection on day 1 and day 29 | Euthanized day 36 |
| C- mouse 3 | 4.5 mg/kg AD04872 + 1.5 mg/kg AD05070 | Single injection on day 1 and day 29 | Euthanized day 40 |

Each mouse was also given a subcutaneous administration of 100 µl per 20 grams body weight containing the amount of HBV RNAi agent(s) formulated in phosphate buffered saline, or an equal volume of phosphate buffered saline without an HBV RNAi agent, on day 1 and on day 29 (if still alive on day 29), pursuant to the schedule as set forth in Table 47, directly above. Each of the HBV RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area.

Serum was collected on day 8, day 15, day 22, day 29, day 36, and day 40 and serum Hepatitis B surface antigen (HBsAg) levels were determined pursuant to the procedure set forth in Example 2, above. Data from the experiment is shown in the following Table:

**Table 48. Average HBsAg levels normalized to pre-treatment (day -3) for each individual HBV-infected humanized FRG model mouse from Example 18.**

| **Group** | **Day 8** | **Day 15** | **Day 22** | **Day 29** | **Day 36** | **Day 40** |
|---|---|---|---|---|---|---|
| A-1 | 0.830 | 0.828 | 0.932 | 0.858 | 1.107 | |
| A-2 | 1.303 | 1.328 | | | | |
| B-1 | 0.548 | 0.314 | 0.272 | 0.207 | 0.138 | |
| B-2 | 0.592 | 0.337 | 0.243 | 0.215 | 0.160 | 0.175 |
| C-1 | 0.643 | 0.460 | 0.415 | 0.251 | 0.164 | |
| C-2 | 0.353 | 0.228 | 0.182 | 0.172 | 0.224 | 0.216 |
| C-3 | 0.814 | 0.674 | | | | |

Additionally, serum HBV DNA levels were determined from serum samples collected on days -10, -3, 8, 15, 22, 29, 36, and 40, pursuant to the procedure set forth in Example 2, above. Data are presented in the following Table 4:

**Table 49. Serum HBV DNA levels normalized to the average of pre-treatment day -10 and day -3 for each HBV-infected FRG humanized mouse following administration of HBV RNAi agents from Example 14.**

| **Group** | **Day -10** | **Day -3** | **Day 8** | **Day 15** | **Day 22** | **Day 29** | **Day 36** | **Day 40** |
|---|---|---|---|---|---|---|---|---|
| A-1 | 0.883 | 1.117 | 0.072 | 0.038 | 0.015 | 0.027 | 0.060 | |
| A-2 | 1.070 | 0.930 | 0.130 | 0.075 | | | | |
| B-1 | 1.538 | 0.462 | 0.032 | 0.017 | 0.011 | 0.006 | 0.010 | |
| B-2 | 1.350 | 0.650 | 0.042 | 0.018 | 0.012 | 0.007 | 0.008 | 0.007 |
| C-1 | 1.348 | 0.652 | 0.041 | 0.020 | 0.016 | 0.005 | 0.004 | |
| C-2 | 1.030 | 0.970 | 0.031 | 0.015 | 0.006 | 0.011 | 0.008 | 0.008 |

As expected, administration of Entecavir reduced viral replication in both the absence and presence of HBV RNAi agents.

### Example 19. Clinical Study of HBV RNAi agents

A Phase 1/2a, randomized, double-blind, single dose escalating study in healthy volunteers (NHV) and open label, multi-dose escalating study in patients with chronic HBV infection (CHB) were conducted. The study was designed to evaluate the safety, tolerability and pharmacokinetic effects of HBV RNAi agents (AD04872 and AD05070) in healthy patients and patients chronically infected with hepatitis B virus (CHB). The study subject population included healthy adult males and females 18-55 years old and adult males and females 18-65 years old who had a diagnosis of HBeAg positive or HBeAg negative chronic (> 6 months) HBV infection.

NHV subjects were divided into five cohorts of 6 subjects per cohort (cohorts 1-5). NHV cohorts (4 active, 2 placebo) received single doses of a combination of AD04872 and AD05070 in an amount of 35, 100, 200, 300, or 400 mg or normal saline in a blinded fashion via subcutaneous injections. CHB cohorts 1, 1c and 2b-5b were HBeAg positive or negative, nucleoside inhibitors (NUC) naive or NUC experienced at baseline, and received three monthly doses of a combination of AD04872 and AD05070 in an amount of 25, 50, 100, 200, 300, or 400 mg via subcutaneous injections. Cohort 6 enrolled 4 CHB patients to receive three 100 mg doses of a combination of AD04872 and AD05070 every two weeks. Cohort 7 enrolled 4 CHB patients to receive three weekly 100 mg doses of a combination of AD04872 and AD05070. CHB cohorts 8 and 9 (4 active) were HBeAg positive, treatment naive or NUC experienced, respectively, that received three monthly doses of a combination of AD04872 and AD05070 in an amount of 300 mg via subcutaneous injections. Cohort 10 and Cohort 11 each enrolled 4 CHB patients to receive 3 weekly 200 mg and 300 mg doses of a combination of AD04872 and AD05070 respectively. NUC experienced CHB patients continued their daily NUC throughout the study and NUC naive CHB patients started daily NUC on day 1. For CHB, viral DNA, viral RNA and antigens HBsAg, HBeAg, and HBcrAg were measured periodically. The study parameters are summarized in Table 50.

**Table 50**

| | |
|---|---|
| Study Title | A Phase 1/2a Single Dose-Escalating Study to Evaluate the Safety, Tolerability and Pharmacokinetic Effects of HBV RNAi agents (AD04872 and AD05070) in Normal Adult Volunteers and Multiple Escalating Doses Evaluating Safety, Tolerability and Pharmacodynamic Effects in HBV Patients |
| Development Phase | Phase 1 First-in-Human, Phase 2a First-in-Patient |
| Study Objectives | Primary Objectives: |
| | • To determine the incidence and frequency of adverse events possibly or probably related to treatment as a measure of the safety and tolerability of HBV RNAi agents (AD04872 and AD05070) using escalating single doses in healthy volunteers (NHV) and escalating multiple doses in patients chronically infected with hepatitis B virus (CHB). |
| | Secondary Objectives: |
| | • To evaluate the single-dose pharmacokinetics of HBV RNAi agents (AD04872 and AD05070) in NHVs |
| | • To determine the reduction from Day 1 pre-dose baseline to post-dose nadir of HBsAg in response to HBV RNAi agents (AD04872 and AD05070) in patients chronically infected with HBV (CHB) as a measure of activity. |
| | Exploratory Objective: |
| | • To determine the reduction from Day 1 pre-dose baseline to post-dose nadir of HBcrAg, HBV RNA (if scientifically feasible) and HBeAg (e+ patients only), in response to HBV RNAi agents (AD04872 and AD05070) in CHB patients as a measure of activity. |
| Study Design | Randomized, double-blind, parallel-group, placebo-controlled |
| Study Population | This study was conducted in NHVs, adult males and females, aged 18-55 years with BMI between 19.0 and 35.0 kg/m² (Cohorts 1 - 5), and in CHB patients, aged 18-65 years with BMI between 19.0 and 38.0 kg/m² (Cohorts 2b, 3b, 4b, 5b, 8 and 9) and had a diagnosis of HBeAg positive or HBeAg negative chronic (> 6 months) HBV infection. |
| | • Cohorts 1-5: NHVs, adult males and females, aged 18-55 years |
| | • Cohorts 1b, 1c, 2b, 3b, 4b and 5b: Any CHB patient regardless of HBeAg or prior therapy status. |
| | • Cohort 6: Any CHB patient regardless of HBeAg or prior therapy status. |
| | • Cohort 7: Any CHB patient regardless of HBeAg or prior therapy status. |
| | • Cohort 8: HBeAg positive treatment naïve patients (no prior chronic exposure to NUCs or Interferon). |
| | • Cohort 9: HBeAg positive NUC experienced patients (on entecavir or tenofovir for at least 12 months). |
| | • Cohort 10: Any CHB patient regardless of HBeAg or prior therapy status. |
| | • Cohort 11: Any CHB patient regardless of HBeAg or prior therapy status. |
| Investigational Product | HBV RNAi agents (AD04872 and AD05070) |
| Dosage and Frequency | Cohorts 1-5 (NHV): randomized to receive HBV RNAi agents (AD04872 and AD05070) or placebo (4 active: 2 placebo) at single escalating doses of 35, 100, 200, 300 and 400 mg administered as a single subcutaneous injection. |
| | Cohorts 1b, 1c, 2b-5b (CHB): received three monthly doses of 25, 50, 100, 200, 300, or 400 mg HBV RNAi agents (AD04872 and AD05070) via subcutaneous injections. |
| | Cohort 6 enrolled 4 HBV patients to receive three 100 mg doses of HBV RNAi agents (AD04872 and AD05070) every two weeks. Cohort 7 enrolled 4 HBV patients to receive three weekly 100 mg doses of HBV RNAi agents (AD04872 and AD05070). |
| | Cohorts 8 and 9 (CHB): received three monthly SQ doses of 300 mg HBV RNAi agents (AD04872 and AD05070) via subcutaneous injections. |
| | Cohort 10 and Cohort 11 each enrolled 4 HBV patients to receive 3 weekly 200 mg and 300 mg doses of HBV RNAi agents (AD04872 and AD05070) respectively. |
| | NUC experienced CHB patients continued their daily NUC throughout the study and NUC naive CHB patients started daily NUC on day 1. |
| Reference Formulation | Placebo (PBO): normal saline (0.9%) administered subcutaneously with matching volume. |
| Efficacy Evaluation Criteria | Virology assessments will be performed (CHB patients only) and included: |
| | • Quantitative assessments: HBV DNA, HBV RNA, HBsAg, HBeAg, HBcrAg, |
| Safety Evaluation Criteria | Safety was assessed by vital signs, clinical laboratory measurements, resting ECG measurements, adverse events, serious adverse events, injection site reactions and 90-day post-EOS pregnancy follow up. |
| Pharmacokinetics Evaluation | Blood samples will be collected from each subject for pharmacokinetic analysis after dose 1 (Cohorts 1-5, NHV only) |
| Data Analysis | Screening, Compliance, Tolerability and Safety Data: |
| | Safety analyses will be performed and the results summarized by cohort. The incidence and frequency of adverse events (AEs), serious adverse events (SAEs), related AEs, related SAEs, and AEs leading to discontinuation, will be summarized by cohort per SOC, PT, and severity. Other safety parameters will be summarized at each scheduled time. |
| | Virology assessments (CHB patients only): |
| | • Quantitative HBsAg (qHBsAg): Log change from baseline to nadir and duration of response from nadir back to approximately 20% of baseline or EOS (Roche Elecsys, LLOQ 0.05 IU/mL) |
| | • Quantitative HBV DNA (when quantifiable at baseline): Log change from baseline to EOS ((Roche Cobas, LLOQ 20 IU/mL) |
| | • Quantitative HBV RNA: Log change from baseline to nadir and duration of response from baseline to EOS (Abbott m2000, LLOQ 1.65 Log U/mL, Butler 2018) |
| | • Quantitative HBerAg: Log change from baseline to nadir and duration of response from baseline to EOS (Fujirebo Lumipulse, LLOQ 1 kU/mL) |
| | • Quantitative HBeAg: Log change from baseline to nadir and duration of response from baseline to EOS (Diasorin Liaison, LLOQ 0.01 PEIU/mL) |
| | Pharmacokinetics (NHV subjects only): |
| | Plasma concentrations of HBV RNAi agents (AD04872 and AD05070) product constituents will be used to calculate the following PK parameters: maximum observed plasma concentration (Cmax), area under the plasma concentration time curve (AUC) from time 0 to 24 hours (AUC0-24), AUC from time 0 extrapolated to infinity (AUCinf), and terminal elimination half-life (t½). |
| | Pharmacokinetic parameters will be determined using non-compartmental methods. |

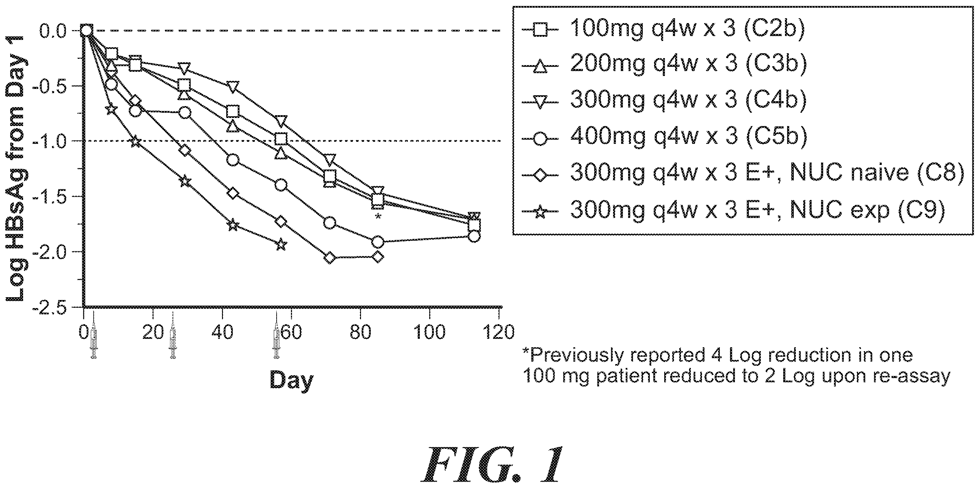

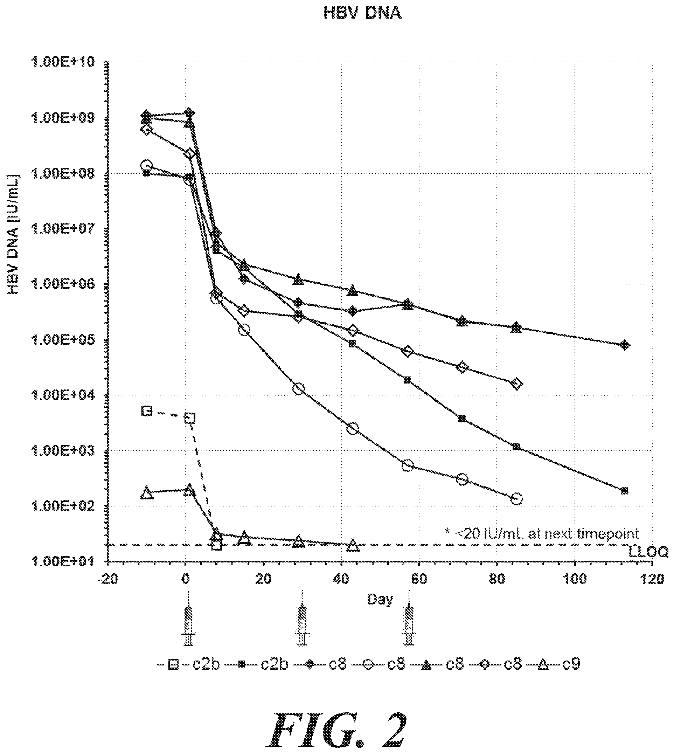

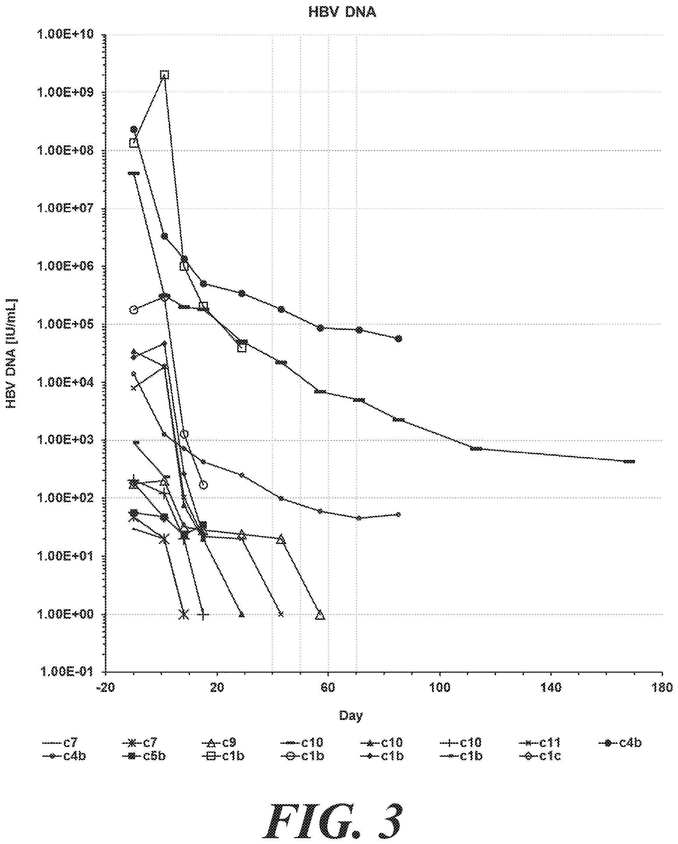

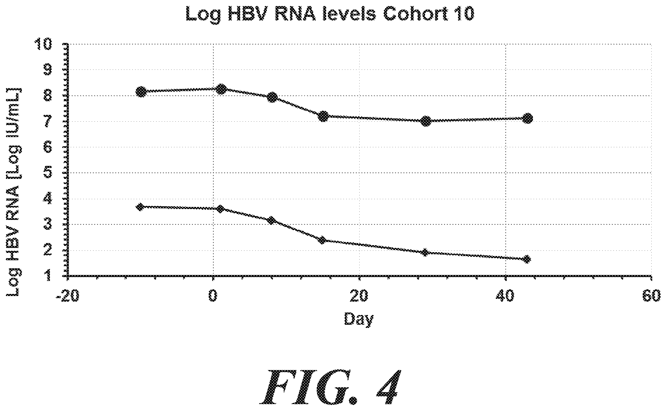

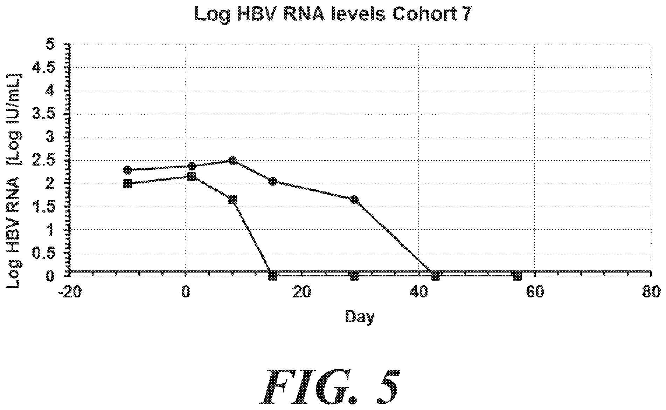

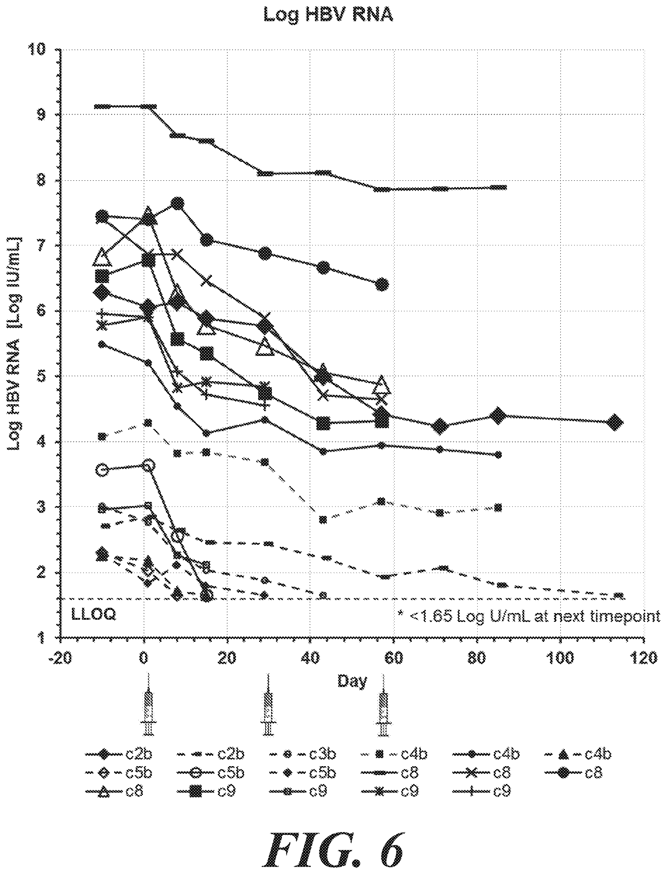

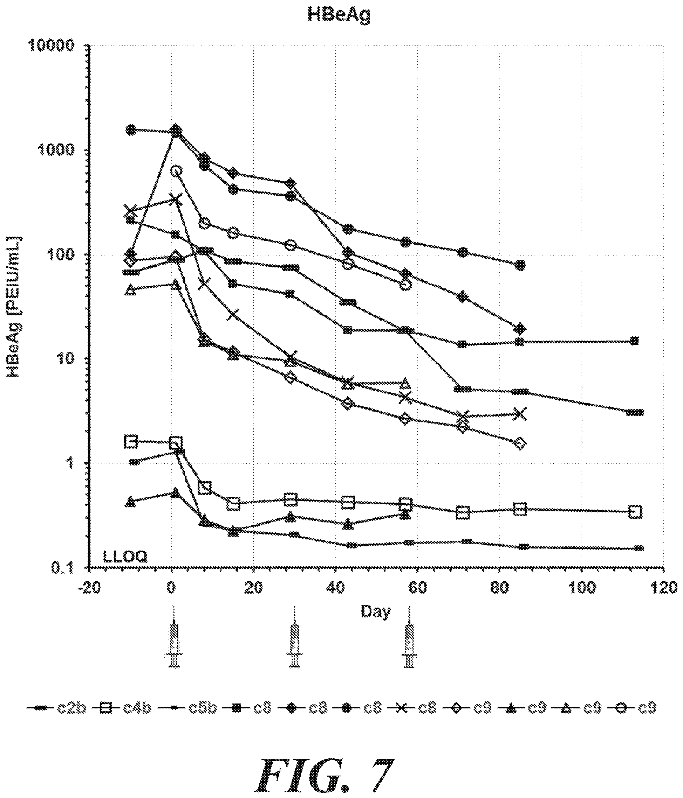

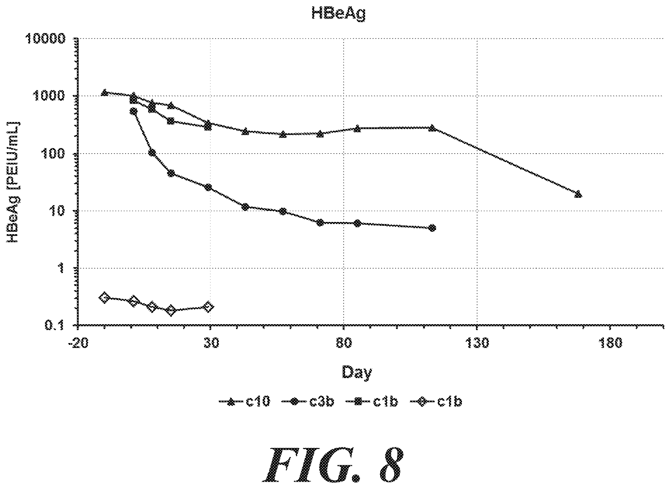

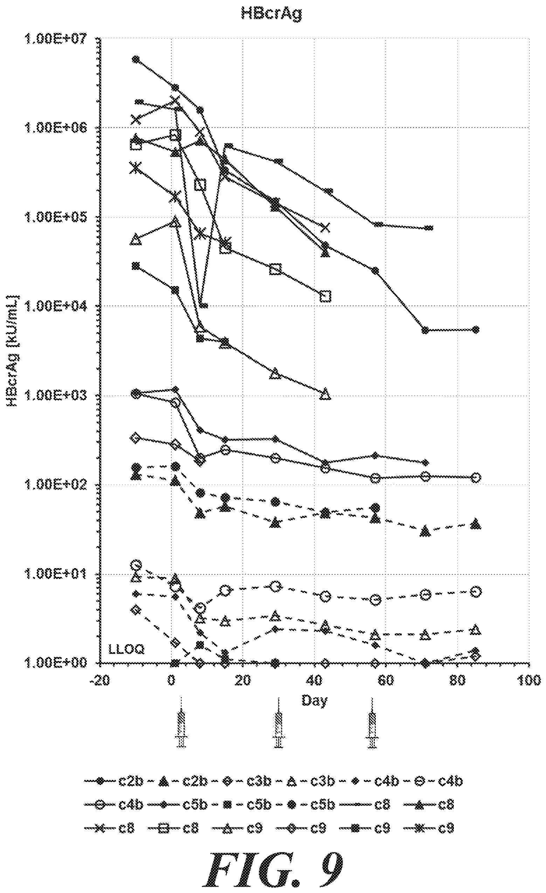

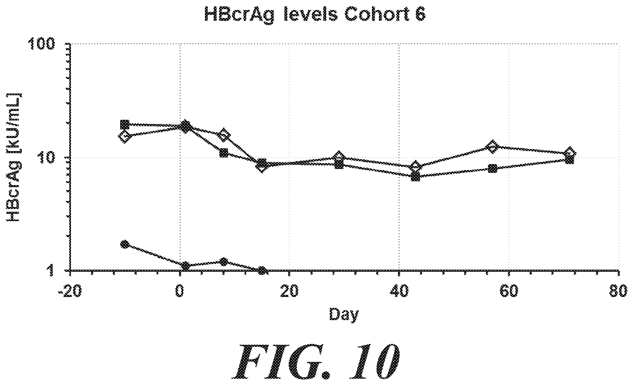

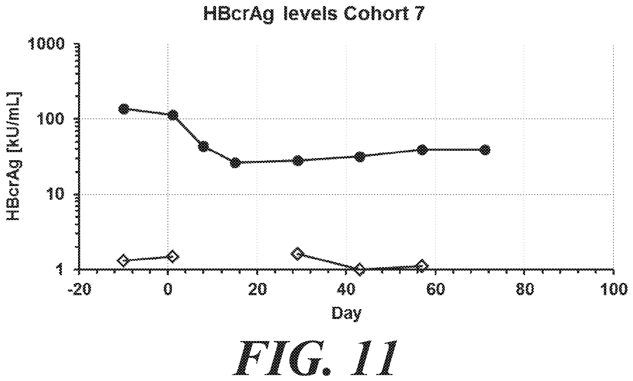

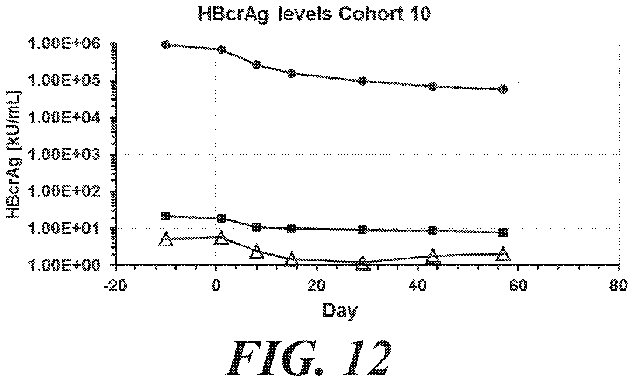

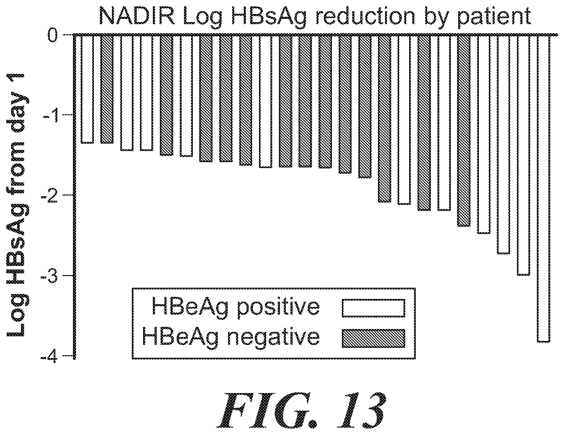

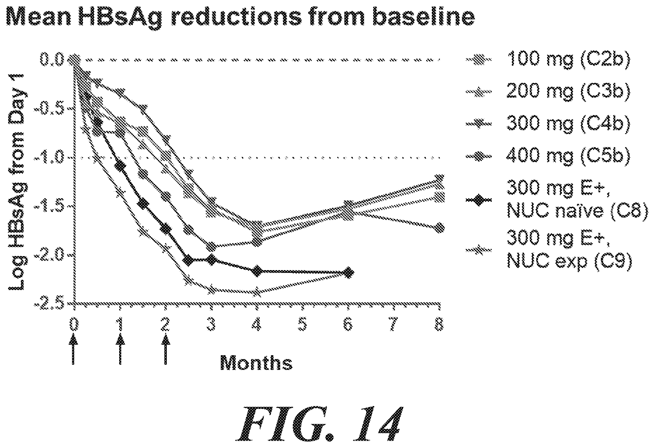

FIGS. 1 and 14 show the mean log change for antigen HBsAg from day 1 for CHB patients (cohorts 2b, 3b, 4b, 5b, 8 and 9). Table 51 below shows the NADIR HBsAg responses for CHB patients with more than 6 weeks of HBsAg data collected. Strong HBsAg responses were observed in all HBV patients with monthly doses and were similar in HBeAg positive and HBeAg negative patients and in NUC naive and NUC experienced patients. Range of HBsAg reductions was from -1.3 to -3.8 Log₁₀. FIGS. 2-12 show the individual changes in HBV DNA, HBV RNA, HBeAg and HBcrAg in CHB patients (cohorts 1b, 1c, 2b, 3b, 4b, 5b, 6, 7, 8, 9, 10 and 11). All these virologic parameters (HBV DNA, HBV RNA, HBeAg and HBcrAg) showed responses to HBV RNAi agents (AD04872 and AD05070). FIG. 13 shows the NADIR Log HBsAg reduction data by CHB patients. Similar responses were observed for patients HBeAg positive and HBeAg negative patients.

**Table 51. NADIR HBsAg responses for CHB patients**

| **NADIR HBsAg responses** | **Percentage of CHB patients** |
|---|---|
| > 1 log (90%) reduction | 100% |
| > 1.5 log (97%) reduction | 83% |
| > 2 log (99%) reduction | 38% |
| > 3 log (99.9%) reduction | 3% |

Table 52 and Table 53 show the safety and tolerability results from the study in healthy patients and CHB patients, respectively. HBV RNAi agents (AD04872 and AD05070) administered subcutaneously appear to be well tolerated at single or multiple monthly doses up to 400 mg. Mild injection site reactions were observed with approximately 12% of subcutaneous injections.

**Table 52. Safety and Tolerability in Healthy Patients**

| AEs in Healthy Volunteers | Cohort 1 35 mg | Cohort 2 100 mg | Cohort 3 200 mg | Cohort 4 300 mg | Cohort 5 400 mg | All active | All PBO | Total AEs |
|---|---|---|---|---|---|---|---|---|
| AE Reported Terms | HBV RNAi agents N=4 | HBV RNAi agents N=4 | HBV RNAi agents N=4 | HBV RNAi agents N=4 | HBV RNAi agents N=4 | HBV RNAi agents N=4 | Placebo N=10 | |
| Hot flush, Feeling hot, Subjective Pyrexia | 1 | | | 1 | | 2 | 1 | 3 |
| Headache | 1 | 2 | 1 | 1 | 2 | 7 | 2 | 9 |
| Abdominal pain | 1 | 2 | 1 | | | 4 | 1 | 5 |
| Upper respiratory tract infection | 1 | 1 | | | 2 | 4 | 2 | 6 |
| Lethargy, Fatigue | | 1 | 2 | | | 3 | 2 | 5 |
| Myalgia | | 1 | | | | 1 | 1 | 2 |
| Sore Throat | | 1 | 1 | | | 2 | 2 | 4 |
| Sensation of feeling dehydrated | | 1 | | | | 1 | 1 | 2 |
| Discomfort / bruising at cannula site | | 1 | | 2 | 1 | 4 | 1 | 5 |
| Nausea | | 1 | 1 | 2 | | 4 | 1 | 5 |
| Dizziness, Lightheadedne ss, Vertigo | | 1 | | | | 1 | 2 | 3 |
| Flu like illness, Non Specific Viral Illness | | 1 | 1 | | | 2 | | 2 |
| Emesis | | | 1 | 1 | | 2 | 1 | 3 |
| Bruising / tenderness at injection site | | | | 1 | 1 | 2 | | 2 |
| Total AEs in >1 NHV | 4 | 13 | 8 | 8 | 6 | 39 | 17 | 56 |

**Table 53. Safety and Tolerability in CHB Patients**

| AEs in HBV Patients | Cohort 2b 100 mg | Cohort 3b 200 mg | Cohort 4b 300 mg | Cohort 5b 400 mg | Cohort 8 300 mg | Cohort 9 300 mg | Total AEs N=24 |
|---|---|---|---|---|---|---|---|
| AE Reported Terms | HBV RNAi agents N=4 | HBV RNAi agents N=4 | HBV RNAi agents N=4 | HBV RNAi agents N=4 | HBV RNAi agents N=4 | HBV RNAi agents N=4 | |
| Insect bites | 1 | | 1 | | | | 2 |
| Upper respiratory infection, sore throat | 1 | | 1 | | 1 | | 3 |
| Erythema, redness, hematoma, rash at injection site | | | 1 | 2 | 2 | 2 | 7 |
| Acne | | | | | 2 | | 2 |
| Headache | | | 2 | | | | 2 |
| Raised creatine kinase | | | 1 | | 1 | | 2 |
| Diarrhea | | | 1 | 1 | | | 2 |
| Lower back ache/pain | | | 1 | | 1 | | 2 |
| Total AEs in >1 CHB | 2 | 0 | 8 | 3 | 7 | 2 | 22 |

### Example 20. Clinical Study of HBV RNAi agents

A Phase 1/2a, randomized, double-blind, multiple dose escalating study in healthy volunteers (NHV) and open label, multi-dose escalating study in patients with chronic HBV infection (CHB) were conducted. The study was designed to evaluate the safety, tolerability and pharmacokinetic effects of HBV RNAi agents (AD04872 and AD05070) in healthy patients and patients chronically infected with hepatitis B virus (CHB). The study subject population included healthy adult males and females 18-55 years old and adult males and females 18-65 years old who had a diagnosis of HBeAg positive or HBeAg negative chronic (> 6 months) HBV infection.

NHV subjects were divided into five cohorts of 6 subjects per cohort (cohorts 1-5). NHV cohorts (4 active, 2 placebo) received single doses of a combination of AD04872 and AD05070 in an amount of 35, 100, 200, 300, or 400 mg or normal saline in a blinded fashion via subcutaneous injections. CHB cohorts 1, 1c and 2b-5b were HBeAg positive or negative, nucleoside inhibitors (NUC) naive or NUC experienced at baseline, and received three doses of a combination of AD04872 and AD05070 in an amount of 35, 50, 100, 200, 300, or 400 mg via subcutaneous injections in 28-day intervals. Cohort 6 enrolled 4 CHB patients to receive three 100 mg doses of a combination of AD04872 and AD05070 every two weeks. Cohort 7 enrolled 4 CHB patients to receive three weekly 100 mg doses of a combination of AD04872 and AD05070. CHB cohorts 8 and 9 (4 active) were HBeAg positive, treatment naive or NUC experienced, respectively, that received three doses of a combination of AD04872 and AD05070 in an amount of 300 mg via subcutaneous injections in 28-day intervals. Cohort 10 and Cohort 11 each enrolled 4 CHB patients to receive 3 weekly 200 mg and 300 mg doses of a combination of AD04872 and AD05070 respectively. NUC experienced CHB patients continued their daily NUC throughout the study and NUC naive CHB patients started daily NUC on day 1. For CHB, viral DNA, viral RNA and antigens HBsAg, HBeAg, and HBcrAg were measured periodically. The study parameters are summarized in Table 54.

**Table 54**

| | |
|---|---|
| Study Title | A Phase 1/2a Single Dose-Escalating Study to Evaluate the Safety, Tolerability and Pharmacokinetic Effects of HBV RNAi agents (AD04872 and AD05070) in Normal Adult Volunteers and Multiple Escalating Doses Evaluating Safety, Tolerability and Pharmacodynamic Effects in HBV Patients |
| Development Phase | Phase 1 First-in-Human, Phase 2a First-in-Patient |
| Study Objectives | Primary Objectives: |
| | • To determine the incidence and frequency of adverse events possibly or probably related to treatment as a measure of the safety and tolerability of HBV RNAi agents (AD04872 and AD05070) using escalating single doses in healthy volunteers (NHV) and escalating multiple doses in patients chronically infected with hepatitis B virus (CHB). |
| | Secondary Objectives: |
| | • To evaluate the single-dose pharmacokinetics of HBV RNAi agents (AD04872 and AD05070) in NHVs |
| | • To determine the reduction from Day 1 pre-dose baseline to post-dose nadir of HBsAg in response to HBV RNAi agents (AD04872 and AD05070) in patients chronically infected with HBV (CHB) as a measure of activity. |
| | Exploratory Objective: |
| | • To determine the reduction from Day 1 pre-dose baseline to post-dose nadir of HBcrAg, HBV RNA (if scientifically feasible) and HBeAg (e+ patients only), in response to HBV RNAi agents (AD04872 and AD05070) in CHB patients as a measure of activity. |
| Study Design | Randomized, double-blind, parallel-group, placebo-controlled |
| Study Population | This study was conducted in NHVs, adult males and females, aged 18-55 years with BMI between 19.0 and 35.0 kg/m² (Cohorts 1 - 5), and in CHB patients, aged 18-65 years with BMI between 19.0 and 38.0 kg/m² (Cohorts 2b, 3b, 4b, 5b, 8 and 9) and had a diagnosis of HBeAg positive or HBeAg negative chronic (> 6 months) HBV infection. |
| | • Cohorts 1-5: NHVs, adult males and females, aged 18-55 years |
| | • Cohorts 1b, 1c, 2b, 3b, 4b and 5b: Any CHB patient regardless of HBeAg or prior therapy status. |
| | • Cohort 6: Any CHB patient regardless of HBeAg or prior therapy status. |
| | • Cohort 7: Any CHB patient regardless of HBeAg or prior therapy status. |
| | • Cohort 8: HBeAg positive treatment naïve patients (no prior chronic exposure to NUCs or Interferon). |
| | • Cohort 9: HBeAg positive NUC experienced patients (on entecavir or tenofovir for at least 12 months). |
| | • Cohort 10: Any CHB patient regardless of HBeAg or prior therapy status. |
| | • Cohort 11: Any CHB patient regardless of HBeAg or prior therapy status. |
| Investigational Product | HBV RNAi agents (AD04872 and AD05070) |
| Dosage and Frequency | Cohorts 1-5 (NHV): randomized to receive HBV RNAi agents (AD04872 and AD05070) or placebo (4 active: 2 placebo) at single escalating doses of 35, 100, 200, 300 and 400 mg administered as a single subcutaneous injection. |
| | Cohorts 1b, 1c, 2b-5b (CHB): received three doses of 25, 50, 100, 200, 300, or 400 mg HBV RNAi agents (AD04872 and AD05070) via subcutaneous injections in 28-day intervals. |
| | Cohort 6 enrolled 4 HBV patients to receive three 100 mg doses of HBV RNAi agents (AD04872 and AD05070) every two weeks. Cohort 7 enrolled 4 HBV patients to receive three weekly 100 mg doses of HBV RNAi agents (AD04872 and AD05070). |
| | Cohorts 8 and 9 (CHB): received three SQ doses of 300 mg HBV RNAi agents (AD04872 and AD05070) via subcutaneous injections in 28-day intervals. |
| | Cohort 10 and Cohort 11 each enrolled 4 HBV patients to receive 3 weekly 200 mg and 300 mg doses of HBV RNAi agents (AD04872 and AD05070) respectively. |
| | NUC experienced CHB patients continued their daily NUC throughout the study and NUC naive CHB patients started daily NUC on day 1. |
| Reference Formulation | Placebo (PBO): normal saline (0.9%) administered subcutaneously with matching volume. |
| Efficacy Evaluation Criteria | Virology assessments will be performed (CHB patients only) and included: |
| | • Quantitative assessments: HBV DNA, HBV RNA, HBsAg, HBeAg, HBcrAg, |
| Safety Evaluation Criteria | Safety was assessed by vital signs, clinical laboratory measurements, resting ECG measurements, adverse events, serious adverse events, injection site reactions and 90-day post-EOS pregnancy follow up. |
| Pharmacokinetics Evaluation | Blood samples will be collected from each subject for pharmacokinetic analysis after dose 1 (Cohorts 1-5, NHV only) |
| Data Analysis | Screening, Compliance, Tolerability and Safety Data: |
| | Safety analyses will be performed and the results summarized by cohort. The incidence and frequency of adverse events (AEs), serious adverse events (SAEs), related AEs, related SAEs, and AEs leading to discontinuation, will be summarized by cohort per SOC, PT, and severity. Other safety parameters will be summarized at each scheduled time. |
| | Virology assessments (CHB patients only): |
| | • Quantitative HBsAg (qHBsAg): Log change from baseline to nadir and duration of response from nadir back to approximately 20% of baseline or EOS (Roche Elecsys, LLOQ 0.05 IU/mL) |
| | • Quantitative HBV DNA (when quantifiable at baseline): Log change from baseline to EOS ((Roche Cobas, LLOQ 20 IU/mL) |
| | • Quantitative HBV RNA: Log change from baseline to nadir and duration of response from baseline to EOS (Abbott m2000, LLOQ 1.65 Log U/mL, Butler 2018) |
| | • Quantitative HBerAg: Log change from baseline to nadir and duration of response from baseline to EOS (Fujirebo Lumipulse, LLOQ 1 kU/mL) |
| | • Quantitative HBeAg: Log change from baseline to nadir and duration of response from baseline to EOS (Diasorin Liaison, LLOQ 0.01 PEIU/mL) |
| | Pharmacokinetics (NHV subjects only): |
| | Plasma concentrations of HBV RNAi agents (AD04872 and AD05070) product constituents will be used to calculate the following PK parameters: maximum observed plasma concentration (Cmax), area under the plasma concentration time curve (AUC) from time 0 to 24 hours (AUC0-24), AUC from time 0 extrapolated to infinity (AUCinf), and terminal elimination half-life (t½). |
| | Pharmacokinetic parameters will be determined using non-compartmental methods. |

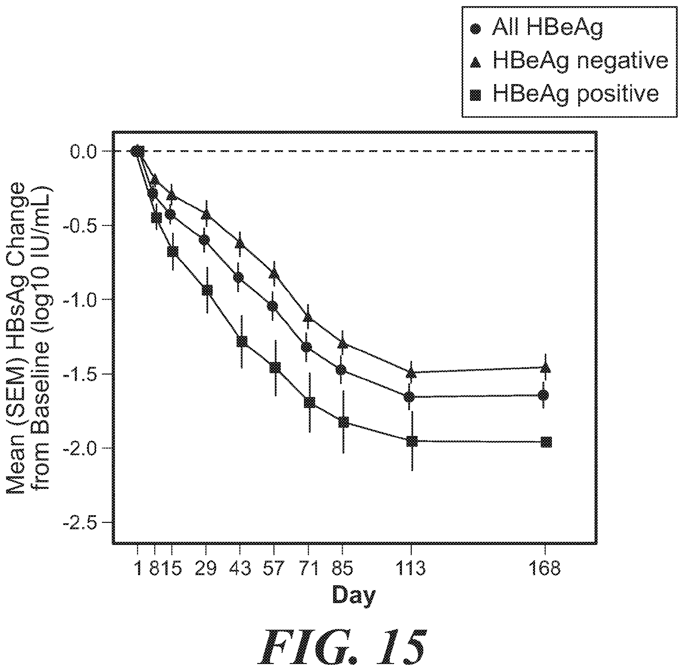

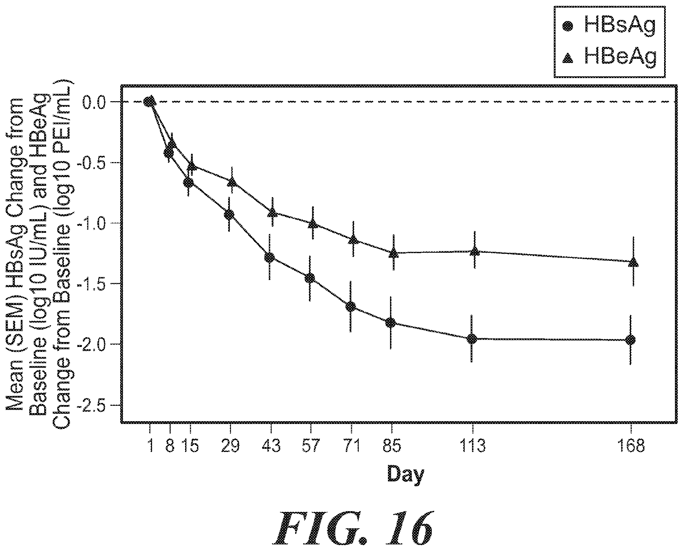

**FIG. 15** shows the mean change for antigen HBsAg from day 1 for CHB patients receiving three doses of HBV RNAi agents (AD04872 and AD05070) via subcutaneous injections in 28-day intervals (cohorts 1b, 1c, 2b, 3b, 4b, 5b, 8 and 9). The mean HBsAg decline was more pronounced in HBeAg positive than in HBeAg negative participants. In general, HBsAg declines were sustained over time at least until Day 168 across all doses. **FIG. 16** shows the mean changes for antigen HBsAg and HBeAg from day 1 for HBsAg-positive CHB patients receiving three doses of HBV RNAi agents (AD04872 and AD05070) via subcutaneous injections in 28-day intervals (cohorts 1b, 1c, 2b, 3b, 4b, 5b, 8 and 9). The RNAi agents showed target activity on both HBeAg and HBsAg with a more pronounced reduction in HBsAg.

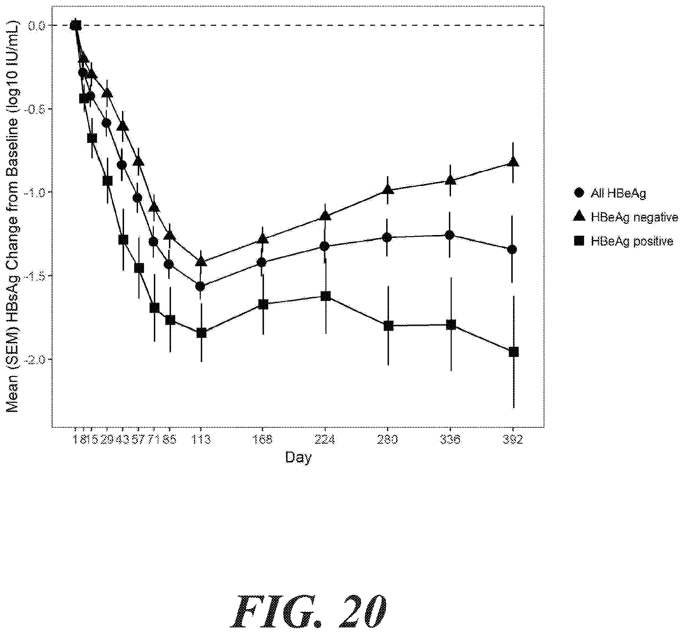

**FIG.** 20 shows the mean change for antigen HBsAg from day 1 for CHB patients receiving three doses of HBV RNAi agents (AD04872 and AD05070) via subcutaneous injections in 28-day intervals (cohorts 1b, 1c, 2b, 3b, 4b, 5b, 8 and 9). The mean HBsAg decline was more pronounced in HBeAg positive than in HBeAg negative participants. In general, HBsAg declines were sustained over time at least until day 392 across all doses.

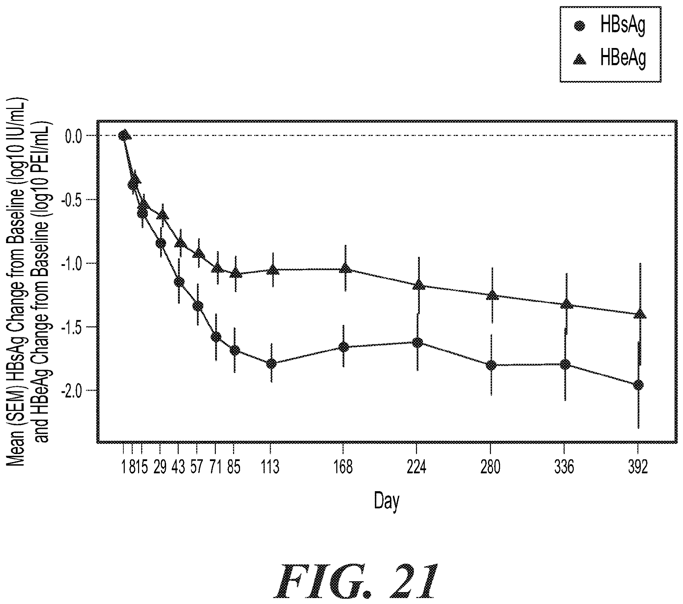

**FIG. 21** shows the mean changes for antigen HBsAg and HBeAg from day 1 for HBsAg-positive CHB patients receiving three doses of HBV RNAi agents (AD04872 and AD05070) via subcutaneous injections in 28-day intervals (cohorts 1b, 1c, 2b, 3b, 4b, 5b, 8 and 9). The RNAi agents showed target activity on both HBeAg and HBsAg with a more pronounced reduction in HBsAg and with sustained mean HBeAg and HBsAg responses.

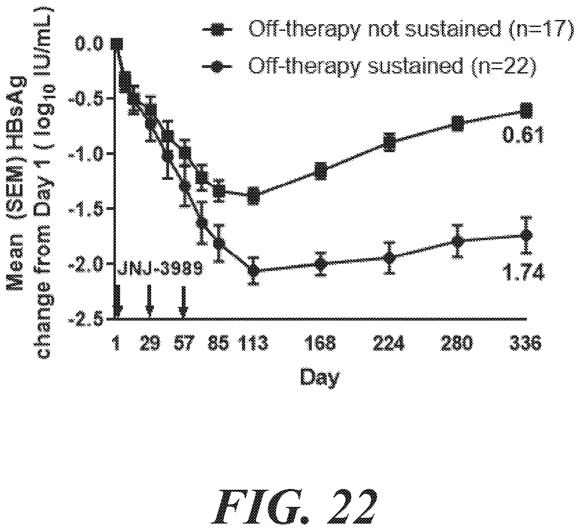

For all parameters, sustained suppression was defined as a ≥1.0 log₁₀ reduction from day 1 or a value <lower limit of quantification at day 336. Safety and viral parameters (HBsAg, HBeAg, HBV DNA, HBV RNA, HBcrAg) were assessed in 8 CHB pts/cohort (NA experienced or naive; HBeAg +ve or -ve) who received 3 subcutaneous JNJ-3989 doses (days 1, 27, 57) of 100, 200, 300 (n=16) or 400mg. Patients started/continued with an NA on day 1 and continued throughout the study. For patients with quantifiable parameters on day 1 and available data at day 336, a sustained suppression was observed for HBV RNA in 15/26 patients, HBeAg in 9/14 patients and HBcrAg in 10/24 patients. JNJ-3989 (100-400mg) with an NA was well tolerated in CHB patients. A ≥1.0 log₁₀ reduction in HBsAg at nadir was achieved in 98% of patients. A subset of patients had sustained suppression of HBsAg ~9 months after the last RNAi dose (mean 1.74); cf. FIG. 22. Sustained suppression of other viral parameters was also seen.

### Example 21. Clinical Study of HBV RNAi agents

A Phase 1/2a, randomized, double-blind, multiple dose escalating study in healthy volunteers (NHV) and open label, multi-dose escalating study in patients with chronic HBV infection (CHB) were conducted. The study was designed to evaluate the safety, tolerability and pharmacokinetic effects of HBV RNAi agents (AD04872 and AD05070) in NHV and patients chronically infected with hepatitis B virus (CHB). The study subject population included healthy adult males and females 18-55 years old and adult males and females 18-65 years old who had a diagnosis of HBeAg positive or HBeAg negative chronic (HBsAg positive for > 6 months) HBV infection.

NHV subjects were divided into five cohorts of 6 subjects per cohort (cohorts 1-5). NHV cohorts (4 active, 2 placebo) received single doses of a combination of AD04872 and AD05070 in an amount of 35, 100, 200, 300, or 400 mg or normal saline in a blinded fashion via subcutaneous injections. CHB cohorts 1b, 1c and 2b-5b were HBeAg positive or negative, nucleoside inhibitors (NUC) naive or NUC experienced at baseline, and received three doses of a combination of AD04872 and AD05070 in an amount of 25, 50, 100, 200, 300, or 400 mg via subcutaneous injections in 28-day intervals. Cohort 6 enrolled 4 CHB patients to receive three 100 mg doses of a combination of AD04872 and AD05070 every two weeks. Cohort 7 enrolled 4 CHB patients to receive three weekly 100 mg doses of a combination of AD04872 and AD05070. CHB cohorts 8 and 9 (4 active) were HBeAg positive, NUC treatment naive or NUC experienced, respectively, that received three doses of a combination of AD04872 and AD05070 in an amount of 300 mg via subcutaneous injections in 28-day intervals. Cohort 10 and Cohort 11 each enrolled 4 CHB patients to receive 3 weekly 200 mg and 300 mg doses of a combination of AD04872 and AD05070 respectively. NUC experienced CHB patients continued their daily NUC throughout the study and NUC naive CHB patients started daily NUC on day 1. For CHB cohort, viral DNA, viral RNA and antigens HBsAg, HBeAg, and HBcrAg were measured periodically.

HBV RNAi agents (AD04872 and AD05070) was designed to silence HBV RNA transcripts from episomal cccDNA and host-integrated HBV DNA. HBV RNAi agents reduces serum HBsAg levels by ≥ 1 log10 in all patients receiving three HBV RNAi agents doses (100-400 mg, one every 4 weeks (Q4w) with an NA), regardless of baseline HBeAg status or prior NA therapy, as shown in Example 19. Given the lack of a dose response with 100-400 mg Q4w HBV RNAi agents, in this Example, cohorts 2b-5b were expanded from four to eight patients, and two lower dose cohorts 1b and 1c (25 and 50 mg Q4w, respectively) were added. Efficacy data and safety data up to Day 113 are presented (*i.e.,* two months post HBV RNAi agents dosing) for cohorts 2b-5b and 1b and 1c, as discussed below.

In cohorts 1b, 1c and 2b-5b, HBeAg positive or negative, NA-experienced or - naive CHB patients were enrolled and received three subcutaneous HBV RNAi agents doses of 25, 50, 100, 200, 300 or 400 mg Q4w on Days 1, 27 and 57. All patients either started (NA-naïve) or continued (NA-experienced) with daily NA (TDF or ETV) treatment on Day 1 and continued beyond the end of HBV RNAi agents dosing. Study visits were at screening and on Days 1, 8, 15, 29, 43, 57, 85 and 113, then extended follow up approximately every 2 months for 12 months. Serum viral parameters were assessed, *i.e.,* HBV DNA (lower limit of quantification [LLOQ]: 20 IU/mL), HBV RNA (LLOQ: 1.65 log₁₀ U/mL⁴), HBsAg (LLOQ: 0.05 IU/mL), HBeAg (LLOQ: 0.01 PEIU/mL; value below 0.11 PEIU/mL are reported as not detected), and HBcrAg (LLOQ: 1 kU/mL). Safety assessments included clinical laboratory assessments and adverse events (AEs) assessed from screening through Day 113, as reported here, and through the extended follow-up period. The study parameters are summarized in Table 55.

**Table 55**

| | |
|---|---|
| Study Title | A Phase 1/2a Single Dose-Escalating Study to Evaluate the Safety, Tolerability and Pharmacokinetic Effects of HBV RNAi agents (AD04872 and AD05070) in Normal Adult Volunteers and Multiple Escalating Doses Evaluating Safety, Tolerability and Pharmacodynamic Effects in HBV Patients |
| Development Phase | Phase 1 First-in-Human, Phase 2a First-in-Patient |
| Study Objectives | Primary Objectives: |
| | • To determine the incidence and frequency of adverse events possibly or probably related to treatment as a measure of the safety and tolerability of HBV RNAi agents (AD04872 and AD05070) using escalating single doses in healthy volunteers (NHV) and escalating multiple doses in patients chronically infected with hepatitis B virus (CHB). |
| | Secondary Objectives: |
| | • To evaluate the single-dose pharmacokinetics of HBV RNAi agents (AD04872 and AD05070) in NHVs |
| | • To determine the reduction from Day 1 pre-dose baseline to post-dose nadir of HBsAg in response to HBV RNAi agents (AD04872 and AD05070) in patients chronically infected with HBV (CHB) as a measure of activity. |
| | Exploratory Objective: |
| | • To determine the reduction from Day 1 pre-dose baseline to post-dose nadir of HBcrAg, HBV RNA (if scientifically feasible) and HBeAg (e+ patients only), in response to HBV RNAi agents (AD04872 and AD05070) in CHB patients as a measure of activity. |
| Study Design | Randomized, double-blind, parallel-group, placebo-controlled |
| Study Population | This study was conducted in NHVs, adult males and females, aged 18-55 years with BMI between 19.0 and 35.0 kg/m² (Cohorts 1 - 5), and in CHB patients, aged 18-65 years with BMI between 19.0 and 38.0 kg/m² (Cohorts 2b, 3b, 4b, 5b, 8 and 9) and had a diagnosis of HBeAg positive or HBeAg negative chronic (> 6 months) HBV infection. |
| | • Cohorts 1-5: NHVs, adult males and females, aged 18-55 years |
| | • Cohorts 1b, 1c, 2b, 3b, 4b and 5b: Any CHB patient regardless of HBeAg or prior therapy status. |
| | • Cohort 6: Any CHB patient regardless of HBeAg or prior therapy status. |
| | • Cohort 7: Any CHB patient regardless of HBeAg or prior therapy status. |
| | • Cohort 8: HBeAg positive treatment naïve patients (no prior chronic exposure to NUCs or Interferon). |
| | • Cohort 9: HBeAg positive NUC experienced patients (on entecavir or tenofovir for at least 12 months). |
| | • Cohort 10: Any CHB patient regardless of HBeAg or prior therapy status. |
| | • Cohort 11: Any CHB patient regardless of HBeAg or prior therapy status. |
| Investigational Product | HBV RNAi agents (AD04872 and AD05070) |
| Dosage and Frequency | Cohorts 1-5 (NHV): randomized to receive HBV RNAi agents (AD04872 and AD05070) or placebo (4 active: 2 placebo) at single escalating doses of 35, 100, 200, 300 and 400 mg administered as a single subcutaneous injection. |
| | Cohorts 1b, 1c, 2b-5b (CHB) (8 patients each cohort): received three doses of 25, 50, 100, 200, 300, or 400 mg HBV RNAi agents (AD04872 and AD05070) via subcutaneous injections in 28-day intervals. |
| | Cohort 6 enrolled 4 HBV patients to receive three 100 mg doses of HBV RNAi agents (AD04872 and AD05070) every two weeks. Cohort 7 enrolled 4 HBV patients to receive three weekly 100 mg doses of HBV RNAi agents (AD04872 and AD05070). |
| | Cohorts 8 and 9 (CHB): received three SQ doses of 300 mg HBV RNAi agents (AD04872 and AD05070) via subcutaneous injections in 28-day intervals. |
| | Cohort 10 and Cohort 11 each enrolled 4 HBV patients to receive 3 weekly 200 mg and 300 mg doses of HBV RNAi agents (AD04872 and AD05070) respectively. |
| | NUC experienced CHB patients continued their daily NUC throughout the study and NUC naive CHB patients started daily NUC on day 1. |
| Reference Formulation | Placebo (PBO): normal saline (0.9%) administered subcutaneously with matching volume. |
| Efficacy Evaluation Criteria | Virology assessments will be performed (CHB patients only) and included: |
| | • Quantitative assessments: HBV DNA, HBV RNA, HBsAg, HBeAg, HBcrAg, |
| Safety Evaluation Criteria | Safety was assessed by vital signs, clinical laboratory measurements, resting ECG measurements, adverse events, serious adverse events, injection site reactions and 90-day post-EOS pregnancy follow up. |
| Pharmacokinetics Evaluation | Blood samples will be collected from each subject for pharmacokinetic analysis after dose 1 (Cohorts 1-5, NHV only) |
| Data Analysis | Screening, Compliance, Tolerability and Safety Data: |
| | Safety analyses will be performed and the results summarized by cohort. The incidence and frequency of adverse events (AEs), serious adverse events (SAEs), related AEs, related SAEs, and AEs leading to discontinuation, will be summarized by cohort per SOC, PT, and severity. Other safety parameters will be summarized at each scheduled time. |
| | Virology assessments (CHB patients only): |
| | • Quantitative HBsAg (qHBsAg): Log change from baseline to nadir and duration of response from nadir back to approximately 20% of baseline or EOS (Roche Elecsys, LLOQ 0.05 IU/mL) |
| | • Quantitative HBV DNA (when quantifiable at baseline): Log change from baseline to EOS ((Roche Cobas, LLOQ 20 IU/mL) |
| | • Quantitative HBV RNA: Log change from baseline to nadir and duration of response from baseline to EOS (Abbott m2000, LLOQ 1.65 Log U/mL, Butler 2018) |
| | • Quantitative HBcrAg: Log change from baseline to nadir and duration of response from baseline to EOS (Fujirebo Lumipulse, LLOQ 1 kU/mL) |
| | • Quantitative HBeAg: Log change from baseline to nadir and duration of response from baseline to EOS (Diasorin Liaison, LLOQ 0.01 PEIU/mL) |
| | Pharmacokinetics (NHV subjects only): |
| | Plasma concentrations of HBV RNAi agents (AD04872 and AD05070) product constituents will be used to calculate the following PK parameters: maximum observed plasma concentration (Cmax), area under the plasma concentration time curve (AUC) from time 0 to 24 hours (AUC0-24), AUC from time 0 extrapolated to infinity (AUCinf), and terminal elimination half-life (t½). |
| | Pharmacokinetic parameters will be determined using non-compartmental methods. |

Baseline characteristics and demographics information for cohorts 1b, 1c, 2b, 3b, 4b and 5b is shown in Table 56. Most patients were NA-experienced (40/48, 83%). All patients received their planned HBV RNAi agents doses with no treatment discontinuations.

**Table 56**

| Cohort | | 1b | 1c | 2b | 3b | 4b | 5b | All patients |
|---|---|---|---|---|---|---|---|---|
| | | 25 mg | 50 mg | 100 mg | 200 mg | 300 mg | 400 mg | N=48 |
| | | N=8 | N=8 | N=8 | N=8 | N=8 | N=8 | |
| Age, years; mean (range) | | 43 (31-52) | 48 (36-58) | 51 (32-66) | 48 (41-57) | 52 (40-63) | 42 (29-61) | 47 (29-66) |
| Male, n (%) | | 5 (63) | 6 (75) | 6 (75) | 5 (63) | 8 (100) | 6 (75) | 36 (75) |
| Race, n (%) | | | | | | | | |
| | Asian | 6 (75) | 5 (63) | 8 (100) | 8 (100) | 5 (63) | 6 (75) | 38 (79) |
| | White | 0 | 0 | 0 | 0 | 1 (13) | 0 | 1 (2) |
| | Native Hawaiian/other | | | | | | | |
| | Pacific Islander | 1 (13) | 1 (13) | 0 | 0 | 2 (25) | 0 | 4 (8) |
| | Black/AA | 1 (13) | 0 | 0 | 0 | 0 | 0 | 1 (2) |
| | Other | 0 | 2 (25) | 0 | 0 | 0 | 2 (25) | 4 (8) |
| BMI, kg/m²; mean (range) | | 27 (20-44) | 26 (18-40) | 24 (22-29) | 25 (19-32) | 27 (21-36) | 25 (21-36) | 26 (18-44) |
| HBeAg positive/negative, n | | 2/6 | 3/5 | 1/7 | 1/7 | 3/5 | 1/7 | 11/37 |
| NA experienced, n (%) | | 4 (50) | 7 (88) | 6 (75) | 8 (100) | 8 (100) | 7 (88) | 40 (83) |
| Mean (SEM) HBsAg on Day 1 (IU/mL) | | 6477 (2876) | 4595 (1986) | 3937 (2142) | 3212 (2453) | 9381 (8275) | 4032 (1652) | 5272 (1558) |

| | | | | | | | | |
|---|---|---|---|---|---|---|---|---|
| AA: African American; BMI: body mass index; HBeAg: hepatitis B e-antigen; HBsAg: hepatitis B surface antigen; NA: nucleos(t)ide analogue; SEM: standard error of the mean. | | | | | | | | |

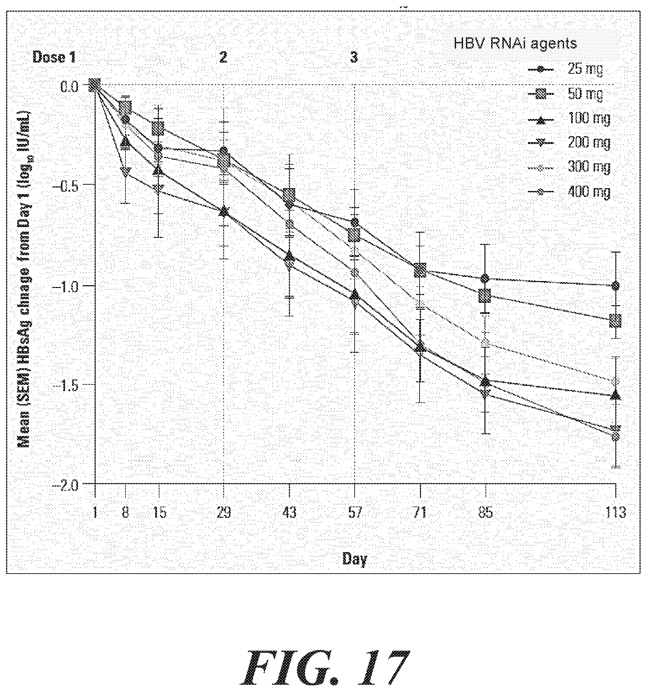

Mean HBsAg changes from Day 1 to Day 113 for CHB patients in cohorts 1b, 1c, 2b, 3b, 4b and 5b are shown in FIG. 17. The patients were given three subcutaneous doses of the HBV RNAi agents in the amount of: 25 mg (cohort 1b), 50 mg (cohort 1c), 100 mg (cohort 2b), 200 mg (cohort 3b), 300 mg (cohort 4b) or 400 mg (cohort 5b) on Day 1, 29 and 57. And all patients were given NA daily. HBV RNAi agents reduced HBsAg levels at all doses evaluated. At Day 113 (typical mean nadir after three doses, 56 days after last dose), mean HBsAg (SEM) log₁₀ reduction from Day 1 was 1.00 (0.18) with 25 mg, 1.18 (0.08) with 50 mg, 1.54 (0.18) with 100 mg, 1.71 (0.15) with 200 mg, 1.48 (0.11) with 300 mg and 1.75 (0.16) with 400 mg HBV RNAi agents.

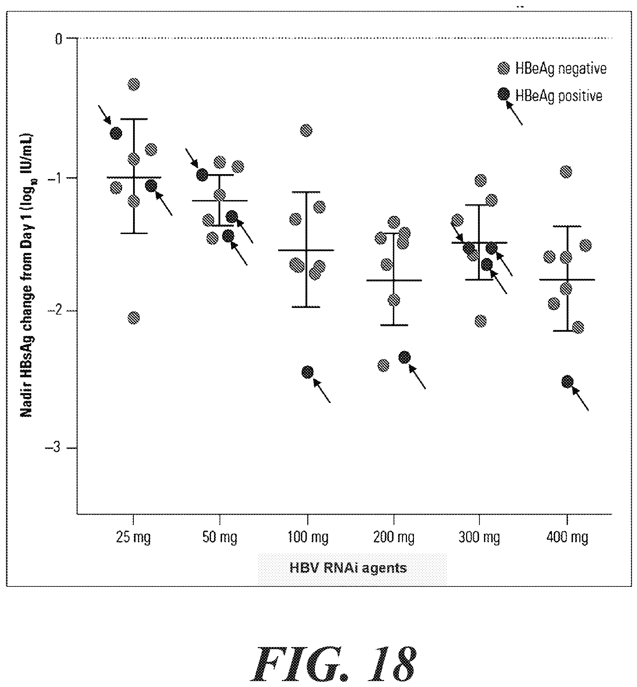

Mean and individual HBsAg changes on Day 113 from Day 1 for all patients in cohorts 1b, 1c, 2b, 3b, 4b and 5b are shown in FIG. 18. The proportion of patients with Day 113 data who achieved ≥ 1.0 log₁₀ reduction in HBsAg from Day 1 at nadir was 4/8 (25 mg), 6/8 (50 mg), 7/8 (100 mg), 8/8 (200 mg), 8/8 (300 mg) and 8/8 (400 mg). Similar responses were observed for HBeAg positive and negative patients. HBeAg positive patients (n=11) presented a reduction in HBsAg of -1.52 log₁₀ IU/mL (mean nadir). HBeAg negative patients (n=37) presented a reduction in HBsAg of -1.62 log₁₀ IU/mL (mean nadir). One patient receiving 200 mg had undetectable HBsAg at Day 113, and achieved 1.6 log10 reduction on Day 15 prior to HBsAg seroclearance. For patients with HBsAg >100 IU/mL (Day 1) and with Day 113 data, 2/7 (25 mg), 3/8 (50 mg), 5/7 (100 mg), 5/6 (200 mg), 6/8 (300 mg) and 5/7 (400 mg) achieved HBsAg <100 IU/mL with HBV RNAi agents treatment.

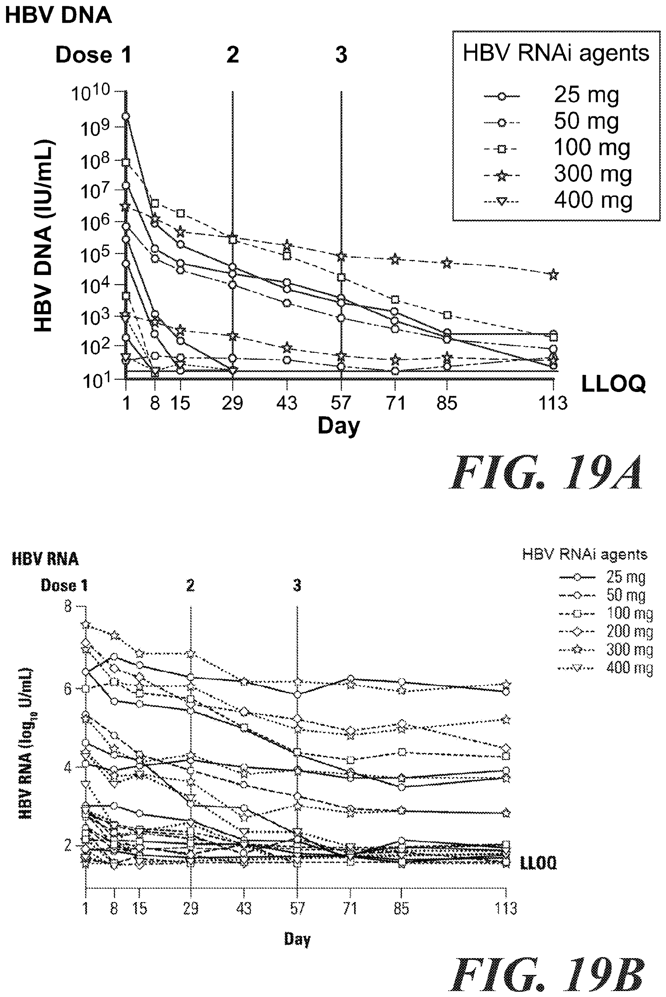

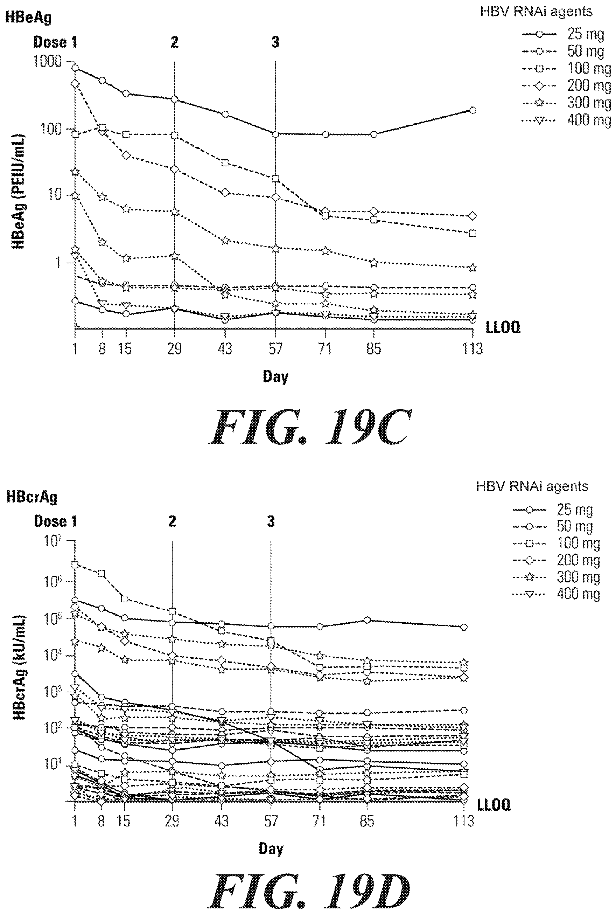

HBV DNA, HBV RNA, HBeAg and HBcrAg changes for individual participants (cohorts 1b, 1c, 2b, 3b, 4b and 5b) receiving 25 mg (cohort 1b), 50 mg (cohort 1c), 100 mg (cohort 2b), 200 mg (cohort 3b), 300 mg (cohort 4b) or 400 mg (cohort 5b) on Day 1, 29 and 57 are shown in FIGS. 19A-19D. All patients were given NA daily. For patients with measurable parameters on Day 1, patients had a robust decline in HBV DNA and HBV RNA levels, and reductions in HBeAg and HBcrAg were less pronounced with the HBV RNAi agents treatment. In addition to HBs, a sustained suppression was observed in a proportion of patients for HBV RNA, HBeAg and HBcrAg. For patients with quantifiable parameters on day 1 and available data at day 336, a sustained suppression was observed for HBV RNA in 15/26, HBeAg in 9/14 and HBcrAg in 10/24 pts.

Adverse events (AE) possibly or probably drug related occurring up to and including Day 113 of treatment for participants in cohorts 1b, 1c, 2b, 3b, 4b and 5b are shown in Table 57 below. Safety data for Cohorts 1b to 5b through Day 113 showed that three monthly doses of HBV RNAi agents at 25-400 mg with an NA were generally well tolerated in CHB patients. Three non-drug related serious adverse events were reported (anxiety with depression in a single patient and menorrhagia, each requiring hospitalization). The most commonly reported AEs at least possibly drug related consisted of various AEs at the injection site (e.g, discoloration, erythema, bruising, rash), which were all mild and reported in five patients. There were no reports of thrombocytopenia and one report of possibly drug-related Stage 1 acute kidney injury with creatinine increase (from 1.10 mg/dL Day 1 pre-dose to a peak of 1.55 mg/dL on Day 8 and return to 1.06 mg/dL on Day 15), which was treatment emergent but likely due to creatine supplementation and did not lead to treatment interruption or adjustment. A single AE reported of mild, possibly related, abnormal liver function tests (peak alanine aminotransferase [ALT] 136 U/L) was reported, representing the highest treatment-emergent ALT elevation in cohorts 1b to 5b through Day 113 (end of study). There were no cases of simultaneous elevations of ALT >3x upper limit of normal and total bilirubin >2x upper limit of normal.

**Table 57**

| **Possibly or probably drug-related AEs in ≥ 2 patients ^{a}** | **1b** | **1c** | **2b** | **3b** | **4b** | **5b** | **All patients** |
|---|---|---|---|---|---|---|---|
| | 25 mg | 50 mg | 100 mg | 200 mg | 300 mg | 400 mg | N=48 |
| | N=8 | N=8 | N=8 | N=8 | N=8 | N=8 | |
| **Injection site discoloration, injection site erythema, injection site bruising** | 1 mild | 0 | 0 | 0 | 2 mild | 2 mild | 5 |
| **Fatigue** | 1 mild | 0 | 1 mild | 0 | 0 | 1 mild | 3 |
| **Blood creatine phosphokinase elevated** | 1 mild | 0 | 0 | 0 | 1 severe | 0 | 2 |
| **Hot flush, flushing** | 0 | 1 mild | 0 | 0 | 0 | 1 mild | 2 |
| **Blood bilirubin increased, hyperbilirubinemia** | 0 | 0 | 0 | 1 mild | 1 mild | 0 | 2 |
| **Pruritus** | 1 mild | 0 | 1 mild | 0 | 0 | 0 | 2 |

| | | | | | | | |
|---|---|---|---|---|---|---|---|
| ^{a}MedDRA preferred term aggregated based on similarity | | | | | | | |

In CHB patients, HBV RNAi agents with an NA had strong activity against HBsAg, HBV DNA and HBV RNA. Reductions in HBeAg and HBcrAg were generally less pronounced. HBsAg reductions were similar in HBeAg positive and HBeAg negative patients. Expanded cohorts with 100-400 mg HBV RNAi agents confirmed previous findings that HBsAg declines were similar with these doses; 97% (31/32) of these patients achieved a ≥1.0 log₁₀ (90%) reduction in HBsAg. The 25 mg and 50 mg HBV RNAi agents doses were active in reducing HBsAg, and appeared less effective than higher doses. HBsAg responses with HBV RNAi agents are consistent with its ability to silence HBV RNA from cccDNA and host-integrated viral DNA (which is a major source of HBsAg in certain CHB populations). HBV RNAi agents (and NA) treatment was well tolerated at doses up to 400 mg Q4w for three doses. Overall, HBV RNAi agents demonstrated anti-HBV characteristics desirable for an RNAi therapy.

### OTHER EMBODIMENTS

The following embodiments are exemplary and should not be considered as limiting the invention as described herein.

**Embodiment 1** A method for inhibiting expression of a Hepatitis B Virus gene in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO: 111, SEQ ID NO:126, SEQ ID NO:127, SEQ ID NO:128, SEQ ID NO:171, SEQ ID NO: 175, SEQ ID NO: 179, and SEQ ID NO: 180, and
   (ii) a sense strand comprising a nucleotide sequence of any one of the following: SEQ ID NO:229, SEQ ID NO: 235, SEQ ID NO:252, SEQ ID NO:253, SEQ ID NO:273, SEQ ID NO: 307, SEQ ID NO:302 and SEQ ID NO:319, and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137, SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 50-400 mg per month.

**Embodiment 2** A method for treating or preventing a symptom or a disease associated with an infection caused by Hepatitis B Virus in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO:126, SEQ ID NO:127, SEQ ID NO:128, SEQ ID NO:171, SEQ ID NO: 179 and SEQ ID NO: 180, an
   (ii) a sense strand comprising a nucleotide sequence of any one of the following: SEQ ID NO:229, SEQ ID NO:252, SEQ ID NO:253, SEQ ID NO:273, SEQ ID NO:302, and SEQ ID NO:319·and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 50-400 mg per month.

**Embodiment 3** The method of Embodiment 1 or Embodiment 2, wherein the first and/or second RNAi agent further comprises a targeting ligand conjugated to the 5' end of the sense or antisense strand.

**Embodiment 4** The method of Embodiment 3, wherein the targeting ligand comprises N-acetyl-galactosamine.

**Embodiment 5** The method of Embodiment 4, wherein the targeting ligand is (NAG25), (NAG25)s, (NAG31), (NAG31)s, (NAG37), or (NAG37)s.

**Embodiment 6** The method of any one of Embodiments 3-5, wherein the targeting ligand is conjugated to the sense strand of the RNAi agent.

**Embodiment 7** The method of any one of Embodiments 3-5, wherein the targeting ligand is conjugated to the antisense strand of the RNAi agent.

**Embodiment 8.** The method of any one of Embodiments 1-7, wherein the first RNAi agent is administered in the amount of about 20-275 mg.

**Embodiment 9.** The method of any one of Embodiments 1-8, wherein the second RNAi agent is administered in the amount of about 10-150 mg.

**Embodiment 10.** The method of any one of Embodiments 1-9, wherein the pharmaceutical composition comprises RNAi agent comprising a duplex structure of AD04511 (SEQ ID NO: 100 and SEQ ID NO:229), AD04872 (SEQ ID NO: 126 and SEQ ID NO:252), AD04873 (SEQ ID NO: 127 and SEQ ID NO:252), AD04874 (SEQ ID NO: 128 and SEQ ID NO:253), AD05070 (SEQ ID NO:140 and SEQ ID NO:262), AD05148 (SEQ ID NO:140 and SEQ ID NO:271), AD05164 (SEQ ID NO:126 and SEQ ID NO:273), AD05165 (SEQ ID NO:140 and SEQ IDNO:274), or a mixture of any of the foregoing.

**Embodiment 11.** The method of Embodiment 10, wherein the pharmaceutical composition comprises an RNAi agent comprising the duplex structure of AD04872 (SEQ ID NO: 126 and SEQ ID NO:252) and an RNAi agent comprising the duplex structure of AD05070 (SEQ ID NO:140 and SEQ ID NO:262).

**Embodiment 12.** The method of Embodiment 11, wherein the duplex structure of AD04872 is conjugated to (NAG37) and the duplex structure of AD05070 is conjugated to (NAG37).

**Embodiment 13.** The method of Embodiment 12, wherein the (NAG37) is conjugated to the 5' end of the respective sense strands of the duplex of AD04872 and the duplex of AD05070.

**Embodiment 14.** The method of any one of Embodiments 11-13, wherein the ratio of the duplex of AD04872 and the duplex of AD05070 is between about 1:2 to about 5:1.

**Embodiment 15.** The method of Embodiment 14, wherein the ratio of the duplex of AD04872 and the duplex of AD05070 is about 2:1.

**Embodiment 16.** The method of any one of Embodiments 1-15, wherein the composition further comprises a pharmaceutically acceptable excipient.

**Embodiment 17.** The method of any one of Embodiments 1-16, wherein the composition is administered subcutaneously.

**Embodiment 18.** The method of any one of Embodiments 1-17, wherein the first and second RNAi agents are administered in a combined amount of about 25, 35, 50, 100, 200, 300 mg or 400 mg per month.

**Embodiment 19.** The method of any one of Embodiments 1-18, wherein the first and second RNAi agents are administered in 7-day, 14-day, 21-day or 28-day intervals.

**Embodiment 20.** The method of any one of Embodiments 1-19, wherein the composition is administered to the subject for up to 6 months.

**Embodiment 21.** The method of any one of Embodiments 1-20 further comprising administering to the subject a second active agent.

**Embodiment 22.** The method of Embodiment 21, wherein the second active agent is a nucleoside analog.

**Embodiment 23.** The method of Embodiment 22, wherein the nucleoside analog is Entecavir or Tenofovir.

**Embodiment 24.** The method of any one of Embodiments 1-23, wherein the level of HBsAg, HBeAg, or serum HBV DNA in the human subject is reduced by at least 40% after the administration of the composition.

**Embodiment 25.** The method of any one of Embodiments 2-24, wherein the symptom or disease associated with the HBV infection is a chronic liver disease or disorder, liver inflammation, fibrotic condition, a proliferative disorder, hepatocellular carcinoma, Hepatitis D virus infection, acute HBV infection or chronic HBV infection.

**Embodiment 26.** The method of any one of Embodiments 2-25, wherein the symptom or disease associated with the HBV infection is chronic HBV infection.

**Embodiment 27.** A method for inhibiting expression of a Hepatitis B Virus gene in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO: 111, SEQ ID NO:126, SEQ ID NO:127, SEQ ID NO:128, SEQ ID NO:171, SEQ ID NO: 175, SEQ ID NO: 179, and SEQ ID NO: 180, and
   (ii) a sense strand comprising a nucleotide sequence of any one of the following: SEQ ID NO:229, SEQ ID NO: 235, SEQ ID NO:252, SEQ ID NO:253, SEQ ID NO:273, SEQ ID NO: 307, SEQ ID NO:302 and SEQ ID NO:319, and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137, SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 25-400 mg per month.

**Embodiment 28.** A method for treating or preventing a symptom or a disease associated with an infection caused by Hepatitis B Virus in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO:126, SEQ ID NO:127, SEQ ID NO:128, SEQ ID NO:171, SEQ ID NO: 179 and SEQ ID NO: 180, and
   (ii) a sense strand comprising a nucleotide sequence of any one of the following: SEQ ID NO:229, SEQ ID NO:252, SEQ ID NO:253, SEQ ID NO:273, SEQ ID NO:302, and SEQ ID NO:319-and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 25-400 mg per month.

**Embodiment 29.** The method of Embodiment 27 or Embodiment 28, wherein the first and/or second RNAi agent further comprises a targeting ligand conjugated to the sense or antisense strand.

**Embodiment 30.** The method of Embodiment 29, wherein the targeting ligand comprises N-acetyl-galactosamine.

**Embodiment 31.** The method of Embodiment 30, wherein the targeting ligand is (NAG25), (NAG25)s, (NAG31), (NAG31)s, (NAG37), or (NAG37)s.

**Embodiment 32.** The method of any one of Embodiments 29-31, wherein the targeting ligand is conjugated to the sense strand of the RNAi agent.

**Embodiment 33.** The method of any one of Embodiments 29-31, wherein the targeting ligand is conjugated to the antisense strand of the RNAi agent.

**Embodiment 34.** The method of any one of Embodiments 27-33, wherein the first RNAi agent is administered in the amount of about 20-275 mg.

**Embodiment 35.** The method of any one of Embodiments 28-34, wherein the second RNAi agent is administered in the amount of about 10-150 mg.

**Embodiment 36.** The method of any one of Embodiments 27-35, wherein the pharmaceutical composition comprises RNAi agent comprising a duplex structure of AD04511 (SEQ ID NO: 100 and SEQ ID NO:229), AD04872 (SEQ ID NO: 126 and SEQ ID NO:252), AD04873 (SEQ ID NO: 127 and SEQ ID NO:252), AD04874 (SEQ ID NO: 128 and SEQ ID NO:253), AD05070 (SEQ ID NO:140 and SEQ ID NO:262), AD05148 (SEQ ID NO:140 and SEQ ID NO:271), AD05164 (SEQ ID NO:126 and SEQ ID NO:273), AD05165 (SEQ ID NO:140 and SEQ IDNO:274), or a mixture of any of the foregoing.

**Embodiment 37.** The method of Embodiment 36, wherein the pharmaceutical composition comprises an RNAi agent comprising the duplex structure of AD04872 (SEQ ID NO: 126 and SEQ ID NO:252) and an RNAi agent comprising the duplex structure of AD05070 (SEQ ID NO:140 and SEQ ID NO:262).

**Embodiment 38.** The method of Embodiment 37, wherein the duplex structure of AD04872 is conjugated to (NAG37) and the duplex structure of AD05070 is conjugated to (NAG37).

**Embodiment 39.** The method of Embodiment 38, wherein the (NAG37) is conjugated to the 5' end of the respective sense strands of the duplex of AD04872 and the duplex of AD05070.

**Embodiment 40.** The method of any one of Embodiments 37-39, wherein the ratio of the duplex of AD04872 and the duplex of AD05070 is between about 1:2 to about 5:1.

**Embodiment 41.** The method of Embodiment 40, wherein the ratio of the duplex of AD04872 and the duplex of AD05070 is about 2:1.

**Embodiment 42.** The method of any one of Embodiments 27-41, wherein the composition further comprises a pharmaceutically acceptable excipient.

**Embodiment 43.** The method of any one of Embodiments 27-42, wherein the composition is administered subcutaneously.

**Embodiment 44.** The method of any one of Embodiments 27-43, wherein the first and second RNAi agents are administered in a combined amount of about any of 25, 35, 50, 100, 200, 300 mg or 400 mg per month.

**Embodiment 45.** The method of any one of Embodiments 27-43, wherein the first and second RNAi agents are administered in a combined amount of about 40 mg, about 100 mg, or about 200 mg per month.

**Embodiment 46.** The method of Embodiment 45, wherein the first and second RNAi agents are administered in a combined amount of about 40 mg per month.

**Embodiment 47.** The method of Embodiment 45, wherein the first and second RNAi agents are administered in a combined amount of about 100 mg per month.

**Embodiment 48.** The method of Embodiment 45, wherein the first and second RNAi agents are administered in a combined amount of about 200 mg per month.

**Embodiment 49.** The method of any one of Embodiments 27-48, wherein the first and second RNAi agents are administered in 7-day, 14-day, 21-day or 28-day intervals.

**Embodiment 50.** The method of any one of Embodiments 27-48, wherein the first and second RNAi agents are administered in 28-day intervals.

**Embodiment 51.** The method of Embodiment 50, wherein the first and second RNAi agents are administered in a combined amount of about 40 mg, about 100 mg, or about 200 mg per month and in 28-day intervals.

**Embodiment 52.** The method of Embodiment 51, wherein the first and second RNAi agents are administered in a combined amount of about 40 mg per month and in 28-day intervals.

**Embodiment 53.** The method of Embodiment 51, wherein the first and second RNAi agents are administered in a combined amount of about 100 mg per month and in 28-day intervals.

**Embodiment 54.** The method of Embodiment 51, wherein the first and second RNAi agents are administered in a combined amount of about 200 mg per month and in 28-day intervals.

**Embodiment 55.** The method of any one of Embodiments 27-54, wherein the composition is administered to the subject for up to 6 months.

**Embodiment 56.** The method of any one of Embodiments 27-55 further comprising administering to the subject a second active agent.

**Embodiment 57.** The method of Embodiment 56, wherein the second active agent is a nucleoside analog.

**Embodiment 58.** The method of Embodiment 57, wherein the nucleoside analog is Entecavir or Tenofovir.

**Embodiment 59.** The method of any one of Embodiments 27-58, wherein the level of HBsAg, HBeAg, or serum HBV DNA in the human subject is reduced by at least 40% after the administration of the composition.

**Embodiment 60.** The method of any one of Embodiments 28-59, wherein the symptom or disease associated with the HBV infection is a chronic liver disease or disorder, liver inflammation, liver fibrotic condition, a proliferative hepatocellular disorder, hepatocellular carcinoma, Hepatitis D virus infection, acute HBV infection, chronic hepatitis B or chronic HBV infection.

**Embodiment 61.** The method of any one of Embodiments 28-60, wherein the symptom or disease associated with the HBV infection is chronic HBV infection or chronic hepatitis B.

**Embodiment 62.** A method for inhibiting expression of a Hepatitis B Virus gene in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO: 111, SEQ ID NO:126, SEQ ID NO:127, SEQ ID NO:128, SEQ ID NO:171, SEQ ID NO: 175, SEQ ID NO: 179, and SEQ ID NO: 180, and
   (ii) a sense strand comprising a nucleotide sequence of any one of the following: SEQ ID NO:229, SEQ ID NO: 235, SEQ ID NO:252, SEQ ID NO:253, SEQ ID NO:273, SEQ ID NO: 307, SEQ ID NO:302 and SEQ ID NO:319, and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137, SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 25-400 mg per 28 days.

**Embodiment 63.** A method for treating or preventing a symptom or a disease associated with an infection caused by Hepatitis B Virus in a human subject in need thereof comprising administering to the human subject an effective amount of a pharmaceutical composition comprising:
(a) a first RNAi agent comprising:
   (i) an antisense strand comprising a nucleotide sequence of any one of the following: SEQ ID NO:100, SEQ ID NO:126, SEQ ID NO:127, SEQ ID NO:128, SEQ ID NO:171, SEQ ID NO: 179 and SEQ ID NO: 180, and
   (ii) a sense strand comprising a nucleotide sequence of any one of the following: SEQ ID NO:229, SEQ ID NO:252, SEQ ID NO:253, SEQ ID NO:273, SEQ ID NO:302, and SEQ ID NO:319-and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 25-400 mg per 28 days.

**Embodiment 64.** The method of Embodiment 62 or Embodiment 63, wherein the first and/or second RNAi agent further comprises a targeting ligand conjugated to the 5' end of the sense or antisense strand.

**Embodiment 65.** The method of Embodiment 64, wherein the targeting ligand comprises N-acetyl-galactosamine.

**Embodiment 66.** The method of Embodiment 65, wherein the targeting ligand is (NAG25), (NAG25)s, (NAG31), (NAG31)s, (NAG37), or (NAG37)s.

**Embodiment 67.** The method of any one of Embodiments 64-66, wherein the targeting ligand is conjugated to the sense strand of the RNAi agent.

**Embodiment 68.** The method of any one of Embodiments 64-66, wherein the targeting ligand is conjugated to the antisense strand of the RNAi agent.

**Embodiment 69.** The method of any one of Embodiments 62-68, wherein the first RNAi agent is administered in the amount of about 20-275 mg.

**Embodiment 70.** The method of any one of Embodiments 62-69, wherein the second RNAi agent is administered in the amount of about 10-150 mg.

**Embodiment 71.** The method of any one of Embodiments 62-70, wherein the pharmaceutical composition comprises RNAi agent comprising a duplex structure of AD04511 (SEQ ID NO: 100 and SEQ ID NO:229), AD04872 (SEQ ID NO: 126 and SEQ ID NO:252), AD04873 (SEQ ID NO: 127 and SEQ ID NO:252), AD04874 (SEQ ID NO: 128 and SEQ ID NO:253), AD05070 (SEQ ID NO:140 and SEQ ID NO:262), AD05148 (SEQ ID NO:140 and SEQ ID NO:271), AD05164 (SEQ ID NO:126 and SEQ ID NO:273), AD05165 (SEQ ID NO:140 and SEQ IDNO:274), or a mixture of any of the foregoing.

**Embodiment 72.** The method of Embodiment 71, wherein the pharmaceutical composition comprises an RNAi agent comprising the duplex structure of AD04872 (SEQ ID NO: 126 and SEQ ID NO:252) and an RNAi agent comprising the duplex structure of AD05070 (SEQ ID NO:140 and SEQ ID NO:262).

**Embodiment 73.** The method of Embodiment 72, wherein the duplex structure of AD04872 is conjugated to (NAG37) and the duplex structure of AD05070 is conjugated to (NAG37).

**Embodiment 74.** The method of Embodiment 73, wherein the (NAG37) is conjugated to the 5' end of the respective sense strands of the duplex of AD04872 and the duplex of AD05070.

**Embodiment 75.** The method of any one of Embodiments 72-74, wherein the ratio of the duplex of AD04872 and the duplex of AD05070 is between about 1:2 to about 5:1.

**Embodiment 76.** The method of Embodiment 75, wherein the ratio of the duplex of AD04872 and the duplex of AD05070 is about 2:1.

**Embodiment 77.** The method of any one of Embodiments 62-76, wherein the composition further comprises a pharmaceutically acceptable excipient.

**Embodiment 78.** The method of any one of Embodiments 62-77, wherein the composition is administered subcutaneously.

**Embodiment 79.** The method of any one of Embodiments 62-78, wherein the first and second RNAi agents are administered in a combined amount of about any of 25, 35, 50, 100, 200, 300 mg or 400 mg per 28 days.

**Embodiment 80.** The method of any one of Embodiments 62-78, wherein the first and second RNAi agents are administered in a combined amount of about 40 mg, about 100 mg, or about 200 mg per 28 days.

**Embodiment 81.** The method of Embodiment 80, wherein the first and second RNAi agents are administered in a combined amount of about 40 mg per 28 days.

**Embodiment 82.** The method of Embodiment 80, wherein the first and second RNAi agents are administered in a combined amount of about 100 mg per 28 days.

**Embodiment 83.** The method of Embodiment 80, wherein the first and second RNAi agents are administered in a combined amount of about 200 mg per 28 days.

**Embodiment 84.** The method of any one of Embodiments 62-83, wherein the first and second RNAi agents are administered in 7-day, 14-day, 21-day or 28-day intervals.

**Embodiment 85.** The method of any one of Embodiments 62-84, wherein the first and second RNAi agents are administered in 28-day intervals.

**Embodiment 86.** The method of Embodiment 85, wherein the first and second RNAi agents are administered in a combined amount of about 40 mg, about 100 mg, or about 200 mg per 28 days and in 28-day intervals.

**Embodiment 87.** The method of Embodiment 86, wherein the first and second RNAi agents are administered in a combined amount of about 40 mg per 28 days and in 28-day intervals.

**Embodiment 88.** The method of Embodiment 86, wherein the first and second RNAi agents are administered in a combined amount of about 100 mg per 28 days and in 28-day intervals.

**Embodiment 89.** The method of Embodiment 86, wherein the first and second RNAi agents are administered in a combined amount of about 200 mg per 28 days and in 28-day intervals.

**Embodiment 90.** The method of any one of Embodiments 62-89, wherein the composition is administered to the subject for up to 6 months.

**Embodiment 91.** The method of any one of Embodiments 62-90, further comprising administering to the subject a second active agent.

**Embodiment 92.** The method of Embodiment 91, wherein the second active agent is a nucleoside analog.

**Embodiment 93.** The method of Embodiment 92, wherein the nucleoside analog is Entecavir or Tenofovir.

**Embodiment 94.** The method of any one of Embodiments 62-93, wherein the level of HBsAg, HBeAg, or serum HBV DNA in the human subject is reduced by at least 40% after the administration of the composition.

**Embodiment 95.** The method of any one of Embodiments 63-94, wherein the symptom or disease associated with the HBV infection is a chronic liver disease or disorder, liver inflammation, liver fibrotic condition, a proliferative hepatocellular disorder, hepatocellular carcinoma, Hepatitis D virus infection, acute HBV infection, chronic hepatitis B or chronic HBV infection.

**Embodiment 96.** The method of any one of Embodiments 63-95, wherein the symptom or disease associated with the HBV infection is chronic HBV infection or chronic hepatitis B.

**Embodiment 97.** A pharmaceutical composition for use in inhibiting expression of a Hepatitis B Virus gene in a human subject, wherein said use comprises the administration of an effective amount of the pharmaceutical composition to the human subject, the pharmaceutical composition comprising:
(a) a first RNAi agent comprising:
   (i) an antisense strand comprising a nucleotide sequence of any one of the following: SEQ ID NO:100, SEQ ID NO: 111, SEQ ID NO:126, SEQ ID NO:127, SEQ ID NO:128, SEQ ID NO:171, SEQ ID NO: 175, SEQ ID NO: 179, and SEQ ID NO: 180, and
   (ii) a sense strand comprising a nucleotide sequence of any one of the following: SEQ ID NO:229, SEQ ID NO: 235, SEQ ID NO:252, SEQ ID NO:253, SEQ ID NO:273, SEQ ID NO: 307, SEQ ID NO:302 and SEQ ID NO:319, and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137, SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 25-400 mg per 28 days.

**Embodiment 98.** A pharmaceutical composition for use in treating or preventing a symptom or a disease associated with an infection caused by Hepatitis B Virus in a human subject, wherein said use comprises the administration of an effective amount of the pharmaceutical composition to the human subject, the pharmaceutical composition comprising:
(a) a first RNAi agent comprising:
   (i) an antisense strand comprising a nucleotide sequence of any one of the following: SEQ ID NO:100, SEQ ID NO:126, SEQ ID NO:127, SEQ ID NO:128, SEQ ID NO:171, SEQ ID NO: 179 and SEQ ID NO: 180, and
   (ii) a sense strand comprising a nucleotide sequence of any one of the following: SEQ ID NO:229, SEQ ID NO:252, SEQ ID NO:253, SEQ ID NO:273, SEQ ID NO:302, and SEQ ID NO:319-and
(b) a second RNAi agent comprising:
   (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
   (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
and wherein the first and second RNAi agents are administered in a combined amount of about 25-400 mg per 28 days.

**Embodiment 99.** The pharmaceutical composition of Embodiment 97 or Embodiment 98, wherein the first and/or second RNAi agent further comprises a targeting ligand conjugated to the 5' end of the sense or antisense strand.

**Embodiment 100.** The pharmaceutical composition of Embodiment 99, wherein the targeting ligand comprises N-acetyl-galactosamine.

**Embodiment 101.** The pharmaceutical composition of Embodiment 100, wherein the targeting ligand is (NAG25), (NAG25)s, (NAG31), (NAG31)s, (NAG37), or (NAG37)s.

**Embodiment 102.** The pharmaceutical composition of any one of Embodiments 99-101, wherein the targeting ligand is conjugated to the sense strand of the RNAi agent.

**Embodiment 103.** The pharmaceutical composition of any one of Embodiments 99-101, wherein the targeting ligand is conjugated to the antisense strand of the RNAi agent.

**Embodiment 104.** The pharmaceutical composition of any one of Embodiments 97-103, wherein the first RNAi agent is administered in the amount of about 20-275 mg.

**Embodiment 105.** The pharmaceutical composition of any one of Embodiments 97-104, wherein the second RNAi agent is administered in the amount of about 10-150 mg.

**Embodiment 106.** The pharmaceutical composition of any one of Embodiments 97-105, wherein the pharmaceutical composition comprises RNAi agent comprising a duplex structure of AD04511 (SEQ ID NO: 100 and SEQ ID NO:229), AD04872 (SEQ ID NO: 126 and SEQ ID NO:252), AD04873 (SEQ ID NO: 127 and SEQ ID NO:252), AD04874 (SEQ ID NO: 128 and SEQ ID NO:253), AD05070 (SEQ ID NO:140 and SEQ ID NO:262), AD05148 (SEQ ID NO:140 and SEQ ID NO:271), AD05164 (SEQ ID NO:126 and SEQ ID NO:273), AD05165 (SEQ ID NO:140 and SEQ IDNO:274), or a mixture of any of the foregoing.

**Embodiment 107.** The pharmaceutical composition of Embodiment 106, wherein the pharmaceutical composition comprises an RNAi agent comprising the duplex structure of AD04872 (SEQ ID NO: 126 and SEQ ID NO:252) and an RNAi agent comprising the duplex structure of AD05070 (SEQ ID NO:140 and SEQ ID NO:262).

**Embodiment 108.** The pharmaceutical composition of Embodiment 107, wherein the duplex structure of AD04872 is conjugated to (NAG37) and the duplex structure of AD05070 is conjugated to (NAG37).

**Embodiment 109.** The pharmaceutical composition of Embodiment 108, wherein the (NAG37) is conjugated to the 5' end of the respective sense strands of the duplex of AD04872 and the duplex of AD05070.

**Embodiment 110.** The pharmaceutical composition of any one of Embodiments 107-109, wherein the ratio of the duplex of AD04872 and the duplex of AD05070 is between about 1:2 to about 5:1.

**Embodiment 111.** The pharmaceutical composition of Embodiment 110, wherein the ratio of the duplex of AD04872 and the duplex of AD05070 is about 2:1.

**Embodiment 112.** The pharmaceutical composition of any one of Embodiments 97-111, wherein the composition further comprises a pharmaceutically acceptable excipient.

**Embodiment 113.** The pharmaceutical composition of any one of Embodiments 97-112, wherein the composition is administered subcutaneously.

**Embodiment 114.** The pharmaceutical composition of any one of Embodiments 97-113, wherein the first and second RNAi agents are administered in a combined amount of about any of 25, 35, 50, 100, 200, 300 mg or 400 mg per 28 days.

**Embodiment 115.** The pharmaceutical composition of any one of Embodiments 97-114, wherein the first and second RNAi agents are administered in a combined amount of about 40 mg, about 100 mg, or about 200 mg per 28 days.

**Embodiment 116.** The pharmaceutical composition of Embodiment 115, wherein the first and second RNAi agents are administered in a combined amount of about 40 mg per 28 days.

**Embodiment 117.** The pharmaceutical composition of Embodiment 115, wherein the first and second RNAi agents are administered in a combined amount of about 100 mg per 28 days.

**Embodiment 118.** The pharmaceutical composition of Embodiment 115, wherein the first and second RNAi agents are administered in a combined amount of about 200 mg per 28 days.

**Embodiment 119.** The pharmaceutical composition of any one of Embodiments 97-118, wherein the first and second RNAi agents are administered in 7-day, 14-day, 21-day or 28-day intervals.

**Embodiment 120.** The pharmaceutical composition of any one of Embodiments 97-118, wherein the first and second RNAi agents are administered in 28-day intervals.

**Embodiment 121.** The pharmaceutical composition of Embodiment 120, wherein the first and second RNAi agents are administered in a combined amount of about 40 mg, about 100 mg, or about 200 mg per 28 days and in 28-day intervals.

**Embodiment 122.** The pharmaceutical composition of Embodiment 121, wherein the first and second RNAi agents are administered in a combined amount of about 40 mg per 28 days and in 28-day intervals.

**Embodiment 123.** The pharmaceutical composition of Embodiment 121, wherein the first and second RNAi agents are administered in a combined amount of about 100 mg per 28 days and in 28-day intervals.

**Embodiment 124.** The pharmaceutical composition of Embodiment 121, wherein the first and second RNAi agents are administered in a combined amount of about 200 mg per 28 days and in 28-day intervals.

**Embodiment 125.** The pharmaceutical composition of any one of Embodiments 97-124, wherein the composition is administered to the subject for up to 6 months.

**Embodiment 126.** The pharmaceutical composition of any one of Embodiments 97-125, further comprising administering to the subject a second active agent.

**Embodiment 127.** The pharmaceutical composition of Embodiment 126, wherein the second active agent is a nucleoside analog.

**Embodiment 128.** The pharmaceutical composition of Embodiment 127, wherein the nucleoside analog is Entecavir or Tenofovir.

**Embodiment 129.** The pharmaceutical composition of any one of Embodiments 97-128, wherein the level of HBsAg, HBeAg, or serum HBV DNA in the human subject is reduced by at least 40% after the administration of the composition.

**Embodiment 130.** The pharmaceutical composition of any one of Embodiments 98-129, wherein the symptom or disease associated with the HBV infection is a chronic liver disease or disorder, liver inflammation, liver fibrotic condition, a proliferative hepatocellular disorder, hepatocellular carcinoma, Hepatitis D virus infection, acute HBV infection, chronic hepatitis B, or chronic HBV infection.

**Embodiment 131.** The pharmaceutical composition of any one of Embodiments 98-130, wherein the symptom or disease associated with the HBV infection is chronic HBV infection or chronic hepatitis B.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### Clauses

**Clause 1.** A pharmaceutical composition for use in treating or preventing a disease associated with an infection caused by Hepatitis B Virus in a human subject, wherein said use comprises the administration of an effective amount of the pharmaceutical composition to the human subject, the pharmaceutical composition comprising:
   (a) a first RNAi agent comprising:
      (i) an antisense strand comprising a nucleotide sequence of any one of the following: SEQ ID NO:100, SEQ ID NO:126, SEQ ID NO:127, SEQ ID NO:128, SEQ ID NO:171, SEQ ID NO: 179 and SEQ ID NO: 180, and
      (ii) a sense strand comprising a nucleotide sequence of any one of the following: SEQ ID NO:229, SEQ ID NO:252, SEQ ID NO:253, SEQ ID NO:273, SEQ ID NO:302, and SEQ ID NO:319 and
   (b) a second RNAi agent comprising:
      (i) an antisense strand comprising a nucleotide sequence of any one of the following: SEQ ID NO:140, SEQ ID NO: 107, SEQ ID NO: 136, SEQ ID NO: 137,SEQ ID NO:188, SEQ ID NO: 154 and SEQ ID NO: 162, and
      (ii) a sense strand comprising a nucleotide sequence of any one of the following: SEQ ID NO:262, SEQ ID NO:271, SEQ ID NO: 216, SEQ ID NO: 248, SEQ ID NO:274, SEQ ID NO:328, SEQ ID NO: 292, and SEQ ID NO: 294;
   and wherein the first and second RNAi agents are administered in a combined amount of about 25-400 mg per 28 days.
**Clause 2.** The pharmaceutical composition of Clause 1, wherein the first and/or second RNAi agent further comprises a targeting ligand conjugated to the sense or antisense strand.
**Clause 3.** The pharmaceutical composition of Clause 2, wherein the targeting ligand comprises N-acetyl-galactosamine.
**Clause 4.** The pharmaceutical composition of Clause 3, wherein the targeting ligand is (NAG25), (NAG25)s, (NAG31), (NAG31)s, (NAG37), or (NAG37)s.
**Clause 5.** The pharmaceutical composition of any one of Clauses 2-4, wherein the targeting ligand is conjugated to the 5' end or to the 3' end of sense strand of the RNAi agent.
**Clause 6.** The pharmaceutical composition of any one of Clauses 2-4, wherein the targeting ligand is conjugated to the 3' end of the antisense strand of the RNAi agent.
**Clause 7.** The pharmaceutical composition of any one of Clauses 1-6, wherein the first RNAi agent is administered in the amount of about 20-275 mg.
**Clause 8.** The pharmaceutical composition of any one of Clauses 1-7, wherein the second RNAi agent is administered in the amount of about 10-150 mg.
**Clause 9.** The pharmaceutical composition of any one of Clauses 1-8, wherein the pharmaceutical composition comprises RNAi agent comprising a duplex structure of AD04511 (SEQ ID NO: 100 and SEQ ID NO:229), AD04872 (SEQ ID NO: 126 and SEQ ID NO:252), AD04873 (SEQ ID NO: 127 and SEQ ID NO:252), AD04874 (SEQ ID NO: 128 and SEQ ID NO:253), AD05070 (SEQ ID NO:140 and SEQ ID NO:262), AD05148 (SEQ ID NO:140 and SEQ ID NO:271), AD05164 (SEQ ID NO:126 and SEQ ID NO:273), AD05165 (SEQ ID NO:140 and SEQ IDNO:274), or a mixture of any of the foregoing.
**Clause 10.** The pharmaceutical composition of Clause 9, wherein the pharmaceutical composition comprises an RNAi agent comprising the duplex structure of AD04872 (SEQ ID NO: 126 and SEQ ID NO:252) and an RNAi agent comprising the duplex structure of AD05070 (SEQ ID NO:140 and SEQ ID NO:262).
**Clause 11.** The pharmaceutical composition of Clause 10, wherein the duplex structure of AD04872 is conjugated to (NAG37) and the duplex structure of AD05070 is conjugated to (NAG37).
**Clause 12.** The pharmaceutical composition of Clause 11, wherein the (NAG37) is conjugated to the 5' end of the respective sense strands of the duplex of AD04872 and the duplex of AD05070.
**Clause 13.** The pharmaceutical composition of any one of Clauses 10-12, wherein the ratio of the duplex of AD04872 and the duplex of AD05070 is between about 1:2 to about 5:1.
**Clause 14.** The pharmaceutical composition of Clause 13, wherein the ratio of the duplex of AD04872 and the duplex of AD05070 is about 2:1.
**Clause 15.** The pharmaceutical composition of any one of Clauses 1-14, wherein the composition further comprises a pharmaceutically acceptable excipient.
**Clause 16.** The pharmaceutical composition of any one of Clauses 1-15, wherein the composition is administered subcutaneously.
**Clause 17.** The pharmaceutical composition of any one of Clauses 1-16, wherein the first and second RNAi agents are administered in a combined amount of about any of 25, 35, 50, 100, 200, 300 mg or 400 mg per 28 days.
**Clause 18.** The pharmaceutical composition of any one of Clauses 1-16, wherein the first and second RNAi agents are administered in a combined amount of about 40 mg, about 100 mg, or about 200 mg per 28 days.
**Clause 19.** The pharmaceutical composition of Clause 18, wherein the first and second RNAi agents are administered in a combined amount of about 40 mg per 28 days.
**Clause 20.** The pharmaceutical composition of Clause 18, wherein the first and second RNAi agents are administered in a combined amount of about 100 mg per 28 days.
**Clause 21.** The pharmaceutical composition of Clause 18, wherein the first and second RNAi agents are administered in a combined amount of about 200 mg per 28 days.
**Clause 22.** The pharmaceutical composition of any one of Clauses 1-21, wherein the first and second RNAi agents are administered in 7-day, 14-day, 21-day or 28-day intervals.
**Clause 23.** The pharmaceutical composition of any one of Clauses 1-21, wherein the first and second RNAi agents are administered in 28-day intervals.
**Clause 24.** The pharmaceutical composition of Clause 23, wherein the first and second RNAi agents are administered in a combined amount of about 40 mg, about 100 mg, or about 200 mg per 28 days and in 28-day intervals.
**Clause 25.** The pharmaceutical composition of Clause 24, wherein the first and second RNAi agents are administered in a combined amount of about 40 mg per 28 days and in 28-day intervals.
**Clause 26.** The pharmaceutical composition of Clause 24, wherein the first and second RNAi agents are administered in a combined amount of about 100 mg per 28 days and in 28-day intervals.
**Clause 27.** The pharmaceutical composition of Clause 24, wherein the first and second RNAi agents are administered in a combined amount of about 200 mg per 28 days and in 28-day intervals.
**Clause 28.** The pharmaceutical composition of any one of Clauses 1-27, wherein the composition is administered to the subject for up to 6 months.
**Clause 29.** The pharmaceutical composition of any one of Clauses 1-28, further comprising administering to the subject a second active agent.
**Clause 30.** The pharmaceutical composition of Clause 29, wherein the second active agent is a nucleoside analog.
**Clause 31.** The pharmaceutical composition of Clause 30, wherein the nucleoside analog is Entecavir or Tenofovir.
**Clause 32.** The pharmaceutical composition of any one of Clauses 1-31, wherein the level of HBsAg, HBeAg, or serum HBV DNA in the human subject is reduced by at least 40% after the administration of the composition.
**Clause 33.** The pharmaceutical composition of any one of Clauses 1-32, wherein the disease associated with the HBV infection is a chronic liver disease or disorder, liver inflammation, liver fibrotic condition, a proliferative hepatocellular disorder, hepatocellular carcinoma, Hepatitis D virus infection, acute HBV infection, chronic hepatitis B or chronic HBV infection.
**Clause 34.** The pharmaceutical composition of Clause 33, wherein the disease associated with the HBV infection is chronic hepatitis B or chronic HBV infection.

## Claims

1. A pharmaceutical composition for use in a method of treating a chronic HBV infection in a human subject, wherein said method comprises the administration of an effective amount of the pharmaceutical composition to the human subject, the pharmaceutical composition comprising:
(a) a first RNAi agent (AD04872) comprising:
(i) an antisense strand having the structure:
asGfsasAfaAfuUfgAfgAfgAfaGfuCfcasc (SEQ ID NO: 126), and
(ii) a sense strand having the structure:
(NAG37)s (invAb)sguggacuuCfUfCfucaauuuucus(invAb) (SEQ ID NO:252), and
(b) a second RNAi agent (AD05070) comprising:
(i) an antisense strand having the structure:
usAfscsCfaAfuUfuAfuGfcCfuAfcAfgcsg (SEQ ID NO: 140), and
(ii) a sense strand having the structure:
(NAG37)s (invAb)scgcuguagGfCfAfuaaauugguas(invAb) (SEQ ID NO:262);
and wherein the first and second RNAi agents are administered in a combined amount of about 50 mg per 4 weeks or 1 month;
wherein (NAG37)s has the structure: 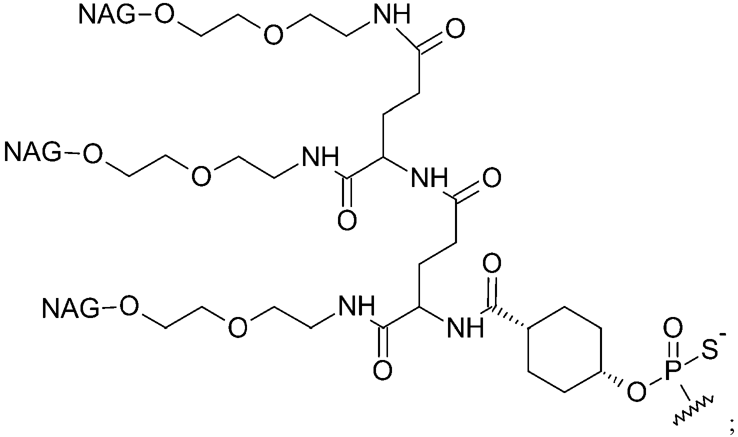 and
NAG is N-acetyl-galactosamine;
a, g, c and u are 2'-O-methyl modified nucleotides;
Af, Cf, Gf, and Uf are 2'-fluoro modified nucleotides;
s is a phosphorothioate internucleoside linkage;
(invAb)s is an inverted (3'-3' linked) abasic deoxyribonucleotide-5'-phosphorothioate; and
(invAb) is an inverted (3'-3' linked) abasic deoxyribonucleotide.

2. The pharmaceutical composition for use of claim 1, wherein the first RNAi agent (AD04872) has the structure represented by the following: 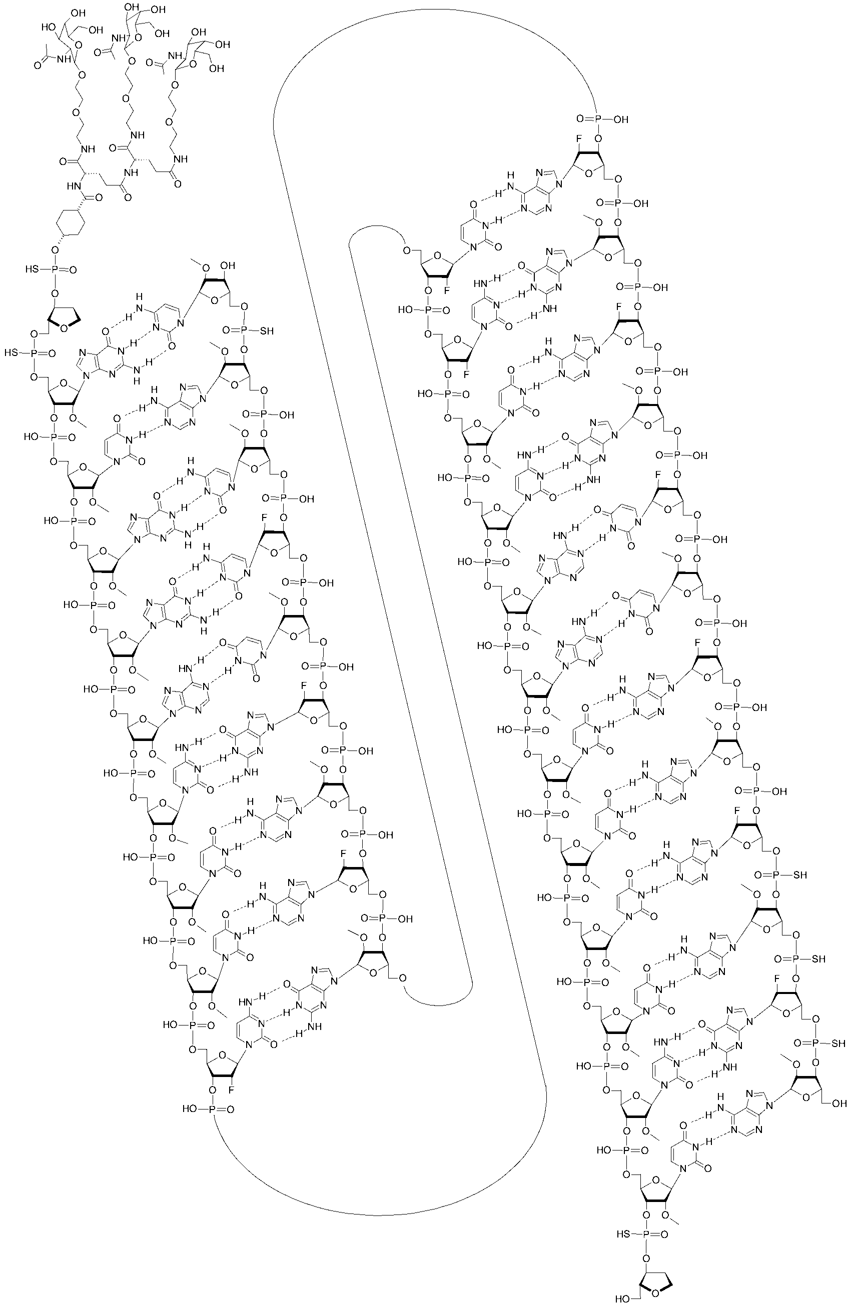
and is provided as a salt, mixed salt, or a free-acid;
and wherein the second RNAi agent (AD05070) has the structure represented by the following: 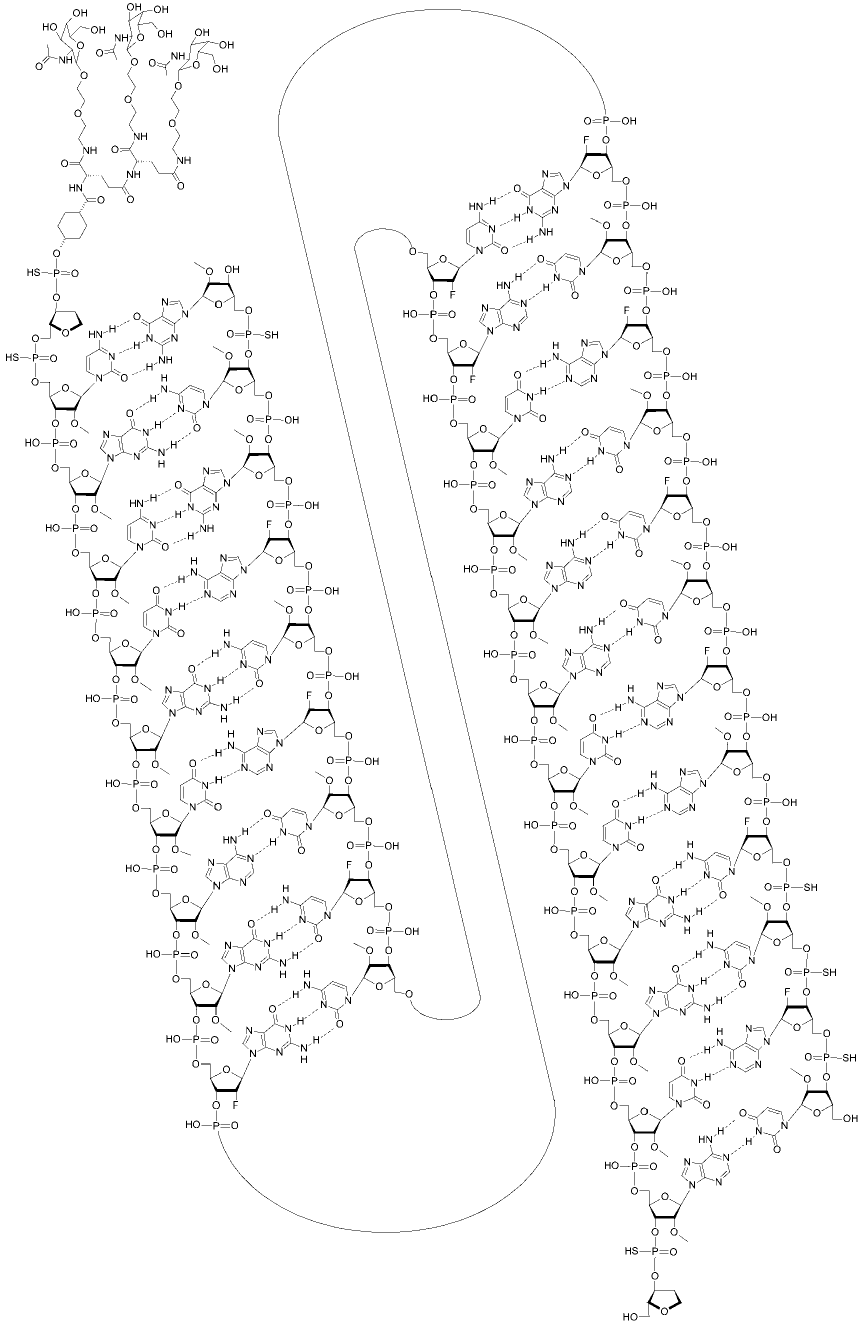
and is provided as a salt, mixed salt, or a free-acid.

3. The pharmaceutical composition for use of claim 1, wherein the first RNAi agent (AD04872) and the second RNAi agent (AD05070) are each provided as a sodium salt.

4. The pharmaceutical composition for use of any one of claims 1-3, wherein the first RNAi agent (AD04872) has the structure represented by the following: 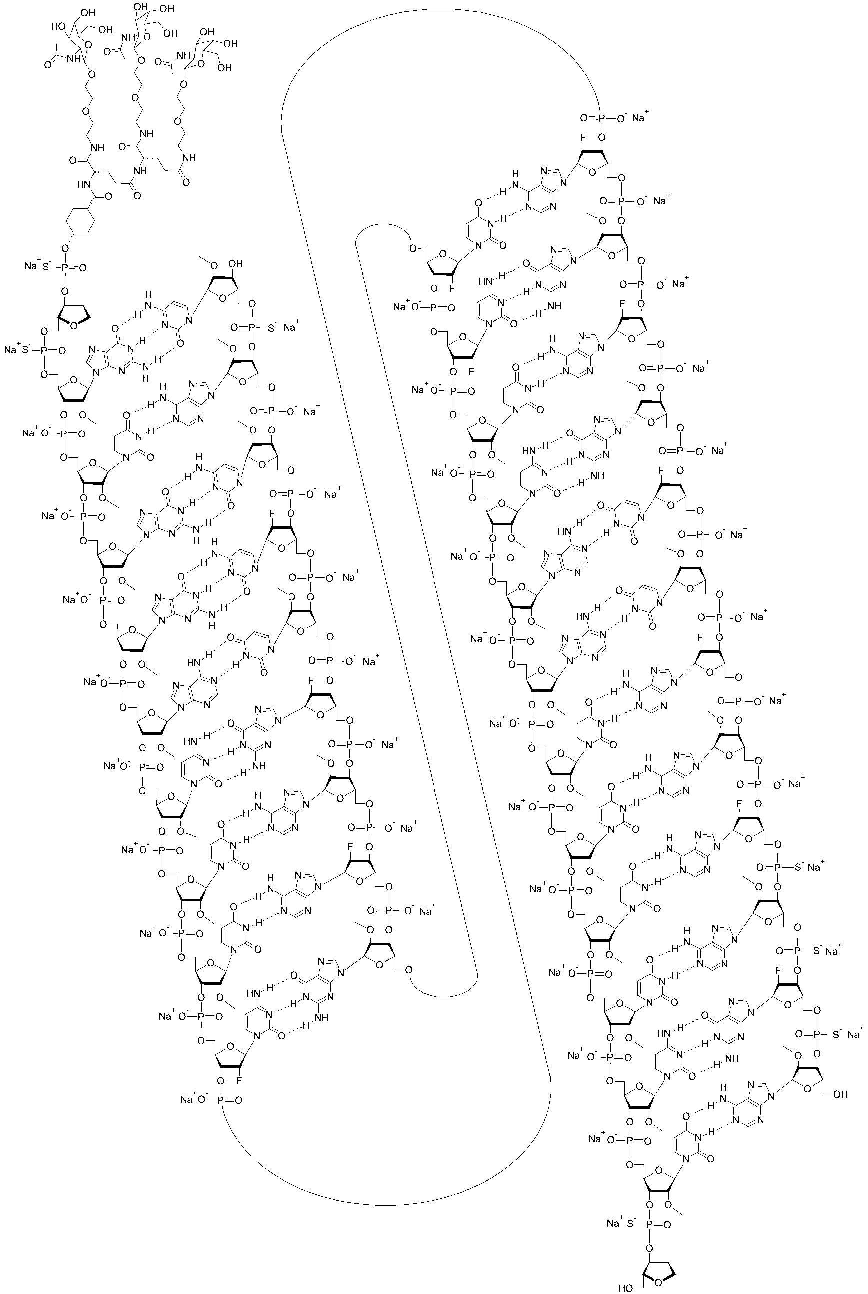 and wherein the second RNAi agent (AD05070) has the structure represented by the following:

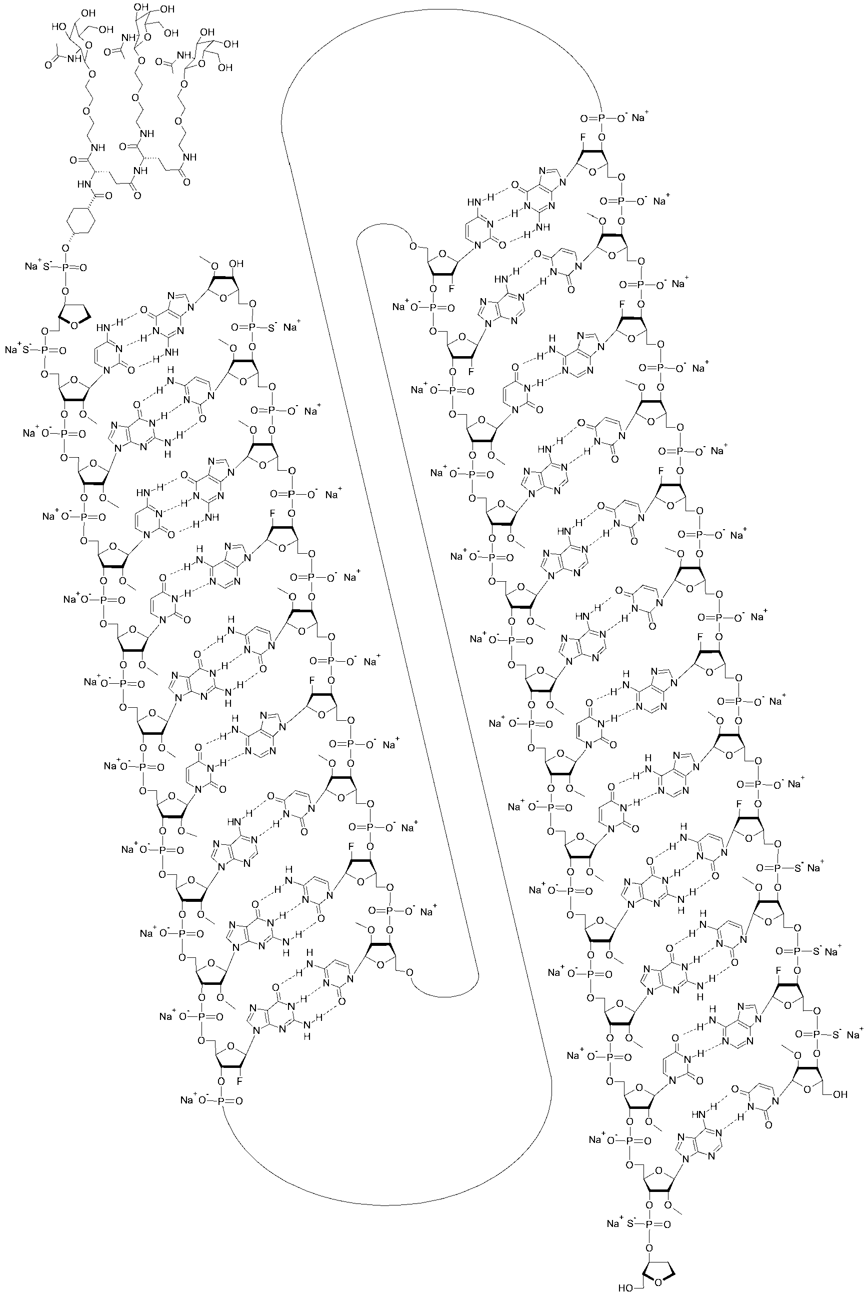

5. The pharmaceutical composition for use of any one of claims 1-4, wherein the ratio of the first RNAi agent (AD04872) and the second RNAi agent (AD05070) is about 2:1.

6. The pharmaceutical composition for use of any one of claims 1-5, wherein the first RNAi agent (AD04872) is administered in an amount of about 33 mg and the second RNAi agent (AD05070) is administered in an amount of about 17 mg for a combined dose of about 50 mg.

7. The pharmaceutical composition for use of any one of claims 1-6, wherein the first and second RNAi agents are administered for a duration of at least about 16 weeks.

8. The pharmaceutical composition for use of any one of claims 1-7, wherein the composition is administered subcutaneously.

9. The pharmaceutical composition for use of claim 8, which is a sterile injectable solution.

10. The pharmaceutical composition for use of claim 8, which is a sterile powder for the preparation of a sterile injectable solution.

11. The pharmaceutical composition for use of claim 9 or claim 10, which is packaged in a pre-filled syringe or vial.

12. The pharmaceutical composition for use of any one of claims 1-11, wherein the method further comprises administering to the subject a second active agent.

13. The pharmaceutical composition for use of claim 12, wherein the second active agent is a nucleoside analog.

14. The pharmaceutical composition for use of claim 13, wherein the nucleoside analog is Entecavir or Tenofovir.
